# EUROPEAN PATENT SPECIFICATION

(11) **EP 2 509 595 B1**
(45) Date of publication and mention of the grant of the patent: **11.04.2018**
(21) Application number: 10788125.2
(22) Date of filing: 10.12.2010
(51) Int. Cl.: A61K 31/4155, A61K 45/06, G01N 33/50, A61P 31/16, A61K 31/5377, C12N 15/113, A61K 31/496, A61K 31/4439, G01N 33/569, A61K 31/506

(54) **INHIBITORS OF HEMOPOIETIC CELL KINASE (P59-HCK) AND THEIR USE IN THE TREATMENT OF INFLUENZA INFECTION**
HEMMER DER HÄMOPOETISCHEN ZELLKINASE (P59-HCK) UND IHER VERWENDUNG BEI DER BEHANDLUNG VON INFLUENZA-NFEKTIONEN
INHIBITEURS DE KINASE DE CELLULES HÉMOPOÏÉTIQUES (P59-HCK) ET LEUR UTILISATION DANS LE TRAITEMENT D'UNE INFECTION PAR LE VIRUS DE LA GRIPPE

(30) Priority: 11.12.2009 GB 0921730
(43) Date of publication of application: 17.10.2012
(73) Proprietor: Respivert Limited, High Wycombe Buckinghamshire HP12 4EG (GB)
(72) Inventor: CHARRON, Catherine, Elisabeth, London SW7 2AZ (GB); FENTON, Robert, London W5 1LX (GB); CROWE, Scott, Hertfordshire SG6 3UE (GB); ITO, Kazuhiro, London SW7 2AZ (GB); STRONG, Peter, London SW7 2AZ (GB); RAPEPORT, Garth, London SW7 2AZ (GB); RAY, Keith, Buckinghamshire HP16 9QU (GB)
(74) Representative: Teuten, Andrew John
(86) International application number: PCT/GB2010/052067
(87) International publication number: WO 2011/070369

(56) References cited:
- WO-A1-2008/157518
- WO-A1-2010/067131
- WO-A1-2010/112936
- WO-A2-2010/038085
- WO-A2-2010/038086
- US-A1- 2004 009 173
- CHANG Y -J ET AL: "Interferon-[gamma]-induced epithelial ICAM-1 expression and monocyte adhesion. Involvement of protein kinase c-dependent c-Src tyrosine kinase activation pathway", JOURNAL OF BIOLOGICAL CHEMISTRY 20020301 US LNKD- DOI:10.1074/JBC.M109924200, vol. 277, no. 9, 1 March 2002 (2002-03-01) , pages 7118-7126, XP002265665, ISSN: 0021-9258
- SU L ET AL: "Distinct mechanisms of STAT phosphorylation via the interferon-[alpha]/[beta] receptor. Selective inhibition of STAT3 and STAT5 by piceatannol", JOURNAL OF BIOLOGICAL CHEMISTRY 20000428 US LNKD- DOI:10.1074/JBC.275.17.12661, vol. 275, no. 17, 28 April 2000 (2000-04-28), pages 12661-12666, XP009143824, ISSN: 0021-9258
- DUFAU I ET AL: "Optimization of a homogeneous assay for kinase inhibitors in plant extracts", ASSAY AND DRUG DEVELOPMENT TECHNOLOGIES, MARY ANN LIEBERT, INC. PUBLISHERS, US, vol. 6, 1 January 2008 (2008-01-01), pages 673-682, XP007917594, ISSN: 1540-658X, DOI: DOI:10.1089/ADT.2008.143 [retrieved on 2008-11-27]

## Description

The present application relates to the use of compounds capable of inhibiting p59-HCK activity in the treatment and/or prophylaxis of infection with influenza virus. The compounds may be administered as a monotherapy or in combination with other anti-viral agents, either concomitantly or sequentially.

### Background to the invention

Seasonal influenza is a sub-acute illness caused by infection with influenza virus (A, B or C strains) and is usually characterised by a sudden rise in temperature and general aches and pains and may include appetite loss and cough. Seasonal influenza causes significant mortality in well defined sub-sets of the population, in particular the very young, the elderly and those suffering from chronic diseases, such as congestive heart failure, being most at risk. In the United States of America, 30,000-40,000 people are estimated to die as a result of infection with influenza virus each year. Public health policy recognises that environmental conditions are an important additional factor when assessing risk. For example, living in a residential or nursing home is considered an additional risk during outbreaks of seasonal influenza since the probability of contact with infection can be significantly elevated. In addition to an increased risk of death, infection with the influenza virus produces significant morbidity in the wider population (Nichol K.L. et al., N. Engl. J. Med., 1995, 333:889-93).

Seasonal influenza usually follows an epidemic pattern of infection, affecting many people at once within a defined geographic region. Although the virus affects all age groups, the highest incidence of infection usually occurs in school children. The public health impact of seasonal influenza is limited by the presence of immunity in the population and widespread vaccination. Influenza disease may also follow a pandemic pattern of infection when a new influenza virus emerges for which the global population has little or no immunity and for which there is no effective vaccine that is available to be deployed rapidly. The public health impact of a pandemic depends on the properties of the influenza virus that is responsible. In the case of the "Spanish 'flu" (1918-1920), the pattern of mortality was uncharacteristic of influenza. Estimates suggest that 99% of the 40-100 million people killed were aged under 65, and in excess of 50% were adults 20-40 years old (Simonsen L. et al., J. Infect. Dis. 1998, 178:53-60). Interestingly, reports from the time suggest that this influenza virus produced unusually severe symptoms, including haemorrhage from mucous membranes, especially from the nose, stomach, and intestine (Barry J.M., Workshop Summary, The National Academies Press, 2005, 60-61).

While the cause of the unusual pattern and extent of mortality is uncertain, the most widely held hypothesis is that the virus was able to exploit the immune system in previously healthy individuals and invoke a "cytokine storm" which led to the changed pattern of mortality. The most recent pandemic in human history, resulting from H1N1 influenza A virus infection, was detected in Mexico in March 2009 and is on-going.

Vaccination is the principal intervention deployed to control the impact of seasonal influenza in both at-risk groups and the wider population. Many groups of at-risk patients are suitable for preventative treatment by vaccination, such as patients suffering from diabetes, congestive heart failure, renal failure, asthma or chronic obstructive pulmonary disease. However, vaccination does not provide effective protection in a percentage of the general population and it is not suitable for immuno-suppressed groups, such as cancer patients undergoing chemotherapy. In addition, as these products are traditionally produced by incubation in eggs, they are not suitable for administration to individuals who have an allergy to eggs. In addition, the time course between vaccination and effective protection can be somewhat extended, leaving a time window when individuals remain vulnerable to infection in the period between the vaccine being administered and the immune system's response being complete. Furthermore, for vaccination to be maximally effective, a new vaccine has to be produced for each new influenza virus which emerges as a cause of influenza disease. While the relatively predictable pattern of spread of seasonal influenza allows timely production of a new vaccine, the rapid spread of pandemic influenza across a wider geographic area is more challenging in this regard.

The commonest form of vaccination involves intra-muscular injection of a product containing inactivated virus having the phenotype of the influenza virus that is currently circulating. In addition, a new mist vaccine, FluMist, was first approved in 2003 and is indicated for patients between the age of 5 and 49. The vaccine is sprayed into the nose and works in a manner similar to injectable products. One major difference is that this vaccine includes live influenza virus, so it cannot be given to persons with weak immune systems or pregnant women.

Public health bodies have decided that control of the threat posed by influenza pandemics requires that this regimen of vaccination be supplemented by the use of treatments which target the propagation of the virus directly, thereby limiting clinical disease in infected individuals and the risk of the virus being transmitted, albeit that infected individuals can be infectious for a day before symptoms begin. Currently, the principle medicines available for clinical use target the viral neuraminidase enzyme and include the products zanamivir (Relenza) and oseltamivir (Tamiflu). The effectiveness of these medicines is limited by several factors which include the need for administration in a prophylactic regimen or very soon after the onset of infection to gain best effect (Gubareva L.V. et al., Lancet, 2000: 355:827-35). The benefit (symptom relief and duration) arising from the treatment of an established (12-24 hr) infection in the wider population has been revealed to be modest, although high doses of neuraminidase inhibitors are used as part of the treatment protocol on intensive care units. In common with other viruses, influenza strains have emerged that express mutant enzymes which render the virus resistant to these therapies (for example, see Aoki F.Y. et al., Antivir. Ther. 2007;12:603-16). Interestingly, the vast majority of strains of pandemic H1N1 influenza A virus circulating in 2009 appeared to be sensitive *in vitro* to the neuraminidase inhibitors, oseltamivir and zanamivir, but all strains tested have been resistant to the older anti-influenza products, amantadine and rimantadine and one mutant is resistant to oseltamivir (Centers for Disease and Prevention (CDC), Morb. Mortal. Wkly. Rep., 2009; 58:433-435; Wang B. et al., Antiviral. Res., 2010, 87:16-21).

The clinical presentation of disease arising from influenza virus infection can be difficult to distinguish from that resulting from other viruses which cause upper respiratory tract infections. It is noteworthy that the mechanism of action of zanamavir and oseltamivir means that the products will only provide benefit where disease arises from infection by either an influenza A or influenza B virus.

It is also noteworthy that combination therapy can be a highly effective approach in the treatment of infectious diseases and is particularly effective as a strategy to minimise the risk of resistant strains arising from treatment. This is well illustrated by the evolution of drug treatments for HIV infection, which began as monotherapy but developed steadily to the point where triple therapy, using drugs directed against two distinct viral proteins, is regarded as the gold standard. Indeed, in the context of treating influenza virus infection, a recent publication has reported that an anti-influenza combination incorporating oseltamivir, amantadine, and ribavirin, displayed synergistic activity against multiple influenza virus strains *in vitro* (Nguyen J.T., et al., Antimicrob. Agents Chemother., 2009, 53:4115-4126). It is therefore likely, to be advantageous for new medicines to be suitable for use in combination with existing neuraminidase inhibitors, particularly in the setting of the intensive care unit.

In summary, vaccination underpins the public health approach to combat influenza viral infection. However, it is an approach which has limitations and its use needs to be augmented with the use of drugs directed against viral entry and replication to deliver optimum effects. Resistant strains are now common to the first class of influenza drugs approved, the amantadines, and have begun to emerge against the neuraminidase inhibitors. These factors make it highly desirable that new, safe and more effective medicines be identified, which inhibit the propagation of the influenza virus and provide greater benefit in alleviating or preventing the clinical consequences of influenza disease, both seasonal and pandemic.

Chang et al. discloses that Src inhibitor PP2 inhibits IFN-γ- or TPA-induced ICAM-1 expression dose-dependently.

Su et al. discloses that IFNα-induced serine phosphorylation of STAT1 and STAT3 is sensitive to the Src kinase-specific inhibitor PP2.

WO2008/157518 discloses biomarkers and biomarker combinations said to have prognostic value as predictors of the immune response of an individual, especially elderly individuals, to influenza vaccine.

Dufua et al. discloses a heterogeneous enzyme-linked immunosorbent assay and a homogenous time-resolved fluorescence assay said to be of use in identifying natural and original kinase inhibitors from plant extracts. HCK was used as a model kinase.

US2004/009173 relates to leukocyte activation and platelet proliferation, and provides nucleic acids and proteins which are said to be capable of modulating leukocyte activation and platelet proliferation. Also disclosed are disorders related to the dysfunction or dysregulation of HCK.

WO2010/067131, WO2010/038085, WO2010/112936 and WO2010/038086 disclose compounds which are inhibitors of p38 mitogen-activated protein kinase enzymes, particularly alpha and gamma kinase sub-types, for use in the treatment of inflammatory diseases such as COPD.

### Description of the Figures

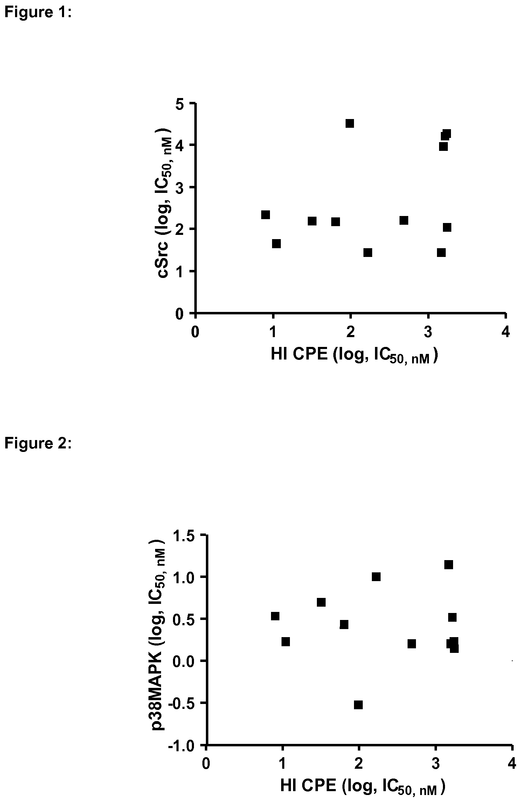

**Figure 1** shows the correlation between inhibitory potency at p38 α and human influenza (HI) induced CPE (Spearman r=-0.29, p=0.36).
**Figure 2** shows the correlation between inhibitory potency at c-SRC and human influenza (HI) induced CPE (Spearman r=0.18, p=0.59).

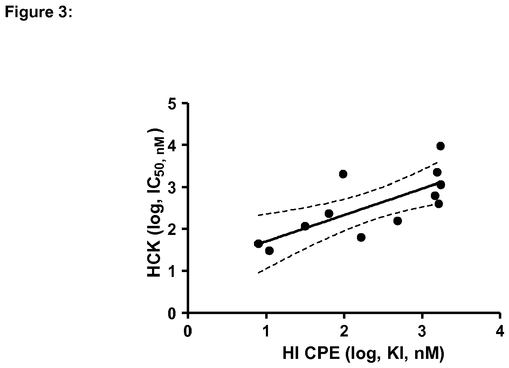

**Figure 3** shows the correlation between inhibitory potency at HCK and influenza-induced CPE (Spearman r=0.76, p=0.004)

### Detailed description of the invention

The present application relates to the identification for the first time of the role of hemopoietic cell kinase (p59-HCK) in influenza virus infection. p59-HCK is a little studied kinase which is a member of the Src B family of enzymes. It has only been previously described as being involved in linking activation of the Fc receptor to activation of the respiratory burst in neutrophils and macrophages (Guiet R., Poincloux R. et al., Eur. J. Cell Biol., 2008, 87:527-42). Surprisingly, it has been found that compounds that inhibit p59-HCK also result in a decrease in influenza virus load in both *in vitro* and *in vivo* models.

Accordingly, in a first aspect the present invention provides for the use of compounds which inhibit the activity of p59-HCK in the manufacture of a medicament to treat or prevent (i.e. prophylaxis of) infection by influenza virus (A, B and C strains including subtypes influenza A virus, influenza B virus, avian strain H5N1, A/H1N1, H3N2 and/or pandemic influenza), wherein the compound is not N-(4-(4-(3-(3-*tert*-butyl-1-*p*-tolyl-1*H*-pyrazol-5-yl) ureido) naphthalen-1-yloxy)pyridin-2-yl)-2-methoxyacetamide or a pharmaceutically acceptable salt or solvate thereof. Similarly, the present invention also provides a compound capable of inhibiting hemopoietic cell kinase (p59-HCK) activity for use in the treatment or prophylaxis of infection by influenza virus (A, B and C strains including subtypes influenza A virus, influenza B virus, avian strain H5N1, A/H1 N1, H3N2 and/or pandemic influenza), wherein the compound is not *N*-(4-(4-(3-(3-*tert*-butyl-1-*p*-tolyl-1*H*-pyrazol-5-yl) ureido) naphthalen-1-yloxy)pyridin-2-yl)-2-methoxyacetamide or a pharmaceutically acceptable salt or solvate thereof. The compounds capable of inhibiting the activity of p59-HCK may either act directly on the p59-HCK enzyme, act to reduce the activity of p59-HCK enzyme present, by, for example, decreasing the level of the enzyme or decreasing the activity of the enzyme present, or may act to reduce the availability of the substrates of p59-HCK enzyme.

In one embodiment the compound is a chemical inhibitor (e.g. a small molecule with for instance a molecular weight of less than 1500, less than 1000 or less than 750 Daltons) of p59-HCK activity. In particular the chemical inhibitor may be a compound of formula (**I**) or a pharmaceutically acceptable salt thereof, provided it is not *N*-(4-(4-(3-(3-*tert*-butyl-1-*p*-tolyl-1*H-*pyrazol-5-yl)ureido)naphthalen-1-yloxy)pyridin-2-yl)-2-methoxyacetamide or a pharmaceutically acceptable salt or solvate thereof. In another embodiment the chemical inhibitor is not a compound of formula (**I**) or a pharmaceutically acceptable salt thereof.

In another embodiment the compound is a biochemical inhibitor of p59-HCK activity. In particular the compound may be a RNAi molecule or microRNA (miRNA) molecule that suppresses the expression of p59-HCK. The RNAi molecules may be antisense oligonucleotides, small interfering RNA (siRNA) molecules. Antisense oligonucleotides are short single stranded oligonucleotides in the region of 16 bases in length which act by employing the naturally occurring, intracellular enzyme RNase H to cleave and degrade the mRNA. siRNAs are doublestranded oligonucleotides in the region of 19 base pairs in length which act by employing the naturally occurring, intracellular mechanism of the RNA-induced silencing complex (RISC). MicroRNA molecules are evolutionary conserved, small (20-24 bases), non-coding molecules that regulate gene expression at the level of translation in humans. The RNAi molecules may be modified to improve their stability and potency through the incorporation of a modified backbone, incorporating for instance 2'deoxynucleotide phosphothioate, 2'deoxynucleotide methylphosponate or peptide nucleic acids. The RNAi
molecules and miRNA may be delivered as aptomer conjugated siRNA, by incorporation into lipid nanoparticles, which may optionally be targeted to specific cells, as dynamic polyconjugates, by incorporation into cyclodextrin nanoparticles, as lipophihilic conjugated siRNA, or simply as "naked" RNAi molecules.

It will be appreciated by the person skilled in the art that the compounds suitable for use according to the present invention may act to inhibit the activity of the p59-HCK through competitive inhibition, uncompetitive inhibition, mixed inhibition and/or non-competitive inhibition.

The use of the present invention is particularly suited to the treatment or prophylaxis of patients classified as "at-risk patients". Examples of "at-risk patients" include, but are not limited to, pregnant women or patients undergoing chemotherapy. Similarly, the use of the present invention is suited to the treatment or prophylaxis of patients suffering complications (pulmonary and systemic) arising from infection by influenza virus (A, B and C strains). The use of the present invention is also suited to the treatment or prophylaxis of patients with chronic diseases, such as, but not limited to, diabetes, congestive heart failure, renal failure, chronic obstructive pulmonary disease

In an embodiment of the invention the compounds capable of inhibiting p59-HCK activity are administered as a monotherapy.

In another embodiment of the invention the compounds capable of inhibiting p59-HCK activity are administered in combination with one or more anti-viral drugs. Examples of suitable anti-viral drugs include, but are not limited to, zanamivir, oseltamivir, laninamivir, peramivir, ribavarin and (exogenous) interferons (including all their physiologically acceptable derivatives such as salts). The use of such combinations allows for more rapid control and/or more rapid resolution of symptoms associated with infection by influenza virus (A, B and C strains) than could be achieved using the anti-viral drug alone. The use of such combinations also allows for the anti-viral drugs to be administered at a lower dose or reduced frequency than is conventionally used in clinical practice when the anti-viral drugs are administered as monotherapies. For example, the dose range for zanamavir when administered in combination with a compound capable of inhibiting p59-HCK activity could be reduced to 0.03 to 3 mg per treatment or preferably 0.3 to 3 mg per treatment.

It will be appreciated by the person skilled in the art that the dosage regime for such combinations may be optimised for the particular compounds to be administered. For instance, the anti-viral drugs may be administered simultaneously or sequentially with the compounds capable of inhibiting p59-HCK activity. If the anti-viral drugs and compounds capable of inhibiting p59-HCK activity are administered simultaneously it will also be appreciated that they may be administered in a single formulation or in separate formulations.

In one embodiment envisaged by the present invention, the compounds capable of inhibiting p59-HCK activity may be administered at a dosage suitable for once per day administration.

In an embodiment of the invention the administration of the compounds capable of inhibiting p59-HCK activity is via the inhaled route. Such administration may use a metered dose inhaler, a dry powder delivery device, a nasal pump or a nebuliser. In other embodiments of the invention the compounds capable of inhibiting p59-HCK activity are administered via the intranasal route or via the oral route. Administration locally to the lung or nose has the advantage that it delivers the drug to a target organ of interest and may typically result in an improved side effect profile by avoiding systemic exposure.

In an aspect of the invention novel combination therapeutics comprising a compound capable of inhibiting p59-HCK activity and an anti-viral agent (such as, but not limited to, zanamivir, oseltamivir, laninamivir, peramivir, ribavarin and (exogenous) interferons) are provided. In an embodiment the compound capable of inhibiting p59-HCK activity may be a compound of formula (I). Such combination therapeutics may be provided in particulate form, especially particulate form suitable for inhalation as a dry powder. An example of such a particulate form is one that has a fine particle fraction of at least 50%, but ideally greater than 80%. The combination therapeutics may be suitable for inhalation via a pMDI. Such combination therapeutics may also be provided as solution formulations further comprising a propellant, a solvent and water.

In an aspect of the disclosure a method of screening for substances capable of inhibiting p59-HCK activity is provided, as are p59-HCK inhibitors and their use in therapy.

In an aspect of the disclosure a method of screening for a candidate drug substance intended to prevent or treat influenza virus infection (A, B and C strains) in a subject is provided, said method comprising identifying a test substance capable of inhibiting p59-HCK activity by measuring the effects of said test substance on p59-HCK activity.

In one embodiment the method of screening for a candidate drug substance intended to prevent or treat influenza virus infection (A, B and C strains) in a subject, comprises the steps of:
a. contacting p59-HCK with a test substance in the presence of FRET peptide and ATP;
b. measuring the level of phosphorylation of FRET peptide after a set period; and
c. comparing the level of phosphorylation measured to that observed when no test substance is added.

Also disclosed is an *in vitro* method of screening for a candidate drug substance intended to prevent or treat influenza viral infection in a subject, e.g. a human subject, which comprises:
**a.** contacting said substance with p59-HCK or cells expressing p59-HCK; and
**b.** determining whether p59-HCK enzymatic activity (e.g. phosphorylation of a substrate) is inhibited;
whereby inhibition of p59-HCK enzymatic activity indicates that the substance is a candidate drug substance intended to prevent or treat influenza viral infection in a subject.

It will be appreciated by the person skilled in the art that the methods of screening provide concrete guidance for the identification of compounds capable of inhibiting p59-HCk activity. Such compounds are an object of the present invention provided that they are not compounds of formula (**I**).

### Compounds of Formula (I)

Compound of formula (I) for use in the present invention are defined by the following formula: 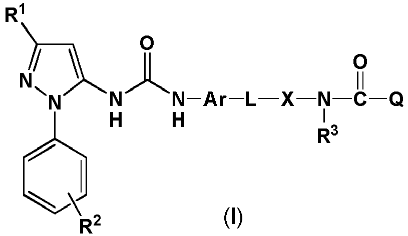
wherein R¹ is C₁₋₆ alkyl optionally substituted by a hydroxyl group;
R² is H or C₁₋₆ alkyl optionally substituted by a hydroxyl group;
R³ is H, C₁₋₆ alkyl or C₀₋₃ alkylC₃₋₆ cycloalkyl;
Ar is a naphthyl or a phenyl ring either of which may be optionally substituted by one or more groups (for example 1 to 3, such as 1, 2 or 3 groups) independently selected from C₁₋₆ alkyl, C₁₋₆ alkoxy, amino, C₁₋₄ mono or C₂₋₈ di-alkyl amino;
L is a saturated or unsaturated branched or unbranched C₁₋₈ alkylene chain, wherein one or more carbons (for example 1 to 3, such as 1, 2 or 3 carbons) are optionally replaced by -O- and the chain is optionally substituted by one or more halogen atoms (for example 1 to 6);
X is 5 or 6 membered heteroaryl group containing at least one nitrogen atom and optionally including 1 or 2 further heteroatoms selected from O, S and N;
Q is selected from:
   a) a saturated or unsaturated, branched or unbranched C₁₋₁₀ alkyl chain, wherein at least one carbon (for example 1, 2 or 3 carbons, suitably 1 or 2, in particular 1 carbon) is replaced by a heteroatom selected from O, N, S(O)ₚ, wherein said chain is optionally, substituted by one or more groups (for example 1, 2 or 3 groups) independently selected from oxo, halogen, an aryl group, a heteroaryl group, a heterocyclyl group or a C₃₋₈ cycloalkyl group,
      each aryl, heteroaryl, heterocyclyl or C₃₋₈ cycloalkyl group bearing 0 to 3 substituents selected from halogen, hydroxyl, C₁₋₆ alkyl, C₁₋₆ alkoxy, C₁₋₆ haloalkyl, amino, C₁₋₄ mono or C₂₋₈ di-alkyl amino, C₁₋₄ mono or C₂₋₈ di-acyl amino, S(O)_{q}C₁₋₆ alkyl, C₀₋₆ alkylC(O)C₁₋₆ alkyl or C₀₋₆ alkylC(O)C₁₋₆ heteroalkyl,
      with the proviso that the atom linked directly to the carbonyl in -NR³C(O)- is not an oxygen or a sulfur atom; and
   b) a C₀₋₈ alkyl-heterocycle said heterocyclyl group comprising at least one heteroatom (for example 1, 2 or 3, suitably 1 or 2, in particular 1 heteroatom) selected from O, N, and S, and which is optionally substituted by one, two or three groups independently selected from halogen, hydroxyl, C₁₋₆ alkyl, C₁₋₆ alkoxy, C₁₋₆ haloalkyl, amino, C₁₋₄ mono and C₂₋₈ di-alkyl amino, C₁₋₄ mono or C₂₋₈ di-acyl amino, S(O)_{q}C₁₋₆ alkyl, C₀₋₆ alkylC(O)C₁₋₆ alkyl, C₀₋₆ alkylC(O)NC₀₋₆ alkyl C₀₋₆ alkyl or C₀₋₆ alkylC(O)C₀₋₆ heteroalkyl; and
p is 0, 1 or 2;
q is 0, 1 or 2; or
a pharmaceutically acceptable salt thereof, including all stereoisomers, tautomers and isotopic derivatives thereof,
wherein the compound is not *N*-(4-(4-(3-(3-*tert*-butyl-1-*p*-tolyl-1*H*-pyrazol-5-yl) ureido) naphthalen-1-yloxy)pyridin-2-yl)-2-methoxyacetamide or a pharmaceutically acceptable salt or solvate thereof.

Alkyl as used herein refers to straight chain or branched chain alkyl, such as, without limitation, methyl, ethyl, *n*-propyl, *iso*-propyl, butyl, *n*-butyl and *tert*-butyl. In one embodiment alkyl refers to straight chain alkyl.

Alkoxy as used herein refers to straight or branched chain alkoxy, for example methoxy, ethoxy, propoxy, butoxy. Alkoxy as employed herein also extends to embodiments in which the oxygen atom is located within the alkyl chain, for example -C₁₋₃ alkylOC₁₋₃ alkyl, such as -CH₂CH₂OCH₃ or -CH₂OCH₃. Thus in one embodiment the alkoxy is linked through carbon to the remainder of the molecule. In one embodiment the alkoxy is linked through oxygen to the remainder of the molecule, for example -Co alkylOC₁₋₆ alkyl. In one embodiment the disclosure relates to straight chain alkoxy.

Heteroalkyl as employed herein is intended to refer to a branched or straight chain alkyl wherein one or more, such as 1, 2 or 3 carbons are replaced by a heteroatom, selected from N, O or S(O)_{q}, wherein q represents 0, 1 or 2. The heteroatom may replace a primary, secondary or tertiary carbon, that is, for example, SH, OH or NH₂ for CH₃, or NH or O or SO₂ for -CH₂- or N for a -CH- or a branched carbon group, as technically appropriate.

Haloalkyl as employed herein refers to alkyl groups having 1 to 6 halogen atoms, for example 1 to 5 halogens, such as *per* haloalkyl, in particular perfluoroalkyl, more specifically -CF₂CF₃ or CF₃.

C₁₋₄ mono or C₂₋₈ di-acyl amino is intended to refer to -NHC(O)C₁₋₃ alkyl and to (-NC(O)C₁₋₃ alkyl) C(O)C₁₋₃ alkyl) respectively.

C₁₋₄ mono or C₂₋₈ di-alkyl amino is intended to refer to -NHC₁₋₄ alkyl and -N(C₁₋₄ alkyl) (C₁₋₄ alkyl) respectively.

Aryl as used herein refers to, for example C₆₋₁₄ mono or polycyclic groups having from 1 to 3 rings wherein at least one ring is aromatic including phenyl, naphthyl, anthracenyl, 1,2,3,4-tetrahydronaphthyl and the like, such as phenyl and naphthyl.

Heteroaryl is a 6 to 10 membered aromatic monocylic ring or bicyclic ring system wherein at least one ring is an aromatic nucleus comprising one or more, for example 1, 2, 3 or 4 heteroatoms independently selected from O, N and S. Examples of heteroaryls include: pyrrole,
oxazole, thiazole, isothiazole, imidazole, pyrazole, isoxazole, pyridine, pyridazine, pyrimidine, pyrazine, benzothiophene, benzofuran, or 1, 2, 3 and 1, 2, 4 triazole.

Heterocyclyl as employed herein refers to a 5 to 6 membered saturated or partially unsaturated non-aromatic ring comprising one or more, for example 1, 2, 3 or 4 heteroatoms independently selected from O, N and S optionally one or two carbons in the ring may bear an oxo substituent. The definition of C₅₋₆ heterocycle as employed herein refers to a is a 5 to 6 membered saturated or partially unsaturated non-aromatic carbocyclic ring comprising one or more, for example 1, 2, 3 or 4 heteroatoms independently selected from O, N and S, wherein each heteroatom replaces a carbon atom and optionally one or two carbons may bear an oxo substitutent. Clearly any valancies of a heteroatom not employed in forming or retaining the ring structure may be filled by hydrogen or a substituent, as appropriate. Thus subsituents on heterocycles may be on carbon or on a heteroatom, such as N as appropriate. Examples of heterocycles and C₅₋₆ heterocycles include pyrroline, pyrrolidine, tetrahydrofuran, tetrahydrothiophene, pyrazoline, imidazoline, pyrazolidine, imidazolidine, oxoimidazolidine, dioxolane, thiazolidine, isoxazolidine, pyran, dihydropyran, piperidine, piperazine, morpholine, dioxane, thiomorpholine and oxathiane.

Halogen includes fluoro, chloro, bromo or iodo, in particular fluoro, chloro or bromo, especially fluoro or chloro.

Oxo as used herein refers to C=O and will usually be represented as C(O).

C₃₋₈ cycloalkyl as employed herein is intended to refer to a saturated or partially unsaturated non-aromatic ring containing 3 to 8 carbon atoms.

C₁₋₁₀ alkyl includes C₂, C₃, C₄, C₅, C₆, C₇, C₈ or C₉ as well as C₁ and C₁₀

C₀₋₈ alkyl includes C₁, C₂, C₃, C₄, C₅, C₆, or C₇ as well as C₀ and C₈.

In relation to a saturated or unsaturated, branched or unbranched C₁₋₁₀ alkyl chain, wherein at least one carbon (for example 1, 2 or 3 carbons, suitably 1 or 2, in particular 1) is replaced by a heteroatom selected from O, N, S(O)ₚ, wherein said chain is optionally, substituted by one or more groups independently selected from oxo, halogen, an aryl group, a heteroaryl group or a heterocyclyl group, it will be clear to persons skilled in the art that the heteroatom may replace a primary, secondary or tertiary carbon, that is CH₃, -CH₂- or a -CH- or a branched carbon group, as technically appropriate.

In one embodiment of the disclosure there is provided compounds of formula (I), wherein R¹ is methyl, ethyl, propyl, *iso*-propyl, butyl or *tert*-butyl, in particular *tert*-butyl.

In one embodiment R¹ is -C(CH₃)₂CH₂OH.

In one embodiment R² is methyl, ethyl, *n*-propyl, *iso*-propyl, *n*-butyl or *tert-*butyl*,* in particular methyl.

In one embodiment R² is -CH₂OH.

In one embodiment R² is in the 2, 3, or 4 position (i.e. *ortho*, *meta* or *para* position), in particular the *para* (4) position.

In one embodiment Ar is naphthyl.

In one embodiment Ar is not substituted with optional substituents.

In one embodiment Ar is substituted with 1 or 2 groups.

In one embodiment Ar is phenyl optionally substituted by 1 or 2 substituents independently selected from C₁₋₃ alkyl or C₁₋₃ alkoxy, for example tolyl, xylyl, anisoyl, di-methoxybenzene or methoxy-methylbenzene. The phenyl ring may, for example, be linked to the nitrogen of the urea through carbon 1 and and to the group L through carbon 4. In such a case the optional one or two substituents selected from C₁₋₃ alkyl or C₁₋₃ alkoxy may be located in any of the unoccupied positions in the aromatic ring, for example in position 2 or in position 3 or in positions 2 and 3 or in positions 2 and 6 or in positions 3 and 5. Embodiments encompassing other possible regioisomers also form an aspect of the present disclosure.

In one embodiment L is a straight chain linker, for example:
-(CH₂)ₙ- wherein n is 1, 2, 3, 4, 5, 6, 7 or 8; or
-(CH₂)ₙO(CH₂)ₘ- wherein n and m are independently 0, 1, 2, 3, 4, 5, 6 or 7, with the proviso that n+m is zero or an integer from 1 to 7, for example where n is 0 and m is 1 or 2 or alternatively, for example, where n is 1 or 2 and m is 0.

In one embodiment L is -OCH₂-, -OCH₂CH₂-, -CH₂O- or -CH₂CH₂O-. For example, L may represent -OCH₂-.

In one embodiment L is a branched chain linker R^{a}O(CH₂)ₘ wherein m is zero or an integer 1, 2, 3, 4 or 5 and R^{a} is a C₂₋₇ branched alkyl, with the proviso that the number of carbons in R^{a} added to m is an integer from 2 to 7, especially where m is zero, such as -CH(CH₃)O-, -C(CH₃)₂O-, -CH₂CH(CH₃)O-, -CH(CH₃)CH₂O-, -C(CH₃)₂CH₂O- or -CH₂C(CH₃)₂O, in particular-CH(CH₃)O-.

In one embodiment L is a branched chain linker (CH₂)ₙOR^{b} wherein n is zero or an integer 1, 2, 3, 4 or 5 and R^{b} is a C₂₋₇ branched alkyl, with the proviso that the number of carbons in R^{b} added to n is an integer from 2 to 7, for example n is zero, such as -OCH(CH₃)-, -OC(CH₃)₂-, -OCH₂CH(CH₃)-, -OCH(CH₃)CH₂-, -OC(CH₃)₂CH₂- or -OCH₂C(CH₃)₂ and in particular -OCH(CH₃)- or -OC(CH₃)₂CH₂-.

In one embodiment L is a branched chain linker R^{a}OR^{b} wherein R^{a} and R^{b} are independently selected from a C₂₋₇ branched alkylene with the proviso that the total number of carbons in R^{a} and R^{b} is an integer from 4 to 7.

In one embodiment L is a saturated unbranched C₁-C₈ alkylene chain or a saturated branched or unbranched C₂₋₈ alkylene chain.

In one embodiment at least one carbon in L is replaced by -O-.

In one embodiment L is -O-.

Alkylene as employed herein refers to branched or unbranched carbon radicals, such as methylene (-CH₂-) or chains thereof. In the context of the present specification where alkyl is a linker then the latter is used interchangeably with the term alkylene.

In one embodiment the chain L includes 1, 2 or 3 halogen atom substituents, independently selected from fluoro, chloro, and bromo, for example an alkylene carbon may incorporate one or two chlorine atoms or one or two fluorine atoms and a terminal carbon atom, for example of a branch of an alkylene chain, may be bonded to one, two or three fluorine atoms or one, two or three chlorine atoms to provide a radical such as a trifluoromethyl or a trichloromethyl group.

In one embodiment the chain L does not include a halogen atom or atoms.

In one embodiment R³ is H.

In one embodiment R³ is methyl, ethyl, *n*-propyl or *iso*-propyl.

In one embodiment R³ is cyclopropyl.

In one embodiment X is selected from, pyrrole, oxazole, thiazole, isothiazole, imidazole, pyrazole, isoxazole, oxadiazole, pyridazine, pyrimidine, pyrazine, or 1,2,3 and 1,2,4 triazole, such as pyrazole, isoxazole, oxadiazole, pyridine, pyridazine, pyrimidine, pyrazine, or 1,2,3 and 1,2,4 triazole, in particular, pyrimidine, imidazole or pyridine, and especially pyridine or pyrimidine, more specifically pyridine.

In one embodiment 1, 2, 3 or 4 carbon atoms are replaced in the alkyl chain of Q by heteroatoms independently selected from O, N, S(O)ₚ.

In one embodiment the heteroatom(s) replacing carbon(s) in the alkyl chain fragment of Q are selected from N and O.

In one embodiment Q is a saturated or unsaturated, branched or unbranched C₁₋₈ alkyl chain or a C₁₋₆ alkyl chain, wherein at least one carbon is replaced by a heteroatom selected from -O,-N, S(O)ₚ. Alternatively, in this embodiment the alkyl chain may be a C₂₋₈ alkyl or a C₃₋₆ alkyl group, such as a C₄ alkyl or a C₅ alkyl group.

In one embodiment a nitrogen atom in the alkyl chain is directly bonded to the carbonyl of the fragment -NR³C(O) and additionally may, for example, be a terminal amino group.

In one embodiment Q represents C₁₋₆ alkylNH₂ or NH₂.

In one embodiment Q represents -NHC₁₋₆ alkyl such as -NHCH₃ or -NHCH₂CH₃ or -NHCH(CH₃)₂.

In one embodiment the fragment Q is a saturated or unsaturated, branched or unbranched C₁₋₁₀ alkyl chain wherein at least one carbon (for example 1, 2, 3 or 4 carbons, in particular 1 or 2 carbons) is replaced by a heteroatom selected from O, N, S(O)ₚ, for example in such a manner as to provide a stable *N*-acyl group, NR³C(O)Q, wherein said chain is optionally substituted by one or more groups selected from oxo, halogen, an aryl group, a heteroaryl group, a heterocyclyl group, or or C₃₋₈ cycloalkyl each aryl, heteroaryl or heterocyclyl or C₃₋₈ cycloalkyl group bearing 0 to 3 substituents independently selected from a relevant substituent listed above for compounds of formula (**I**).

In one embodiment the fragment Q is a saturated or unsaturated, branched or unbranched C₁₋₁₀ alkyl chain wherein at least one carbon (for example 1, 2, 3 or 4 carbons, in particular 1 or 2 carbons) is replaced by a heteroatom selected from O, N, S(O)ₚ, for example in such a manner as to provide a stable *N*-acyl group, NR³C(O)O, wherein said chain is optionally substituted by one or more groups selected from oxo, halogen, an aryl group, a heteroaryl group or a heterocyclyl group, each aryl, heteroaryl or heterocyclyl group bearing 0 to 3 substituents independently selected from a relevant substituent listed above for compounds of formula (**I**), for example halogen, C₁₋₆ alkyl, C₁₋₆ alkoxy, C₁₋₆ haloalkyl, amino, C₁₋₄ mono or C₂₋₈ di-alkyl amino and C₁₋₄ mono or C₂₋₈ di-acyl amino.

In one embodiment the latter chain is optionally substituted by one or more groups selected from oxo, halogen, an aryl group, a heteroaryl group or a heterocyclyl group, each aryl, heteroaryl or heterocyclyl group bearing 0 to 3 substituents selected from halogen, C₁₋₆ alkyl, C₁₋₆ alkoxy, C₁₋₆ haloalkyl, amino, and C₁₋₄ mono or C₂₋₈ di-alkyl amino.

In one embodiment Q is C₁₋₄alkyl-V-R⁴, such as C₁₋₃alkyl-V-R⁴ wherein:
V is a heteroatom selected from NR^{V}, O or S(O)ₚ;
R^{V} represents H or C₁₋₃ alkyl;
R⁴ is H or -C₁₋₃ alkyl, and p is as defined above,
with the proviso that the total alkyl chain length is not more than 10 carbon atoms, including replacement heteroatoms and that the resulting radical Q is a stable group, for example -CH₂SCH₃, -CH₂SO₂CH₃, -CH₂NHCH₃, -CH₂N(CH₃)₂ -C(CH₃)₂NHCH₃, -CH(CH₃)N(CH₃)₂, -(CH₂)₃CHNHCH₃, -(CH₂)₃N(CH₃)₂, -CH₂OH, -CH₂OCH₃, -CH(CH₃)OCH₃, or -(CH₂)₂OCH₃.

In one embodiment Q is C₁₋₃ alkyl-V-(C₁₋₃ alkyl-Z-R⁵)ₖ such as C₁₋₃ alkyl-V-(C₂₋₃ alkyl-Z-R⁵)ₖ wherein:
V is a heteroatom selected from N, NH, O or S(O)ₚ, such as N or NH
(V is N in the case where k = 2, or will be selected from NH, O or S(O)ₚ, in the case where k =1, in particular NH);
Z is independently selected from NH, O or S(O)ₚ;
R⁵ is H or -C₁₋₃alkyl;
k is an integer 1 or 2 (such as 1); and
p is as defined above,
with the proviso that the total alkyl chain length is not more than 10 carbon atoms, including replacement heteroatoms and that the resulting radical Q is a stable group. Suitably Q is C₁₋₃alkyl-V-C₁₋₃alkyl-OCH₃ for example C₁₋₃alkyl-V-C₂₋₃alkyl-OCH₃ such as C₁₋₃alkyl-V-(CH₂)₂OCH₃, in particular -CH₂O(CH₂)2OCH₃ and CH₂S(CH₂)₂OCH₃, or -CH₂NH(CH₂)₂OCH₃, C₁₋₃alkyl-V-(C₁₋₃alkyl-OCH₃)ₖ wherein k represents 2, for example C₁₋₃alkyl-V-(C₂₋₃alkyl-OCH₃)ₖ such as-CH₂N[(CH₂)₂OCH₃]₂.

In one embodiment Q is C₁₋₃ alkyl-V-C₁₋₂ alkyl-Z-C₁₋₂ alkyl-Y-R⁶, or C₁₋₃ alkyl-V-C₂₋₃ alkyl-Z-C₂₋₃ alkyl-Y-R⁶, wherein V, Z and Y are independently a heteroatom selected from NH, O or S(O)ₚ,
R⁶ is H or methyl, and
p is as defined above,
with the proviso that the total alkyl chain length is not more than 10 carbon atoms, including replacement heteroatoms and that the resulting radical Q is a stable group. Suitably Q is -CH₂V(CH₂)₂O(CH₂)₂OCH₃, such as -CH₂O(CH₂)₂O(CH₂)₂OCH₃, or alternatively -CH₂NH(CH₂)₂O(CH₂)₂OCH₃, or -CH₂S(CH₂)₂O(CH₂)₂OCH₃.

In one embodiment Q represents -NR⁷R⁸ and -NR³C(O)Q forms a urea, where R⁷ and R⁸ independently represent hydrogen or a C₁₋₉ saturated or unsaturated, branched or unbranched alkyl chain, wherein one or more carbons, such as 1, 2 or 3 are optionally replaced by a heteroatom selected from O, N or S(O)ₚ. Said chain is optionally substituted by one or more groups independently selected from oxo, halogen, an aryl group, a heteroaryl group, a heterocyclyl or C₃₋₈ cycloalkyl group, each aryl, heteroaryl or heterocyclyl group bearing 0 to 3 substituents independently selected from the relevant substituents listed above for compounds of formula (I), for example halogen, C₁₋₆ alkyl, C₁₋₆ alkoxy, C₁₋₆ haloalkyl, amino, C₁₋₄ mono or C₂₋₈ di-alkyl amino and C₁₋₄ mono or C₂₋₈ di-acyl amino with the proviso that the total alkyl chain length is not more than 10 carbon atoms, including replacement heteroatoms and that the resulting radical Q is a stable group.

In one embodiment Q represents -NR⁷R⁸ and -NR³C(O)Q forms a urea, where R⁷ and R⁸ independently represent hydrogen or a C₁₋₉ saturated or unsaturated, branched or unbranched alkyl chain, wherein one or more carbons, such as 1, 2 or 3 are optionally replaced by a heteroatom selected from O, N or S(O)ₚ. Said chain is optionally substituted by one or more groups independently selected from oxo, halogen, an aryl group, a heteroaryl group or a heterocyclyl group, each aryl, heteroaryl or heterocyclyl group bearing 0 to 3 substituents independently selected from the relevant substituents listed above for compounds of formula (I), for example halogen, C₁₋₆ alkyl, C₁₋₆ alkoxy, C₁₋₆ haloalkyl, amino, C₁₋₄ mono or C₂₋₈ di-alkyl amino and C₁₋₄ mono or C₂₋₈ di-acyl amino with the proviso that the total alkyl chain length is not more than 10 carbon atoms, including replacement heteroatoms and that the resulting radical Q is a stable group.

In this urea embodiment in one sub-embodiment R⁷ represents hydrogen.

Examples of ureas include those in which R⁷ and R⁸ are both hydrogen and Q is -NH₂, or where Q is -NHCH₃ or -N(CH₃)₂ to provide, for example, a fragment -NR³C(O)NH₂ or -NR³C(O)NHCH₃ or -NR³C(O)N(CH₃)₂.

Examples of ureas containing a heteroatom in the alkyl chain include those in which Q is-NH(CH₂)₂OCH₃ or -N[(CH₂)₂OCH₃)]₂. In one embodiment Q represents -NHC₂₋₆alkylOC₁₋₃alkyl, such as-NHCH₂CH₂OCH₃.

Examples of ureas containing an oxo substitutent include those in which Q is -NHCH₂C(O)NH-C₂₋₃alkyl-X¹-C₁₋₃ alkyl, wherein X¹ is a heteroatom selected from N, O or S(O)ₚ and p is defined as above. Examples of the latter include those wherein Q is -NHCH₂C(O)NHCH₂CH₂OCH₃. Thus in one embodiment Q represents -NHC₁₋₄ alkylC(O)NHC₂alkylOCH₃ such as-NHCH₂C(O)NHCH₂CH₂OCH₃.

In one embodiment Q represents -NHC₁₋₄alkylC(O)R^{Q} wherein R^{Q} is selected from OH or-NR'R" where R' is hydrogen or C₁₋₃ alkyl and R" is hydrogen or C₁₋₃ alkyl, for example -NHCH₂C(O)OH, -NHCH₂C(O)NH₂ or -NHCH₂C(O)NHCH₃ such as -NHCH₂C(O)OH or -NHCH₂C(O)NHCH₃.

In one embodiment the radical Q represents -NHC₁₋₄alkylC(O)OC₁₋₃alkyl, such as -NHCH₂C(O)OCH₂CH₃.

In a further urea sub-embodiment Q represents -N-R⁹C₁₋₃ alkyl-V-(C₁₋₃ alkyl-Z-R¹⁰)ₖ for example -N-R⁹C₂₋₃ alkyl-V-(C₂₋₃ alkyl-Z-R¹⁰)ₖ wherein:
V represents N, NH, O, S(O)ₚ;
Z represents NH, O, S(O)ₚ;
k is an integer 1 or 2;
p is an integer 0, 1 or 2
R⁹ represents H or C₁₋₃ alkyl-V-(C₁₋₃ alkyl-Z-R¹⁰)ₖ such as C₂₋₃ alkyl-V-(C₂₋₃ alkyl-Z-R¹⁰)ₖ; and
R¹⁰ is H or C₁₋₃ alkyl such as C₁₋₃ alkyl;
with the proviso that the total alkyl chain length is not more than 10 carbon atoms, including replacement heteroatoms and that the resulting radical Q is a stable group.

In one embodiment Q is a saturated or unsaturated, branched or unbranched C₁₋₁₀ alkyl chain, wherein at least one carbon is replaced by a heteroatom selected from O, N, and S(O)ₚ, wherein said chain is substituted by an aryl group bearing 0 to 3 substituents, for example 1, 2 or 3, such as 1 or 2 substituents independently selected from the relevant substituents listed above for compounds of formula (I), for example from halogen, C₁₋₆ alkyl, C₁₋₆ alkoxy, C₁₋₆ haloalkyl, amino and C₁₋₄ mono or C₂₋₈ di-alkyl amino and C₁₋₄ mono or C₂₋₈ di-acyl amino, such as a saturated or unsaturated, branched or unbranched C₁₋₁₀ alkyl chain, wherein at least one carbon is replaced by a heteroatom selected from O, N, and S(O)ₚ, wherein said chain is substituted by an aryl group bearing 0 to 3 substituents, for example 1, 2 or 3, such as 1 or 2 substituents independently selected from halogen, C₁₋₆ alkyl, C₁₋₆ alkoxy, C₁₋₆ haloalkyl, amino and C₁₋₄ mono or C₂₋₈ di-alkyl amino. In one embodiment the said aryl group is phenyl, for example substituted phenyl or unsubstituted phenyl.

In one embodiment Q represents -NHC₀₋₆ alkylphenyl, such as -NHphenyl or NHbenzyl.

Examples of the fragment -NR³C(O)Q wherein Q comprises substituted benzyl include: -NR³C(O)CH₂NHCH₂C₆H₄(OCH₃) such as -NHC(O)CH₂NHCH₂C₆H₄(OCH₃), for example where the methoxy substituent is in the *ortho*, *meta* or *para* position, such as the *para* position.

In one embodiment Q is a saturated or unsaturated, branched or unbranched C₁₋₁₀ alkyl chain, wherein at least one carbon is replaced by a heteroatom selected from O, N, and S(O)ₚ, wherein said chain is substituted by a heteroaryl group bearing 0 to 3 substituents (for example 1, 2 or 3, such as 1 or 2 substituents) independently selected from the relevant substituents listed above for compounds of formula (**I**), for example halogen, C₁₋₆ alkyl, C₁₋₆ alkoxy, C₁₋₆ alkyl amino, C₁₋₄ mono or C₂₋₈ di-alkyl amino and C₁₋₄ mono or C₂₋₈ di-acyl amino, such as a saturated or unsaturated, branched or unbranched C₁₋₁₀ alkyl chain, wherein at least one carbon is replaced by a heteroatom selected from O, N, and S(O)ₚ, wherein said chain is substituted by a heteroaryl group bearing 0 to 3 substituents for example 1, 2 or 3, such as 1 or 2 substituents selected from halogen, C₁₋₆ alkyl, C₁₋₆ alkoxy, C₁₋₆ alkyl amino, C₁₋₄ mono or C₂₋₈ di-alkyl amino. In one embodiment the said heteroaryl group is selected from, thiophene, oxazole, thiazole, isothiazole, imidazole, pyrazole, isoxazole, isothiazole, oxadiazole, 1,2,3 or 1,2,4 triazole, pyridine, pyridazine, pyrimidine, pyrazine and, in particular pyridine and pyrimidine, especially pyridine.

In one embodiment Q represents -NHC₁₋₆ alkylheteroaryl, for example -NH(CH₂)₃imidazolyl or -NHCH₂isoxazole wherein the isoxazole is optionally substituted such as -NHCH₂isoxazole(CH₃).

In one embodiment Q represents -NHC₁₋₄ alkylC(O)NHC₁₋₃alkylheteroaryl, for example a nitrogen containing heteroaryl group or a nitrogen and oxygen containing heteroaryl, more specifically -NHCH₂C(O)NHCH₂CH₂pyridinyl, in particular where pyridinyl is linked through carbon, for example pyridin-4-yl or -NHCH₂C(O)NHCH₂CH₂CH₂imidazolyl, in particular where imidazolyl is linked through nitrogen.

In one embodiment Q is a saturated or unsaturated, branched or unbranched C₁₋₁₀ alkyl chain, wherein at least one carbon is replaced by a heteroatom selected from O, N and S(O)ₚ wherein said chain is substituted by a heterocyclyl group bearing 0 to 3 substituents (for example 1, 2 or 3, such as 1 or 2 substituents) independently selected from the relevant substituents listed above for compounds of formula (**I**), for example halogen, C₁₋₆ alkyl, C₁₋₆ alkoxy, C₁₋₆ haloalkyl amino, C₁₋₄ mono or C₂₋₈ di-alkyl amino and C₁₋₄ mono or C₂₋₈ di-acyl amino, such as a saturated or unsaturated, branched or unbranched C₁₋₁₀ alkyl chain, wherein at least one carbon is replaced by a heteroatom selected from O, N and S(O)ₚ wherein said chain is substituted by a heterocyclyl group bearing 0 to 3 substituents, for example 1, 2 or 3, such as 1 or 2 substituents selected from halogen, C₁₋₆ alkyl, C₁₋₆ alkoxy, C₁₋₆ haloalkyl amino, C₁₋₄ mono or C₂₋₈ di-alkyl amino.

In one embodiment said heterocyclyl is selected, from a 5 or 6 membered saturated or partially unsaturated ring system comprising one or more (for example 1, 2 or 3 in particular 1 or 2) heteroatoms independently selected from O, N and S, for example pyrrolidine, tetrahydrofuran, tetrahydrothiophene, piperidine, piperazine, morpholine, 1,4-dioxane, pyrrolidine and oxoimidazolidine such as pyrrolidine, tetrahydrofuran, tetrahydrothiophene, piperidine, piperazine, morpholine, and 1,4-dioxane, in particular piperidine, piperazine, and morpholine.

A heterocyclic group may be linked to the alkyl chain of Q or to the carbonyl of -NR³C(O)- through carbon or nitrogen, in particular a nitrogen atom.

In one embodiment Q is -C₀₋₃alkylheterocycle (for example-C₀₋₁alkylheterocycle) said heterocyclyl group comprising at least one heteroatom (for example 1, 2 or 3, in particular 1 or 2, heteroatoms) selected from O, N and S, and is optionally substituted by one or two or three groups independently selected from the relevant substituents listed above for compounds of formula (I), for example halogen, C₁₋₆ alkyl, C₁₋₆ alkoxy, C₁₋₆ haloalkyl, amino, C₁₋₄ mono and C₂₋₈ di-alkyl amino and C₁₋₄ mono or C₂₋₈ di-acyl amino.

In one embodiment Q is -C₀alkylheterocycle, for example a tetrahydropyranyl or a pyrrolidinyl or a morpholinyl or a piperazinyl or an oxoimidazolinyl group, such as 2-oxoimidazolidinyl group.

In one embodiment in which Q is -C₀alkylheterocycle, the heterocycle is linked through carbon, and is, for example, a C-linked tetrahydropyran or a C-linked piperidine or a C-linked morpholine or a C-linked piperazine.

In one embodiment in which Q is -C₀alkylheterocycle, the heterocyclic group containing one or more N atoms is linked through N. This embodiment provides for ureas in which one of the urea nitrogens is embedded within a heterocyclic ring. Examples of this embodiment include, but are not limited to, an *N*-linked morpholine or an *N*-linked piperidine or an *N*-linked piperazine, said *N*-linked piperizinyl group optionally bearing an additional *C*- or *N*- substituent (such as an *N-*methyl group or *N*-CH₂CH₂OCH₃ group . In one embodiment Q is a heterocyclyl linked through nitrogen such as piperidinyl, in particular 4-hydroxypiperidinyl or piperazinyl, such as 4-methyl pierazinyl.

In one embodiment Q represents a heterocyclyl group, for example a nitrogen containing heterocyclyl group, in particular linked through N, such as morpholinyl or piperazinyl optionally substituted by methyl, especially 4-methyl, or piperidinyl.

In one embodiment Q is a -C₁alkylheterocycle, for example tetrahydropyranylmethyl or a *C*- or *N*-linked piperazinylmethyl optionally bearing a substituent (for example a C₁₋₆ alkyl substitutent such as methyl or a C₁₋₆ alkoxy substituent such as -CH₂CH₂OCH₃). Additional examples include a *C*- or *N*-linked pyrrolidinylmethyl, or a *C*- or *N*- linked oxoimidazolinylmethyl (such as 2-oxoimidazolidinylmethyl, said heterocycle optionally bearing a substitutent (such as *N*-methyl or *N*-SO₂CH₃).

In one embodiment Q represents -NHheterocyclyl (wherein the heterocyclyl bears 0 to 3 substituents selected from the relevant list of substituents listed above for compounds of formula (I), for example halogen, hydroxy, C₁₋₆ alkyl, C₁₋₆ alkoxy, C₁₋₆ haloalkyl, amino, C₁₋₄ mono or C₂₋₈ di-alkyl amino, -S(O)_{q}C₁₋₆ alkyl, C₁₋₄ mono or C₂₋₈ di-acyl amino, C₀₋₆ alkylC(O)C₁₋₆ alkyl or C₀₋₆ alkylC(O)C₁₋₆ heteroalkyl), such as where the ring is linked through carbon, for example 2-piperidinyl or 3-piperidinyl or 4-piperidinyl, in particular 1-acetylpiperidin-4-yl, 1-methylpiperidin-4-yl, 1-(methylsulfonyl)piperidin-4-yl or 1-(2-(2-methoxyethoxy)acetyl) piperidin-4-yl

In one embodiment Q represents -NHC₁₋₆ alkylheterocyclyl for example a nitrogen containing heterocyclyl group, in particular one linked through nitrogen, such as -NHCH₂CH₂morpholine, -NH(CH₂)₃morpholine or-NH(CH₂)₄morpholine.

In one embodiment Q represents -NHC₁₋₆ alkylC(O)heterocyclyl (wherein the heterocyclyl bears 0 to 3 substituents selected from the relevant list of substituents listed above for compounds of formula (**I**), for example halogen, hydroxy, C₁₋₆ alkyl, C₁₋₆ alkoxy, C₁₋₆ haloalkyl, amino, C₁₋₄ mono or C₂₋₈ di-alkyl amino, C₁₋₄ mono or C₂₋₈ di-acyl amino, C₀₋₆ alkylC(O)C₁₋₆ alkyl or C₀₋₆ alkylC(O)C₁₋₆ heteroalkyl) for example a nitrogen containing heterocyclyl group, in particular one linked through nitrogen, such as -NHCH₂C(O)-1-pyrrolindinyl, -NHCH₂C(O)-1-piperidinyl, -NHCH₂C(O)-4-morpholinyl or alternatively -NHCH₂C(O)piperazinyl such as -NHCH₂C(O)-4-methyl-1-piperazinyl.

In one embodiment Q represents -NHC₁₋₄ alkylC(O)NHC₁₋₃alkylheterocyclyl for example a nitrogen containing heterocyclyl group or a nitrogen and/or oxygen containing heterocyclyl, such as -NHCH₂C(O)NHCH₂CH₂morpholinyl, in particular where morpholinyl is linked through nitrogen.

In one embodiment Q represents -N(C₁₋₃ alkyl)C₁₋₆ alkylheterocyclyl, for example a nitrogen containing heterocyclyl group, in particular linked through nitrogen, such as -N(CH₃)CH₂CH₂morpholine, -N(CH₃)(CH₂)₃morpholine or -N(CH₃)(CH₂)₄morpholine.

In one embodiment Q is -C₁₋₃alkyl-G-C₁₋₃alkylheterocycle wherein G is a heteroatom selected from NH, O or S(O)ₚ said heterocyclyl group comprising at least one heteroatom (for example 1, 2 or 3, in particular 1 or 2, heteroatoms) selected from O, N, and S, and is optionally substituted by one or two or three groups independently selected from relevant substituents listed above for compounds of formula (I), for example halogen, C₁₋₆ alkyl, C₁₋₆ alkoxy, C₁₋₆ haloalkyl, amino, C₁₋₄ mono and C₂₋₈ di-alkyl amino and C₁₋₄ mono or C₂₋₈ di-acyl amino such as one or two or three groups halogen, C₁₋₆ alkyl, C₁₋₆ alkoxy, C₁₋₆ haloalkyl, amino, C₁₋₄ mono and C₂₋₈ di-alkyl amino. Suitably Q is -CH₂G(CH₂)₂heterocycle for example -CH₂G(CH₂)₂tetrahydropyranyl; or -CH₂G(CH₂)₂morpholinyl in which the heterocyclyl is linked through nitrogen or carbon; or CH₂G(CH₂)₂piperazinyl in which the heterocyclyl is linked through nitrogen or carbon and optionally bearing a further *C-* or *N-* substituent (for example a C₁₋₆ alkyl substitutent such as methyl or a C₁₋₆ alkoxy substituent such as -CH₂CH₂OCH₃); or-CH₂G(CH₂)₂pyrrolidinyl, in which the heterocyclyl is linked through nitogen or carbon, for example linked through nitrogen; or -CH₂G(CH₂)₂oxoimidazolinyl (such as 2-oxoimidazolidinyl) for example linked through N and optionally bearing an additional *C*- or *N*- substitutent (such as *N*-methyl or *N*-SO₂CH₃), and in which G is O or NH.

In one embodiment G is O.

In one embodiment G is NH.

In one embodiment Q is a saturated or unsaturated C₁₋₁₀ alkyl chain wherein at least one carbon (for example 1, 2 or 3 carbons) is replaced by a heteroatom selected from O, N, S(O)ₚ wherein said chain is substituted by a C₃₋₈ carbocyclyl group and said alkyl chain is optionally substituted by one or more (for example 1 or 2) groups selected from oxo and halogen. In one embodiment said C₃₋₈ carbocyclyl group bears one or more groups (for example 1, 2 or 3 groups) independently selected from halogen, hydroxyl, C₁₋₆ alkyl, C₁₋₆ alkoxy, C₁₋₆ haloalkyl, amino, C₁₋₄ mono or C₂₋₈ di-alkyl amino, C₁₋₄ mono or C₂₋₈ di-acyl amino, S(O)_{q}C₁₋₆ alkyl, C₀₋₆ alkylC(O)C₁₋₆ alkyl or C₀₋₆ alkylC(O)C₁₋₆ heteroalkyl.

In one embodiment Q represents -NHC₃₋₆ cycloalkyl, such as -NHcyclopropyl, -NHcyclopentyl or-NHcyclohexyl.

In one embodiment the aryl, heteroaryl or heterocyclyl group bears at least one -S(O)_{q}C₁₋₆ alkyl substitutent and optionally bears one or two further relevant substituents independently selected from the list of substituents defined above for compounds of formula (I).

In one embodiment the C₅₋₆ heterocycle bears at least one -S(O)_{q}C₁₋₆ alkyl substitutent and optionally bears one or two further substituents independently selected from the relevant list of substituents defined above for compounds of formula (**I**).

In one embodiment the aryl, heteroaryl or heterocyclyl group bears at least one hydroxyl substituent and optionally bears one or two further substituents independently selected from the relevant list of substituents defined above for compounds of formula (**I**).

In one embodiment the C₅₋₆heterocycle bears at least one hydroxyl substituent and optionally bears one or two further substituents independently selected from the relevant list of substituents defined above for compounds of formula (**I**).

In one embodiment the aryl, heteroaryl or heterocyclyl group bears at least one C₁₋₄ mono and/or C₂₋₈ di-acyl amino substituent and optionally bears one or two further substituents independently selected from the relevant list defined above for compounds of formula (**I**).

In one embodiment the C₅₋₆heterocycle bears at least one C₁₋₄ mono and/or C₂₋₈ di-acyl amino substituent and optionally bears one or two further substituents independently selected from the relevant list defined above for compounds of formula (**I**).

In one embodiment the aryl, heteroaryl or heterocyclyl group bears at least one C₀₋₆ alkylC(O)C₁₋₆ heteroalkyl substituent and optionally bears one or two further substituents independently selected from the relevant list defined above for compounds of formula (**I**).

In one embodiment the C₅₋₆heterocycle bears at least one C₀₋₆ alkylC(O)C₁₋₆ heteroalkyl substituent and optionally bears one or two further substituents independently selected from the relevant list defined above for compounds of formula (**I**).

In one embodiment the aryl, heteroaryl or heterocyclyl group bears at least one C₀₋₆ alkylC(O)C₁₋₆ alkyl substituent and optionally bears one or two further substituents independently selected from the relevant list defined above for compounds of formula (**I**).

In one embodiment the C₅₋₆heterocycle bears at least one C₀₋₆ alkylC(O)C₁₋₆ alkyl substituent and optionally bears one or two further substituents independently selected from the relevant substituents defined above for compounds of formula (**I**).

In one embodiment Q represents tetrahydrofuranyl, morpholinyl, piperidinyl such as piperidinyl bearing one hyroxyl substituent, piperazinyl such as piperazinyl bearing one methyl substituent or pyrrolidinyl such a pyrrolidinyl bearing one di-methyl amino substituent. The ring may be linked through the heteroatom, such as nitrogen. Alternatively, the ring may be linked through carbon. The substituent may, for example be para relative to the atom through which the ring is linked to the remainder of the molecule.

In one embodiment the alkyl chain fragment of Q does not bear any optional substituents.

In one embodiment the alkyl chain is saturated.

In one embodiment the alkyl chain is unbranched.

In one embodiment the alkyl chain fragment of Q bears 1, 2, or 3, for example 1 or 2, in particular 1 optional substituent.

It will be clear to persons skilled in the art that the heteroatom may replace a primary, secondary or tertiary carbon, that is a CH₃, -CH₂- or a -CH-, group, as technically appropriate.

In one embodiment p is 0 or 2.

In one embodiment p is 1.

In one embodiment compounds of the disclosure include those in which the fragment O is:
-CH₂OH;
-CH₂OC₁₋₆ alkyl, in particular -CH₂OCH₃;
-CH₂CH₂OCH₃;
-CH₂O(CH₂)₂OCH₃;
-CH(CH₃)OCH₃;
-CH₂NHCH₃ or -CH₂N(CH₃)₂
-CH₂NHCH₂CH₂OCH₃ or -CH₂NHC(O)CH₂0CH₃;
-CH₂SCH₃, -CH₂S(O)₂CH₃ or -CH₂NHC(O)CH₂S(O)₂CH₃; or
-CH₂NHC(O)CH₂.

In one embodiment compounds of the disclosure include those in which the fragment -NR³C(O)O in formula (**I**) is represented by:
-NR³C(O)CH₂OH, and in particular -NHC(O)CH₂OH;
-NR³C(O)CH₂OC₁₋₆ alkyl, in particular -NR³C(O)CH₂OCH₃, especially -NHC(O)CH₂OCH₃;
-NR³C(O)CH₂O(CH₂)₂OCH₃, and in particular -NHC(O)CH₂O(CH₂)₂OCH₃;
-NR³C(O)CH(CH₃)OCH₃, and in particular -NHC(O)CH(CH₃)OCH₃;
-NR³C(O)CH(CH₃)NHC₁₋₃alkyl, and in particular -NHC(O)CH(CH₃)NHCH₃;
-NR³C(O)CH(CH₃)N(C₁₋₃alkyl)₂, and in particular -NHC(O)CH(CH₃)N(CH₃)₂;
-NR³C(O)C(CH₃)₂NHCH₃, and in particular -NHC(O)C(CH₃)₂NHCH₃;
-NR³C(O)(CH₂)₂OC₁₋₆alkyl, such as -NR³C(O)(CH₂)₂OCH₃, in particular -NHC(O)(CH₂)₂OCH₃;
-NR³C(O)(CH₂)₃NHC₁₋₃alkyl, and in particular -NHC(O)(CH₂)₃NHCH₃;
-NR³C(O)(CH₂)₃N(C₁₋₃alkyl)₂, and in particular -NHC(O)(CH₂)₃N(CH₃)₂;
-NR³C(O)CH₂NHC₁₋₃alkyl, and in particular -NHC(O)CH₂NHCH₃;
-NR³C(O)CH₂NH(CH₂)₂OCH₃ , and in particular -NHC(O)CH₂NH(CH₂)₂OCH₃;
-NR³C(O)CH₂SCH₃, in particular -NHC(O)CH₂SCH₃;
-NR³C(O)CH₂S(CH₂)₂OCH₃, in particular -NHC(O)CH₂S(CH₂)₂OCH₃;
-NR³C(O)CH₂S(CH₂)₂O(CH₂)₂OCH₃, and in particular -NHC(O)CH₂S(CH₂)₂O(CH₂)₂OCH₃
-NR³C(O)CH₂SOCH₃, and in particular -NHC(O)CH₂SOCH₃
-NR³C(O)CH₂S(O)₂CH₃, and in particular -NHC(O)CH₂S(O)₂CH₃;
-NR³C(O)CH₂N[(CH₂)₂OCH₃]₂, and in particular -NHC(O)CH₂N[(CH₂)₂OCH₃]₂;
-NR³C(O)NH₂, and in particular -NHC(O)NH₂;
-NR³C(O)NHC₁₋₉ alkyl, such as NR³C(O)NHC₁₋₇ alkyl, and in particular -NHC(O)NHCH₃
-NR³C(O)N(C₁₋₄ alkyl)C₁₋₅ alkyl, and in particular -NHC(O)N(CH₃)₂; or
-NR³C(O)NHCH₂CONH(CH₂)₂OCH₃, in particular -NHC(O)NHCH₂CONH(CH₂)₂OCH₃.

In one embodiment compounds of the disclosure include compounds of formula (**I**) in which the fragment -NR³C(O)C₀₋₈alkylheterocyclyl is represented by:
-NHC(O)-(tetrahydropyranyl), such as -NHC(O)-(tetrahydro-2*H*-pyran-4-yl):
-NHC(O)-(morpholinyl) such as -NHC(O)-(4-morpholinyl) or -NHC(O)-(3-morpholinyl);
-NHC(O)-(pyrrolidinyl), such as -NHC(O)-(pyrrolidin-1-yl);
-NHC(O)-(piperazinyl), such as -NHC(O)-(piperazin-1-yl);
-NHC(O)-(methylpiperazinyl), such as -NHC(O)-(4-methylpiperazin-1-yl);
-NHC(O)-[(methoxyethyl)piperazinyl], such as -NHC(O)-[4-(2-methoxyethyl)piperazin-1-yl];
-NHC(O)-(oxoimidazolidinyl) such as -NHC(O)-(2-oxoimidazolidinyl), in particular -NHC(O)-(2-oxoimidazolidin-1-yl);
-NHC(O)CH₂-(tetrahydropyranyl), such as -NHC(O)CH₂-(tetrahydro-2*H*-pyran-4-yl);
-NHC(O)CH₂-(morpholinyl), such as -NHC(O)CH₂-(4-morpholinyl);
-NHC(O)CH₂-(pyrrolidinyl), such as -NHC(O)CH₂-(pyrrolidin-1-yl);
-NHC(O)CH₂-(piperazinyl), such as -NHC(O)CH₂-(piperazin-1-yl);
-NHC(O)CH²-(methylpiperazinyl), such as -NHC(O)CH₂-(4-methylpiperazin-1-yl);
-NHC(O)CH₂-[(methoxyalkyl)piperazinyl], such as -NHC(O)CH₂-[4-(2-methoxyethyl)piperazin
-1-yl];
-NHC(O)CH₂SCH₂CH₂-(morpholinyl), such as -NHC(O)CH₂SCH₂CH₂-(4-morpholinyl), or
-NHC(O)CH₂SCH₂CH₂-(3-morpholinyl); and
-NHC(O)CH₂SO₂CH₂CH₂-(morpholinyl), such as -NHC(O)CH₂SO₂CH₂CH₂-(4-morpholinyl), or
-NHC(O)CH₂SO₂CH₂CH₂-(3-morpholinyl).

In one embodiment of the fragment Q, the saturated or unsaturated, branched or unbranched C₁₋₁₀ alkyl chain, wherein at least one carbon is replaced by a heteroatom selected from -O, -N, S(O)ₚ is a linker selected from: -CH₂OCH₂-, -CH₂NHCH₂-, -CH₂NH- and -CH₂OCH₂CH₂-. These fragments may optionally terminate in an aryl group, a heteroaryl group a heterocyclyl group or C₃₋₈ cycloalkyl group, such as an aryl group, a heteroaryl group a heterocyclyl group as defined for fragment Q above.

In one embodiment the disclosure relates to compounds of formula (**IA**): 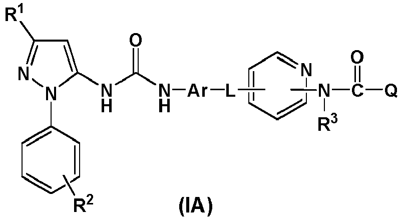 wherein R¹, R², Ar, L, R³ and Q are as defined above.

In one embodiment of the compounds of formula (**IA**) the substituent -NR³C(O)Q is in the 2 or 3 position.

In a further embodiment the disclosure relates to compounds of formula (**IB**): 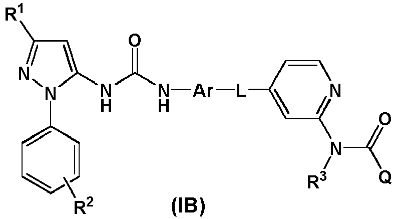 wherein R¹, R², Ar, L, R³ and Q are as defined above.

In yet another embodiment the disclosure relates to compounds of formula (**IC**): 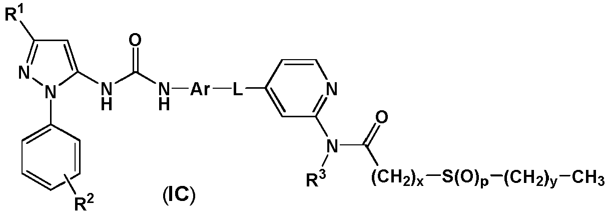 wherein R¹, R², Ar, L and R³ are as defined above and p is 0, 1 or 2, in particular 0 or 2, and x is an integer from 1 to 6 (including 2, 3, 4 and 5) and y is zero or an integer from 1 to 5 (including 2, 3 and 4) with the proviso that the sum of x and y is an integer from 1 to 8 such as 1 to 6, for example x is 1 and y is 1.

In one embodiment the disclosure relates to compounds of formula (**ID**): 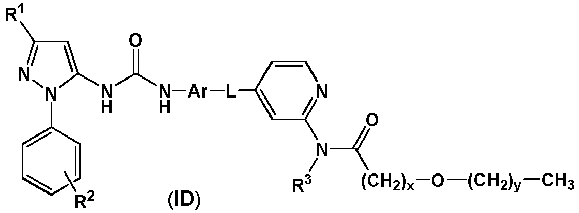
wherein R¹, R², Ar, L and R³ are as defined above
x is an integer from 1 to 6 (including 2, 3, 4 and 5) and y is zero or an integer from 1 to 5 (including 2, 3 and 4), with the proviso that the sum of xand y is an integer from 1 to 6, for example x is 1 and y is 0.

In one embodiment of the compounds of formula (**ID**) the fragment represented by -NR³C(O)(CH₂)ₓO(CH₂)_{y}CH₃ is: -NR³C(O)CH₂OCH₃, especially -NHC(O)CH₂OCH₃.

In one embodiment the disclosure relates to compounds of formula (**IE**): 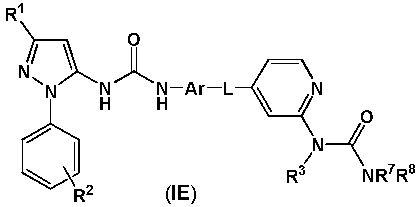 wherein R¹, R², Ar, L, R³, R⁷ and R⁸ are as defined above.

In one embodiment the disclosure relates to compounds of formula (**IF**): 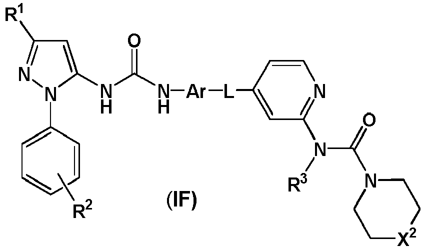 wherein R¹, R², Ar, L and R³ are as defined above and X² represents O, CH₂, NH, NCH₃ or NCH₂CH₂OCH₃.

In one aspect there is provided a compound of formula (**IG**): 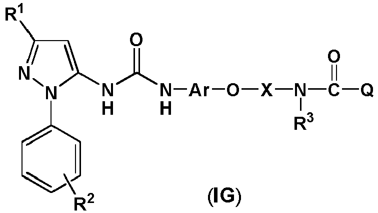
wherein R¹ is C₁₋₆ alkyl optionally substituted by a hydroxyl group;
R² is H or C₁₋₆ alkyl optionally substituted by a hydroxyl group;
R³ is H, C₁₋₆ alkyl or C₀₋₃ alkylC₃₋₆ cycloalkyl;
Ar is a naphthyl or a phenyl ring either of which may be optionally substituted by one or more groups independently selected from C₁₋₆ alkyl, C₁₋₆ alkoxy, amino, C₁₋₄ mono or C₂₋₈ di-alkyl amino;
X is 5 or 6 membered heteroaryl group containing at least one nitrogen atom
and optionally including 1 or 2 further heteroatoms selected from O, S and N;
Q is selected from:
   a) a saturated or unsaturated, branched or unbranched C₁₋₁₀ alkyl chain, wherein at least one carbon (for example 1, 2 or 3 carbons, suitably 1 or 2, in particular 1) is replaced by a heteroatom selected from O, N, S(O)ₚ, wherein said chain is optionally, substituted by one or more groups (for example 1, 2 or 3) independently selected from oxo, halogen, an aryl group, a heteroaryl group, a heterocyclyl group or a C₃₋₈ cycloalkyl,
      each aryl, heteroaryl or heterocyclyl group bearing 0 to 3 substituents selected from halogen, hydroxyl, C₁₋₆ alkyl, C₁₋₆ alkoxy, C₁₋₆ haloalkyl, amino, C₁₋₄ mono or C₂₋₈ di-alkyl amino, C₁₋₄ mono or C₂₋₈ di-acyl amino, S(O)_{q}C₁₋₆ alkyl, C₀₋₆ alkylC(O)C₁₋₆ alkyl or C₀₋₆ alkylC(O)C₁₋₆ heteroalkyl,
      with the proviso that the atom linked directly to the carbonyl in -NR³C(O)- is not an oxygen or a sulfur atom; and
   b) a C₀₋₈ alkylC₅₋₆ heterocycle or said heterocyclyl group comprising at least one heteroatom (for example 1, 2 or 3, suitably 1 or 2, in particular 1 heteroatom) selected from O, N, and S, and is optionally substituted by one, two or three groups independently selected from halogen, hydroxyl, C₁₋₆ alkyl, C₁₋₆ alkoxy, C₁₋₆ haloalkyl, amino, C₁₋₄ mono and C₂₋₈ di-alkyl amino, C₁₋₄ mono or C₂₋₈ di-acyl amino, S(O)_{q}C₁₋₆ alkyl, C₀₋₆ alkylC(O)C₁₋₆ alkyl or C₀₋₆ alkylC(O)C₁₋₆ heteroalkyl; and
p is 0, 1 or 2;
or a pharmaceutically acceptable salt thereof, including all stereoisomers, tautomers and isotopic derivatives thereof.

In one embodiment the disclosure relates to compounds of formula (**IH**): 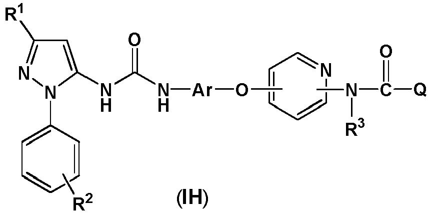 wherein R¹, R², Ar, R³ and Q are as defined above.

In a further embodiment the disclosure relates to compounds of formula (**IJ**): 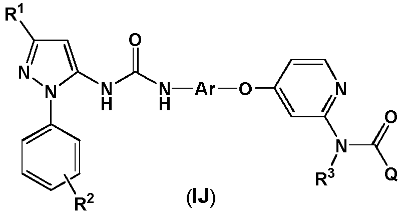 wherein R¹, R², Ar, R³ and Q are as defined above.

In yet another embodiment the disclosure relates to compounds of formula (**IK**): 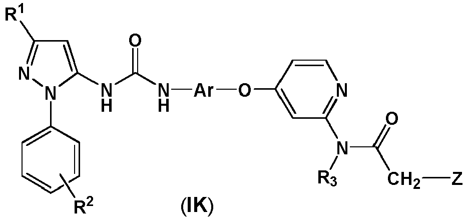
wherein R¹, R², Ar and R³ are as defined above and
Z represents a saturated or unsaturated, branched or unbranched C₁₋₉ alkyl chain, wherein at least one carbon (for example 1, 2 or 3 carbons, suitably 1 or 2, in particular 1) is replaced by a heteroatom selected from O, N, S(O)ₚ, or
a C₀₋₇ alkylC₅₋₆ heterocycle said heterocyclyl group comprising at least one heteroatom (for example 1, 2 or 3, suitably 1 or 2, in particular 1 heteroatom) selected from O, N and S, and is optionally substituted by one or two or three groups independently selected from the relevant substituents listed above for compounds of formula (I), for example halogen, C₁₋₆ alkyl, C₁₋₆ alkoxy, C₁₋₆ haloalkyl, amino, C₁₋₄ mono and C₂₋₈ di-alkyl amino.

In one embodiment of formula (**IK**) Z is -OCH₃ or -OCH₂CH₂OCH₃.

In one embodiment of formula (**IK**) Z is -SO₂CH₃.

In one embodiment of formula (**IK**) Z is -NR^{A}R^{B} wherein R^{A} and R^{B} are independently selected from hydrogen, C₁₋₆ alkyl, and C₃₋₆ alkoxy such that for example Z represents -NH₂, -NHCH₃, -N(CH₃)₂ or -NHCH₂CH₂OCH₃.

In one embodiment of formula (**IK**) Z is -S(O)_{q}CH₃ wherein n is 0, 1 or 2, such as 0 or 2.

In one embodiment of formula (**IK**) Z represents a -C₅₋₆ heterocycle said heterocyclyl group comprising at least one heteroatom (for example 1, 2 or 3, suitably 1 or 2, in particular 1 heteroatom) selected from O, N and S, and is optionally substituted by one, two or three groups independently selected from the relevant substituents listed above for compounds of formula (I) for example halogen, C₁₋₆ alkyl, C₁₋₆ alkoxy, C₁₋₆ haloalkyl, amino, C₁₋₄ mono and C₂₋₈ di-alkyl amino, for example:
morpholinyl (in particular linked through nitrogen) or
tetrahydropyranyl, or
   piperazinyl (in particular linked through nitrogen) optionally substituted on the second nitrogen by -CH₃ or -CH₂CH₂OCH₃.

In one embodiment the disclosure relates to compounds of formula (**IL**): 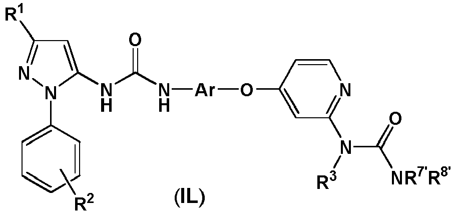
wherein R¹, R², Ar and R³ are as defined above and
R^{7'} and R^{8'} independently represent hydrogen, C₁₋₆ alkyl, or
R^{7'} and R^{8'} together with the nitrogen to which they are attached represent a 5 or 6 membered heterocycle optionally comprising a further heteroatom selected from O, N and S, wherein said heterocycle is optionally substituted by one or two or three groups independently selected from the relevant sustituents listed above for compounds of formula (**I**), for example halogen, C₁₋₆ alkyl, C₁₋₆ alkoxy, C₁₋₆ haloalkyl, amino, C₁₋₄ mono and C₂₋₈ di-alkyl amino.

In one embodiment of compounds of formula (**IL**) the group -NR^{7'}R^{8'} represents -NH₂, -NHCH₃ or NHCH₂CH₃.

In one embodiment of compounds of formula (**IL**) -NR^{7'}R^{8'} represents morpholinyl or piperazinyl.

In an alternative embodiment the disclosure relates to compounds of formula (**IM**): 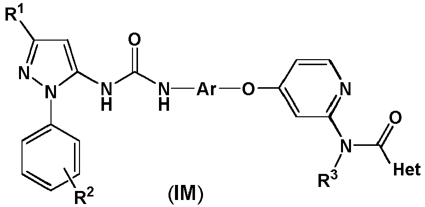 wherein R¹, R², Ar and R³ are as defined above and
Het represents a C₅₋₆ heterocycle said heterocyclyl group comprising at least one heteroatom (for example 1, 2 or 3, suitably 1 or 2, in particular 1 heteroatom) selected from O, N and S, and is optionally substituted by one or two or three groups independently selected from the relevant substituents listed above for compounds of formula (**I**) for example halogen, C₁₋₆ alkyl, C₁₋₆ alkoxy, C₁₋₆ haloalkyl, amino, C₁₋₄ mono and C₂₋₈ di-alkyl amino.

In one embodiment of compounds of formula (**IM**) Het is morpholinyl or tetrahydropyranyl.

In one embodiment the compound is::
*N*-(4-((4-(3-(3-*tert*-Butyl-1-*p*-tolyl-1*H*-pyrazol-5-yl)ureido)naphthalen-1-yloxy)methyl)pyridin-2-yl)-2-methoxyacetamide or a pharmaceutically acceptable salt thereof, including all stereoisomers, tautomers and isotopic derivatives thereof.

In one embodiment the compound is:
Methyl 4-((4-(3-(3-*tert*-butyl-1-*p*-tolyl-1*H*-pyrazol-5-yl)ureido)naphthalen-1-yloxy)methyl) pyridin-2-ylurea;
*N*-(4-((4-(3-(3-*tert*-Butyl-1-*p*-tolyl-1*H*-pyrazol-5-yl)ureido)naphthalen-1-yloxy)methyl)pyridin-2-yl)tetrahydro-2*H*-pyran-4-carboxamide;
(*S*)-*N*-(4-((4-(3-(3-*tert*-Butyl-1-*p*-tolyl-1*H*-pyrazol-5-yl)ureido)naphthalen-1-yloxy)methyl) pyridin-2-yl)-2-methoxypropanamide;
(*R*)-*N*-(4-((4-(3-(3-*tert*-Butyl-1-*p*-tolyl-1*H*-pyrazol-5-yl)ureido)naphthalen-1-yloxy)methyl) pyridin-2-yl)-2-methoxypropanamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(methylsulfonyl)acetamide
*N*-(4-((4-(3-(3-*tert*-Butyl-1-*p*-tolyl-1*H*-pyrazol-5-yl)ureido)naphthalen-1-yloxy)methyl)pyridin-2-yl)-2-hydroxyacetamide;
*N*-(4-((4-(3-(3-tert-Butyl-1-*p*-tolyl-1*H*-pyrazol-5-yl)ureido)naphthalen-1-yloxy)methyl)pyridin-2-yl)-2-methyl-2-(methylamino)propanamide;
(*S*)-*N*-(4-((4-(3-(3-*tert*-Butyl-1-*p*-tolyl-1*H*-pyrazol-5-yl)ureido)naphthalen-1-yloxy)methyl) pyridin-2-yl)-2-(methylamino)propanamide;
(*R*)-*N*-(4-((4-(3-(3-*tert*-Butyl-1-*p*-tolyl-1*H*-pyrazol-5-yl)ureido)naphthalen-1-yloxy)methyl) pyridin-2-yl)morpholine-3-carboxamide;
(*S*)-*N*-(4-((4-(3-(3-*tert*-Butyl-1-*p*-tolyl-1*H*-pyrazol-5-yl)ureido)naphthalen-1-yloxy)methyl) pyridin-2-yl)morpholine-3-carboxamide;
*N*-(4-((4-(3-(3-*tert*-Butyl-1-p-tolyl-1*H*-pyrazol-5-yl)ureido)naphthalen-1-yloxy)methyl)pyridin-2-yl)-4-methylpiperazine-1-carboxamide;
*N*-(4-((4-(3-(3-*tert*-Butyl-1-*p*-tolyl-1*H*-pyrazol-5-yl)ureido)naphthalen-1-yloxy)methyl)pyridin-2-yl)morpholine-4-carboxamide;
*N*-(4-((4-(3-(3-*tert*-Butyl-1-*p*-tolyl-1*H*-pyrazol-5-yl)ureido)naphthalen-1-yloxy)methyl)pyridin-2-yl)-3-methoxypropanamide;
2-(3-(4-((4-(3-(3-*tert*-Butyl-1-p-tolyl-1*H*-pyrazol-5-yl)ureido)naphthalen-1-yloxy)methyl) pyridin-2-yl)ureido)-*N*-(2-methoxyethyl)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-4-(dimethylamino)butanamide;
*N*-(4-((4-(3-(3-*tert*-Butyl-1-*p*-tolyl-1*H*-pyrazol-5-yl)ureido)naphthalen-1-yloxy)methyl)pyridin-2-yl)-3-(methylsulfonyl)propanamide;
*N*-(4-((4-(3-(3-*tert*-Butyl-1-*p*-tolyl-1*H*-pyrazol-5-yl)ureido)naphthalen-1-yloxy)methyl)pyridin-2-yl)-3-(methylsulfonyl)-2-oxoimidazolidine-1-carboxamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(methylthio)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(methylsulfinyl)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-morpholinoacetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(pyrrolidin-1-yl)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(4-methylpiperazin-1-yl)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(4-(2-methoxyethyl)piperazin-1-yl)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(2-methoxyethylamino)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(dimethylamino)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(methylamino)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-((4-methoxybenzyl)(methyl)amino)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(2-methoxyethylthio)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(2-(2-methoxyethoxy)ethylthio)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(2-morpholinoethylthio)acetamide;
*N*-(4-((4-(3-(3-*tert*-Butyl-1-*p*-tolyl-1*H*-pyrazol-5-yl)ureido)naphthalen-1-yloxy)methyl)pyridin-2-yl)-2-(2-morpholinoethylsulfonyl)acetamide;
2-(Bis(2-methoxyethyl)amino)-*N*-(4-((4-(3-(3-*tert*-butyl-1-*p*-tolyl-1*H*-pyrazol-5-yl)ureido) naphthalen-1-yloxy)methyl)pyridin-2-yl)acetamide;
1-(4-((3-Methylureidopyridin-4-yl)methoxy)naphthalen-1-yl)-3-(3-*tert*-butyl-1-*p*-tolyl-1*H*-pyrazol-5-yl)urea;
*N*-(4-((4-(3-(3-*tert*-Butyl-1-*p*-tolyl-1*H*-pyrazol-5-yl)ureido)naphthalen-1-yloxy)methyl)pyridin-3-yl)-2-methoxyacetamide;
*N*-(4-((4-(3-(3-*tert*-Butyl-1-*p*-tolyl-1*H*-pyrazol-5-yl)ureido)naphthalen-1-yloxy)methyl)pyridin-3-yl)-2-(2-methoxyethoxy)acetamide;
*N*-(4-(2-(4-(3-(3-*tert*-Butyl-1-*p*-tolyl-1*H*-pyrazol-5-yl)ureido)naphthalen-1-yloxy)ethyl)pyridin-2-yl)-2-methoxyacetamide;
*N*-(4-(2-(4-(3-(3-*tert*-Butyl-1-*p*-tolyl-1*H*-pyrazol-5-yl)ureido)naphthalen-1-yloxy)ethyl)pyridin-2-yl)-2-(2-methoxyethoxy)acetamide;
4-(2-(4-(3-(3-*tert*-Butyl-1-*p*-tolyl-1*H*-pyrazol-5-yl)ureido)naphthalen-1-yloxy)ethyl)-1-methyl-3-(pyridin-2-yl)urea;
4-(2-(4-(3-(3-*tert*-Butyl-1-*p*-tolyl-1*H*-pyrazol-5-yl)ureido)naphthalen-1-yloxy)ethyl)-3-(pyridin-2-yl)urea;
*N*-(4-(2-(4-(3-(3-*tert*-Butyl-1-*p*-tolyl-1*H*-pyrazol-5-yl)ureido)naphthalen-1-yloxy)ethyl)pyridin-3-yl)-2-methoxyacetamide;
*N*-(4-(2-(4-(3-(3-*tert*-Butyl-1-*p*-tolyl-1*H*-pyrazol-5-yl)ureido)naphthalen-1-yloxy)ethyl)pyridin-3-yl)-2-(2-methoxyethoxy)acetamide;
N-(4-(2-(4-(3-(3-*tert*-butyl-1-*p*-tolyl-1*H*-pyrazol-5-yl)ureido)naphthalen-1-yl)ethoxy)pyridin-2-yl)-2-methoxyacetamide;
*N*-(4-(1-(4-(3-(3-*tert*-Butyl-1-p-tolyl-1H-pyrazol-5-yl)ureido)naphthalen-1-yloxy)ethyl)pyridin-2-yl)-2-methoxyacetamide;
*N*-(4-(2-(4-(3-(3-*tert*-Butyl-1-*p*-tolyl-1*H*-pyrazol-5-yl)ureido)naphthalen-1-yloxy)-2-methyl propyl)pyridin-2-yl)-2-methoxyacetamide;
*N*-(4-(2-(4-(3-(3-*tert*-Butyl-1-*p*-tolyl-1*H*-pyrazol-5-yl)ureido)naphthalen-1-yloxy)propyl)pyridin-2-yl)-2-methoxyacetamide;
*N*-(4-(1-(4-(3-(3-*tert*-Butyl-1-*p*-tolyl-1*H*-pyrazol-5-yl)ureido)naphthalen-1-yloxy)propan-2-yl) pyridin-2-yl)-2-methoxyacetamide;
*N*-(4-(1-(4-(3-(3-*tert*-Butyl-1-*p*-tolyl-1-pyrazol-5-yl)ureido)naphthalen-1-yloxy)-2-methyl propan-2-yl)pyridin-2-yl)-2-methoxyacetamide;
*N*-(4-((4-(3-(3-*tert*-Butyl-1-(4-(hydroxymethyl)phenyl)-1*H*-pyrazol-5-yl)ureido)naphthalen-1-yloxy)methyl)pyridin-2-yl)-2-methoxyacetamide;
*N*-(4-((4-(3-(3-*tert*-Butyl-1-*p*-tolyl-1*H*-pyrazol-5-yl)ureido)naphthalen-1-yloxy)methyl)pyrimidin -2-yl)-2-methoxyacetamide;
*N*-(4-(4-(3-(3-*tert*-Butyl-1-*p*-tolyl-1*H*-pyrazol-5-yl)ureido)naphthalen-1-yloxy)pyridin-2-yl)-2-(methylsulfonyl)acetamide
*N*-(4-(4-(3-(3-*tert*-Butyl-1-*p*-tolyl-1*H*-pyrazol-5-yl)ureido)naphthalen-1-yloxy)pyridin-2-yl)-2-(2-methoxyethoxy)acetamide;
*N*-(4-(4-(3-(3-*tert*-Butyl-1-*p*-tolyl-1*H*-pyrazol-5-yl)ureido)naphthalen-1-yloxy)pyridin-2-yl) tetrahydro-2*H*-pyran-4-carboxamide;
*N*-(4-(4-(3-(3-*tert*-Butyl-1-*p*-tolyl-1*H*-pyrazol-5-yl)ureido)naphthalen-1-yloxy)pyridin-2-yl)-2-(methylthio)acetamide;
*N*-(4-(4-(3-(3-*tert*-Butyl-1-*p*-tolyl-1*H*-pyrazol-5-yl)ureido)naphthalen-1-yloxy)pyridin-2-yl)-3-methoxypropanamide;
*N*-(4-(4-(3-(3-*tert*-Butyl-1-*p*-tolyl-1*H*-pyrazol-5-yl)ureido)naphthalen-1-yloxy)pyridin-2-yl)-2-hydroxyacetamide;
*N*-(4-(4-(3-(3-Isopropyl-1-*p*-tolyl-1*H*-pyrazol-5-yl)ureido)naphthalen-1-yloxy)pyridin-2-yl)-2-methoxyacetamide;
*N*-(4-(4-(3-(3-Ethyl-1-*p*-tolyl-1*H*-pyrazol-5-yl)ureido)naphthalen-1-yloxy)pyridin-2-yl)-2-methoxyacetamide;
*N*-(4-(4-(3-(3-(1-Hydroxy-2-methylpropan-2-yl)-1-*p*-tolyl-1*H*-pyrazol-5-yl)ureido)naphthalen-1-yloxy)pyridin-2-yl)-2-methoxyacetamide;
*N*-(4-(4-(3-(3-*tert*-butyl-1-(2,3,5,6-tetradeutero-4-(trideuteromethyl)phenyl)-1*H*-pyrazol-5-yl)ureido)naphthalen-1-yloxy)pyridin-2-yl)-2-methoxyacetamide;
*N*-(4-(4-(3-(3-*tert*-Butyl-1-*p*-tolyl-1*H*-pyrazol-5-yl)ureido)naphthalen-1-yloxy)pyridin-2-yl)-2-morpholinoacetamide;
*N*-(4-(4-(3-(3-*tert*-Butyl-1-*p*-tolyl-1*H*-pyrazol-5-yl)ureido)naphthalen-1-yloxy)pyridin-2-yl)-(dimethylamino)acetamide;
*N*-(4-(4-(3-(3-*tert*-Butyl-1-*p*-tolyl-1*H*-pyrazol-5-yl)ureido)naphthalen-1-yloxy)pyridin-2-yl)-2-(2-methoxyethylamino)acetamide;
*N*-(4-(4-(3-(3-*tert*-Butyl-1-*p*-tolyl-1*H*-pyrazol-5-yl)ureido)naphthalen-1-yloxy)pyridin-2-yl)-2-ureidoacetamide;
*N*-(4-(4-(3-(3-*tert*-Butyl-1-*p*-tolyl-1*H*-pyrazol-5-yl)ureido)naphthalen-1-yloxy)pyridin-2-yl)-2-(2-methoxyacetamido)acetamide;
*N*-(2-(4-(4-(3-(3-*tert*-Butyl-1-*p*-tolyl-1*H*-pyrazol-5-yl)ureido)naphthalen-1-yloxy)pyridin-2-ylamino)-2-oxoethyl)tetrahydro-2*H*-pyran-4-carboxamide;
*N*-(2-(4-(4-(3-(3-*tert*-Butyl-1-*p*-tolyl-1*H*-pyrazol-5-yl)ureido)naphthalen-1-yloxy)pyridin-2-ylamino)-2-oxoethyl)isonicotinamide;
*N*-(4-(4-(3-(3-*tert*-Butyl-1-*p*-tolyl-1*H*-pyrazol-5-yl)ureido)naphthalen-1-yloxy)pyridin-2-yl)-2-(2-(methylsulfonyl)acetamido)acetamide;
*N*-(2-(4-(4-(3-(3-*tert*-Butyl-1-*p*-tolyl-1*H*-pyrazol-5-yl)ureido)naphthalen-1-yloxy)pyridin-2-ylamino)-2-oxoethyl)-3-morpholinopropanamide;
*N*-(2-(4-(4-(3-(3-*tert*-Butyl-1-*p*-tolyl-1*H*-pyrazol-5-yl)ureido)naphthalen-1-yloxy)pyridin-2-ylamino)-2-oxoethyl)morpholine-4-carboxamide;
*N*-(2-(4-(4-(3-(3-*tert*-Butyl-1-*p*-tolyl-1*H*-pyrazol-5-yl)ureido)naphthalen-1-yloxy)pyridin-2-ylamino)-2-oxoethyl)-2,6-difluoro-3-(2-(2-methoxyethoxy)ethoxy)benzamide;
*N*-(4-(4-(3-(3-*tert*-Butyl-1-*p*-tolyl-1*H*-pyrazol-5-yl)ureido)phenoxy)pyridin-2-yl)-2-methoxy acetamide;
*N*-(4-(4-(3-(3-*tert*-Butyl-1-*p*-tolyl-1*H*-pyrazol-5-yl)ureido)-2-methylphenoxy)pyridin-2-yl)-2-methoxyacetamide;
*N*-(4-(4-(3-(3-*tert*-Butyl-1-*p*-tolyl-1*H*-pyrazol-5-yl)ureido)-3-methylphenoxy)pyridin-2-yl)-2-methoxyacetamide;
*N*-(4-(4-(3-(3-*tert*-Butyl-1-*p*-tolyl-1*H*-pyrazol-5-yl)ureido)-2-methoxyphenoxy)pyridin-2-yl)-2-methoxyacetamide;
*N*-(4-(4-(3-(3-*tert*-Butyl-1-*p*-tolyl-1*H*-pyrazol-5-yl)ureido)-2,3-dimethylphenoxy)pyridin-2-yl)-2-methoxyacetamide;
*N*-(4-(4-(3-(3-*tert*-Butyl-1-*p*-tolyl-1*H*-pyrazol-5-yl)ureido)-3-methoxyphenoxy)pyridin-2-yl)-2-methoxyacetamide;
N-Ethyl-N'-4-(4-(3-(3-*tert*-butyl-1-*p*-tolyl-1*H*-pyrazol-5-yl)ureido)naphthalen-1-yloxy)pyridin-2-ylurea;
4-(4-(3-(3-*tert*-Butyl-1-*p*-tolyl-1*H*-pyrazol-5-yl)ureido)naphthalen-1-yloxy)pyridin-2-ylurea;
*N*-Propan-2-yl-*N*'-4-(4-(3-(3-*tert*-butyl-1-*p*-tolyl-1*H*-pyrazol-5-yl)ureido)naphthalen-1-yloxy)pyridin-2-ylurea;
1-(3-(*tert*-Butyl)-1-(*p*-tolyl)-1*H*-pyrazol-5-yl)-3-(4-((2-(3-phenylureido)pyridin-4-yl)oxy) naphthalen-1-yl)urea;
1-(4-((2-(3-Benzylureido)pyridin-4-yl)oxy)naphthalen-1-yl)-3-(3-(*tert*-butyl)-1-(*p*-tolyl)-1*H-*pyrazol-5-yl)urea;
1-(4-((2-(3-Cyclopropylureido)pyridin-4-yl)oxy)naphthalen-1-yl)-3-(3-(*tert*-butyl)-1-(*p*-tolyl)-1*H-*pyrazol-5-yl)urea;
1-(3-(*tert*-Butyl)-1-(*p*-tolyl)-1*H*-pyrazol-5-yl)-3-(4-((2-(3-(2-methoxyethyl)ureido)pyridin-4-yl)oxy)naphthalen-1-yl)urea;
1-(3-(*tert*-Butyl)-1-(*p*-tolyl)-1*H*-pyrazol-5-yl)-3-(4-((2-(3-cyclopentyl)ureido)pyridin-4-yl)oxy) naphthalen-1-yl)urea;
1-(3-(*tert*-Butyl)-1-(*p*-tolyl)-1*H*-pyrazol-5-yl)-3-(4-((2-(3-methyl)ureido)pyridin-4-yl)oxy) naphthalen-1-yl)urea;
Ethyl 2-(3-(4-((4-(3-(3-(*tert*-butyl)-1-(p-tolyl)-1*H*-pyrazol-5-yl)ureido)naphthalen-1-yl)oxy) pyridin-2-yl)ureido)acetate;
4-(3-(4-(4-(3-(3-*tert*-Butyl-1-*p*-tolyl-1*H*-pyrazol-5-yl)ureido)naphthalen-1-yloxy)pyridin-2-yl) ureido)piperidine;
*N*-Acetyl 4-(3-(4-(4-(3-(3-*tert*-butyl-1-*p*-tolyl-1*H*-pyrazol-5-yl)ureido)naphthalen-1-yloxy) pyridin-2-yl)ureido)piperidine;
2-(2-Methoxyethoxy)-1-(4-(3-(4-(4-(3-(3-*tert*-butyl-1-*p*-tolyl-1*H*-pyrazol-5-yl)ureido) naphthalen-1-yloxy)pyridin-2-yl)ureido)piperidin-1-yl)ethanone;
*N*-Methylsulfonyl-4-(3-(4-(4-(3-(3-*tert*-butyl-1-*p*-tolyl-1*H*-pyrazol-5-yl)ureido)naphthalen-1-yloxy)pyridin-2-yl)ureido)piperidine;
N-(4-(4-(3-(3-*tert*-Butyl-1-*p*-tolyl-1*H*-pyrazol-5-yl)ureido)naphthalen-1-yloxy)pyridin-2-yl) morpholine-4-carboxamide;
*N*-(4-((4-(3-(3-(*tert*-butyl)-1-(*p*-tolyl)-1*H*-pyrazol-5-yl)ureido)naphthalen-1-yl)oxy)pyridin-2-yl)-4-methylpiperazine-1-carboxamide;
3-(4-((4-(3-(3-(*tert*-butyl)-1-(*p*-tolyl)-1*H*-pyrazol-5-yl)ureido)naphthalen-1-yl)oxy)pyridin-2-yl)-1,1-dimethylurea;
*N*-(4-((4-(3-(3-(*tert*-Butyl)-1-(*p*-tolyl)-1*H*-pyrazol-5-yl)ureido)naphthalen-1-yl)oxy)pyridin-2-yl)piperidine-1-carboxamide;
*N*-Methyl-*N*-(2-(morpholin-4-yl)ethyl)-*N'*-4-(4-(3-(3-*tert*-butyl-1-*p*-tolyl-1*H*-pyrazol-5-yl)ureido) naphthalen-1-yloxy)pyridin-2-ylurea;
*N*-(4-(morpholin-4-yl)butyl)-*N'*-4-(4-(3-(3-*tert*-butyl-1-*p*-tolyl-1*H*-pyrazol-5-yl)ureido) naphthalen-1-yloxy)pyridin-2-ylurea;
*N*-(2-(morpholin-4-yl)ethyl)-*N'*-4-(4-(3-(3-*tert*-butyl-1-*p*-tolyl-1*H*-pyrazol-5-yl)ureido) naphthalen-1-yloxy)pyridin-2-ylurea;
*N*-(3-methylisoxazol-5-yl)methyl-*N*'-4-(4-(3-(3-*tert*-butyl-1-*p*-tolyl-1*H*-pyrazol-5-yl)ureido) naphthalen-1-yloxy)pyridin-2-ylurea;
*N*-(1-methyl)piperidin-4-yl-*N*'-4-(4-(3-(3-*tert*-butyl-1-*p*-tolyl-1*H*-pyrazol-5-yl)ureido) naphthalen-1-yloxy)pyridin-2-ylurea;
*N*-(4-(4-(3-(3-*tert*-Butyl-1-*p*-tolyl-1*H*-pyrazol-5-yl)ureido)naphthalen-1-yloxy)pyridin-2-yl)-4-hydroxypiperidine-1-carboxamide;
*N*-(3-(imidazol-1-yl)propyl)-*N'*-4-(4-(3-(3-*tert*-butyl-1-*p*-tolyl-1*H*-pyrazol-5-yl)ureido) naphthalen-1-yloxy)pyridin-2-ylurea;
*N*-(2-(3-(4-(4-(3-(3-*tert*-Butyl-1-*p*-tolyl-1*H*-pyrazol-5-yl)ureido)naphthalen-1-yloxy)pyridin-2-yl)ureido)acetyl)pyrrolidine;
(*R*)-*N*-(4-(4-(3-(3-*tert*-Butyl-1-*p*-tolyl-1*H*-pyrazol-5-yl)ureido)naphthalen-1-yloxy)pyridin-2-yl)-3-(dimethylamino)pyrrolidine-1-carboxamide;
*N*-(4-(4-(3-(3-*tert*-Butyl-1-*p*-tolyl-1*H*-pyrazol-5-yl)ureido)naphthalen-1-yloxy)pyridin-2-yl)pyrrolidine-1-carboxamide;
2-(3-(4-(4-(3-(3-*tert*-Butyl-1-*p*-tolyl-1*H*-pyrazol-5-yl)ureido)naphthalen-1-yloxy)pyridin-2-yl)ureido)-*N*-methylacetamide;
2-(3-(4-(4-(3-(3-*tert*-Butyl-1-*p*-tolyl-1*H*-pyrazol-5-yl)ureido)naphthalen-1-yloxy)pyridin-2-yl)ureido)-*N*-(2-morpholinoethyl)acetamide;
2-(3-(4-(4-(3-(3-*tert*-Butyl-1-*p*-tolyl-1*H*-pyrazol-5-yl)ureido)naphthalen-1-yloxy)pyridin-2-yl)ureido)acetyl morpholine;
2-(3-(4-(4-(3-(3-*tert*-Butyl-1-*p*-tolyl-1*H*-pyrazol-5-yl)ureido)naphthalen-1-yloxy)pyridin-2-yl)ureido)-*N*-(2-(pyridin-4-yl)ethyl)acetamide;
*N*-(3-(1*H*-Imidazol-1-yl)propyl)-2-(3-(4-(4-(3-(3-*tert*-butyl-1-*p*-tolyl-1*H*-pyrazol-5-yl)ureido) naphthalen-1-yloxy)pyridin-2-yl)ureido)acetamide;
1-(2-(3-(4-(4-(3-(3-*tert*-Butyl-1-*p*-tolyl-1*H*-pyrazol-5-yl)ureido)naphthalen-1-yloxy)pyridin-2-yl)ureido)acetyl)-4-methylpiperazine;
*N*-(3-(1*H*-Imidazol-1-yl)propyl)-2-(3-(4-(4-(3-(3-*tert*-butyl-1-*p*-tolyl-1*H*-pyrazol-5-yl)ureido) naphthalen-1-yloxy)pyridin-2-yl)ureido)acetamide;
*N*-(6-(4-(3-(3-*tert*-Butyl-1-*p*-tolyl-1*H*-pyrazol-5-yl)ureido)naphthalen-1-yloxy)pyrimidin-4-yl)-2-methoxyacetamide;
*N*-(6-(4-(3-(3-*tert*-Butyl-1-*p*-tolyl-1*H*-pyrazol-5-yl)ureido)phenoxy)pyrimidin-4-yl)-2-methoxy acetamide;
*N*-(4-(4-(3-(3-*tert*-Butyl-1-*p*-tolyl-1*H*-pyrazol-5-yl)ureido)naphthalen-1-yloxy)pyrimidin-2-yl)-2-methoxyacetamide;
3-(4-(4-(3-(3-*tert*-Butyl-1-*p*-tolyl-1*H*-pyrazol-5-yl)ureido)naphthalen-1-yloxy)pyrimidin-2-yl) urea;
1-Methyl-3-(4-(4-(3-(3-*tert*-butyl-1-*p*-tolyl-1*H*-pyrazol-5-yl)ureido)naphthalen-1-yloxy) pyrimidin-2-yl)urea;
1,1-Dimethyl-3-(4-(4-(3-(3-*tert*-butyl-1-*p*-tolyl-1*H*-pyrazol-5-yl)ureido)naphthalen-1-yloxy) pyrimidin-2-yl)urea;
1-Cyclopropyl-3-(4-(4-(3-(3-*tert*-butyl-1-*p*-tolyl-1*H*-pyrazol-5-yl)ureido)naphthalen-1-yloxy) pyrimidin-2-yl)urea;
(4-(4-(3-(3-*tert*-Butyl-1-*p*-tolyl-1*H*-pyrazol-5-yl)ureido)naphthalen-1-yloxy)pyrimidin-2-yl) morpholine-4-carboxamide;
3-(6-(4-(3-(3-*tert*-Butyl-1-p-tolyl-1*H*-pyrazol-5-yl)ureido)naphthalen-1-yloxy)pyrimidin-4-yl)urea; 2-(3-(4-(4-(3-(3-*tert*-Butyl-1-*p*-tolyl-1*H*-pyrazol-5-yl)ureido)naphthalen-1-yloxy)pyridin-2-yl)ureido)acetic acid
or a pharmaceutically acceptable salt thereof, including all stereoisomers, tautomers and isotopic derivatives thereof.

Examples of salts of compound (**I**) include all pharmaceutically acceptable salts, such as, without limitation, acid addition salts of mineral acids such as HCl and HBr salts and addition salts of organic acids such as a methansulfonic acid salt.

The disclosure herein extends to solvates of compounds of formula (**I**). Examples of solvates include hydrates.

The compounds of the disclosure include those where the atom specified is a naturally occurring or non-naturally occurring isotope. In one embodiment the isotope is a stable isotope. Thus the compounds of the disclosure include, for example deuterium containing compounds and the like.

The compounds described herein may include one or more chiral centres, and the disclosure extends to include racemates, both enantiomers (for example each substantially free of the other enantiomer) and all stereoisomers resulting therefrom. In one embodiment one enantiomeric form is present in a substantially purified form that is substantially free of the corresponding entaniomeric form.

The disclosure also extends to all polymorphic forms of the compounds herein defined.

Compounds of formula (**I**) can be prepared by a process comprising reacting compounds of formula (**II**): 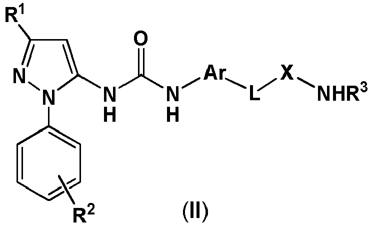 wherein Q is not -NHR* (wherein R* is the remainder of the Q fragment) with a compound of formula (**IIIa**): 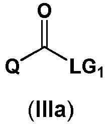 where LG₁ is a leaving group for example halogen, such as chloro.
When NR³C(O)Q is NR³C(O)NHR* compounds of formula (**I**) can be prepared by reacting compounds of formula (**II**) with a compound of formula (**IIIb**):

Q=C=O (**IIIb**)

The reaction is suitably carried out in the presence of a base (e.g. DIPEA). The reaction is suitably carried out in an aprotic solvent or solvent mixture, e.g. DCM and DMF.

Compounds of formula (**II**) can be prepared by reacting a compound of formula (**IV**): 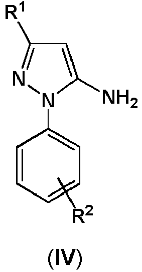 where R¹ and R² are as defined above for compounds of formula (**I**), with a compound of formula (**VI**): 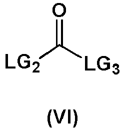 wherein LG₂ and LG₃ each independently represent leaving groups (e.g. LG₂ and LG₃ both represent imidazolyl followed by reaction with a compound of formula (**V**): 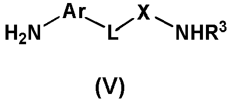 wherein Ar, L, X and R³ are defined above for compounds of formula (**I**)

The reaction is suitably carried out in an aprotic solvent (e.g. dichloromethane), using appropriate protecting groups for chemically sensitive groups and a base, for example DIPEA.

Specifically compounds of formula (**II**) can be prepared by reacting a compound of formula (**IVa**): 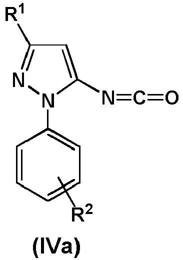 where R¹ and R² are as defined above for compounds of formula (**I**), with a compound of formula (**V**).

The reaction may be performed in the presence of a sterically hindered base such as DIPEA, in a suitable inert solvent such as dichloromethane.

DIPEA, in a suitable inert solvent such as dichloromethane.

Compounds of formula (**I**) wherein R² is a hydroxyalkyl may be prepared by reacting a (hydrazinylphenyl)alkanoic acid with an alkanoyl acetonitrile such as R¹C(O)CH₂CN, for example. The coupling may be effected in presence of an alcohol solvent such as ethanol and a mineral acid, such as HCl followed by treatment with a lithium hydroxide in a solvent such as THF. The substituent R² comprising a hydroxyalkyl may be revealed by a reduction employing borane in a suitable solvent, for example THF to afford a compound of formula (**IV**) where R² is hydroxylated alkyl. The hydroxyl may then be protected, for example as a silyl ether and and (**IV**) carried through one of the routes described elsewhere in this section to generate a compound of formula (**I**) in which R² is a protected hydroxyalkyl group. The hydroxyl can be revealed by cleavage of the sillyl group, for example with tetrabutylammonium fluoride.

Compounds of formula (**I**) wherein R¹ is a hydroxylated alkyl species may be prepared by reacting protected benzyloxyalkanoyl acetonitrile an aryl hydrazine employing analogous conditions to those described directly above.

A compound of formula (**IVa**) can be prepared by reacting a compound of formula (**IV**) with phosgene or a phosgene equivalent such as diphosgene or triphosgene in the presence of a base such DIPEA. It will be understood by persons skilled in the art that the compound of formula (**IVa**) is generally a reactive intermediate, and may be isolated and used directly in subsequent transformations or may be a transient intermediate, that is generated *in situ* and used without isolation.

More specifically compounds of formula (**II**) may be prepared by reacting a compound of formula (**IVb**): 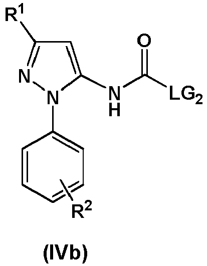 where LG₂ is as defined above with a compound of formula (**VI**).

The reaction may be performed in the presence of a sterically hindered base such as DIPEA, in a suitable inert solvent such as dichloromethane.

A compound of (**IVb**) can be prepared by reacting a compound of formula (**IV**) with a compound of formula (**VI**) in the presence of a base such as DIPEA. It will be understood by persons skilled in the art that the compound of formula (**IVb**) may be an intermediate, including a transient intermediate, that is not isolated.
A compound of formula (**V**) may be prepared by reduction of a compound of formula (**VII**): 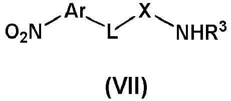 wherein Ar, L, X and R³ are as defined above for compounds of formula (**I**), for example by hydrogenation in the presence of a catalyst such as platinum supported on carbon.

The reaction is suitably carried out in polar protic solvent or mixture of solvents (e.g. methanol and acetic acid).

Alternatively, a compound of formula (**V**) where L is O may be prepared by deprotecting a compound of formula **(VIIa):** 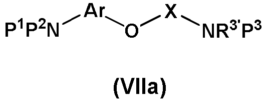 wherein P¹, P² and P³ are protecting groups and R³ is a protecting group, for example acetyl such as -C(O)CH₂OCH₃ or R³ as defined above for compounds of formula (**I**).

A compound of formula (**VII**) wherein L represents -(CH₂)ₙO(CH₂)ₘ or (CH₂)ₙOR^{b}, as defined above, wherein n is zero and the linker L contains at least one -CH₂- may be prepared by reaction of a compound of formula (**VIIIa**) or (**VIIIb**): 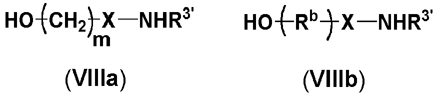 or analogues thereof and wherein m, X and R^{b} are as defined above for compounds of formula (**I**) and R^{3'} is a protecting group or R³ as defined above for compounds of formula (**I**) with a compound of formula (**IX**) or (**X**): 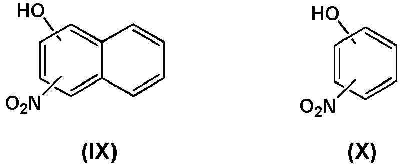 wherein compounds (**IX**) and (**X**) may bear optional substitutents as defined above for compounds of formula (**I**).

The reaction may be performed under Mitsunobu conditions, such as in the presence of triphenylphosphine and diisopropylazodicarboxylate. The reaction is suitably carried out in a polar aprotic solvent (e.g. tetrahydrofuran, in particular anhydrous tetrahydrofuran).

In an alternative process, certain compounds of formula (**V**), wherein Ar, L and X are as defined above for compounds of formula (I) may be prepared by reacting a compound of formula (**XI**): 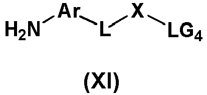 or a protected derivative thereof, such as a carbamate, wherein Ar, L and X are as defined above and LG₄ represents a leaving group such as chloro (in particular where L represents 0) with an amidation reagent, for example with the carbamate (**XII**): 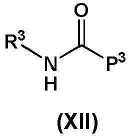 wherein P³ and R³ are as defined above in the presence of an dry inert solvent such as THF and a suitable palladium catalyst, for example under a nitrogen atmosphere, followed by deprotection of both the original and newly introduced protected amines, for example employing dichloromethane and TFA.

In one embodiment the compound formula (**XII**) is:

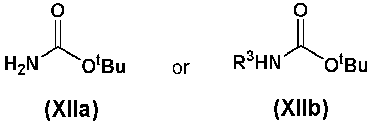

Compounds of formula (**XI**), where L is linked to X through O, for example, wherein L represents -(CH₂)ₙO(CH₂)ₘ or R^{a}O(CH₂)ₘ, as defined above, wherein m is zero, may be prepared by reacting a compound of formula (**XIII**): 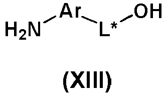 or a protected derivative thereof, for example where the free amine is protected as a carbamate, wherein Ar is as defined above and L* and OH taken together represent L (in particular L* represents alkylene or a bond), with a compound of formula (**XIV**): 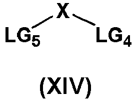 wherein X is as defined above and LG₄ represents a leaving group such as chloro and LG₅ represents a leaving group such a fluoro.

The reaction may be performed in the presence of a strong base such as sodium hydride in a polar aprotic solvent such as DMF.

Compounds of formula (**XIII**) may be prepared from compounds of formula (**XV**): 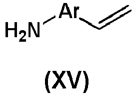 wherein the free amine is suitably protected, for example as a carbamate, and Ar is as defined above, and wherein L represents -(CH₂)ₙO(CH₂)ₘ, as defined above, wherein n is 2 and m is zero by hydroboration with a reagent such as 9-BBN followed by oxidation using hydrogen peroxide in the present of a base such as sodium hydroxide.

Compounds of formula (**XV**) may be prepared in a two step transformation from compounds of formula (**XVI**) *via* compounds of formula (**XVII**), wherein Ar is as defined above and the free amine is suitably protected, for example as a carbamate:

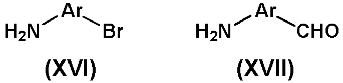

Treatment of a compound of formula (**XVI**) with a base such as n-butyl lithium in an inert solvent such as THF followed by the addition of DMF provides compounds of formula (**XVII**). Compounds of formula (**XVII**) may be transformed into compounds of formula (**XV**) by an olefination step such as by reaction with a Wittig reagent generated *in situ*, such as the ylid generated from methyltriphenylphosphonium bromide in the presence of a base such potassium *tert*-butoxide. Generally the reaction will be performed in an inert solvent, for example THF, and under an inert atmosphere such as nitrogen at a low temperature, such a - 78°C.

Compounds of formula (I) wherein Q is linked to -NR³C(O) by -CH₂V', wherein V' is a heteroatom selected from N, O, or S, can be prepared by the process comprising of a nucleophilic displacement reaction on a compound of formula (**IIa**): 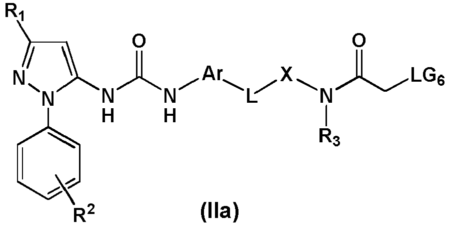 wherein R¹, R², Ar, L, X, NR³ are as defined above for compounds of formula (**I**) and LG₆ represents a leaving group, for example halogen such as chloro, with a compound of formula (**XVIII**):

**H-V'-q** **(XVIII)**

wherein H represents hydrogen, V' represents a heteroatom selected from N, NH, O, or S and q represents the residual portion of Q (i.e. -CH₂V'-q = Q).

The reaction may, be performed in the presence of a sterically hindered base, for example DIPEA, in an inert solvent, for example dichloromethane.

Compounds of formula (**IIa**) may be prepared by reacting a compound of formula (**II**) with a compound of formula (**XVI**): 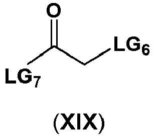 wherein LG₆ is defined above for compounds of formula (**IIa**), and LG₇ is a leaving group, for example a halogen such as chloro.

The reaction may, for example be performed in the presence of a sterically hindered base, for example DIPEA, in an inert solvent, for example dichloromethane.

Compounds of formula (**I**) wherein Q is NH-(CH₂)_{d}-C(O)NHR⁸, can be prepared by the process comprising of an amide coupling between (**IIb**): 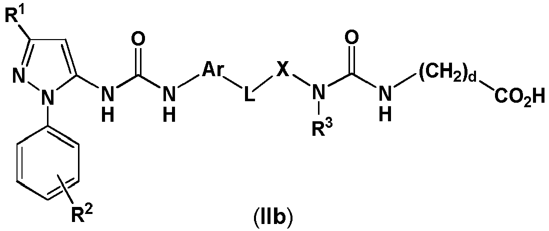 wherein R¹, R², Ar, L, X and R^{3 9} are as defined above for compounds of formula (**I**) and d is an integer 1 to 5 (such as 1 to 4), and an amine R⁸NH₂ using a coupling reagent such as EDC.

Compounds of formula (**IIb**) can be synthesisized by reaction of Compound (**II**) with an isocyanate of formula (**IIIb**) in which Q is N-(CH₂)ₚ-CO₂Et, followed by hydrolysis of the resulting ethyl ester product using, for example, aqueous lithium hydroxide in THF.

The reaction may, be performed in the presence of a sterically hindered base, for example DIPEA, in an inert solvent, for example dichloromethane.

Compounds of formula (**I**) wherein Q is NR⁷R⁸ can be prepared by the process comprising of reaction between an amine RR'NH and a compound of formula (**IIc**): 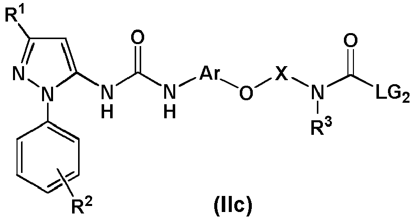 wherein R¹, R², Ar, X and R³ are as defined above for compounds of formula (**I**) and LG₂ is a leaving group such as 2-isopropenyloxy.

Compounds of formula (**IIc**) can be synthesized by reaction of Compound (**II**) with a compound of formula (**VI**), such as isopropenylchloroformate in the presence of a hindered base such as DIPEA.

The reaction may, be performed in the presence of a sterically hindered base, for example DIPEA, in an inert solvent, for example dichloromethane.

The preparation of classes of intermediates of broad synthetic utility and generic routes providing access to various embodiments of the invention are summarised below (**Schemes 1 to 16**).

A synthestic approach for the preparation of aminopyridine building blocks represented by **Intermediate *a*** is outlined below (**Scheme 1**) wherein R¹, R², R³ and L are as defined above for compounds of formula (**I**).

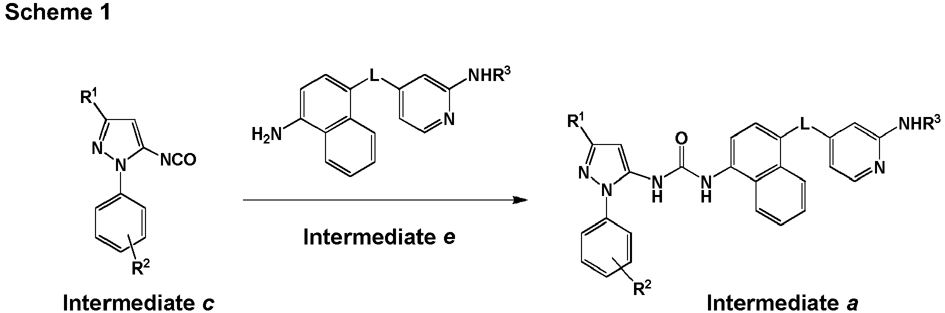

The preparation of activated aminopyrazoles represented by **Intermediate *b*** and **Intermediate *c*** from commercially available compounds, wherein R¹, and R² are as defined above for compounds of formula (**I**), is illustrated below (**Scheme 2**).

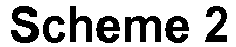

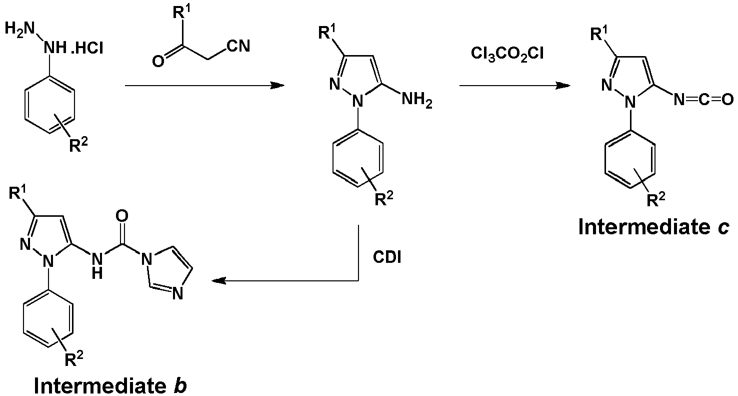

A preparative route to compounds represented by **Intermediate *d*** and **Intermediate *e,*** where L therein represents O(CH₂)ₙ is shown below (**Scheme 3**):

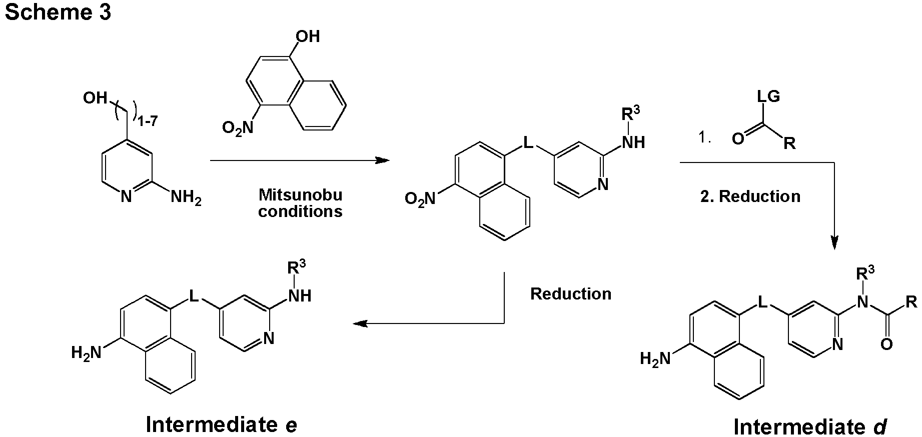

Methodologies which may be exploited to prepare compounds of the genus represented by **Intermediate *d*** for which L therein represents O and the specific diamine **Intermediate e*1*** are summarised below (**Scheme 4**).

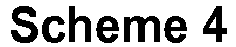

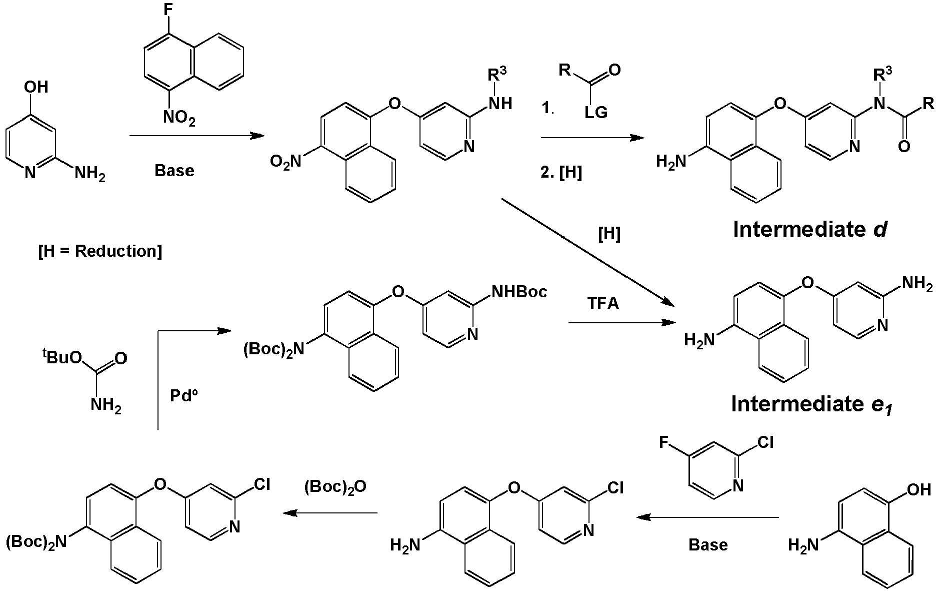

The preparation of compounds represented by **Intermediate *f*** wherein R¹, R² and L are as defined above for compounds of formula (**I**), Ar is naphthyl and X is pyridinyl is outlined below (**Scheme 5**).

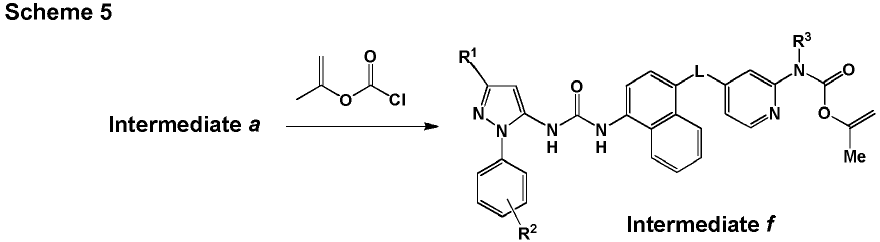

A generic process for the preparation of compounds represented by **Intermediate *g*** wherein L and R³ are as defined above for compounds of formula (I), Ar is naphthyl, X is pyridinyl and Q is NRR' is summarised below (**Scheme 6**).

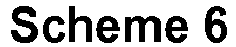

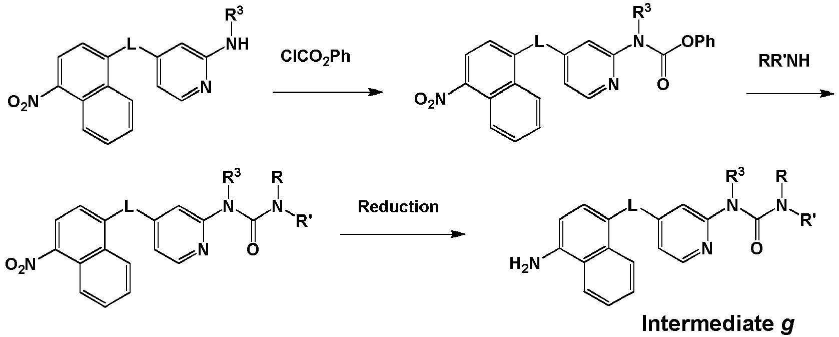

The preparation of compounds represented by **Intermediate *h*** and **Intermediate *j*** wherein L, R¹, R², and R³ are as defined above for compounds of formula (**I**), Ar is naphthyl and X is pyridinyl is summarised below (**Scheme 7**).

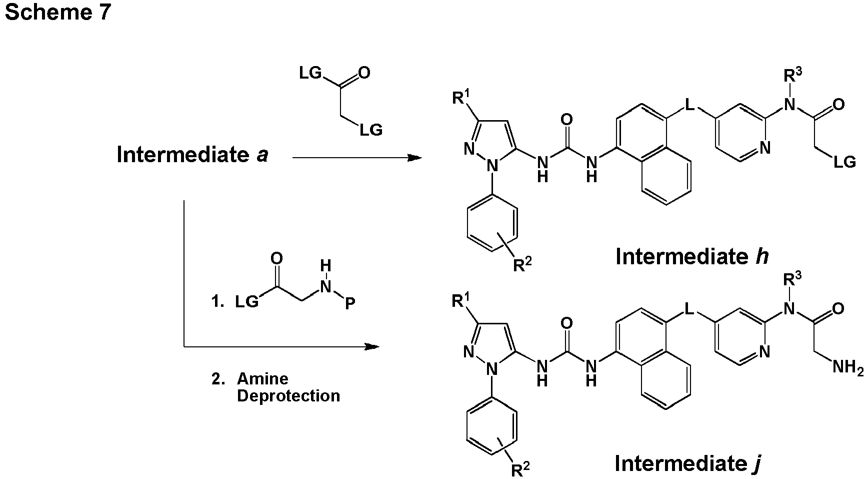

From the **Intermediates** described herein above, examples of the disclosure wherein L, R¹, R², R³ and Q are as defined above for compounds of formula (**I**), Ar is naphthyl and X is pyridinyl, may be prepared according to the transformations set out below (**Schemes 8a-f**). Particular routes disclosed below (**Scheme 8b** and **8c**) provide for examples of compounds of formula (**I**) wherein NHR or NRR' represent Q and wherein Q together with NHC(O) forms a urea.

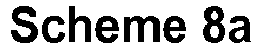

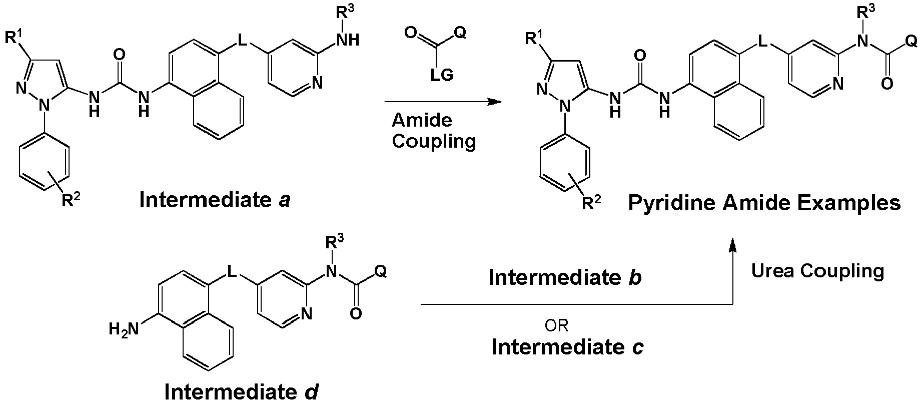

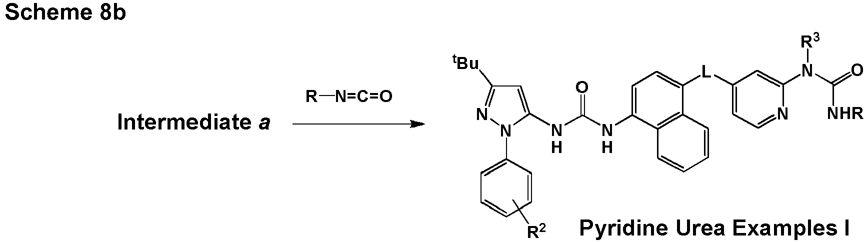

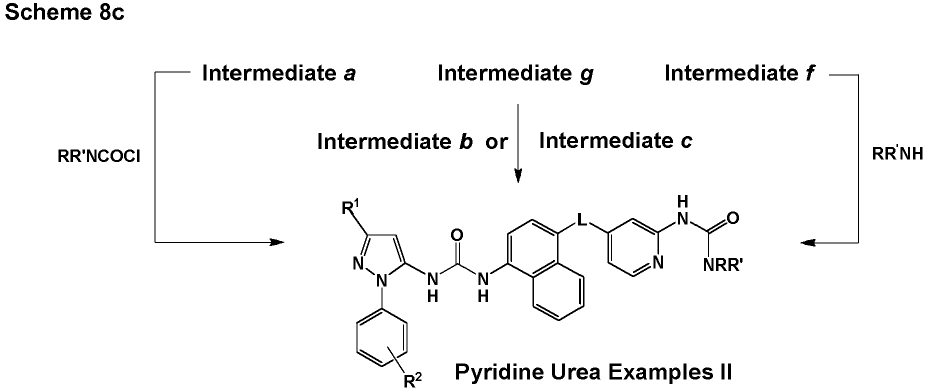

Similar methods may be used to prepare compounds of formula (**I**) wherein Ar is phenyl and X is as defined above for compounds of formula (**I**).

A general method of preparing compounds of formula (**I**) is provided below (**Scheme 9**) wherein Ar is naphthyl and X is pyridinyl and the fragment NR³C(O)Q is a glycinamide derivative

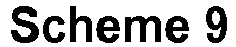

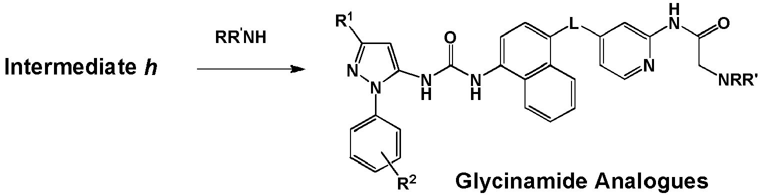

Similar methods may be used to prepare compounds of formula (**I**) wherein Ar is phenyl and X is as defined above for compounds of formula (**I**).

A synthetic approach for preparing compounds of formula (**I**) wherein Ar is naphthyl and X is pyridinyl and the fragment NR³C(O)Q is an *N*-acyl glycinamide derivative is provided below (**Scheme 10**), where LG is a leaving group and RC(O)NHCH₂ is Q.

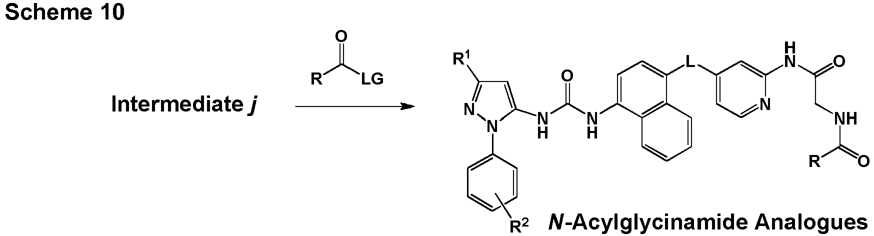

Similar methods may be used to prepare compounds of formula (**I**) wherein Ar is phenyl and X is as defined above for compounds of formula (**I**).

Synthetic routes are disclosed below suitable for the generation of additional intermediates, required for the preparation of examples of compounds of formula (**I**) wherein the fragment XNHR³ represents 2-aminopyrimidinyl or 4-aminopyrimidinyl.

Processes for the synthesis of the fragment H₂N-Ar-L-X-NHR³ wherein Ar is naphthyl and L is O and the fragment X-NHR³ is 2-aminopyrimidinyl are outlined below (**Scheme 11**).

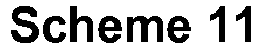

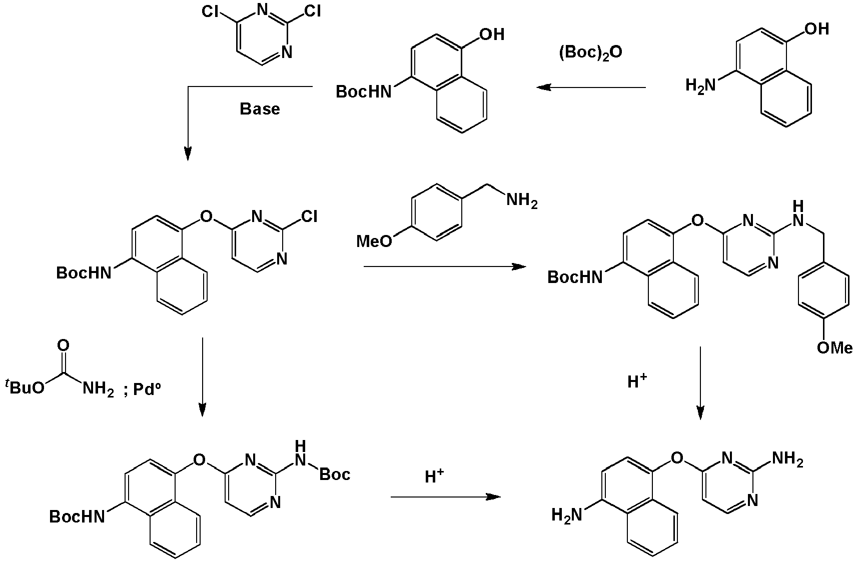

A procedure for the preparation of the fragment O₂N-Ar-L-X-NHR³ wherein Ar is naphthyl and L is O and the fragment X-NHR³ is 4-aminopyrimidinyl is dislclosed below (**Scheme 12**).

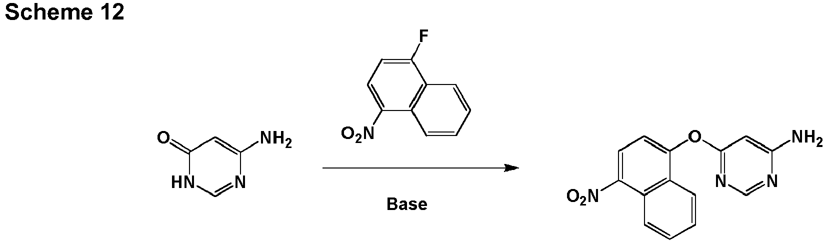

A procedure for the preparation of the fragment O₂N-Ar-L-X-NHR³ wherein Ar is phenyl and L is O and the fragment X-NHR³ is 4-aminopyrimidinyl is dislclosed below (**Scheme 13**).

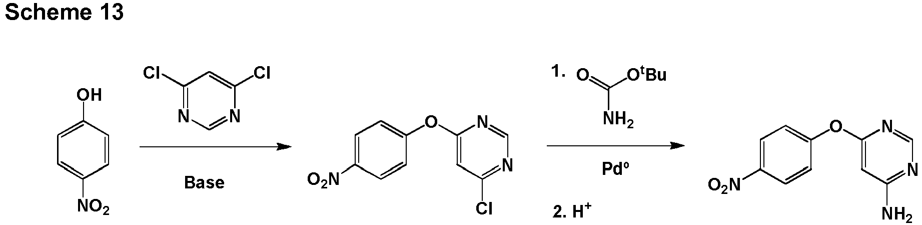

Those starting materials of the genera H₂N-Ar-L-X-NHR³ and O₂N-Ar-L-X-NHR³ in which the fragment X-NHR³ is an aminopyrimidinyl group and where L is a linker other than O can be prepared by analogous methods to those shown above.

A generic process for the preparation of compounds represented by **Intermediate *k*** wherein L and R³ are as defined above for compounds of formula (**I**), Ar is naphthyl and the fragment XNR³ is either 2-aminopyrimidinyl [**Y'=CH**; **X'=N**] or 4-aminopyrimidinyl [**Y'=N**; **X'=CH**] and NR³C(O)Q taken together represent an amide, is summarised below (**Scheme 14**).

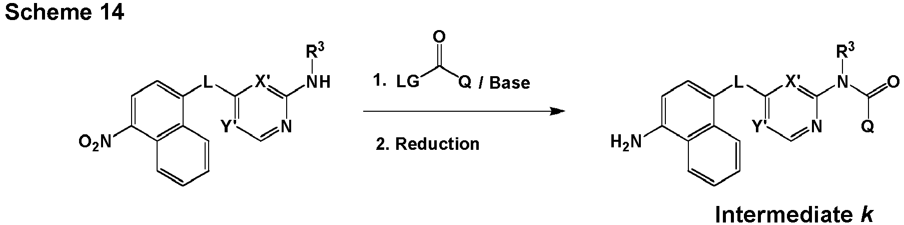

Analogous structures represented by **Intermediate *m*** wherein L and R³ are as defined above for compounds of formula (**I**), Ar is naphthyl and the fragment XNR³ is either 2-aminopyrimidinyl [**Y'=CH**; **X'=N**] or 4-aminopyrimidinyl [**Y'=N**; **X'=CH**] and NR³C(O)Q taken together represent a urea, are accessible by the processes illustrated below (**Scheme 15**).

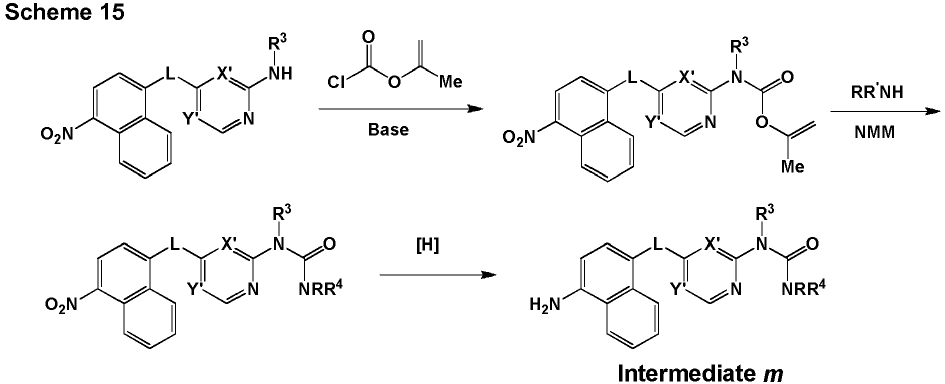

Compounds represented by **Intermediate *n*** wherein R¹, R², are as defined above for compounds of formula (**I**), Ar is naphthyl and L is O and the fragment XNR³ is 2-aminopyrimidinyl and R³ is H, may be prepared by the processes illustrated below (**Scheme 16**).

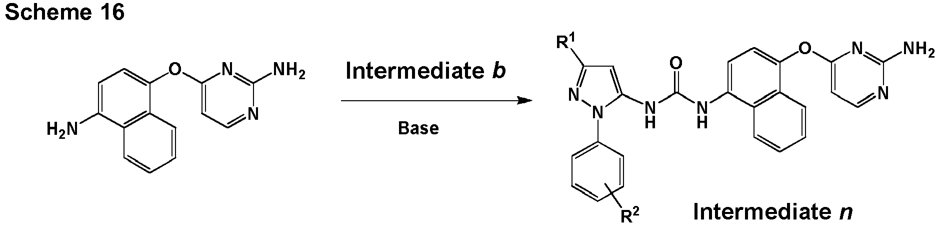

Compounds represented by **Intermediate *p*** wherein R¹ and R² are as defined above for compounds of formula (**I**), Ar is naphthyl and L is OCH₂ and the fragment XNR³ is 2-aminopyrimidinyl and R³ is H, may be prepared as disclosed below (**Scheme 17**).

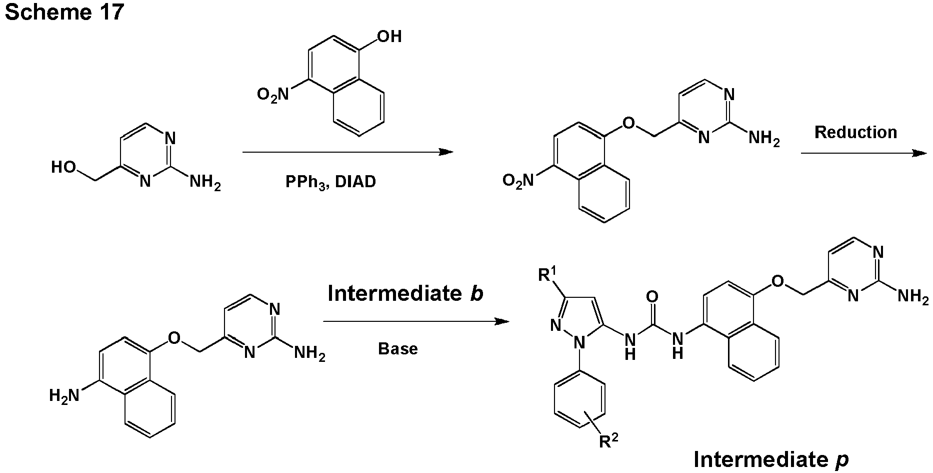

From the **Intermediates** described herein above, examples of the disclosure wherein L, R¹, R², R³ and Q are as defined above for compounds of formula (**I**), Ar is naphthyl and wherein the fragment XNHR³ represents 2-aminopyrimidinyl or 4-aminopyrimidinyl, may be prepared according to the transformations set out below (**Schemes 18a-d**). Particular routes disclosed below (**Scheme 18c** and **18d**) provide for examples of compounds of formula (**I**) wherein NHR or NRR' represent Q and wherein Q together with NHC(O) forms a urea.

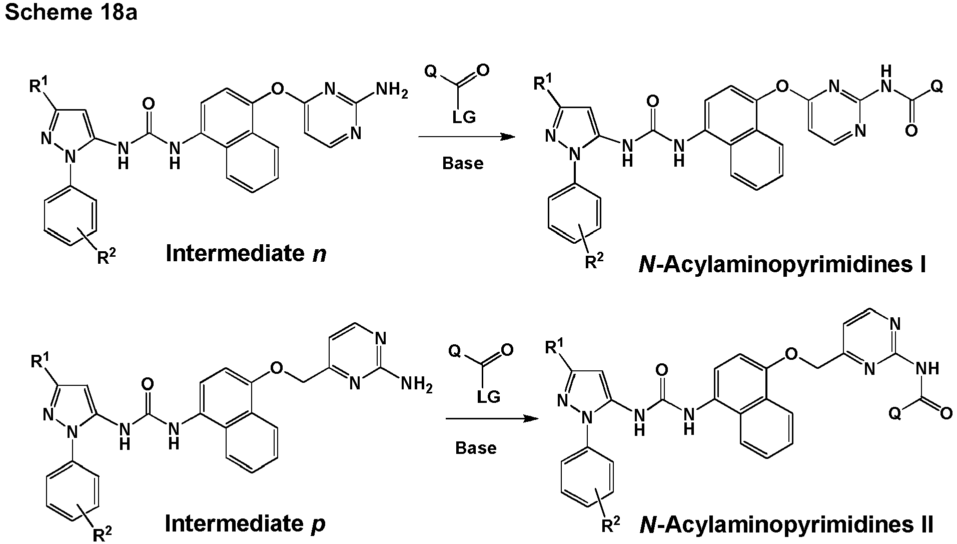

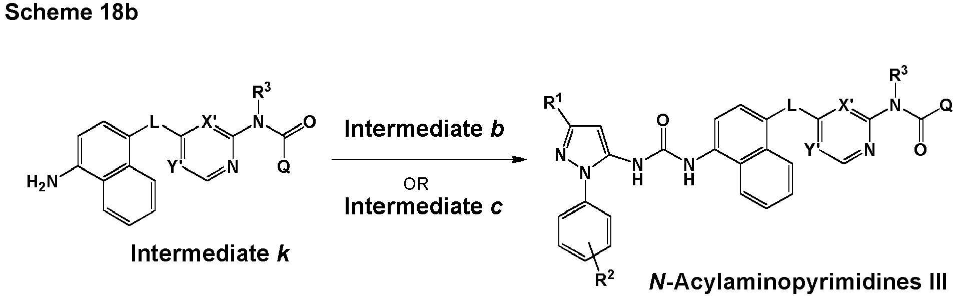

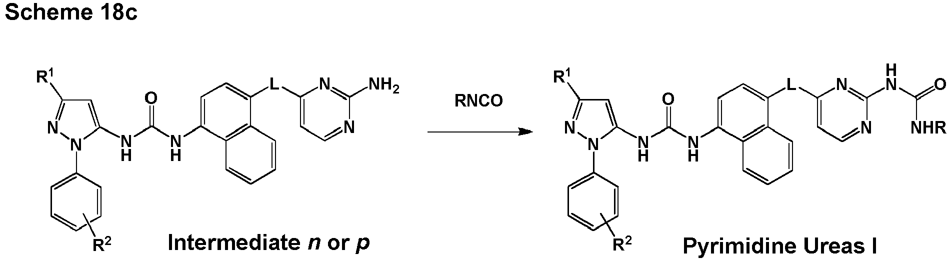

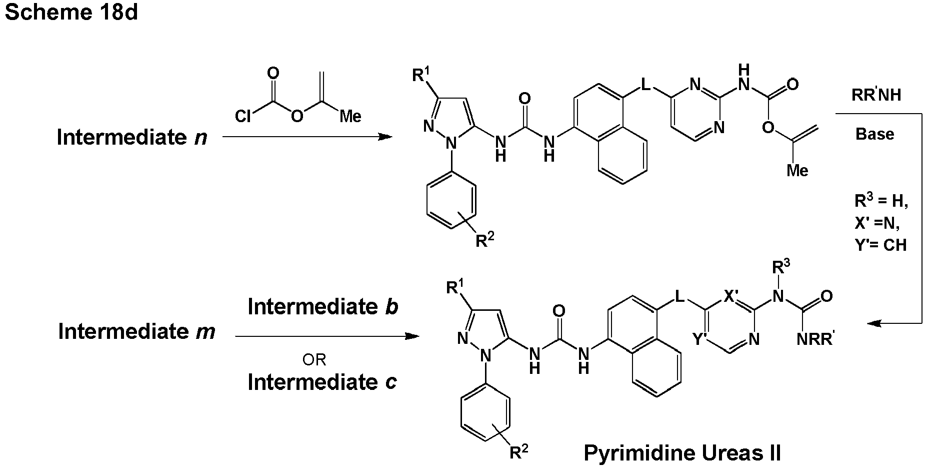

Similar methods may be used to prepare compounds of formula (**I**) wherein L, R¹, R² and R³ are as defined above for compounds of formula (**I**), the fragment XNHR³ represents 2-aminopyrimidinyl or 4-aminopyrimidinyl and wherein NHR or NRR' represent Q and wherein Q together with NHC(O) forms a urea and wherein Ar represents phenyl.

Compounds of formulae (**IIIa**), (**IIIb**), (**IV**), (**IVa**), (**IVb**), (**VI**), (**VIIIa**), (**VIIIb**), (**IX**), (**X**), (**XII**), (**XIIa**), (**XIIb**), (**XIII**), (**XIV**), (**XV**), (**XVI**), (**XVI**) and (**XIX**) and certain other compounds illustrated in the schemes are either commercially available, or are known, or are novel and can be readily prepared by conventional methods. See for example Regan, J. et al., J. Med. Chem., 2003, 46, 4676-4686, WO 00/043384, WO 2007/087448 and WO 2007/089512.

Protecting groups may be required to protect chemically sensitive groups during one or more of the reactions described above, to ensure that the process is efficient. Thus if desired or necessary, intermediate compounds may be protected by the use of conventional protecting groups. Protecting groups and means for their removal are described in "Protective Groups in Organic Synthesis", by Theodora W. Greene and Peter G.M. Wuts, published by John Wiley & Sons Inc; 4th Rev Ed., 2006, ISBN-10: 0471697540.

### Experimental Section

### Abbreviations

- AcOH: glacial acetic acid
- Akt: protein kinases B
- ATCC: American Tissue Culture Collection
- aq: aqueous
- Ac: acetyl
- ATP: adenosine-5'-triphosphate
- BALF: bronchoalveolae lavage fluid
- 9-BBN: 9-borabicyclo[3.3.1]nonane
- Boc: *tert*-butoxycarbonyl
- br: broad
- BSA: bovine serum albumin
- CatCart®: catalytic cartridge
- CCID₅₀: cell culture infectious dose 50%
- CDI: 1,1-carbonyl-diimidazole
- CFC: chlorofluorocarbon
- COPD: chronic obstructive pulmonary disease
- CPE: cytopathic effect
- d: doublet
- DCM: dichloromethane
- DIAD: diisopropylazadicarboxylate
- DIBAL-H: diisobutylaluminium hydride
- DIPEA: *N*,*N*-diisopropylethylamine
- DMEM: Dulbecco's modified Eagle's medium
- d-U937: differentiated U937 cells
- DMF: *N*,*N*-dimethylformamide
- DMSO: dimethyl sulfoxide
- *E. coli*: *Escherichia coli*
- EDAC.HCl: 1-ethyl-3-(3-dimethylaminopropyl)carbodiimide
- ELISA: enzyme-linked immunosorbent assay
- (ES⁺): electrospray ionization, positive mode
- Et: ethyl
- EtOAc: ethyl acetate
- FCS: foetal calf serum
- HFC: hydrofluorocarbon
- HFC-134a: tetrafluoroethane
- HFC-227: heptafluoropropane
- HIV: human immunodeficiency virus
- HOBt: 1-hydroxybenzotriazole
- HPA: Health Protection Agency
- hr: hour(s)
- HRP: horseradish peroxidase
- HRV: human rhinovirus
- IC₅₀: 50% inhibitory concentration
- i.n.: intra-nasal
- i.p.: intra-peritoneal
- JNK: c-Jun N-terminal kinase
- LPS: lipopolysaccharide
- KHMDS: potassium hexamethyldisilazane
- KD: Knock down
- (M+H)⁺: protonated molecule
- MAPK: mitogen protein activated protein kinase
- MDCK: Madin-Darby canine kidney cells
- Me: methyl
- MEK: Map-erk kinase
- MeOH: methanol
- MHz: megahertz
- min: minute(s)
- MMAD: mass mean diameter
- MOI: multiple of infection
- Mtt: maximum toleration test
- MTT: 3-(4,5-dimethylthiazol-2-yl)-2,5-diphenyltetrazolium bromide
- m/z:: mass-to-charge ratio
- NMP: 1-methylpyrrolidin-2-one (*N*-methyl-2-pyrrolidone)
- NMR: nuclear magnetic resonance (spectroscopy)
- OD: optical density
- *OXONE*®: potassium peroxymonosulfate
- PBS: phosphate buffered saline
- Pd₂(dba)₃: tris(dibenzylideneacetone)dipalladium(0)
- PI3 K: phosphoinositide 3-kinase
- Pfu: plaque forming units
- PMA: phorbol myristate acetate
- pMDI: pressurised meter dose inhaler
- PPh₃: triphenylphosphine
- PyBOP®: (benzotriazol-1-yloxy)tripyrrolidinophosphonium hexafluorophosphate
- q: quartet
- R-EC₅₀:: relative EC₅₀ 50% effective concentration value
- RSV:: respiratory syncytial virus
- RT: room temperature
- RP HPLC: reverse phase high performance liquid chromatography
- s: singlet
- SCX: solid supported cation exchange (resin)
- SDS: sodium dodecyl sulfate
- siRNA: small interfering RNA

- t: triplet
- TCID₅₀: 50% tissue culture infection dose
- TFA: trifluoroacetic acid
- THF: tetrahydrofuran
- TMB: 3,3',5,5'-tetramethylbenzidine
- TNFα: tumor necrosis factor alpha
- TPCK: L-1-tosylamido-2-phenylethyl chloromethyl ketone
- WB: washing buffer
- XantPhos: 4,5-bis(diphenylphosphino)-9,9-dimethylxanthene

### COMPOUND EXAMPLES

Labels given to intermediates in the examples are independent of labels given to intermediates in other parts of the description.

### General Procedures

All starting materials and solvents were either obtained from commercial sources or prepared according to the literature citation. Organic solutions were routinely dried over magnesium sulfate. Hydrogenations were preformed on a Thales H-cube flow reactor under the conditions stated. SCX was purchased from Supelco and treated with 1M hydrochloric acid prior to use. Unless stated otherwise the reaction mixture to be purified was first diluted with MeOH and made acidic with a few drops of AcOH. This solution was loaded directly onto the SCX and washed with MeOH. The desired material was then eluted by washing with 1% NH₃ in MeOH. Column chromatography was performed on pre-packed silica (230-400 mesh, 40-63 µM) cartridges using the amount indicated.

*Preparative Reverse Phase High Performance Liquid Chromatography*: Agilent Scalar column C18, 5 µm (21.2 x 50 mm), flow rate 28 mL.min⁻¹ eluting with a H₂O-MeCN gradient containing 0.1% v/v formic acid over 10 mins using UV detection at 215 and 254 nm. Gradient information: 0.0-0.5 min: 95% H₂O-5% MeCN; 0.5 -7.0 min; Ramped from 95% H₂O-5% MeCN to 5% H₂O-95% MeCN; 7.0-7.9 min: Held at 5% H₂O-95% MeCN; 7.9-8.0 min: Returned to 95% H₂O-5% MeCN; 8.0-10.0 min: Held at 95% H₂O-5% MeCN.

### Analytical Methods

*Reverse Phase High Performance Liquid Chromatography* was performed by one of the two methods described below:
Method 1: Agilent Scalar column C18, 5 µm (4.6 x 50 mm) or Waters XBridge C18, 5 µm (4.6 x 50 mm) flow rate 2.5 mL min⁻¹ eluting with a H₂O-MeCN gradient containing either 0.1% v/v formic acid (Method 1 acidic) or NH₃ (Method 1 basic) over 7 min employing UV detection at 215 and 254 nm. Gradient information: 0.0-0.1 min, 95% H₂O-5% MeCN; 0.1-5.0 min, ramped from 95% H₂O-5% MeCN to 5% H₂O-95% MeCN; 5.0-5.5 min, held at 5% H₂O-95% MeCN; 5.5-5.6 min, held at 5% H₂O-95% MeCN, flow rate increased to 3.5 mL min⁻¹; 5.6-6.6 min, held at 5% H₂O-95% MeCN, flow rate 3.5 mL min⁻¹; 6.6-6.75 min, returned to 95% H₂O-5% MeCN, flow rate 3.5 mL min⁻¹; 6.75-6.9 min, held at 95% H₂O-5% MeCN, flow rate 3.5 mL min⁻¹; 6.9-7.0 min, held at 95% H₂O-5% MeCN, flow rate reduced to 2.5 mL min⁻¹.
Method 2: Agilent Extend C18 column, 1.8 µm (4.6 x 30 mm) at 40°C; flow rate 2.5-4.5 mL.min⁻¹ eluting with a H₂O-MeCN gradient containing 0.1% v/v formic acid over 4 min employing UV detection at 254 nm. Gradient information: 0-3.00 min, ramped from 95% H₂O-5% MeCN to 5% H₂O-95% MeCN; 3.00-3.01 min, held at 5% H₂O-95% MeCN, flow rate increased to 4.5 mL min⁻¹; 3.01-3.50 min, held at 5% H₂O-95% MeCN; 3.50-3.60 min, returned to 95% H₂O-5% MeCN, flow rate reduced to 3.50 mL min⁻¹; 3.60-3.90 min, held at 95% H₂O-5% MeCN; 3.90-4.00 min, held at 95% H₂O-5% MeCN, flow rate reduced to 2.5 mL min⁻¹.
*¹H NMR Spectroscopy* was performed on a Bruker Avance III 400 MHz spectrometer, using residual undeuterated solvent as the internal reference standard.

### Intermediate A: 1-(4-((2-Aminopyridin-4-yl)methoxy)naphthalen-1-yl)-3-(3-tert-butyl-1-p-tolyl-1H-pyrazol-5-yl)urea.

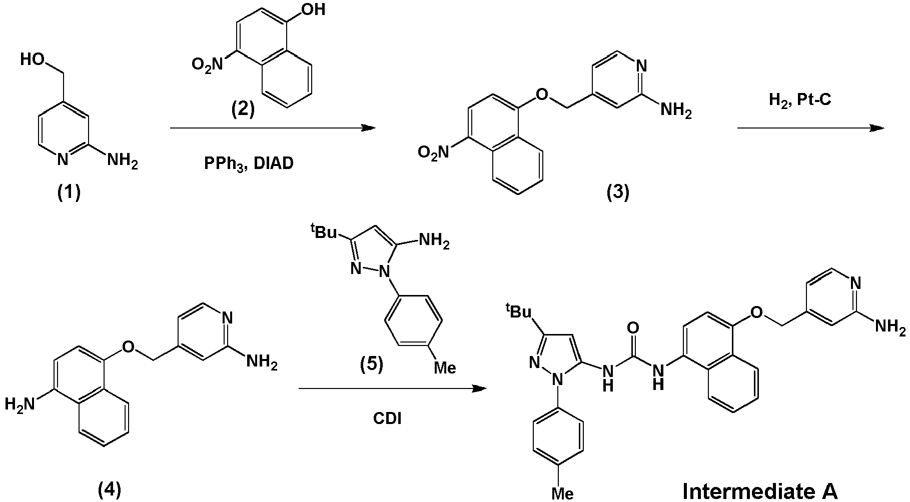

To a solution of 4-nitronaphthalen-1-ol (**2**) (5.17 g, 27.3 mmol), PPh₃ (10.75 g, 41.0 mmol) and 2-aminopyridine-4-methanol (**1**) (5.09g, 41.0 mmol) in THF (50 mL) was added dropwise DIAD (8.07 mL, 41.0 mmol) at -15°C. The mixture was stirred overnight at RT and the volatiles were removed *in vacuo.* The crude product was triturated from EtOAc (150 mL) and was collected by filtration and washed with EtOAc (100 mL). A second trituration from MeOH (100 mL) gave 2-amino-4-((4-nitronaphthalen-1-yloxy)methyl)pyridine (**3**) (4.54 g, 56%) as a yellow solid: m/z 296 (M+H)⁺ (ES⁺).

A solution of (3) (4.50 g, 15.24 mmol) in a mixture of MeOH (200 mL) and AcOH (200 mL) was passed through a Thales H-cube (2.0 mL.min⁻¹, 40°C, 55 mm 10% Pt/C Cat-Cart, full hydrogen mode) and the volatiles were removed *in vacuo.* The crude product was subjected to SCX capture and release eluting with 1% NH₃ in MeOH solution and the solvent was removed *in vacuo* to give 2-amino-4-((4-aminonaphthalen-1-yloxy)methyl)pyridine (**4**) (3.82g, 94%) as a purple solid: m/z 266 (M+H)⁺ (ES⁺).

To a solution of CDI (4.18 g, 25.8 mmol) in DCM (15 mL) was added dropwise under nitrogen a solution of 3-*tert*-butyl-1-*p*-tolyl-1*H*-pyrazol-5-amine (**5**) (WO200/0043384) (5.91g, 25.8 mmol) in DCM (15 mL) over 40 min. The resulting solution was stirred at RT for 1 hr then added dropwise under nitrogen to a solution of 2-amino-4-((4-aminonaphthalen-1-yloxy)methyl)pyridine (**4**) (3.80 g, 12.9 mmol). The mixture was stirred overnight and the volatiles were removed *in vacuo.* The crude material was purified by column chromatography (120 g); eluting with 0 to 6% MeOH in DCM to give the title compound, **Intermediate A** as an off white solid (4.27 g, 63%): m/z 521 (M+H)⁺ (ES⁺).

### Example 1: N-(4-((4-(3-(3-tert-Butyl-1-p-tolyl-1H-pyrazol-5-yl)ureido)naphthalen-1-yloxy)methyl)pyridin-2-yl)-2-methoxyacetamide:

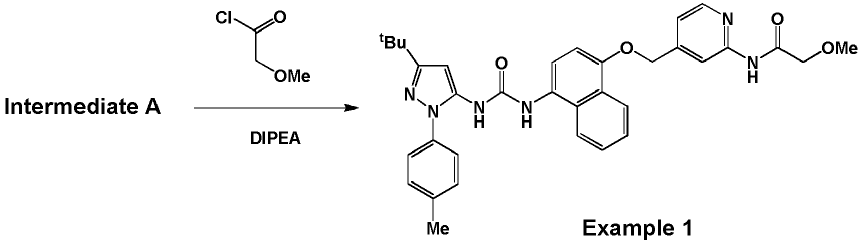

To a mixture of **Intermediate A** (526 mg, 0.96 mmol) and DIPEA (184 µL, 1.06 mmol) in DCM/DMF (10:1, 11 mL) was added methoxyacetyl chloride (92 µL, 1.01 mmol). After stirring for 1 hr at RT, further DIPEA (184 µL, 1.06 mmol) and methoxyacetyl chloride (92 µL, 1.01 mmol) were added sequentially and stirring was continued for 1 hr. After the addition of a solution of 1% NH₃ in MeOH (40 mL), the mixture was stirred for 15 min and evaporated *in vacuo.* The crude product was purified by column chromatography (40 g); eluting with 0 to 6% MeOH in DCM to furnish the title compound, **Example 1**, as a white solid (286 mg, 49%): m/z 593 (M+H)⁺ (ES⁺). ¹H NMR (400 MHz, DMSO-d₆) δ: 1.27 (9 H, s), 2.39 (3 H, s), 3.32 (3 H, s), 4.08 (2H, s), 5.39 (2H, s), 6.36 (1H, s), 7.03 (1H, d), 7.28 (1H, dd), 7.36 (2H, m), 7.44 (2H, m), 7.56-7.64 (3H, m), 7.93 (1H, m), 8.30-8.35 (3H, m), 8.58 (1H, s), 8.79 (1H, s), 10.02 (1H, s).

### Example 2: Methyl 4-((4-(3-(3-tert-butyl-1-p-tolyl-1H-pyrazol-5-yl)ureido)naphthalen-1-yloxy)methyl)pyridin-2-ylurea:

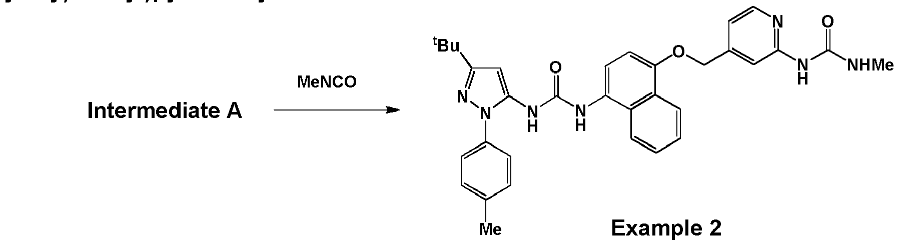

To a solution of **Intermediate A** (70 mg, 0.13 mmol) in anhydrous pyridine (1.5 mL) was added methyl isocyanate (14 µL, 0.24 mmol) and the mixture allowed to stir at RT for 72 hr. Pyridine was removed *in vacuo* and the residue triturated with DCM (3.0 mL). Filtration afforded the title compound, **Example 2**, as an off-white powder, (36 mg, 45 %): m/z 578 (M+H)⁺ (ES⁺). ¹H NMR (400 MHz, DMSO-d₆) δ: 1.27 (9H, s), 2.39 (3H, s), 2.74 (3H, d), 5.30 (2H, s), 6.36 (1H, s), 6.99 (1H, d), 7.05 (d, 1H), 7.35, (2H, d), 7.44 (2H, d), 7.54-7.64 (4H, m), 7.93 (1H, d), 8.19 (1H, d), 8.23 (1H, brs), 8.35 (1H, d), 8.58 (1H, s), 8.79 (1H, s), 9.36 (1H, s).

### Example 3: N-(4-((4-(3-(3-tert-Butyl-1-p-tolyl-1H-pyrazol-5-yl)ureido)naphthalen-1-yloxy)methyl)pyridin-2-yl)tetrahydro-2H-pyran-4-carboxamide:

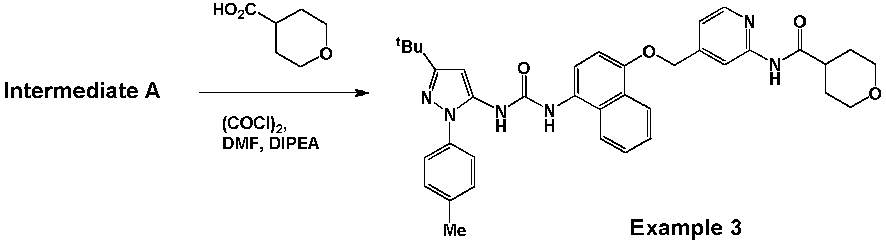

Neat DMF (2 drops) was added to a stirred solution of tetrahydropyran-2*H*-4-carboxylic acid and oxalyl chloride (21 µL, 0.25 mmol) in DCM (1.0 mL) and the resulting solution was stirred at RT for 1 hr. The solution was evaporated *in vacuo* to give a colourless oil, which was redissolved in DCM (1.0 mL) and added dropwise to a stirred mixture of **Intermediate A** (50 mg, 0.10 mmol) and DIPEA (84 µL, 0.50 mmol) in DCM (1.0 mL). Stirring was continued for 18 hr. The reaction mixture was stirred in 1% NH₃ in MeOH (20 mL) for 30 mins, evaporated *in vacuo*, pre-adsorbed on silica, and purified by column chromatography (12 g, 0-5% MeOH in DCM, gradient elution) to give the title compound, **Example 3**, as a light tan solid (18 mg, 28%): m/z 633 (M+H)⁺ (ES⁺). ¹H NMR (400 MHz, DMSO-d₆) δ: 1.26 (9H, s), 1.57-1.72 (4H, m), 2.38 (3H, s), 2.75 (1H, m), 3.28-3.33 (2H, m), 3.88 (2H, m), 5.35 (2H, s), 6.34 (1H, s), 6.99 (1H, d), 7.24 (1H, dd), 7.35 (2H, m), 7.43 (2H, m), 7.55-7.64 (3H, m), 7.92 (1H, m), 8.27-8.33 (3H, m), 8.58 (1H, s), 8.78 (1H, s), 10.50 (1H, s).

### Example 4: (S)-N-(4-((4-(3-(3-tert-Butyl-1-p-tolyl-1H-pyrazol-5-yl)ureido)naphthalen-1-yloxy)methyl)pyridin-2-yl)-2-methoxypropanamide:

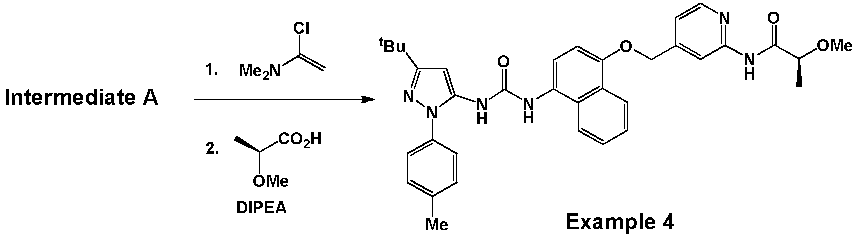

1-Chloro-*N*,*N*-dimethylethenamine (50 µL, 0.48 mmol) was added to a stirring solution of (*S*)-2-methoxypropionic acid (50 mg, 0.48 mmol) in DCM (1.0 mL) and the resulting yellow solution was stirred at RT for 1 hr. The solution was added dropwise to a stirring mixture of **Intermediate A** (50 mg, 0.10 mmol) and DIPEA (167 µL, 0.96 mmol) in DCM (1.0 mL). Stirring was continued overnight. The reaction mixture was stirred in 1% NH₃ in MeOH (20 mL), evaporated *in vacuo*, pre-adsorbed on silica and purified by column chromatography (12 g, 10-50% EtOAc in isohexane, gradient elution) to give the title compound, **Example 4**, as a colourless solid (18 mg, 30%): m/z 607 (M+H)⁺ (ES⁺). ¹H NMR (400 MHz, DMSO-d₆) δ: 1.27 (9H, d), 1.31 (3H, s), 2.38 (3H, s), 3.30 (3H, s), 4.02 (1H, q), 5.39 (2H, s), 6.37 (1H, s), 7.00 (1H, d), 7.29 (1H, dd), 7.35 (2H, m), 7.45 (2H, m), 7.56-7.64 (3H, m), 7.93 (1H, m), 8.30-8.37 (3H, m), 8.58 (1H, s), 8.79 (1H, s), 10.06 (1H, s).

### Example 5: (R)-N-(4-((4-(3-(3-tert-Butyl-1-p-tolyl-1H-pyrazol-5-yl)ureido)naphthalen-1-yloxy)methyl)pyridin-2-yl)-2-methoxypropanamide:

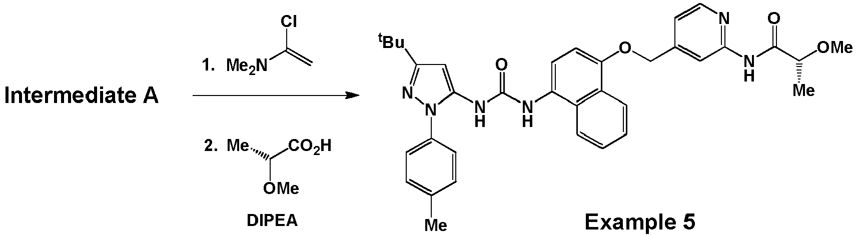

1-Chloro-*N*,*N*-dimethylethenamine (38 µL, 0.36 mmol) was added to a stirred solution of (*R*)-2-methoxypropionic acid (37 mg, 0.36 mmol) in DCM (1.0 mL) and the resulting solution was stirred at RT for 1 hr. The solution was added dropwise to a stirred mixture of **Intermediate A** (75 mg, 0.14 mmol) and DIPEA (75 µL, 0.43 mmol) in DCM (2.0 mL) at 0°C. Stirring was continued for a further 48 hr. The mixture was poured in to 1% NH₃ in MeOH (20 mL) and stirred for 1 hr, and evaporated *in vacuo* to give a yellow residue. Column chromatography (12 g, 20-50% EtOAc in isohexane) gave the title compound, **Example 5**, as a light pink solid (39 mg, 43%): m/z 607 (M+H)⁺ (ES⁺). ¹H NMR (400 MHz, DMSO-d₆) δ: 1.27 (9H, d), 1.30 (3H, s), 2.39 (3H, s), 3.31 (3H, s), 4.02 (1H, q), 5.39 (2H, s), 6.35 (1H, s), 7.02 (1H, d), 7.29 (1H, dd), 7.35 (2H, m), 7.45 (2H, m), 7.56-7.64 (3H, m), 7.93 (1H, m), 8.30-8.37 (3H, m), 8.58 (1H, s), 8.79 (1H, s), 10.09 (1H, s).

### Example 6: N-(4-((4-(3-(3-tert-Butyl-1-p-tolyl-1H-pyrazol-5-yl)ureido)naphthalen-1-yloxy)methyl)pyridin-2-yl)-2-(methylsulfonyl)acetamide:

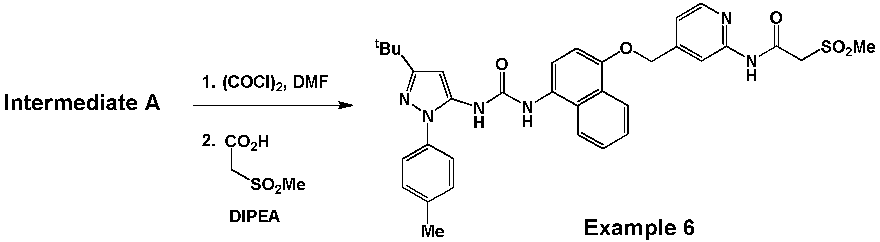

To a stirred suspension of methanesulfonylacetic acid (40 mg, 0.29 mmol) and oxalyl chloride (29 µL, 0.34 mmol) in DCM (1.0 mL) was added DMF (1 drop) and the reaction mixture was stirred at RT for 1 hr. The solution was added dropwise to a stirred mixture of **Intermediate A** (50 mg, 0.10 mmol) and DIPEA (167 µL, 1.0 mmol) in DCM / DMF (10:1 v/v, 1.1 mL) and stirring was continued for 18 hr. The reaction mixture was stirred with 1% NH₃ in MeOH (2.0 mL) and evaporated *in vacuo.* The residue was subjected to capture and release on SCX to afford the title compound, **Example 6**, as a pale yellow solid (11 mg, 18%): m/z 641 (M+H)⁺ (ES⁺). ¹H NMR (400M Hz, DMSO-d₆) δ: 1.27 (9H, s), 2.39 (3H, s), 3.17 (3H, s), 4.44 (2H, s), 5.40 (2H, s), 6.35 (1H, s), 7.02 (1H, d), 7.33 (1H, dd), 7.36 (2H, m), 7.44 (2H, m), 7.56-7.64 (3H, overlapping m), 7.93 (1H, m), 8.30-8.33 (2H, overlapping m), 8.39 (1H, dd), 8.59 (1H, br s), 8.79 (1H, br s), 10.98 (1H, br s).

### Example 7: N-(4-((4-(3-(3-tert-Butyl-1-p-tolyl-1H-pyrazol-5-yl)ureido)naphthalen-1-yloxy)methyl)pyridin-2-yl)-2-hydroxyacetamide:

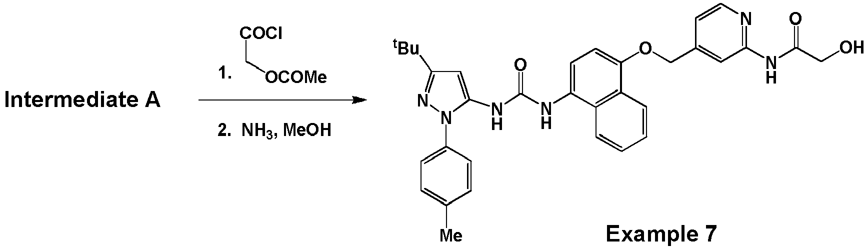

To a solution of **Intermediate A** (75 mg, 0.14 mmol) and DIPEA (125 µL, 0.72 mmol) in DCM / DMF (10:1 v/v, 1.10 mL) was added a solution of acetoxyacetyl chloride (39 µL, 0.36 mmol) in DCM (0.25 mL). The reaction mixture was stirred at RT for 2 hr and then 1% NH₃ in MeOH (3.0 mL) was added and stirring continued for 18 hr. The reaction mixture was evaporated *in vacuo* and the residue was purified by flash column chromatography (SiO₂, 12 g, 30-100% EtOAc in isohexane, gradient elution) to afford the title compound, **Example 7**, as a pale orange solid (24 mg, 28%): m/z 579 (M+H)⁺ (ES⁺). ¹H NMR (400 MHz, DMSO-d₆) δ: 1.27 (9H, s), 2.39 (3H, s), 4.06 (2H, d), 5.39 (2H, s), 5.77 (1H, t), 6.35 (1H, s), 7.02 (1H, d), 7.29 (1H, dd), 7.36 (2H, m), 7.44 (2H, m), 7.56-7.64 (3H, overlapping m), 7.93 (1H, m), 8.32 (1H, m) 8.34 (1H, d), 8.36 (1H, br s), 8.60 (1H, br s), 8.81 (1H, br s), 9.75 (1H, br s).

### Example 8: N-(4-((4-(3-(3-tert-Butyl-1-p-tolyl-1H-pyrazol-5-yl)ureido)naphthalen-1-yloxy)methyl)pyridin-2-yl)-2-methyl-2-(methylamino)propanamide:

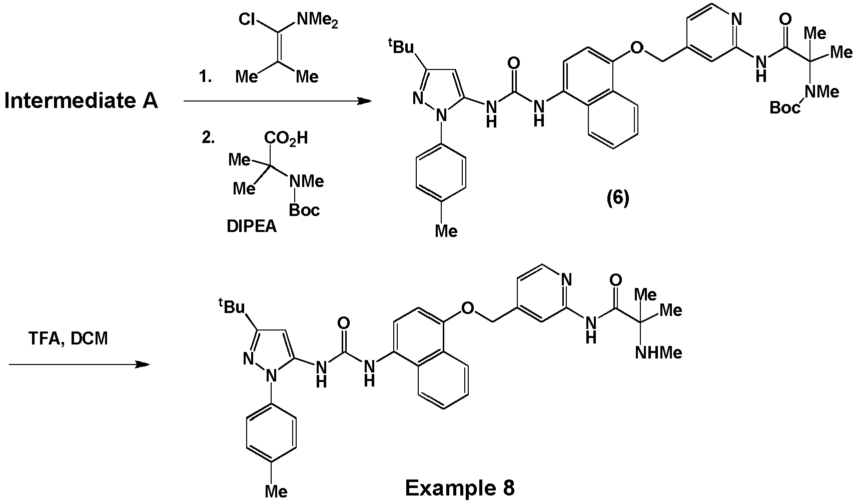

To a stirred suspension of 2-(*tert*-butoxycarbonyl(methyl)amino)-2-methylpropanoic acid (125 mg, 0.58 mmol) in DCM (2.0 mL) was added 1-chloro-*N*,*N*,2-trimethylprop-1-en-1-amine (95 µL, 0.72 mmol) and the mixture was stirred at RT for 2 hr. The reaction mixture was then added to a solution of **Intermediate A** (75 mg, 0.14 mmol) and DIPEA (101 µL, 0.58 mmol) in DCM (1.0 mL) and stirred for 18 hr. A solution of ammonia in MeOH (7M, 1 mL) was added and the mixture was evaporated *in vacuo.* The residue was purified twice by column chromatography (SiO₂, 12 g, 0-100% EtOAc in isohexane, gradient elution) to afford *tert*-butyl 1-(4-((4-(3-(3-*tert-*butyl-1-*p*-tolyl-1*H*-pyrazol-5-yl)ureido)naphthalen-1-yloxy)methyl)pyridin-2-ylamino)-2-methyl-1-oxopropan-2-yl(methyl)carbamate (**6**) as an off-white solid (30 mg, 28%): m/z 620 ((M-Boc)+H)⁺ (ES⁺).

A solution of the carbamate (**6**) (25 mg, 0.04 mmol) in DCM / TFA (1:1 v/v, 2.0 mL) was stirred at RT for 30 min. The reaction mixture was evaporated *in vacuo* and the resulting residue was subjected to SCX capture and release to afford the title compound, **Example 8**, as a pale brown solid (20mg, 89%): m/z 620 (M+H)⁺ (ES⁺). ¹H NMR (400 MHz, DMSO-d₆) δ: 1.25 (6H, s), 1.27 (9H, s), 2.20 (3H, s), 2.39 (3H, s), 5.38 (2H, s), 6.35 (1H, s), 7.00 (1H, d), 7.27 (1H, dd), 7.36 (2H, m), 7.44 (2H, m), 7.58-7.62 (3H, overlapping m), 7.93 (1H, m), 8.29-8.34 (3H, overlapping m), 8.59 (1H, br s), 8.79 (1H, br s).

### Example 9: (S)-N-(4-((4-(3-(3-tert-Butyl-1-p-tolyl-1H-pyrazol-5-yl)ureido)naphthalen-1-yloxy)methyl)pyridin-2-yl)-2-(methylamino)propanamide:

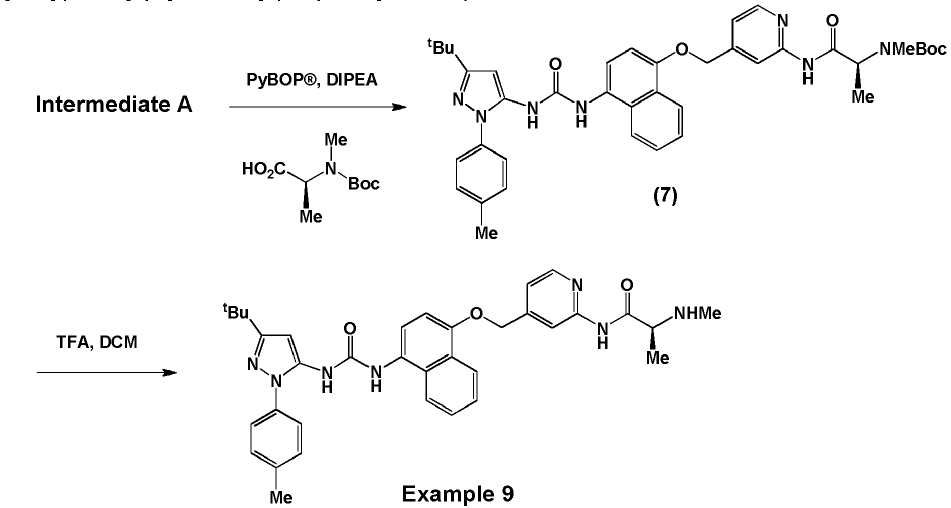

To a stirred suspension of *N*-Boc-methyl-*L*-alanine (117 mg, 0.58 mmol), PyBOP® (300 mg, 0.58 mmol) and DIPEA (101 µL, 0.58 mmol) in DMF (2.0 mL) was added **Intermediate A** (75 mg, 0.14 mmol) in one portion. The reaction mixture was heated to 55°C and stirred for 18 hr and was cooled to RT and partitioned between water (10 mL) and EtOAc (10 mL). The organic layer was separated and evaporated *in vacuo* and the residue was purified by flash column chromatography (SiO₂, 12 g, 0-100% EtOAc in isohexane, gradient elution). The resulting impure product was purified by SCX capture and release to afford (*S*)-*tert*-butyl 1-(4-((4-(3-(3-*tert*-butyl-1-*p*-tolyl-1*H*-pyrazol-5-yl)ureido)naphthalen-1-yloxy)methyl)pyridin-2-ylamino)-1-oxopropan-2-yl(methyl)carbamate (**7**) as a brown solid (25 mg, 23%): m/z 706 (M+H)⁺ (ES⁺).

A solution of the carbamate (**7**) (25 mg, 0.04 mmol) in DCM / TFA (1:1 v/v, 2.0 mL) was stirred at RT for 30 min. The reaction mixture was evaporated *in vacuo* and the resulting residue was subjected to SCX capture and release and then purified by flash column chromatography (SiO₂, 4 g, 0-100% EtOAc in MeOH, gradient elution) to afford the title compound (**Example 9**) as an off-white solid (13 mg, 57%): m/z 606 (M+H)⁺ (ES⁺). ¹H NMR (400 MHz, DMSO-d₆) δ: 1.20 (3H, d), 1.27 (9H, s), 2.26 (3H, s), 2.39 (3H, s), 3.20 (1H, q), 5.38 (2H, s), 6.35 (1H, s), 7.01 (1H, d), 7.28 (1H, dd), 7.36 (2H, m), 7.44 (2H, m), 7.55-7.63 (3H, overlapping m), 7.93 (1H, m), 8.31 (1H, m), 8.34 (1H, dd), 8.36 (1H, br s), 8.60 (1H, s), 8.80 (1H, s).

### Example 10: (R)-N-(4-((4-(3-(3-tert-Butyl-1-p-tolyl-1H-pyrazol-5-yl)ureido)naphthalen-1-yloxy)methyl)pyridin-2-yl)morpholine-3-carboxamide:

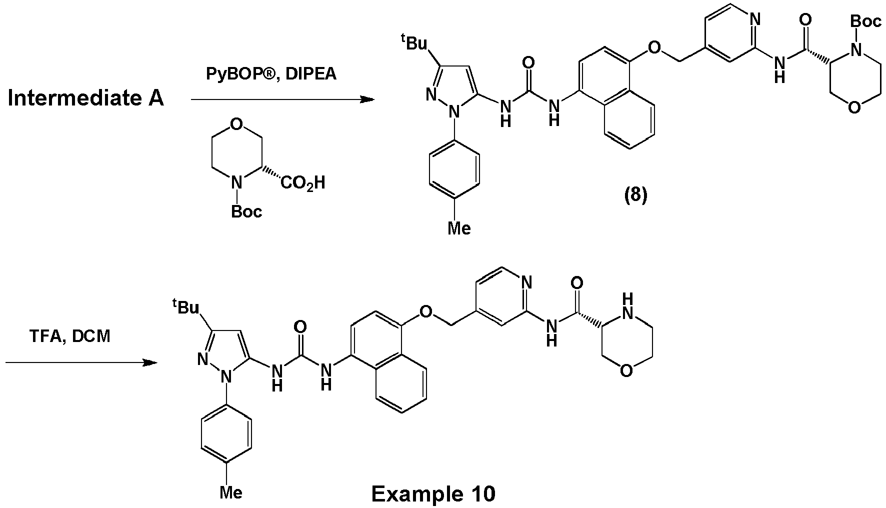

To a stirred suspension of (R)-morpholine-3,4-dicarboxylic acid-4-*tert*-butyl ester (133 mg, 0.58 mmol), PyBOP® (300 mg, 0.58 mmol) and DIPEA (101 µL, 0.58 mmol) in DMF (2.0 mL) was added **Intermediate A** (75 mg, 0.14 mmol) in one portion. The reaction mixture was heated to 55°C and stirred for 18 hr. The reaction mixture was cooled to RT and partitioned between water (10 mL) and EtOAc (10 mL). The organic extract was evaporated *in vacuo* and the residue was purified by flash column chromatography (SiO₂, 12 g, 0-100% EtOAc in isohexane, gradient elution) to afford an impure product which was purified further by SCX capture and release to give (*R*)-*tert*-butyl 3-(4-((4-(3-(3-*tert*-butyl-1-*p*-tolyl-1*H*-pyrazol-5-yl)ureido)naphthalen-1-yloxy)methyl)pyridin-2-ylcarbamoyl)morpholine-4-carboxylate (**8**) as a brown solid (28 mg, 25%): m/z 734 (M+H)⁺ (ES⁺).

A solution of the carbamate (**8**) (28 mg, 0.04 mmol) in DCM / TFA (1:1 v/v, 2.0 mL) was stirred at RT for 30 min. The reaction mixture was evaporated *in vacuo* and the resulting residue was subjected to capture and release on SCX and then triturated from diethyl ether (10 mL) to afford the title compound, **Example 10**, as an off-white solid (13 mg, 53%): m/z 634 (M+H)⁺ (ES⁺). ¹H NMR (400 MHz, DMSO-d₆) δ: 1.26 (9H, s), 2.39 (3H, s), 2.73-2.92 (3H, overlapping m), 3.56-3.64 (4H, overlapping m), 3.82 (1H, m), 5.38 (2H, s), 6.35 (1H, s), 7.00 (1H, d), 7.29 (1H, dd), 7.36 (2H, m), 7.44 (2H, m), 7.58-7.62 (3H, overlapping m), 7.92 (1H, m), 8.28-8.35 (3H, overlapping m), 8.58 (1H, br s), 8.79 (1H, br s), 10.09 (1H, br s) [partial assignment].

### Example 11: (S)-N-(4-((4-(3-(3-tert-Butyl-1-p-tolyl-1H-pyrazol-5-yl)ureido)naphthalen-1-yloxy)methyl)pyridin-2-yl)morpholine-3-carboxamide:

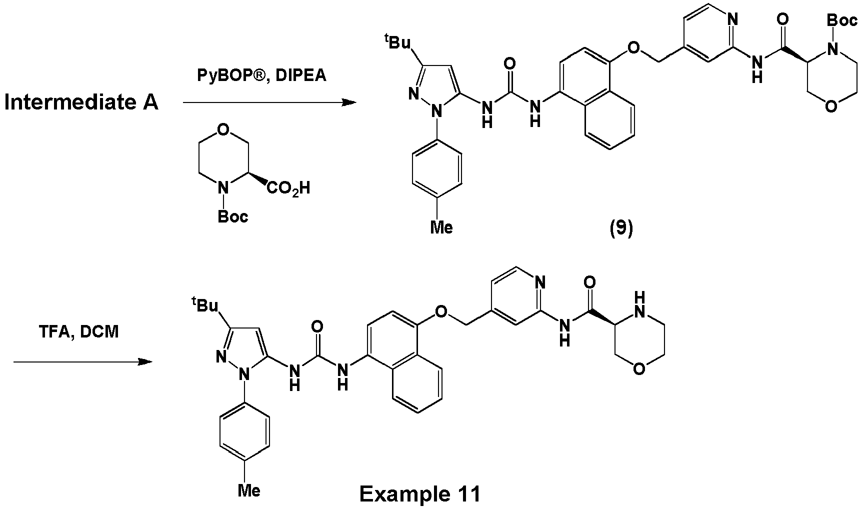

To a stirred suspension of (S)-morpholine-3,4-dicarboxylic acid-4-*tert*-butyl ester (133 mg, 0.58 mmol), PyBOP® (300 mg, 0.58 mmol) and DIPEA (101 µL, 0.58 mmol) in DMF (2.0 mL) was added **Intermediate A** (75 mg, 0.14 mmol) in one portion. The reaction mixture was heated to 55°C in a pre-heated oil bath and stirred for 18 hr. The reaction mixture was cooled to RT and partitioned between water (10 mL) and EtOAc (10 mL). The organic layer was separated, evaporated *in vacuo* and the residue was purified by column chromatography (12 g, 0-100% EtOAc in isohexane, gradient elution). Product fractions were concentrated *in vacuo* and the residue was triturated from DCM (5.0 mL) and isohexane (5.0 mL) to afford (*S*)*-tert*-butyl 3-(4-((4-(3-(3-*tert*-butyl-1-*p*-tolyl-1*H*-pyrazol-5-yl)ureido)naphthalen-1-yloxy)methyl)pyridin-2-ylcarbamoyl) morpholine-4-carboxylate (**9**) as a white solid (50 mg, 43%): m/z 734 (M+H)⁺ (ES⁺).

A solution of the carbamate (**9**) (30 mg, 0.04 mmol) in DCM / TFA (1:1 v/v, 2.0 mL) was stirred at RT for 1 hr and was then evaporated *in vacuo.* the resulting residue was subjected to capture and release on SCX and then triturated from DCM (5.0 mL) and isohexane (5.0 mL) to afford the title compound, **Example 11**, as a brown solid (15 mg, 63%): m/z 634 (M+H)⁺ (ES⁺). ¹H NMR (400 MHz, DMSO-d₆) δ: 1.26 (9H, s), 2.39 (3H, s), 2.73-2.92 (3H, overlapping m), 3.56-3.64 (4H, overlapping m), 3.82 (1H, m), 5.38 (2H, s), 6.34 (1H, s), 7.00 (1H, d), 7.29 (1H, dd), 7.35 (2H, m), 7.42 (2H, m), 7.55-7.62 (3H, overlapping m), 7.91 (1H, m), 8.30 (1H, dd), 8.32 (1H, br s), 8.35 (1H, dd), 8.56 (1H, br s), 8.77 (1H, br s) 10.16 (1H, br s).

### Example 12: N-(4-((4-(3-(3-tert-Butyl-1-p-tolyl-1H-pyrazol-5-yl)ureido)naphthalen-1-yloxy)methyl)pyridin-2-yl)-4-methylpiperazine-1-carboxamide:

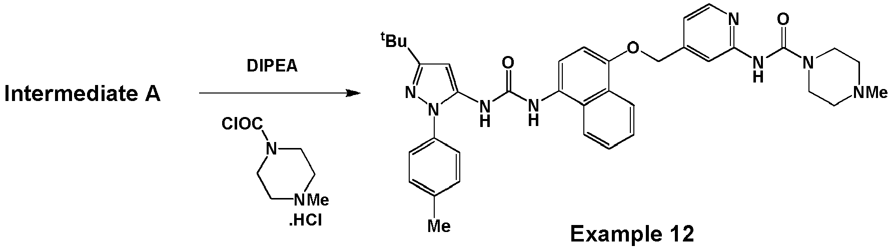

To a solution of **Intermediate A** (50 mg, 0.10 mmol) in pyridine (1.0 mL) was added a suspension of 4-methylpiperazine-1-carbonyl chloride hydrochloride (38 mg, 0.19 mmol) in pyridine (1.50 mL). DIPEA (50 µL, 0.29 mmol) was added and the mixture was stirred at RT for 3 days. The reaction mixture was evaporated *in vacuo* and the residue was partitioned between DCM (10 mL) and water (10 mL). The organic layer was separated and evaporated *in vacuo* and the residue was purified by flash column chromatography (SiO₂, 4 g, 0-10% DCM in MeOH, gradient elution) to afford the title compound, **Example 12**, as a brown solid (17 mg, 27%): m/z 647 (M+H)⁺ (ES⁺). ¹H NMR (400 MHz, DMSO-d₆) δ : 1.27 (9H, s), 2.18 (3H, s), 2.29 (4H, t), 2.39 (3H, s), 3.46 (4H, t), 5.32 (2H, s), 6.35 (1H, s), 6.99 (1H, d), 7.13 (1H, dd), 7.36 (2H, m), 7.44 (2H, m), 7.55-7.63 (3H, overlapping m), 7.92 (1H, m), 7.99 (1H, s), 8.25 (1H, d), 8.29 (1H, m), 8.60 (1H, br s), 8.80 (1H, br s), 9.22 (1H, br s).

### Example 13: N-(4-((4-(3-(3-tert-Butyl-1-p-tolyl-1H-pyrazol-5-yl)ureido)naphthalen-1-yloxy)methyl)pyridin-2-yl)morpholine-4-carboxamide:

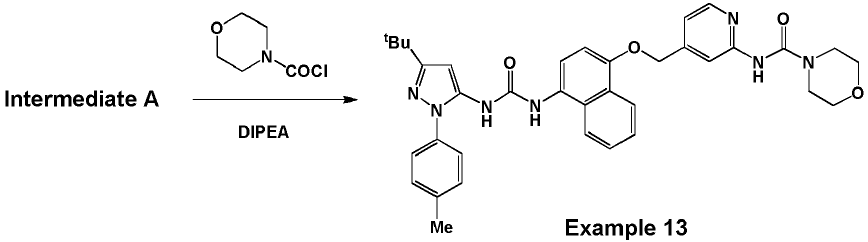

To a solution of **Intermediate A** (70 mg, 0.13 mmol) in pyridine (1.50 mL) was added morpholine-4-carbonyl chloride (28 µL, 0.24 mmol) and the solution was stirred at RT for 18 hr. A further portion of morpholine-4-carbonyl chloride (28 µL, 0.24 mmol) was added to the reaction mixture and stirring continued for 24 hr. The mixture was stirred with 1% NH₃ in MeOH (3.0 mL) and then evaporated *in vacuo.* The residue was purified by flash column chromatography (SiO₂, 12 g, 0-5% MeOH in DCM, gradient elution) and recrystallized from isopropanol (5.0 mL) to afford the title compound, **Example 13**, as a white solid (17 mg, 20%): m/z 634 (M+H)⁺ (ES⁺). ¹H NMR (400 MHz, DMSO-d₆) δ: 1.27 (9H, s), 2.39 (3H, s), 3.45 (4H, t), 3.59 (4H, t), 5.33 (2H, s), 6.35 (1H, s), 6.99 (1H, d), 7.14 (1H, dd), 7.36 (2H, m), 7.44 (2H, m), 7.55-7.63 (3H, overlapping m), 7.92 (1H, m), 8.01 (1H, br s), 8.26 (1H, d), 8.29 (1H, m), 8.58 (1H, br s), 8.79 (1H, br s), 9.27 (1H, br s).

### Example 14: N-(4-((4-(3-(3-tert-Butyl-1-p-tolyl-1H-pyrazol-5-yl)ureido)naphthalen-1-yloxy)methyl)pyridin-2-yl)-3-methoxypropanamide:

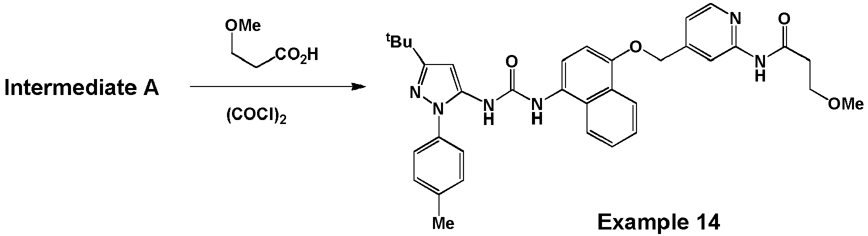

Oxalyl chloride (11.0 µL, 0.115 mmol) and then DMF (1 drop) were added to a suspension of 3-methoxypropionic acid (9.0 µL, 0.096 mmol) in DCM (1 mL) and the mixture was stirred at 0°C for 10 min and then allowed to warm to RT. After 2 hr the solvent was removed *in vacuo* and the residue was taken up in to DCM (1 mL) and added to a solution of **Intermediate A** (50 mg, 0.096 mmol) and DIPEA (33.5 µL, 0.192 mmol) in DCM (1 mL). After 16 hr the mixture was quenched by the addition of saturated aqueous NaHCO₃ solution. The organic layer was separated and a solution of NH₃ in MeOH (7M) was added and the mixture was stirred for 10 min and then evaporated *in vacuo.* The residue was purified by flash chromatography (SiO₂, 4g, 0 to 100% EtOAc in isohexane, gradient elution) to afford the title compound, **Example 14**, as a pale yellow solid (15 mg, 26%). m/z 607 (M+H)⁺ (ES⁺). ¹H NMR (400 MHz, DMSO-d₆) δ: 1.27 (9H, s), 2.39 (3H, s), 2.65 (2H, t), 3.23 (3H, s), 3.61 (2H, t), 5.37 (2H, s), 6.35 (1H, s), 7.00 (1H, d), 7.25 (1H, d), 7.36 (2H, m), 7.44 (2H, m), 7.56-7.63 (3H, overlapping m), 7.83-7.85 (1H, d), 7.93 (1H, d), 8.29-8.34 (3H, overlapping m), 8.62 (1H, br s), 8.82 (1H, br s), 10.54 (1H, br s).

### Example 15: 2-(3-(4-((4-(3-(3-tert-Butyl-1-p-tolyl-1H-pyrazol-5-yl)ureido)naphthalen-1-yloxy)methyl)pyridin-2-yl)ureido)-N-(2-methoxyethyl)acetamide:

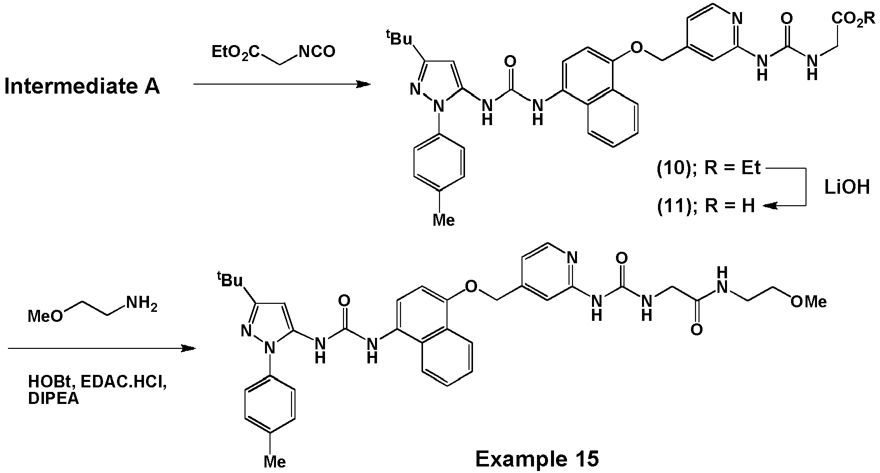

To a solution of **Intermediate A** (200 mg, 0.384 mmol) in pyridine (3 mL) was added ethyl isocyanatoacetate (129 µL, 1.152 mmol) and the reaction was stirred at RT for 16 hr. The mixture was evaporated *in vacuo* and was then co-evaporated with toluene and the residue was triturated with methanol to afford ethyl 2-(3-(4-((4-(3-(3-*tert*-butyl-1-p-tolyl-1H-pyrazol-5-yl)ureido)naphthalen-1-yloxy)methyl)pyridin-2-yl)ureido)acetate (**10**) as a solid (191 mg, 76%): m/z 650 (M+H)⁺, (ES⁺).

To a suspension of the ester (**10**) (200 mg, 0.308 mmol) in a mixture of THF / H₂O (4:1 v/v, 5 mL) was added lithium hydroxide (10.0 mg, 0.418 mmol) and the mixture was stirred at RT for 1 hr. The reaction mixture was acidified to pH3, by the addition of 1M hydrochloric acid and evaporated *in vacuo* to half of its original volume. The resulting preciptate was collected by filtration and was washed with water and dried *in vacuo* to provide 2-(3-(4-((4-(3-(3-)-butyl-1-p-tolyl-1*H*-pyrazol-5-yl)ureido)naphthalen-1-yloxy)methyl)pyridin-2-yl)ureido)acetic acid (**11**) (185mg, 97%): m/z 622 (M+H)⁺, (ES⁺).

To a suspension of the acid (**11**) (60 mg, 0.097 mmol) in DCM (1.5 mL) was added 2-methoxyethylamine (25.0 µL, 0.290 mmol), HOBt (19.56 mg, 0.145 mmol), DIPEA (50.5 µL, 0.290 mmol), and EDC. HCl (27.8 mg, 0.145 mmol) and the mixture was stirred at RT for 1 hr. The reaction mixture became very viscous and was diluted with DMF (1 mL) and stirred for 16 hr. The reaction mixture was diluted with DCM and was washed with water. The organic phase was dried (MgSO₄) and the solvent was evaporated *in vacuo.* The residue was triturated with MeOH to afford the title compound, **Example 15**, as an off white solid (22 mg, 34%): m/z 679 (M+H)⁺, (ES⁺). ¹H NMR (400 MHz, DMSO-d₆) δ: 1.27 (9H, s), 2.39 (3H, s), 3.24 (3H, s), 3.25 (2H, m), 3.34 (2H, m), 3.82 (2H, d), 5.31 (2H, s), 6.35 (1H, s), 7.00 (1H, d), 7.07 (1H, d), 7.36 (2H, m), 7.44 (2H, m), 7.58-7.65 (4H, overlapping m), 7.93 (1H, m), 8.03 (1H, t), 8.21 (1H, d), 8.34 (1H, m), 8.51 (1H, very br s), 8.58 (1H, br s), 8.79 (1H, br s), 9.46 (1H, br s)

### Example 16: N-(4-((4-(3-(3-tert-Butyl-1-p-tolyl-1H-pyrazol-5-yl)ureido)naphthalen-1-yloxy)methyl)pyridin-2-yl)-4-(dimethylamino)butanamide:

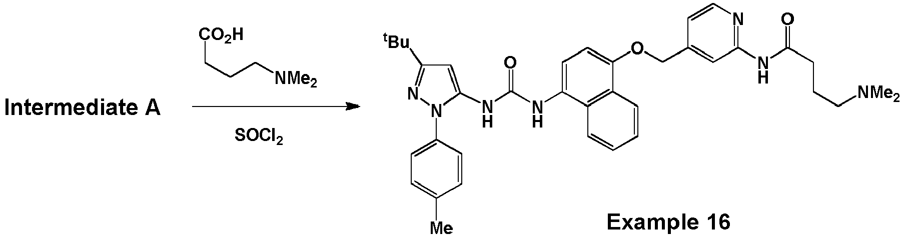

To a stirred suspension of 4-(dimethylamino)butyric acid. HCl (97 mg, 0.576 mmol) in DCM (2 mL) at 0°C was added thionyl chloride (42.0 µL, 0.576 mmol) and DMF (one drop) and the mixture was allowed to warm to RT. After 16 hr the reaction mixture was evaporated *in vacuo* and the residue was dissolved in DCM / THF (1:1 v/v, 2 mL) and added to a solution of **Intermediate A** (60 mg, 0.115 mmol) in THF (1 mL) containing DIPEA (101 µL, 0.576 mmol). The mixture was stirred at 55°C and after 4 hr the mixture was cooled to RT, diluted with water (10 mL) and was extracted with EtOAc (10 mL). The organic layer was separated and was treated with a solution of ammonia (7M in MeOH, 2 mL) for 5 min and the mixture evaporated *in vacuo.* The residue was purified by flash column chromatography (SiO₂, 12g, (10% NH₃ in MeOH) - EtOAc gradient elution) to afford the title compound, **Example 16**, as a pale brown solid (25 mg, 34%): m/z 634 (M+H)⁺ (ES⁺); ¹H NMR (400 MHz, DMSO-d₆) δ: 1.27 (9 H, s), 1.74 (2H, m), 2.21 (6H, s), 2.35 (2H, m), 2.39 (3 H, s), 2.40 (2H, m), 5.36 (2H, s), 6.35 (1H, s), 7.01 (1H, d), 7.24 (1H, d), 7.36 (2H, m), 7.44 (2H, m), 7.54-7.63 (3H, overlapping m), 7.93 (1H, m), 8.30-8.37 (3H, overlapping m), 8.59 (1H, s), 8.79 (1H, s), 10.54 (1H, br s).

### Example 17: N-(4-((4-(3-(3-tert-Butyl-1-p-tolyl-1H-pyrazol-5-yl)ureido)naphthalen-1-yloxy)methyl)pyridin-2-yl)-3-(methylsulfonyl)propanamide:

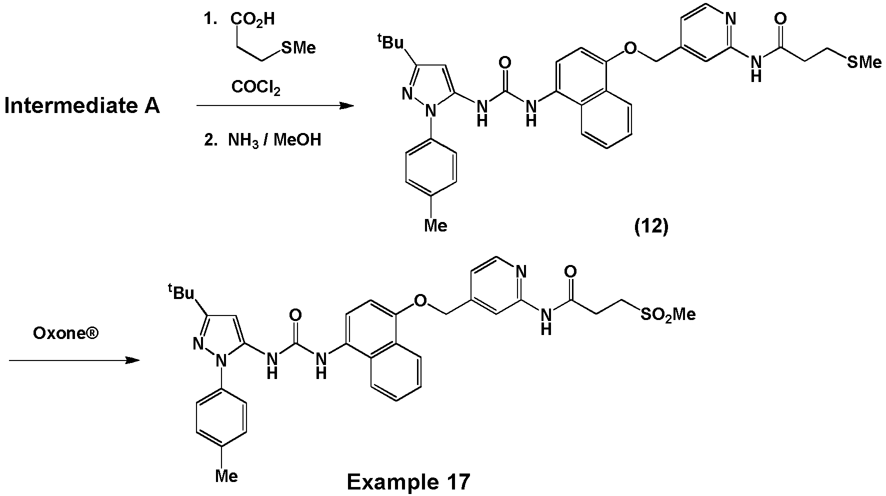

To a suspension of 3-methanethiopropionic acid (117 mg, 0.97 mmol) in DCM (1.0 mL) was added oxalyl chloride (85.0 µL, 0.97 mmol), followed by DMF (2 drops) and the mixture was stirred at RT for 1 hr. The solvent was removed by evaporation *in vacuo* and the residue was redissolved in DCM (2.5 mL) and was then added dropwise to a solution of **Intermediate A** (145 mg, 0.28 mmol) and DIPEA (218 µL, 1.25 mmol) in DCM (2.0 mL). After 2 hr a solution of ammonia in MeOH (7M, 3.0 mL) was added and stirring was continued for 1 hr. The volatiles were evaporated *in vacuo* and the residue was purified by flash column chromatography (SiO₂, 12 g, 0-5% MeOH in DCM, gradient elution) to afford *N*-(4-((4-(3-(3-*tert*-butyl-1-p-tolyl-1*H-*pyrazol-5-yl)ureido)naphthalen-1-yloxy)methyl)pyridin-2-yl)-3-(methylthio)propanamide (**12**) (110 mg, 61%): m/z 623 (M+H)⁺ (ES⁺).

To a stirred solution of the sulfide (**12**) (47 mg, 0.075 mmol) in DMF (0.5 mL) was added a solution of Oxone (93 mg, 0.151 mmol) in water (1.0 mL). After 10 min a precipitate had formed and MeOH (2 mL) was added and stirring continued for 1 hr. The mixture was diluted with a further aliquot of MeOH (2.0 mL) and glacial AcOH (0.5 mL) was added. The suspension was subjected to SCX capture and release and then purified by flash column chromatography (SiO₂, 12 g, 2-8% MeOH in DCM, gradient elution) to afford the title compound, **Example 17**, (20 mg, 38%): m/z 655 (M+H)⁺ (ES⁺). ¹H NMR (400 MHz, DMSO-d₆) δ: 1.27 (9H, s), 2.39 (3H, s), 2.90 (2H, t), 3.01 (3H, s), 3.43 (2H, t), 5.38 (2H, s), 6.35 (1H, s), 7.00 (1H, d), 7.27 (1H, dd), 7.36 (2H, m), 7.44 (2H, m), 7.56-7.65 (3H, overlapping m), 7.93 (1H, m), 8.29-8.33 (2H, overlapping m), 8.34 (1H, d), 8.58 (1H, br s), 8.79 (1H, br s), 10.72 (1H, br s).

### Example 18: N-(4-((4-(3-(3-tert-Butyl-1-p-tolyl-1H-pyrazol-5-yl)ureido)naphthalen-1-yloxy)methyl)pyridin-2-yl)-3-(methylsulfonyl)-2-oxoimidazolidine-1-carboxamide:

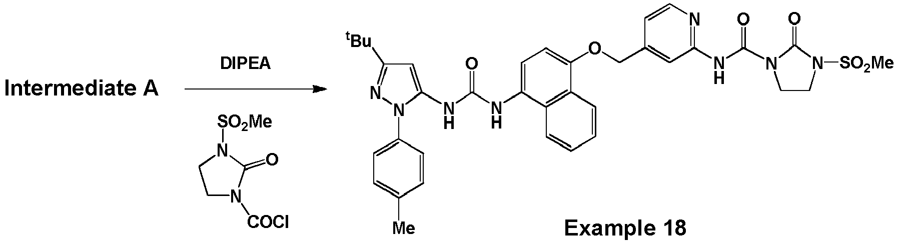

To a solution of **Intermediate A** (100 mg, 0.192 mmol) in pyridine (1.5 mL) was added 3-chlorocarbonyl-1-methanesulfonyl-2-imidazolidinone (131 mg, 0.576 mmol) in pyridine (1.5 mL) and the reaction mixture was stirred at RT. After 3 days the reaction mixture was evaporated *in vacuo* and the residue was stirred with NH₃ (1% v/v in MeOH). The solvent was evaporated *in vacuo* and the residue was purified by flash column chromatography (SiO₂, 4 g, 100% DCM then 2% MeOH in DCM, isocratic elutions) to afford an impure product which was recrystallized from methanol to give the title compound, **Example 18**, as a white crystalline solid (12 mg, 9%): m/z 711 (M+H)⁺ (ES⁺). ¹H NMR (400 MHz, DMSO-d₆) δ: 1.27 (9H, s), 2.39 (3H, s), 3.39 (3H, s), 3.87 (4H, s), 5.40 (2H, s), 6.35 (1H, s), 7.01 (1H, d), 7.31 (1H, dd), 7.36 (2H, m), 7.44 (2H, m), 7.55-7.63 (3H, m), 7.93 (1H, m), 8.21 (1H, br s), 8.31 (1H, m), 8.35 (1H, d), 8.58 (1H, br s), 8.79 (1H, br s), 10.40 (1H, br s).

### Intermediate B: N-(4-((4-(3-(3-tert-Butyl-1-p-tolyl-1H-pyrazol-5-yl)ureido)naphthalen-1-yloxy)methyl)pyridin-2-yl)-2-chloroacetamide.

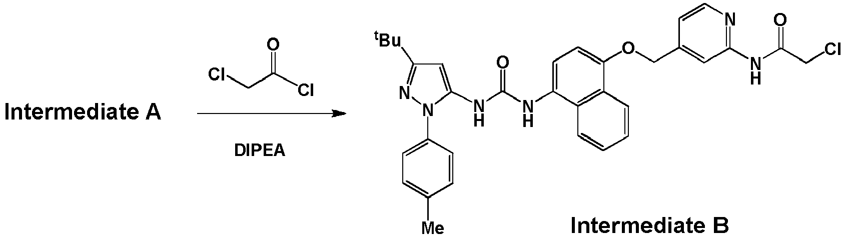

To a solution of DIPEA (1.37 mL, 7.68 mmol) and **Intermediate A** (2.00 g, 3.84 mmol) in DCM (40 mL) and DMF (8.0 mL) was added chloroacetyl chloride (0.61 mL, 7.68 mmol). The reaction mixture was stirred at RT for 1 hr and a further portion of chloroacetyl chloride (100µl, 1.25mmol) was added. After 1 hr at RT, the reaction mixture was partitioned between DCM (40 mL) and saturated aq NaHCO₃ solution (40 mL). The organic phase was concentrated *in vacuo* and purified by column chromatography (80 g, 0-10% MeOH in DCM, gradient elution). Product fractions were concentrated in vacuo and the residue triturated with diethyl ether (20 mL) and isohexane (20 mL). The solid was collected by filtration to afford the title compound, **Intermediate B**, as a pale purple solid (1.07g, 42%): m/z 597, 599 (M+H)⁺ (ES⁺).

### Example 19: N-(4-((4-(3-(3-tert-Butyl-1-p-tolyl-1H-pyrazol-5-yl)ureido)naphthalen-1-yloxy)methyl)pyridin-2-yl)-2-(methylthio)acetamide;

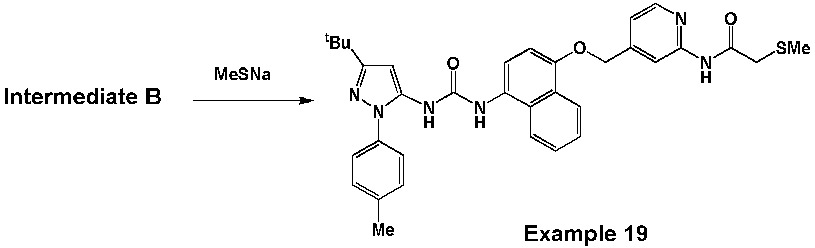

**Intermediate B** (100 mg, 0.17 mmol) was added portionwise to a stirred solution of sodium thiomethoxide (35 mg, 0.50 mmol) in MeOH (5.0 mL) and the resulting mixture was stirred at RT for 1 hr. The mixture was evaporated *in vacuo* and partitioned between brine (20 mL) and DCM (30 mL). The organic layer was concentrated *in vacuo,* the residue pre-adsorbed on silica and purified by column chromatography (SIO₂, 12 g, 10-100% EtOAc in isohexane, gradient elution). Product fractions were evaporated *in vacuo* to give the title compound **Example 19** as a light yellow solid (28 mg, 26%): m/z 610 (M+H)⁺ (ES⁺). ¹H NMR (400 MHz, DMSO-d₆) δ: 1.27 (9H, s), 2.16 (3H, s), 2.39 (3H, s), 3.53 (2H, s), 5.37 (2H, s), 6.35 (1H, s), 7.01 (1H, d), 7.26 (1H, dd), 7.35 (2H, m), 7.44 (2H, m), 7.55-7.64 (3H, m), 7.92 (1H, m), 8.30-8.35 (3H, m), 8.58 (1H, s), 8.78 (1H, s), 10.60 (1H, s).

### Example 20: N-(4-((4-(3-(3-tert-Butyl-1-p-tolyl-1H-pyrazol-5-yl)ureido)naphthalen-1-yloxy)methyl)pyridin-2-yl)-2-(methylsulfinyl)acetamide:

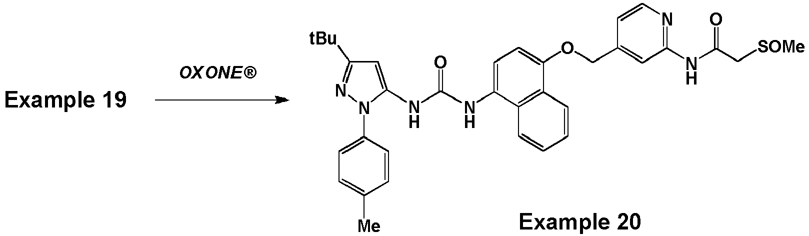

To a solution of compound **Example 19** (20 mg, 0.03 mmol) in a mixture of DMF / H₂O (1:1 v/v, 1.0mL) was added *OXONE*® (10 mg, 0.03 mmol) and the reaction was stirred at RT for 3 days. A second portion of *OXONE*® (10 mg, 0.03 mmol) was added, the mixture was stirred for a further 24 hr and was then partitioned between brine (20 mL) and DCM (20 mL). The organic extract was washed with brine (20 mL), dried (MgSO₄) and evaporated *in vacuo* and the residue was purified by flash column chromatography (SiO₂, 4 g, 30-100% EtOAc in isohexane, gradient elution) and subjected to SCX capture and release to afford the title compound, **Example 20**, as a tan coloured solid (11 mg, 52%): m/z 625 (M+H)⁺ (ES⁺). ¹H NMR (400 MHz, DMSO-d₆) δ: 1.27 (9H, s), 2.39 (3H, s), 2.69 (3H, s), 3.88 (2H, d), 4.04 (2H, d) 5.39 (2H, s), 6.35 (1H, s), 7.02 (1H, d), 7.31 (1H, dd), 7.36 (2H, m), 7.44 (2H, m), 7.58-7.64 (3H, overlapping m), 7.93 (1H, m), 8.30-8.33 (2H, overlapping m), 8.37 (1H, d), 8.59 (1H, br s), 8.79 (1H, br s), 10.85 (1H, br s).

### Example 21: N-(4-((4-(3-(3-tert-Butyl-1-p-tolyl-1H-pyrazol-5-yl)ureido)naphthalen-1-yloxy)methyl)pyridin-2-yl)-2-morpholinoacetamide:

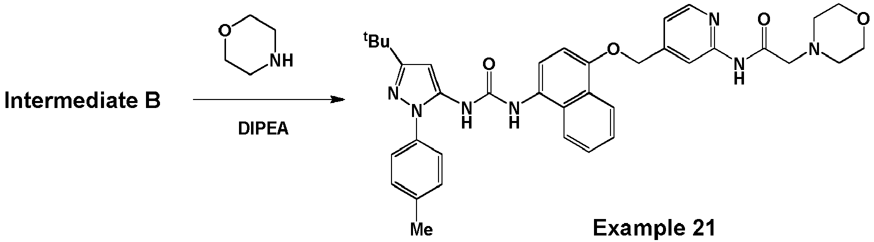

To a stirred solution of **Intermediate B** (50 mg, 0.08 mmol) in a mixture of DCM (1.0 mL), DMF (0.1 mL) and DIPEA (21.9 µL, 0.13 mmol) was added morpholine (11.0 µL, 0.13 mmol) and the reaction mixture stirred at RT for 3 hr and then at 40°C for 12 hr. A further portion of morpholine (11.0 µL, 0.13 mmol) was added and the reaction mixture stirred at 40°C for 5 hr. The crude reaction mixture was purified by column chromatography (12 g, 0-10% MeOH in DCM, gradient elution). Product fractions were concentrated *in vacuo* and the residue was triturated with MeOH (5.0 mL). The solid was collected by filtration to afford the title compound **Example 21** as a light yellow solid (11 mg, 20%): m/z 648 (M+H)⁺ (ES⁺). ¹H NMR (400 MHz, DMSO-d₆) δ: 1.27 (9 H, s), 2.39 (3 H, s), 2.54 (4 H, m), 3.20 (2 H, s), 3.63 (4 H, m), 5.39 (2H, s), 6.35 (1H, s), 7.01 (1H, d), 7.28 (1H, d), 7.35 (2H, d), 7.43 (2H, d), 7.63 - 7.56 (3H, m), 7.92 (1H, d), 8.37 - 8.29 (3H, m), 8.58 (1H, s), 8.79 (1H, s), 10.01 (1H, s).

### Example 22: N-(4-((4-(3-(3-tert-Butyl-1-p-tolyl-1H-pyrazol-5-yl)ureido)naphthalen-1-yloxy)methyl)pyridin-2-yl)-2-(pyrrolidin-1-yl)acetamide:

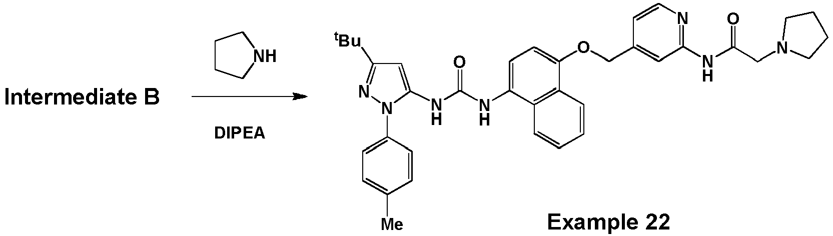

To a solution of **Intermediate B** (50 mg, 0.08 mmol) in DCM (1.0 mL), DMF (0.1 mL) and DIPEA (22 µL, 0.13 mmol) was added pyrrolidine (7.0 µL, 0.08 mmol). The reaction mixture was stirred at RT for 3 hr and then at 40°C for 12 hr. A further portion of pyrrolidine (7.0 µL, 0.08 mmol) was added and the reaction mixture stirred at 40°C for 5 hr. The crude reaction mixture was purified by column chromatography (12 g, 0-10% MeOH in DCM, gradient elution) to afford the title compound, **Example 22,** as a light orange solid (17 mg, 32%): m/z 632 (M+H)⁺ (ES⁺). ¹H NMR (400 MHz, DMSO-d₆) δ: 1.27 (9 H, s), 1.76 (4 H, m), 2.39 (3 H, s), 2.62 (4 H, m), 5.39 (2H, s), 6.35 (1H, s), 7.01 (1H, d), 7.28 (1H, d), 7.34 (2H, d), 7.44 (2H, d), 7.65-7.55 (3H, m), 7.92 (1H, d), 8.36-8.29 (3H, m), 8.58 (1H, s), 8.79 (1H, s), 9.93 (1H, s).

### Example 23; N-(4-((4-(3-(3-tert-Butyl-1-p-tolyl-1H-pyrazol-5-yl)ureido)naphthalen-1-yloxy)methyl)pyridin-2-yl)-2-(4-methylpiperazin-1-yl)acetamide:

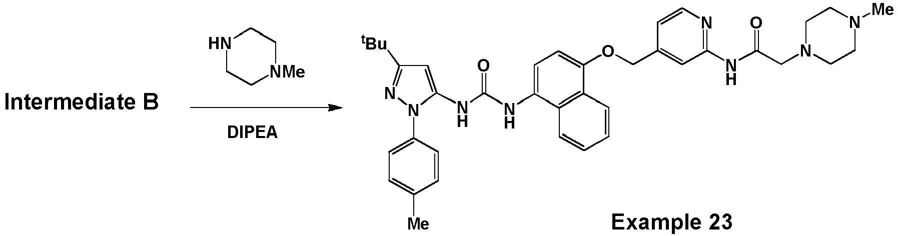

To a solution of **Intermediate B** (50 mg, 0.08 mmol) in a mixture of DCM (1.0 mL), DMF (0.1 mL) and DIPEA (22 µL, 0.13 mmol) was added *N*-methyl piperazine (9.3 µL, 0.08 mmol). The reaction mixture was stirred at RT for 3 hr and then at 40°C for 12 hr. A further portion of N-methyl piperazine (9.0 µL, 0.08 mmol) was added and the reaction mixture stirred at 40°C for 5 hr. The crude reaction mixture was purified by column chromatography (12 g, 0-10% MeOH in DCM, gradient elution). Product fractions were concentrated *in vacuo* and the residue triturated with a mixture of diethyl ether, DCM and isohexane (2:1:2, 5.0 mL) to give the title compound, **Example 23,** as a light orange solid (26 mg, 47%): m/z 661 (M+H)⁺ (ES⁺). ¹H NMR (400 MHz, DMSO-d₆) δ: 1.27 (9 H, s), 2.39 (3 H, s), 2.69-2.60 (3 H, br m), 2.88-2.73 (3 H, br m), 3.17-2.95 (4 H, br m), 5.39 (2H, s), 6.34 (1H, s), 7.00 (1H, d), 7.29 (1H, d), 7.35 (2H, d), 7.45 (2H, d), 7.66-7.56 (3H, m), 7.98 (1H, d), 8.37-8.28 (3H, m), 8.73 (1H, s), 8.91 (1H, s), 10.12 (1H, s).

### Example 24: N-(4-((4-(3-(3-tert-Butyl-1-p-tolyl-1H-pyrazol-5-yl)ureido)naphthalen-1-yloxy)methyl)pyridin-2-yl)-2-(4-(2-methoxyethyl)piperazin-1-yl)acetamide:

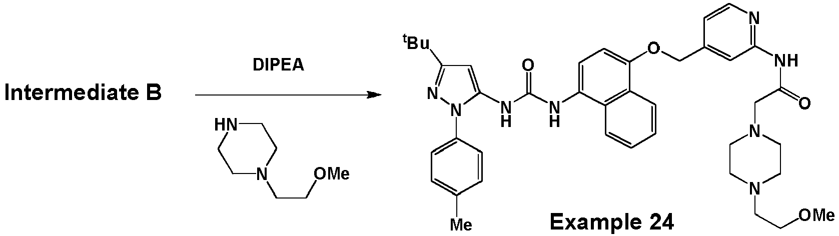

To a solution of **Intermediate B** (50 mg, 0.08 mmol) in a mixture of DCM (1.0 mL), DMF (0.1 mL) and DIPEA (22 µL, 0.13 mmol) was added *N*-methoxyethyl piperazine (12.5 µL, 0.08 mmol). The reaction mixture was stirred at RT for 3 hr and then at 40°C for 12 hr. A further portion of *N*-methoxyethyl piperazine (12.5 µL, 0.08 mmol) was added and the reaction mixture stirred at 40°C for 5 hr. The crude reaction mixture was purified by column chromatography (SiO₂, 12 g, 0-10% MeOH in DCM, gradient elution) to afford the title compound, **Example 24,** as a light orange solid (45 mg, 73%): m/z 705 (M+H)⁺ (ES⁺). ¹H NMR (400 MHz, DMSO) δ: 1.27 (9 H, s), 2.39 (3 H, s), 2.46-2.48 (3 H, m, obscured by DMSO), 2.57-2.50 (4 H, m), 3.17 (2 H, s), 3.23 (3 H, s), 3.42 (2 H, t), 5.39 (2H, s), 6.35 (1H, s), 7.01 (1H, d), 7.29 (1H, d), 7.35 (2H, d), 7.43 (2H, d), 7.65 - 7.55 (3H, m), 7.93 (1H, d), 8.36 - 8.30 (3H, m), 8.58 (1H, s), 8.79 (1H, s), 9.92 (1H, s).

### Example 25: N-(4-((4-(3-(3-tert-Butyl-1-p-tolyl-1H-pyrazol-5-yl)ureido)naphthalen-1-yloxy)methyl)pyridin-2-yl)-2-(2-methoxyethylamino)acetamide:

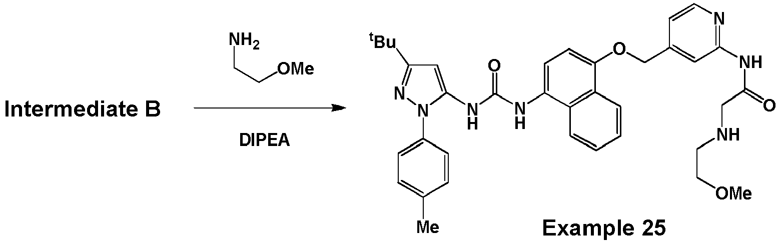

To a solution of **Intermediate B** (50 mg, 0.08 mmol) in a mixture of DCM (1.0 mL), DMF (0.1 mL) and DIPEA (17 µL, 0.10 mmol) was added 2-methoxyethylamine (7.0 µL, 0.08 mmol). The reaction mixture was heated to 40°C and stirred for 12 hr. The crude reaction mixture was purified by column chromatography (SiO₂,.12 g, 0-10% MeOH in DCM, gradient elution). Product fractions were concentrated *in vacuo* and the residue triturated with a mixture of diethyl ether, DCM and iso-hexane (2:1:2, 5.0 mL) to afford the title compound, **Example 25,** as an off-white solid (6 mg, 11%): m/z 637 (M+H)⁺ (ES⁺). ¹H NMR (400 MHz, DMSO-d₆) δ: 1.27 (9 H, s), 2.39 (3 H, s), 2.71 (2 H, t), 3.24 (3 H, s), 3.33 (2 H, m (obscured by DHO)), 3.40 (2 H, t), 5.38 (2H, s), 6.35 (1H, s), 7.01 (1H, d), 7.27 (1H, d), 7.36 (2H, d), 7.43 (2H, d), 7.64 - 7.57 (3H, m), 7.92 (1H, m), 8.36 - 8.30 (3H, m), 8.59 (1H, s), 8.79 (1H, s).

### Example 26: N-(4-((4-(3-(3-tert-Butyl-1-p-tolyl-1H-pyrazol-5-yl)ureido)naphthalen-1-yloxy)methyl)pyridin-2-yl)-2-(dimethylamino)acetamide:

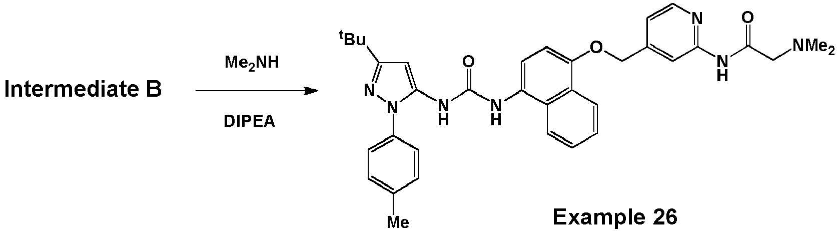

To a solution of **Intermediate B** (50 mg, 0.08 mmol) in DCM (1.0 mL), DMF (0.1 mL) and DIPEA (17 µL, 0.1 mmol) was added dimethylamine (2.0M solution in THF) (41 µL, 0.08 mmol). The reaction mixture was heated to 40°C and stirred for 12 hr. The crude reaction mixture was purified by column chromatography (12 g silica, 0-10% MeOH in DCM, gradient elution). Product fractions were concentrated *in vacuo* and the residue triturated with a mixture of diethyl ether, DCM and isohexane (2:1:2, 5.0 mL) to afford the title compound, **Example 26,** as an orange solid (18 mg, 35%): m/z 607 (M+H)⁺ (ES⁺). ¹H NMR (400 MHz, DMSO-d₆) δ: 1.27 (9 H, s), 2.31 (6 H, s), 2.39 (3 H, s), 3.14 (2 H, s), 5.39 (2H, s), 6.35 (1H, s), 7.01 (1H, d), 7.29 (1H, d), 7.35 (2H, d), 7.44 (2H, d), 7.65-7.55 (3H, m), 7.94 (1H, m), 8.38-8.28 (3H, m), 8.59 (1H, s), 8.79 (1H, s), 9.93 (1H, s).

### Example 27; N-(4-((4-(3-(3-tert-Butyl-1-p-tolyl-1H-pyrazol-5-yl)ureido)naphthalen-1-yloxy)methyl)pyridin-2-yl)-2-(methylamino)acetamide:

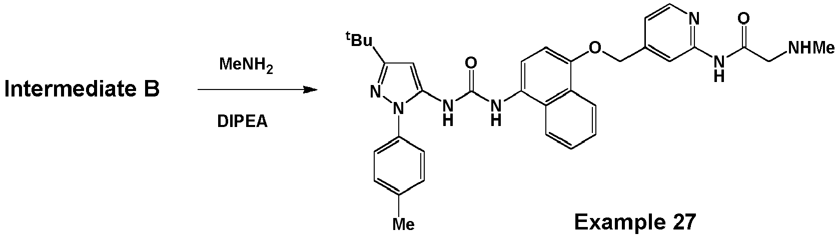

To a solution of **Intermediate B** (50 mg, 0.08 mmol) in a mixture of DCM (1.0 mL), DMF (0.2 mL) and DIPEA (17 µL, 0.10 mmol) was added methylamine (2.0M solution in THF) (41 µL, 0.08 mmol). The reaction mixture was heated to 40°C and stirred for 12 hr. The crude reaction mixture was purified by column chromatography (12 g, 0-10% MeOH in DCM, gradient elution). Product fractions were contaminated with an impurity and the crude material was re-purified by column chromatography (SiO₂,12 g, 0-10% MeOH in DCM, gradient elution) to give the title compound, **Example 27,** as a light brown solid (6 mg, 12%): m/z 593 (M+H)⁺ (ES⁺). ¹H NMR (400 MHz, DMSO-d₆) δ: 1.27 (9 H, s), 2.32 (3 H, s), 2.39 (3 H, s), 3.28 (2 H, s), 5.39 (2H, s), 6.35 (1H, s), 7.01 (1H, d), 7.27 (1H, d), 7.35 (2H, d), 7.44 (2H, d), 7.63-7.55 (3H, m), 7.93 (1H, m), 8.37 - 8.30 (3H, m), 8.59 (1H, s), 8.80 (1H, s).

### Example 28: N-(4-((4-(3-(3-tert-Butyl-1-p-tolyl-1H-pyrazol-5-yl)ureido)naphthalen-1-yloxy)methyl)pyridin-2-yl)-2-((4-methoxybenzyl)(methyl)amino)acetamide:

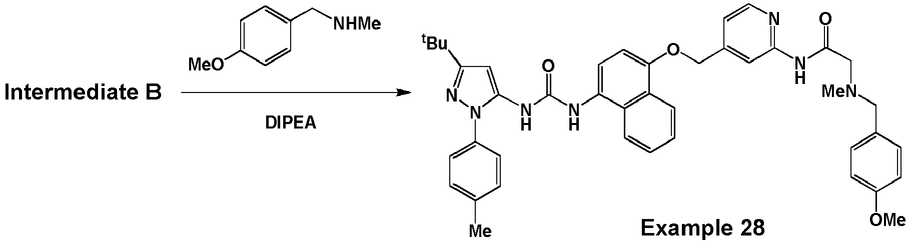

To a solution of **Intermediate B** (50 mg, 0.08 mmol) in a mixture of DCM (1.0 mL), DMF (0.2 mL) and DIPEA (17.5 µL, 0.10 mmol) was added *N*-(4-methoxybenzyl)-*N*-methylamine (15.5 µL, 0.09 mmol) and the reaction mixture was stirred at 55°C for 12 hr. The crude reaction mixture was purified by column chromatography (SiO₂, 12 g, 0-10% MeOH in DCM, gradient elution). Product fractions were concentrated *in vacuo* and the residue triturated with a mixture of diethyl ether, DCM and isohexane (2:1:2, 5.0 mL) to afford the title compound, **Example 28,** as a white solid (7 mg, 11%): m/z 713 (M+H)⁺ (ES⁺). ¹H NMR (400 MHz, DMSO-d₆) δ: 1.27 (9 H, s), 2.25 (3 H, s), 2.39 (3 H, s), 3.22 (2 H, s), 3.59 (2 H, s), 3.72 (3 H, s), 5.38 (2H, s), 6.35 (1H, s), 6.90 (2H, m), 7.01 (1H, m), 7.27 (3H, m), 7.35 (2H, m), 7.43 (2H, m), 7.64-7.55 (3H, m), 7.94 (1H, m), 8.37-8.28 (3H, m), 8.58 (1H, s), 8.79 (1H, s), 9.97 (1H, s).

### Example 29: N-(4-((4-(3-(3-tert-Butyl-1-p-tolyl-1H-pyrazol-5-yl)ureido)naphthalen-1-yloxy)methyl)pyridin-2-yl)-2-(2-methoxyethylthio)acetamide:

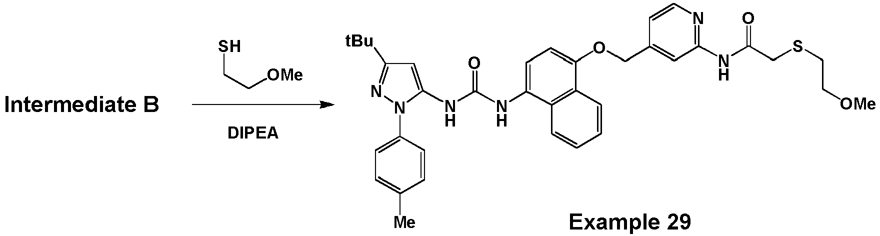

To a solution of **Intermediate B** (50 mg, 0.08 mmol) in a mixture of DCM (1.0 mL), DMF (0.1 mL) and DIPEA (17 µL, 0.10 mmol) was added 2-methoxyethane-1-thiol (8.0 µL, 0.10 mmol). The reaction mixture was heated to 40 °C and stirred for 18 hr, after which further portions of 2-methoxyethane-1-thiol (8.0 µL, 0.10 mmol) and DIPEA (17 µL, 0.10 mmol) were added and stirring continued at 40 °C for 2 days. The reaction mixture was evaporated *in vacuo* and the residue was purified by flash column chromatography (SiO₂, 12 g, 0-10% MeOH in DCM, gradient elution) to afford an impure product which was triturated with diethyl ether (3.0 mL) and further purified by SCX capture and release to give the title compound, **Example 29,** (25 mg, 44%): m/z 653 (M+H)⁺ (ES⁺). ¹H NMR (400 MHz, DMSO-d₆) δ: 1.27 (9H, s), 2.39 (3H, s), 2.80 (2H, t), 3.22 (3H, s), 3.41 (2H, s), 3.51 (2H, t), 5.38 (2H, s), 6.35 (1H, s), 7.01 (1H, d), 7.27 (1H, dd), 7.36 (2H, m), 7.44 (2H, m), 7.56-7.64 (3H, overlapping m), 7.93 (1H, m), 8.29-8.33 (2H, overlapping m), 8.35 (1H, m), 8.58 (1H, br s), 8.79 (1H, br s), 10.61 (1H,br s).

### Example 30: N-(4-((4-(3-(3-tert-Butyl-1-p-tolyl-1H-pyrazol-5-yl)ureido)naphthalen-1-yloxy)methyl)pyridin-2-yl)-2-(2-(2-methoxyethoxy)ethylthio)acetamide:

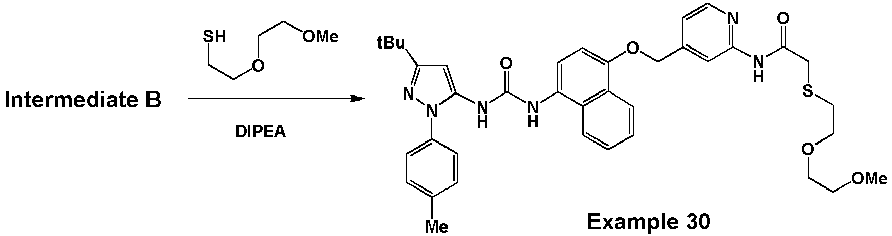

To a solution of **Intermediate B** (50 mg, 0.08 mmol) in a mixture of DCM (1.0 mL), DMF (0.1 mL) and DIPEA (17 µL, 0.10 mmol) was added 2-(2-methoxyethoxy)ethanethiol (14 µL, 0.10 mmol). The reaction mixture was heated to 40 °C and stirred for 18 hr, after which further portions of 2-(2-methoxyethoxy)ethanethiol (14 µL, 0.10 mmol) and DIPEA (17 µL, 0.10 mmol) were added and stirring continued at 40 °C for 2 days. The reaction mixture was evaporated *in vacuo* and the residue was purified by flash column chromatography (SiO₂, 12 g, 0-10% MeOH in DCM, gradient elution) to afford an impure product which was triturated with diethyl ether (3.0 mL) and further purified by SCX capture and release to give the title compound, **Example 30,** (19 mg, 32%): m/z 697 (M+H)⁺ (ES⁺). ¹H NMR (400 MHz, DMSO-d₆) δ: 1.27 (9H, s), 2.39 (3H, s), 2.79 (2H, t), 3.21 (3H, s), 3.39-3.42 (4H, overlapping m), 3.49 (1H, d), 3.50 (1H, dd), 3.59 (2H, t), 5.38 (2H, s), 6.35 (1H, s), 7.01 (1H, d), 7.27 (1H, dd), 7.36 (2H, m), 7.44 (2H, m), 7.56-7.64 (3H, overlapping m), 7.93 (1H, m), 8.29-8.32 (2H, overlapping m), 8.35 (1H, dd), 8.58 (1H, br s), 8.79 (1H, br s), 10.61 (1H, br s).

### Example 31: N-(4-((4-(3-(3-tert-Butyl-1-p-tolyl-1H-pyrazol-5-yl)ureido)naphthalen-1-yloxy)methyl)pyridin-2-yl)-2-(2-morpholinoethylthio)acetamide:

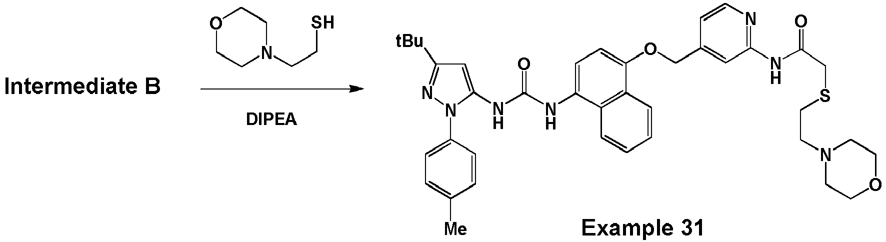

To a solution of **Intermediate B** (50 mg, 0.08 mmol) in a mixture of DCM (1.0 mL), DMF (0.1 mL) and DIPEA (17 µL, 0.10 mmol) was added 2-morpholin-4-yl-ethane-1-thiol (12 µL, 0.10 mmol). The reaction mixture was heated to 40°C for 18 hr after which further portions of 2-morpholin-4-yl-ethane-1-thiol (12 µL, 0.10 mmol) and DIPEA (17 µL, 0.10 mmol) were added and stirring continued at 40 °C for 2 days. The reaction mixture was evaporated *in vacuo* and purified by flash column chromatography (SiO₂, 12 g, 0-10% MeOH in DCM, gradient elution) to afford impure product which was triturated with a mixture of diethyl ether (3.0 mL), DCM (3.0 mL) and isohexane (5.0 mL) and further purified by SCX capture and release to provide the title compound, **Example 31,** (14 mg, 21%): m/z 708 (M+H)⁺ (ES⁺). ¹H NMR (400 MHz, DMSO-d₆) δ: 1.27 (9H, s), 2.39 (3H, s), 2.43 (4H, very br s), 2.59 (2H, very br s), 2.78 (2H, br t), 3.42 (2H, s), 3.55 (4H, br s), 5.38 (2H, s), 6.35 (1H, s), 7.01 (1H, d), 7.27 (1H, dd), 7.36 (2H, m), 7.44 (2H, m), 7.55-7.64 (3H, overlapping m), 7.95 (1H, m), 8.29-8.32 (2H, overlapping m), 8.35 (1H, dd), 8.63 (1H, s), 8.83 (1H, s), 10.63 (1H,s).

### Example 32: N-(4-((4-(3-(3-tert-Butyl-1-p-tolyl-1H-pyrazol-5-yl)ureido)naphthalen-1-yloxy)methyl)pyridin-2-yl)-2-(2-morpholinoethylsulfonyl)acetamide:

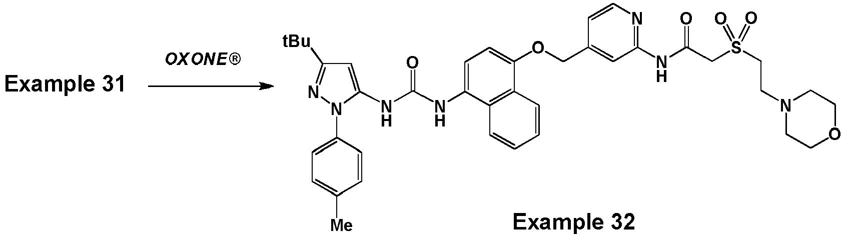

A solution of *OXONE*® (39 mg, 0.13 mmol) in water (0.4 mL) was added to a solution of compound, **Example 31,** (30 mg, 0.04 mmol) in DMF (2.0 mL) and the mixture was stirred at RT for 18 hr. The reaction mixture was diluted with glacial AcOH (1.0 mL) and brine (4.0 mL) and extracted with DCM (4.0 mL). The organic phase was evaporated *in vacuo* and the residue was subjected to SCX capture and release and was then purified by flash column chromatography (SiO₂, 12 g, 0-10% (1% NH₃ in MeOH) in DCM, gradient elution). The impure product so obtained was triturated with DCM (0.5 mL) and isohexane (3.0 mL) to afford the title compound, **Example 32,** as a white solid (7 mg, 21%): m/z 740 (M+H)⁺ (ES⁺). ¹H NMR (400 MHz, DMSO-d₆) δ: 1.27 (9H, s), 2.39 (3H, s), 2.43 (4H, br m), 2.77 (2H, t), 3.51 (2H, t), 3.56 (4H, br t), 4.53 (2H, s), 5.40 (2H, s), 6.35 (1H, s), 7.01 (1H, d), 7.32 (1H, m), 7.35 (2H, m), 7.44 (2H, m), 7.54-7.63 (3H, overlapping m), 7.93 (1H, m), 8.30-8.32 (2H, overlapping m), 8.38 (1H, d), 8.64 (1H, br s), 8.84 (1H, brs), 10.98 (1H, br s).

### Example 33: 2-(Bis(2-methoxyethyl)amino)-N-(4-((4-(3-(3-tert-butyl-1-p-tolyl-1H-pyrazol-5-yl)ureido)naphthalen-1-yloxy)methyl)pyridin-2-yl)acetamide:

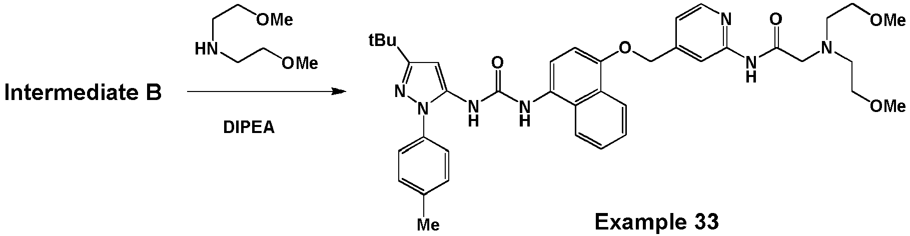

To a solution of **Intermediate B** (50 mg, 0.08 mmol) in a mixture of DCM (1.0 mL), DMF (0.1 mL) and DIPEA (17 µL, 0.10 mmol) was added bis(2-methoxyethyl)amine (15 µL, 0.10 mmol). The reaction mixture was stirred at 40°C, for 18 hr and then further portions of bis(2-methoxyethyl)amine (15 µL, 0.10 mmol) and DIPEA (17 µL, 0.10 mmol) were added and stirring continued at 40°C for 4 days. The reaction mixture was evaporated *in vacuo* and the residue was purified three times by flash column chromatography (SiO₂, 2 x 12 g, 0-20% MeOH in DCM and SiO₂, 4 g, 0-10% [1% NH₃ in MeOH] in DCM, gradient elution) to afford the title compound, **Example 33,** (13 mg, 22%): m/z 694 (M+H)⁺ (ES⁺). ¹H NMR (400 MHz, DMSO-d₆) δ: 1.27 (9H, s), 2.39 (3H, s), 2.79-2.81 (4H, t), 3.21 (6H, s), 3.34 (2H, s), 3.42 (4H, t), 5.38 (2H, s), 6.35 (1H, s), 7.01 (1H, d), 7.27 (1H, dd), 7.36 (2H, m), 7.44 (2H, m), 7.56-7.63 (3H, overlapping m), 7.93 (1H, m), 8.30-8.35 (2H, overlapping m), 8.38 (1H, br s), 8.58 (1H, br s), 8.79 (1H, br s), 10.14 (1H, br s).

### Intermediate C: 1-(4-((3-Aminopyridin-4-yl)methoxy)naphthalen-1-yl)-3-(3-tert-butyl-1-p-tolyl-1H-pyrazol-5-yl)urea.

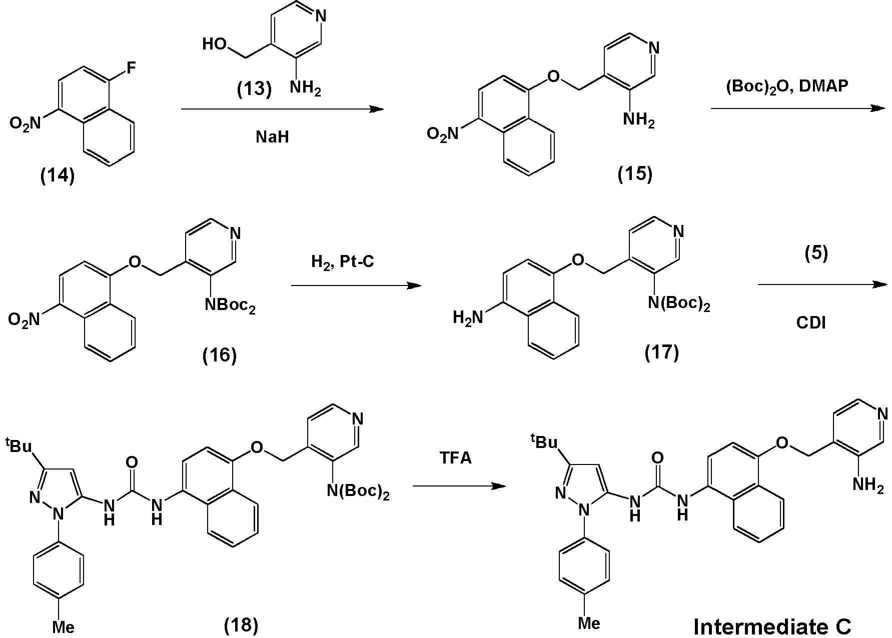

To a solution of (3-amino-pyridin-4-yl)-methanol (**13**) (4.00 g, 32.2 mmol) in anhydrous THF (160 mL) at 0°C was added sodium hydride (1.55 g, 38.7 mmol, 60 wt%). After stirring for 20 min, 1-fluoro-4-nitronaphthalene (**14**) (6.16 g, 32.2 mmol) was added, the ice bath was removed and the reaction mixture left to warm to RT and stirred for 12 hr. The reaction mixture was partitioned between EtOAc (200 mL) and saturated aq NaHCO₃ solution (150 mL). The remaining yellow solid was collected by filtration and washed sequentially with water (50 mL), MeOH (50 mL) and diethyl ether (100 mL) and was identified as the desired product by LC-MS and ¹H NMR. The filtrate was returned to a separating funnel; the organic phase was collected and washed with brine (100 mL), dried and concentrated in vacuo to afford an orange residue. Trituration of the orange residue with MeOH (200 mL) afforded an orange solid which was washed with diethyl ether (200 mL). LC-MS and ¹H NMR analysis of the orange solid was identical to that observed for the insoluble solid obtained earlier. The two products were combined to afford 4-((4-nitronaphthalen-1-yloxy)methyl)pyridin-3-amine (**15**) (7.80 g, 77%): m/z 296 (M+H)⁺ (ES⁺).

To a suspension of (**15**) (3.00 g, 10.2 mmol) and DMAP (0.25 g, 2.03 mmol) in THF (30 mL) was added a solution of di-*tert*-butyldicarbonate (2.33 g, 10.7 mmol) in THF (15 mL). After 2-3 min a solution was obtained. The reaction mixture was stirred at RT for 12 hr whereupon further di-*tert*-butyldicarbonate (2.33 g, 10.7 mmol) was added and the reaction mixture was stirred at RT for 12 hr. The reaction was partitioned between EtOAc (100 mL) and saturated aq NaHCO₃ solution (50 mL). The organic layer was collected, dried and concentrated *in vacuo* to afford an orange oil. The oil was purified by column chromatography (0-50% EtOAc in isohexane, gradient elution) to afford di-*tert*-butyl 4-((4-nitronaphthalen-1-yloxy) methyl) pyridin-3-yliminodicarbonate (**16**) as an orange oil which subsequently crystallised on standing (2.33 g, 43%): m/z 496 (M+H)⁺ (ES⁺).

A solution of (**16**) (2.30 g, 4.64 mmol) in MeOH (100 mL) and AcOH (20 mL) was passed through a Thales H-cube (1.0 mL.min⁻¹, 25°C, 55 mm 10% Pt/C Cat-Cart, full hydrogen mode) and the volatiles were removed *in vacuo* to afford di-*tert*-butyl 4-((4-aminonaphthalen -1-yloxy)methyl)pyridin-3-yliminodicarbonate (**17**) as a brown oil (2.12 g, 82%): m/z 466 (M+H)⁺ (ES⁺).

A solution of (**5**) (1.55 g, 6.77 mmol) in DCM (4.0 mL) was added dropwise over 25 min to a suspension of CDI (1.10 g, 6.77 mmol) in DCM (4.0 mL) at RT. The reaction mixture was stirred for 80 min at RT and a solution of di-*tert*-butyl 4-((4-aminonaphthalen-1-yloxy)methyl)pyridin-3-yliminodicarbonate (**17**) (2.10 g, 4.51 mmol) in DCM (10 mL) was added to the reaction mixture in one portion and stirred for 12 hr. The reaction mixture was partitioned between saturated aq NaHCO₃ solution (20 mL) and DCM (20 mL). The organic layer was collected, dried and concentrated *in vacuo* to afford a purple residue. The crude material was purified by column chromatography (80 g, 0-100% EtOAc in isohexane, gradient elution,) to afford di-*tert*-butyl 4-((4-(3-(3-*tert*-butyl-1-*p*-tolyl-1*H*-pyrazol-5-yl) ureido)) naphthalen-1-yloxy)methyl)pyridin-3-yliminodicarbonate (**18**) as a purple solid (1.77 g, 53%): m/z 721 (M+H)⁺ (ES⁺).

To a solution of the iminodicarbonate (**18**) (1.70 g, 2.36 mmol) in DCM (10 mL) was added TFA (2.0 mL). After 1 hr at RT further TFA (2.0 mL) was added and the reaction mixture stirred for 12 hr at RT. The solvents were removed *in vacuo* and the product purified by SCX capture and release, followed by trituration with DCM (20 mL) to afford the title compound, **Intermediate C,** as a pale buff solid (0.96 g, 77%): m/z 521 (M+H)⁺ (ES⁺). ¹H NMR (400 MHz, DMSO-d₆) δ: 1.27 (9 H, s), 2.39 (3 H, s), 5.16 (2 H, s), 5.38 (2 H, s), 6.35 (1H, s), 7.05 (1H, d), 7.32 (1H, d), 7.35 (2H, d), 7.43 (2H, m), 7.64-7.51 (2H, m), 7.63 (1H, d), 7.82 (1H, d), 7.91 (1H, m), 8.03 (1H, s), 8.29 (1H, m), 8.57 (1H, s), 8.78 (1H, s).

### Example 34: 1-(4-((3-Methylureidopyridin-4-yl)methoxy)naphthalen-1-yl)-3-(3-tert-butyl-1-p-tolyl-1H-pyrazol-5-yl)urea:

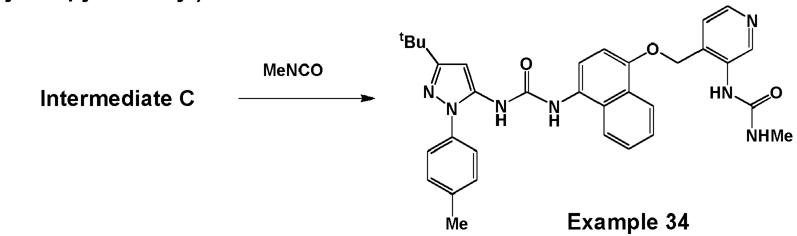

Methyl isocyanate (8.5 µL, 0.14 mmol) was added to a solution of **Intermediate C** (50 mg, 0.10 mmol) in pyridine (1.0 mL). The reaction mixture was stirred for 2 hr at RT and a further portion of methyl isocyanate (8.5 µL, 0.14 mmol) was added and stirring continued for 72 hr at RT. The solvent was removed *in vacuo* and the crude product was purified by column chromatography (SiO₂,4 g, 10-25% MeOH in DCM, gradient elution). The crude product fractions were combined and triturated with DCM (20 mL). The solid was filtered off to afford the title compound (**Example 34**) (8 mg, 14%): m/z 578 (M+H)⁺ (ES⁺). ¹H NMR (400 MHz, DMSO-d₆) δ: 1.27 (9 H, s), 2.39 (3 H, s), 2.68 (3 H, d), 5.27 (2H, s), 6.35 (1H, s), 6.53 (1H, m), 6.98 (1H, d), 7.35 (2H, d), 7.45 (2H, d), 7.65 - 7.52 (4H, m), 7.92 (1H, d), 8.16 (1H, s), 8.28 (2H, m), 8.61 (1H, s), 8.82 (1H, s), 8.88 (1H, s).

### Example 35: N-(4-((4-(3-(3-tert-Butyl-1-p-tolyl-1H-pyrazol-5-yl)ureido)naphthalen-1-yloxy) methyl) pyridin-3-yl)-2-methoxyacetamide:

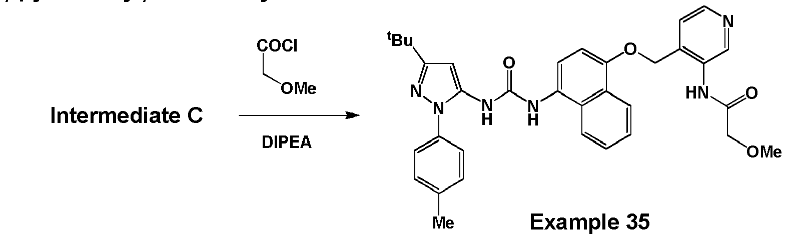

To a solution of **Intermediate C** (50 mg, 0.10 mmol) and DIPEA (33.5 µL, 0.19 mmol) in anhydrous DCM (1.0 mL) and anhydrous DMF (0.1 mL) was added methoxyacetyl chloride (10 µL, 0.11 mmol) and the reaction mixture stirred for 12 hr at RT Additional methoxyacetyl chloride (10 µL, 0.11 mmol) was added and the reaction mixture was stirred at RT for 5 hr. A further portion of methoxyacetyl chloride (8 µL, 0.09 mmol) was added and after 2 hr a solution of ammonia (1% in MeOH, 10 mL) was added and the reaction mixture was stirred for 20 min at RT. The solvents were removed *in vacuo* to afford a purple oily solid. This was dissolved in MeOH (2.0 mL) and 3 drops of AcOH were added. The solution was subjected to SCX capture and release, eluting the product with 1% NH₃ in MeOH. The solvent was removed *in vacuo* and the residue was triturated with diethyl ether (10 mL) to afford the title compound, **Example 35,** as a light purple solid (24 mg, 41%) m/z 593 (M+H)⁺ (ES⁺). ¹H NMR (400 MHz, DMSO-d₆) δ: 1.27 (9 H, s), 2.39 (3 H, s), 3.31 (3 H, s (obscured by DHO peak)), 4.06 (2H, s), 5.34 (2H, s), 6.35 (1H, s), 6.96 (1H, d), 7.35 (2H, d), 7.43 (2H, d), 7.64-7.54 (4H, m), 7.93 (1H, d), 8.29 (1H, dd), 8.45 (1H, d), 8.58 (1H, s), 8.70 (1H, s), 8.79 (1H, s), 9.76 (1H, s).

### Example 36: N-(4-((4-(3-(3-tert-Butyl-1-p-tolyl-1H-pyrazol-5-yl)ureido)naphthalen-1-yloxy)methyl) pyridin-3-yl)-2-(2-methoxyethoxy)acetamide:

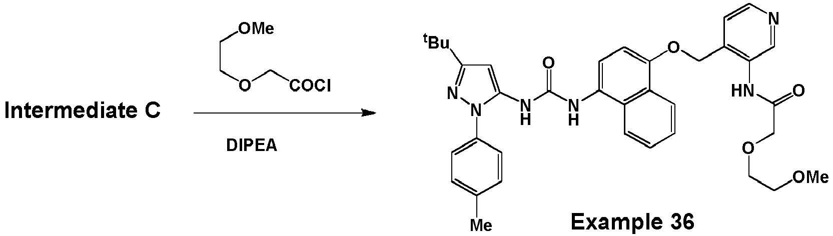

To a solution of **Intermediate C** (50 mg, 0.10 mmol) and DIPEA (33.5 µL, 0.19 mmol) in a mixture of anhydrous DCM (1.0 mL) and anhydrous DMF (0.2 mL) was added 2-(2-methoxyethoxy)acetyl chloride (15 µL 0.11 mmol) and the reaction mixture was stirred for 12 hr at RT. A second aliquot of 2-(2-methoxyethoxy) acetyl chloride (15 µL, 0.11 mmol) was added and the reaction mixture stirred at RT for 6 hr. Methanol (2.0 mL) and AcOH (5 drops) were added and the reaction mixture was subjected to SCX capture and release, eluting with 1% NH₃ in MeOH. The solvent was removed *in vacuo* and the crude material purified by column chromatography (SiO₂, 4 g, 0-10% MeOH in EtOAc, gradient elution) to afford the title compound, **Example 36,** as a white solid (27 mg, 43%): m/z 637 (M+H)⁺ (ES⁺). ¹H NMR (400 MHz, DMSO-d₆) δ: 1.27 (9 H, s), 2.39 (3 H, s), 3.18 (3 H, s), 3.36 (2 H, m), 3.61 (2 H, m), 4.14 (2H, s), 5.33 (2H, s), 6.35 (1H, s), 6.97 (1H, d), 7.35 (2H, d), 7.43 (2H, d), 7.67 - 7.55 (4H, m), 7.92 (1H, d), 8.27 (1H, d), 8.46 (1H, d), 8.58 (1H, s), 8.74 (1H, s), 8.80 (1H, s), 9.65 (1H, s).

### Intermediate D: 1-(4-(2-(2-Aminopyridin-4-yl)ethoxy)naphthalen-1-yl)-3-(3-tert-butyl-1-p-tolyl-1H-pyrazol-5-yl)urea.

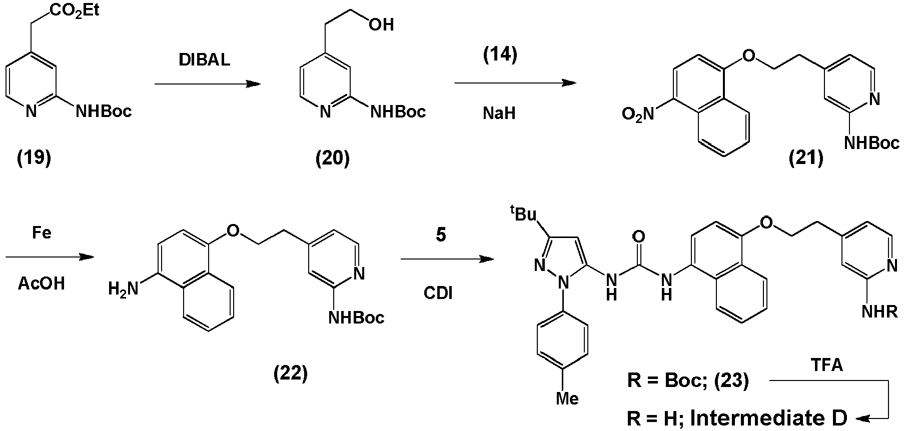

To a stirred solution of ethyl 2-(2-(*tert*-butoxycarbonylamino)pyridin-4-yl)acetate (**19**) (WO 2007/089512) (10.0 g, 35.7 mmol) under nitrogen in THF (100 mL), at -78°C, was added DIBAL (1M solution in THF, 71.3 mL, 71.3 mmol) over 1 hr. The reaction mixture was stirred at -78 to - 60°C for 40 min and was then warmed to -15°C over 1 hr. The solution was re-cooled to -78°C and was treated with further DIBAL (1M solution in THF, 35 mL, 35.7 mmol). The mixture was allowed to warm to -40°C and stirred for 1 hr. Water (10 mL) was added cautiously to quench the reaction followed by MgSO₄ (20 g) and the solids removed by filtration. The filtrate was evaporated to dryness *in vacuo* and the residue subjected to column chromatography (SiO₂, 330 g, 65 % EtOAc in hexanes) to give *tert-butyl* 4-(2-hydroxyethyl)pyridin-2-ylcarbamate (**20**) (6.00 g, 64%) as a yellow solid: m/z 239 (M+H)⁺ (ES⁺).

To a solution of *tert-butyl* 4-(2-hydroxyethyl)pyridin-2-ylcarbamate (**20**) (6.00 g, 25.2 mmol) in THF (70 mL) was added sodium hydride (2.52 g, 63.0 mmol, 60 wt%) at 0 °C. The bright yellow suspension was stirred for 20 min at 0°C and the 1-fluoro-4-nitronaphthalene (**14**) (4.81 g, 25.2 mmol) added in a single portion. After stirring at RT for 2 hr, water (100 mL) was added followed by EtOAc (100 mL). The solid formed between the layers was collected by filtration and the organic phase was washed with saturated aq NaHCO₃ solution (100 mL), brine (100 mL) and dried. The volatiles were removed to give an orange solid. The solids were combined and triturated from MeOH (50 mL) to give *tert-*butyl 4-(2-(4-nitronaphthalen-1-yloxy)ethyl)pyridin-2-ylcarbamate (**21**) as a yellow solid (11.0 g, 98%): m/z 410 (M+H)⁺ (ES⁺).

A mixture of *tert-butyl* 4-(2-(4-nitronaphthalen-1-yloxy)ethyl)pyridin-2-ylcarbamate (**21**) (5.20 g, 12.7 mmol) and iron mesh (4.30 g, 76 mmol) was suspended in AcOH and EtOH (1:2, 120 mL) and was heated to 60°C and stirred rapidly until the reaction was judged to be complete by LC-MS. The mixture was cooled to RT, poured carefully onto saturated aq NaHCO₃ solution (1000 mL) and extracted with EtOAc (500 mL x 2). The combined organic layers were washed with saturated aq NaHCO₃ solution (1000 mL), water (1000 mL), brine (1000 mL) and dried. The solution was filtered and evaporated *in vacuo* to give *tert-butyl* 4-(2-(4-aminonaphthalen-1-yloxy)ethyl)pyridin-2-ylcarbamate (**22**) as a yellow oil (5.00 g, 95%): m/z 380 (M+H)⁺ (ES⁺).

To a stirred suspension of CDI (3.00 g, 18.18 mmol) in DCM (15 mL) was added a solution of the pyrazole amine (**5**) (4.17 g, 18.18 mmol) in DCM (40 mL) over 1.5 hrs. After 2 hr at RT a solution of the naphthyl amine (**22**) (3.00 g, 7.91 mmol) in DCM (15 mL) was added. After stirring overnight, the solution was diluted with MeOH (10 mL) and absorbed onto silica gel (30 g) and subjected to column chromatography (SiO₂, 330 g, 30% to 100% EtOAc in isohexane and then 0% to 6% MeOH in EtOAc) to give *tert*-butyl-4-(2-(4-(3-(3-*tert*-butyl-1-*p*-tolyl-1*H-*pyrazol-5-yl)ureido)naphthalen-1-yloxy)ethyl)pyridin-2-ylcarbamate (**23**) as a beige solid (4.20 g, 80%): m/z 635 (M+H)⁺ (ES⁺).

To a suspension of the carbamate (**23**) (1.35 g, 2.20 mmol) in DCM (10 mL) was added TFA (10 mL). After stirring at RT for 2 hr, the volatiles were evaporated and the residue was taken up in EtOAc (50 mL) and extracted with saturated aq NaHCO₃ solution (50 mL). The organic layer was separated and was washed with brine (50 mL), and then dried and evaporated to give the title compound, **Intermediate D,** as a pale pink solid (1.20 g, 100%): m/z 535 (M+H)⁺ (ES⁺).

### Example 37: N-(4-(2-(4-(3-(3-tert-Butyl-1-p-tolyl-1H-pyrazol-5-yl)ureido)naphthalen-1-yloxy)ethyl)pyridin-2-yl)-2-methoxyacetamide:

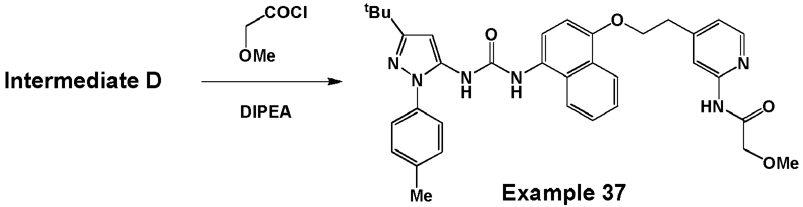

To a suspension of **Intermediate D** (35 mg, 0.065 mmol) in DCM (0.5 mL) was added DIPEA (23 µL, 0.131 mmol) and methoxyacetyl chloride (7 µL, 0.072 mmol) and the mixture stirred at RT, until judged to be complete by LC-MS. The reaction mixture was diluted with saturated aq NaHCO₃ solution (1.5 mL) and the layers were separated through a phase separator cartridge. The organics were collected, evaporated under reduced pressure and the residue subjected to SCX capture and release. The resulting residue was purified further by preparative RP HPLC to give the title compound, **Example 37,** as a white solid (5 mg, 13%): m/z 607 (M+H)⁺ (ES⁺). ¹H NMR (400 MHz, DMSO-d₆) δ: 1.26 (9 H, s), 2.37 (3 H, s), 3.20 (2 H, t), 3.37 (3 H, s), 4.06 (2 H, s), 4.38 (2 H, t), 6.33 (1H, s), 6.95 (1H, d), 7.19 (1H, dd), 7.33 (2 H, m), 7.42-7.47 (3 H, m), 7.54 (1H, m), 7.59 (1H, d), 7.87 (1H, d), 8.12 (1H, d), 8.18 (1H, br s), 8.23 (1H, d), 8.67 (1 H, s), 8.84 (1H, s), 9.89 (1H, s).

### Example 38: N-(4-(2-(4-(3-(3-tert-Butyl-1-p-tolyl-1H-pyrazol-5-yl)ureido)naphthalen-1-yloxy)ethyl)pyridin-2-yl)-2-(2-methoxyethoxy)acetamide:

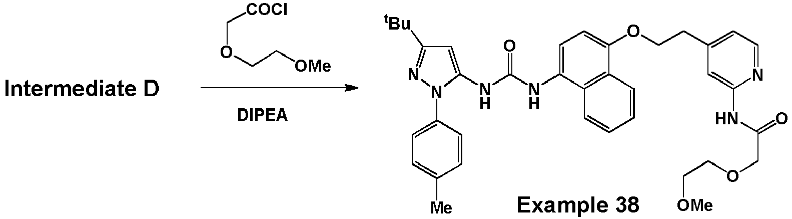

To a suspension of **Intermediate D** (35 mg, 0.065 mmol) in DCM (0.5 mL) was added DIPEA (23 µL, 0.131 mmol) and 2-(2-methoxy)ethoxyacetyl chloride (11 mg, 0.072 mmol) and the mixture stirred at RT until judged to be complete by LC-MS. The reaction mixture was diluted with saturated aq NaHCO₃ solution (1.5 mL) and the layers were separated through a phase separator cartridge. The organics were collected, evaporated under reduced pressure and the residue subjected to SCX capture and release. The resulting residue was purified further by preparative RP HPLC to give the title compound, **Example 38,** as an off white solid (13 mg, 31%): m/z 651 (M+H)⁺ (ES⁺). ¹H NMR (400 MHz, DMSO-d₆) δ: 1.26 (9 H, s), 2.38 (3 H, s), 3.21 (2 H, t), 3.28 (3 H, s), 3.49-3.51 (2 H, m), 3.66-3.68 (2 H, m), 4.13 (2 H, s), 4.38 (2 H, t), 6.34 (1 H, s), 6.95 (1H, d), 7.19 (1H, dd), 7.34 (2 H, m), 7.41-7.48 (3 H, m), 7.51-7.56 (1H, m), 7.59 (1H, d), 7.87 (1H, d), 8.11-8.14 (1H, dd), 8.20 (1H, br s), 8.23-8.25 (1H, dd), 8.55 (1H, s), 8.75 (1H, s), 9.83 (1H, s).

### Example 39: 4-(2-(4-(3-(3-tert-Butyl-1-p-tolyl-1H-pyrazol-5-yl)ureido)naphthalen-1-yloxy)ethyl)-1-methyl-3-(pyridin-2-yl)urea:

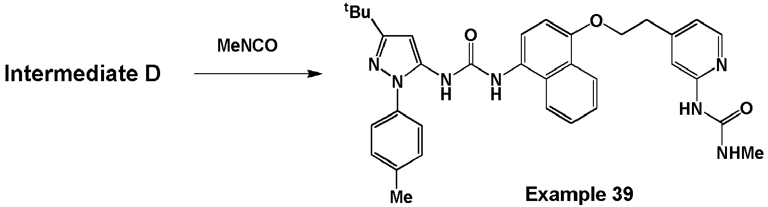

To a solution of **Intermediate D** (50 mg, 0.094 mmol) in pyridine (1.0 mL) was added methyl isocyanate (5.3 mg, 0.094 mmol) and the mixture was stirred at RT for 72 hr. The solvent was evaporated under reduced pressure and the resulting residue was triturated from MeOH (5.0 mL) to give the title compound, **Example 39,** as an off white solid (7 mg, 13%): m/z 592 (M+H)⁺ (ES⁺). ¹H NMR (400 MHz, DMSO-d₆) δ: 1.26 (9 H, s), 2.38 (3 H, s), 3.13 (2 H, t), 3.31 (3 H, s, obscured by H₂O),4.32 (2 H, t), 6.34 (1H, s), 6.93 (1H, d), 7.34 (2 H, m), 7.40-7.48 (6 H, m), 7.53-7.57 (1H, m), 7.61 (1H, d), 7.81-7.83 (2 H, d), 7.86-7.89 (1H, d), 8.02-8.04 (1H, dd), 8.55 (1H, s), 8.75 (1H, s).

### Example 40: 4-(2-(4-(3-(3-tert-Butyl-1-p-tolyl-1H-pyrazol-5-yl)ureido)naphthalen-1-yloxy)ethyl)-3-(pyridin-2-yl)urea:

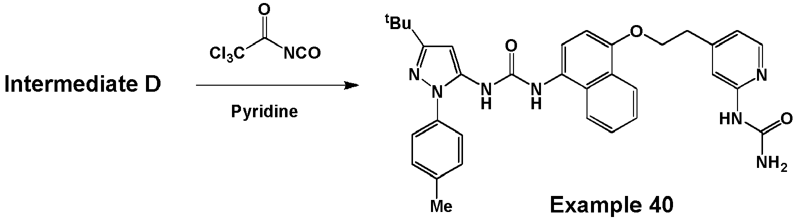

To a solution of **Intermediate D** (50 mg, 0.094 mmol) in pyridine (1.0 mL) was added trichloroacetylisocyanate (12 µL, 0.103 mmol) and the mixture was stirred at RT until judged to be complete by LC-MS. The solvent was evaporated *in vacuo* and the resulting residue was subjected to SCX capture and release and then triturated from DCM (10 mL) to give the title compound, **Example 40,** as an off white solid (25 mg, 44%): m/z 578 (M+H)⁺ (ES⁺). ¹H NMR (400 MHz, DMSO-d₆) δ: 1.26 (9 H, s), 2.38 (3 H, s), 3.12 (2 H, t), 4.35 (2 H, t), 6.34 (1H, s), 6.94-6.99 (2 H, m), 7.19 (1H, dd), 7.33-7.35 (2 H, m), 7.41-7.50 (5 H, m), 7.52-7.56 (1H, m), 7.60 (1H, d), 7.87 (1H, d), 8.09-8.13 (2 H, m), 8.54 (1H, s), 8.75 (1H, s), 9.08 (1H, s).

### Intermediate E: 1-(4-(2-(3-Aminopyridin-4-yl)ethoxy)naphthalen-1-yl)-3-(3-tert-butyl-1-p-tolyl-1H-pyrazol-5-yl)urea.

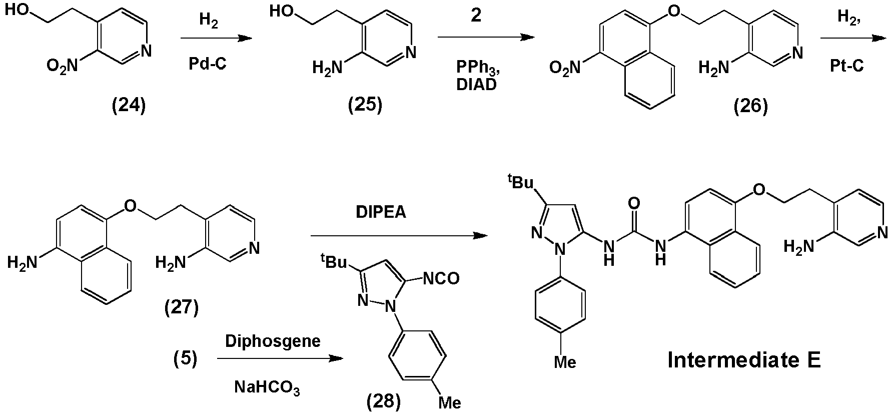

A solution of 2-(3-nitropyridin-4-yl)ethanol (**24**) (WO2006/136562) (2.00 g, 11.89 mmol) in MeOH (150 mL) was passed through a Thales H-cube (2.0 mL.min⁻¹, 30°C, 30 bar, Pd/C Cat-Cart, 55 mm, controlled mode). Analysis by LC-MS showed a significant amount of starting material was still present and the solution was passed through the H-cube twice more (2.0 mL.min⁻¹, full hydrogen mode, RT, and 2.0 mL.min⁻¹, full hydrogen mode, 40°C). Evaporation of the volatiles gave 2-(3-aminopyridin-4-yl)ethanol (**25**) as a purple oil (1.30 g, 81%): m/z 139 (M+H)⁺ (ES⁺).

To a solution of 4-nitronaphthalen-1-ol (**2**) (0.95 g, 5.00 mmol), PPh₃ (1.97 g, 7.50 mmol) and 2-(3-aminopyridin-4-yl)ethanol (**25**) (1.04 g, 7.50 mmol) in THF (20 mL) was added dropwise DIAD (590 µL, 3.75 mmol) at -15 °C. The mixture was stirred for 1 hr at RT and the volatiles removed *in vacuo.* The residues was absorbed on silica (20 g) and purified by column chromatography (SiO₂, 80 g, 50-100% EtOAc in isohexane, gradient elution and then 5% MeOH in EtOAc, isocratic elution) to give 4-(2-(4-nitronaphthalen-1-yloxy) ethyl)pyridin-3-amine (**26**) (1.36 g, 88 %): m/z 310 (M+H)⁺ (ES⁺).

A solution of 4-(2-(4-nitronaphthalen-1-yloxy)ethyl)pyridin-3-amine (**26**) (700 mg, 2.263 mmol) in a mixture of MeOH (50 mL), EtOAc (25 mL), and DCM (25 mL) was passed through a Thales H-cube (10 % Pt/C, 30mm, 1.0 mL.min⁻¹, 40°C, full hydrogen mode). The solvent was removed *in vacuo* to give 4-(2-(4-aminonaphthalen-1-yloxy)ethyl)pyridin-3-amine (**27**) as a brown solid (612 mg, 92%). m/z 280 (M+H)⁺ (ES⁺).

To a solution of 3-*tert*-butyl-1-*p*-tolyl-1*H*-pyrazol-5-amine (**5**) (1.00 g, 4.36 mmol) in DCM (90 mL) was added a saturated aq solution of NaHCO₃ (60 mL). The mixture was stirred vigorously, cooled to 0 °C and diphosgene (2.1 mL, 17.4 mmol) was added in a single portion. After stirring for 1 hr at RT, the layers were separated and the organics dried and evaporated to give a brown oil. The oil was triturated with isohexane (5.0 mL) and the solid filtered. The filtrate was concentrated in vacuo to give 3-*tert*-butyl-5-isocyanato-1-*p*-tolyl-1*H*-pyrazole (**28**) as a light brown oil (1.00 g, 3.92 mmol, 90 %). m/z 288 (in MeOH) (M+H+MeOH)⁺ (ES⁺).

A solution of 3-*tert*-butyl-5-isocyanato-1-*p*-tolyl-1*H*-pyrazole (**28**) (530 mg, 2.076 mmol) in THF (2.0 mL) was added to a solution of 4-(2-(4-aminonaphthalen-1-yloxy)ethyl)pyridin-3-amine (**27**) (580 mg, 2.076 mmol) and DIPEA (1085 µL, 6.23 mmol) in THF (10 mL) and MeCN (1.0 mL) and the reaction mixture stirred at RT overnight. The mixture was poured into brine (25 mL) and extracted with EtOAc (2 x 25 mL), dried, filtered and the solvent removed *in vacuo.* The product was pre-adsorbed onto hyflo (10 g), and purified by reverse phase column chromatography (40 g, C₁₈ [from Silicycle], acetonitrile/water, 0 to 100%) and the product fractions concentrated *in vacuo* to give the title compound. **Intermediate E,** as an off white solid (410 mg, 36 %). m/z 535 (M+H)⁺ (ES⁺).

### Example 41: N-(4-(2-(4-(3-(3-tert-Butyl-1-p-tolyl-1H-pyrazol-5-yl)ureido)naphthalen-1-yloxy)ethyl)pyridin-3-yl)-2-methoxyacetamide:

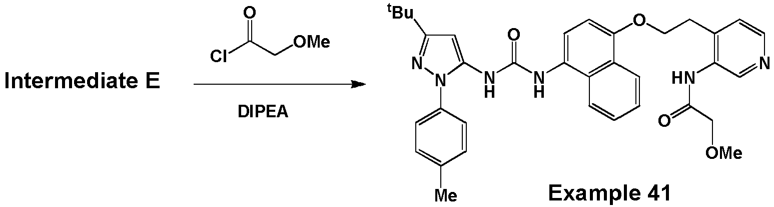

To a solution of **Intermediate E** (50 mg, 0.094 mmol) and DMAP (5.71 mg, 0.047 mmol) in DCM (3.0 mL) was added methoxyacetyl chloride (25.7 µL, 0.281 mmol) at 0°C and the reaction mixture stirred at RT for 1.5 hr. The solvent was removed *in vacuo* and the residue subjected to SCX capture and release. The impure product was purified by flash column chromatography (SiO₂, 4.0 g, 0-10% MeOH in DCM, gradient elution) to give the title compound, **Example 41,** as a pale pink solid (45 mg, 77%): m/z 607 (M+H)⁺ (ES⁺). ¹H NMR (400 MHz, DMSO-d₆) δ: 1.27 (9H, s), 2.39 (3H, s), 3.18 (2H, t), 3.40 (3H, s), 4.08 (2H, s), 4.39 (2H, t), 6.35 (1H, s), 6.96 (1H, d), 7.35 (2H, m), 7.43 (2H, m), 7.50 (2H, m), 7.58 (1H, m), 7.61 (1H, d), 7.88 (1H, d), 8.09 (1H, dd), 8.37 (1H, d), 8.52 (1H, s), 8.56 (1H. br s), 8.76 (1H, br s), 9.66 (1H, brs.).

### Example 42: N-(4-(2-(4-(3-(3-tert-Butyl-1-p-tolyl-1H-pyrazol-5-yl)ureido)naphthalen-1-yloxy)ethyl)pyridin-3-yl)-2-(2-methoxyethoxy)acetamide:

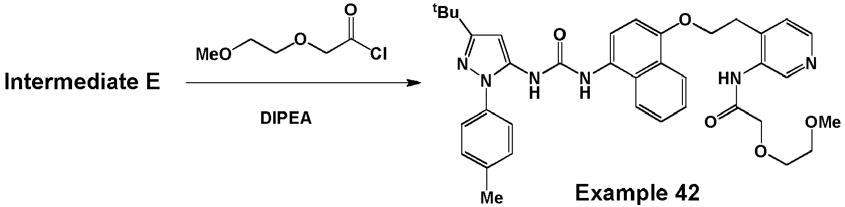

To a solution of **Intermediate E** (50 mg, 0.094 mmol) and DMAP (5.71 mg, 0.047 mmol) in DCM (3.0 mL) was added 2-(2-methoxyethoxy)acetyl chloride (30 µL, 0.281 mmol) at 0°C and the reaction mixture stirred at RT for 1.5 hr. The solvent was removed *in vacuo* and the residue subjected to SCX capture and release. The residue was purified by column chromatography (SiO₂ ,4.0 g, 0-8% MeOH in DCM, gradient elution) and the product fractions concentrated *in vacuo* to give the title compound, **Example 42,** as a light purple solid (35 mg, 56%): m/z 651 (M+H)⁺ (ES⁺). ¹H NMR (400 MHz, DMSO-d₆) δ: 1.25 (9H, s), 2.40 (3H, s), 3.26 (5H, m), 3.50 (2H, m), 3.70 (2H, m), 4.15 (2H, s), 4.40 (2H, t), 6.35 (1H, s), 6.98 (1H, d), 7.35 (2H, m), 7.42 (2H, m), 7.50 (3H, m), 7.62 (1H, d), 7.87 (1H, d), 8.07 (1H, dd), 8.36 (1H, d), 8.56 (1H. br s), 8.60 (1H, s), 8.76 (1H, br s), 9.55 (1H, br s.).

### Intermediate F: 1-(4-(2-(2-Aminopyridin-4-yloxy)ethyl)naphthalen-1-yl)-3-(3-tert-butyl-1-p-tolyl-1H-pyrazol-5-yl)urea.

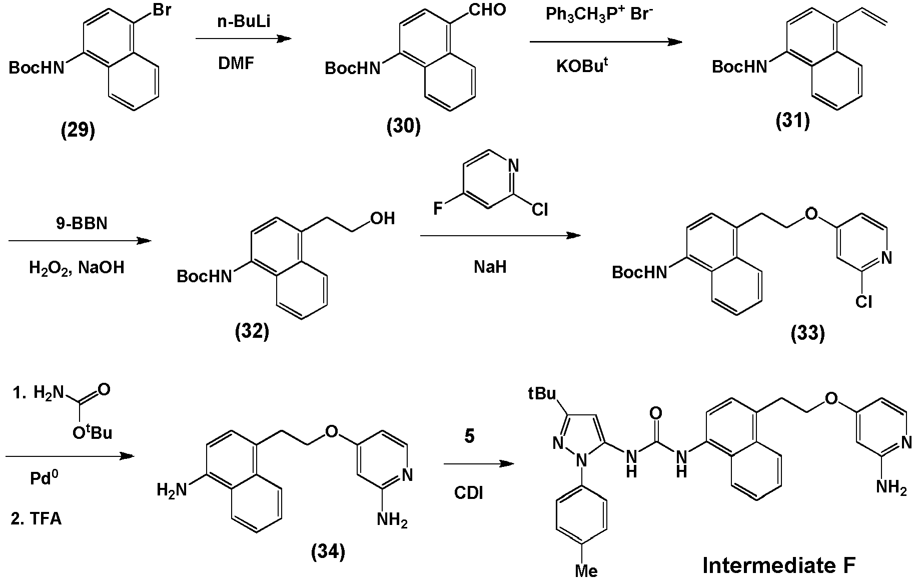

To a solution of *tert-butyl* 4-bromonaphthalen-1-ylcarbamate (**29**)(3.00 g, 9.31 mmol) in THF (100 mL) at -78°C under nitrogen was added n-BuLi (1.6M in hexane, 20.4 mL, 32.6 mmol) and the reaction mixture was stirred at -78°C for 1 hr. Neat DMF (4.50 mL, 58.1 mmol) was added and the reaction mixture was stirred at -78°C for 1 hr, then warmed to RT and stirred for a further 1 hr. Water (5 mL) was added and the mixture was partitioned between ethyl acetate (100 mL) and water (100 mL). The organic phase was washed with brine, dried (MgSO₄) and evaporated *in vacuo.* The residue was triturated with isohexane to afford *tert-butyl* 4-formylnaphthalen-1-ylcarbamate (**30**) as a beige solid (2.20 g, 87%): m/z 272 (M+H)⁺(ES)⁺.

To a suspension of methyltriphenylphosphonium bromide (3.82 g, 10.69 mmol) in THF (20 mL) at 0°C under nitrogen was added potassium *tert*-butoxide (1.20 g, 10.69 mmol) and the reaction mixture was stirred at 0°C for 15 min, then warmed to RT and stirred for a further 45 min. The suspension was cooled to 0°C and a solution of (**30**) (1.16 g, 4.28 mmol) in THF (10 mL) was added dropwise. The reaction mixture was warmed to RT and stirred for 2 hr. Saturated aqueous NH₄Cl solution (30 mL) was added and the mixture was extracted with ethyl acetate. The ethyl acetate extract was washed with brine, dried (MgSO₄) and evaporated *in vacuo.* The residue was purified by flash column chromatography (SiO₂, 0-10% ethyl acetate in isohexane, gradient elution) to afford *tert-butyl* 4-vinylnaphthalen-1-ylcarbamate (**31**) as a white solid (0.831 g, 72%): m/z 268 (M-H)⁻ (ES-).

To a solution of (**31**) (0.83 g, 3.08 mmol) in THF (10 mL) was added 9-BBN (0.5M in THF, 9.9 mL, 4.95 mmol) dropwise under nitrogen at 0°C. The mixture was warmed to RT and stirred for 16 hr. Water (1 mL), aqueous NaOH solution (3M, 10.0 mL, 30 mmol) and hydrogen peroxide (35% in water, 8.0 mL) were added. The reaction mixture was warmed to 50°C and stirred for 2 hr and was then extracted with ethyl acetate. The ethyl acetate extract was washed with water and brine, dried (MgSO₄) and evaporated *in vacuo.* The residue was purified by flash column chromatography (SiO₂, 10-60% ethyl acetate in isohexane, gradient elution) to afford *tert-*butyl 4-(2-hydroxyethyl)naphthalen-1-ylcarbamate (**32**) (763 mg, 86%); m/z 288 (M+H)⁺, (ES⁺)

To a degassed solution of (**32**) (0.64 g, 2.23 mmol) in DMF (10 mL) was added sodium hydride (0.267 g, 60% dispersion in mineral oil, 6.68 mmol) under nitrogen at 0°C. The solution was warmed to RT and was stirred for 30 min and then cooled to 0°C. To this mixture was added a solution of 2-chloro-4-fluoro-pyridine (0.381 g, 2.90 mmol) in DMF (5.0 mL) and the reaction mixture was warmed to RT and stirred for 16 hr. Water was added and the mixture was extracted with ethyl acetate. The ethyl acetate extract was washed with brine (3x), dried (MgSO₄) and evaporated *in vacuo.* The residue was purified by flash column chromatography (SiO₂, 0-40% ethyl acetate in isohexane, gradient elution) to afford *tert-butyl* 4-(2-(2-chloropyridin-4-yloxy)ethyl)naphthalen-1-ylcarbamate (**33**) (670 mg, 75%); m/z 399 (M+H)⁺, (ES⁺)

To a degassed suspension of (**33**) (600 mg, 1.50 mmol), *tert-butyl* carbamate (176 mg, 1.50 mmol), Cs₂CO₃ (733 mg, 2.26 mmol) and Xathphos (43 mg, 0.075 mmol) in THF (10 mL) was added Pd₂dba₃ (34 mg, 0.038 mmol) and the reaction mixture was heated at reflux under nitrogen for 16 hr. Water was added and the reaction mixture was extracted with ethyl acetate. The organic extract was washed with brine, dried and evaporated *in vacuo.* The residue was purified by flash column chromatography (SiO₂, 0-50% hexane in ethyl acetate, gradient elution) to afford a mixture of the desired Boc protected product and starting material, which was dissolved in a mixture of DCM (2 mL) and TFA (2 mL) and stirred at RT for 1 hr. The solvent was evaporated *in vacuo* and the residue was dissolved in DCM and washed with saturated aqueous NaHCO₃ solution and brine, then dried (MgSO₄) and evaporated *in vacuo.* The residue was purified by flash column chromatography (SiO₂, 5% MeOH in DCM, isocratic elution) to afford 4-(2-(4-aminonaphthalen-1-yl)ethoxy)pyridin-2-amine (**34**) (45 mg, 11%); m/z 280 (M+H)⁺, (ES⁺).

To a suspension of CDI (122 mg, 0.752 mmol) in DCM (2 mL) was added a solution of (**5**) (172 mg, 0.752 mmol) in DCM (2.0 mL) and the reaction mixture was stirred at RT for 16 hr. An aliquot (1.0 mL) of this reaction mixture was added to a stirred suspension of (**34**) (42 mg, 0.150 mmol) in DCM (2.0 mL). THF (0.5 mL) was added and the reaction mixture was stirred for 1 hr. A second aliquot (1.0 mL) of the CDI reaction mixture was added and the mixture was stirred for a further 20 hr at RT. Methanol was added and stirring continued for 30 min. The solvent was evaporated and the residue was purified by flash column chromatography (SiO₂, 0-5% MeOH in DCM, gradient elution). The resulting impure product was dissolved in ethyl acetate, washed with water and brine, dried (MgSO₄) and evaporated *in vacuo* to afford the title compound, **Intermediate F,** (42 mg, 52%); m/z 535 (M+H)⁺, (ES⁺).

### Example 43: N-(4-(2-(4-(3-(3-tert-butyl-1-p-tolyl-1H-pyrazol-5-yl)ureido)naphthalen-1-yl)ethoxy)pyridin-2-yl)-2-methoxyacetamide:

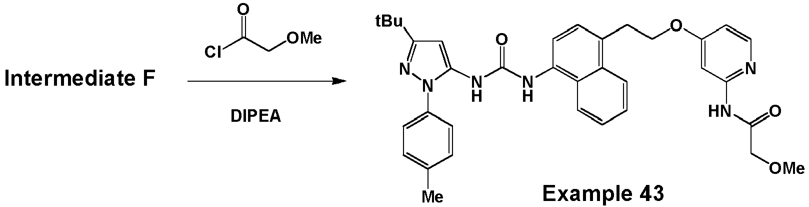

To a solution of **Intermediate F** (40 mg, 0.075 mmol) and DIPEA (0.059 mL, 0.337 mmol) in DCM (1.5 mL) was added methoxyacetyl chloride (32 mg, 0.299 mmol) and the reaction mixture was stirred for 16 hr at RT. A solution of ammonia (1 % in methanol) was added and the stirring continued for 30 min. The solvent was evaporated *in vacuo* and the residue was purified by SCX capture and release. The crude product was purified by flash column chromatography (SiO₂, 0-5% MeOH in DCM, gradient elution) to afford the title compound, **Example 43,** (10 mg, 20%): m/z 607 (M+H)⁺ (ES⁺); ¹H NMR (400 MHz, DMSO-d₆) δ: 1.28 (9H, s), 2.39 (3H, s), 3.35 (3H, s), 3.51 (2H, t), 4.03 (2H, s), 4.35 (2H, t), 6.39 (1H, s), 6.72 (1H, dd), 7.35 (2H, d), 7.42-7.47 (3H, overlapping m), 7.55-7.63 (2H, overlapping m), 7.67 (1H, d), 7.82 (1H, d), 8.03 (1H, m), 8.10 (1H, d), 8.19 (1H, d), 8.72 (1H, brs), 9.01 (1H, brs), 9.87 (1H, brs).

### Intermediate G: 1-(4-(1-(2-aminopyridin-4-yl)ethoxy)naphthalen-1-yl)-3-(3-tert-butyl-1-p-tolyl-1H-pyrazol-5-yl)urea.

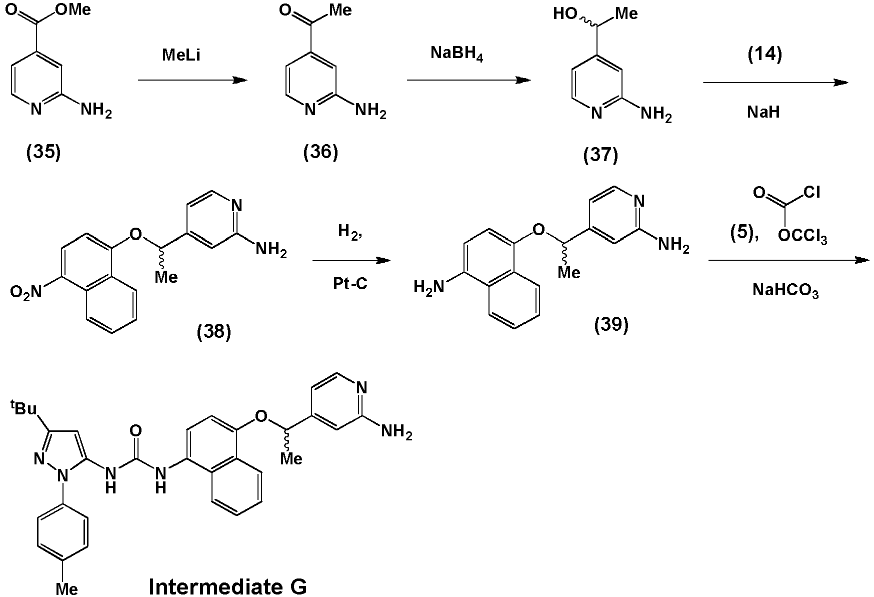

To a stirred solution of methyl-2-aminopyridine-4-carboxylate (**35**) (1.00 g, 6.57 mmol) in THF (100 mL), at -78°C under nitrogen, was added methyllithium (1.6 M in diethyl ether, 16.4 mL, 26.3 mmol), over 10 min. After a further 30 min at -78°C, the viscous reaction mixture was warmed to 0°C. After a further 3 hr, the reaction was quenched at 0°C by the cautious addition of iso-propanol (8.0 mL). The mixture was warmed to RT, brine (200 mL) and EtOAc (150 mL) were added, and the layers were separated. The aqueous layer was extracted with EtOAc (3 x100 mL), and the combined organic extracts were dried and the solvents removed *in vacuo.* The crude residue was purified by column chromatography (SiO₂, 80 g, 0-8% MeOH in EtOAc, gradient elution) to give 1-(2-aminopyridin-4-yl)ethanone (**36**) (176 mg, 20%) as a yellow powder: m/z 137 (M+H)⁺ (ES⁺).

To a mixture of (**36**) (168 mg, 1.234 mmol) in MeOH (10 mL), under nitrogen at 0°C, was added sodium borohydride (46.7 mg, 1.234 mmol). The reaction mixture was stirred at RT for 2 hr, and the volatiles were removed under reduced pressure. The residue was taken up into EtOAc (25 mL), and extracted with saturated aq NaHCO₃ solution (30 mL). The aqueous layer was back extracted with EtOAc (2 x 20 mL), and the combined organic extracts were washed with brine (30 mL), dried and the solvents removed *in vacuo* to give 1-(2-aminopyridin-4-yl)ethanol (**37**) (77 mg, 45%) as a yellow oil: m/z 139 (M+H)⁺ (ES⁺).

To a stirred solution of (**37**) (73 mg, 0.528 mmol) in DMF (1.5 mL), under nitrogen at 0°C, was added sodium hydride (32 mg, 0.793 mmol, 60 wt%). The resulting mixture was stirred at 0°C for 40 min, and a solution of 1-fluoro-4-nitronaphthalene (**14**) (101 mg, 0.528 mmol) in DMF (1.5 mL) was added dropwise. The resulting dark-red mixture was stirred at 0°C for 5 min and at RT for 40 min and was quenched by the addition of 1.0 mL of NH₄Cl solution. Water (20 mL) and EtOAc (20 mL) were added, and the layers were separated. The aqueous layer was extracted with EtOAc (3 x 15 mL). The combined organic extracts were washed with brine, dried and the solvents removed *in vacuo.* The crude material was purified by column chromatography (SiO₂, 12 g, 0-80% EtOAc in isohexane, gradient elution) to give 4-(1-(4-nitronaphthalen-1-yloxy)ethyl)pyridin-2-amine (**38**) (94.6 mg, 57%) as an orange gum: m/z 310 (M+H)⁺ (ES⁺).

A solution of (**38**) (91 mg, 0.294 mmol) in MeOH (15 mL) and AcOH (3.0 mL) was passed through a Thales H-cube (1.0 mL.min⁻¹, 30°C, 55 mm, 10% Pt/C Cat-Cart, full hydrogen mode). The volatiles were removed under reduced pressure, leaving a purple solid, which was then subjected to SCX capture and release to give 4-(1-(4-aminonaphthalen-1-yloxy)ethyl)pyridin-2-amine (**39**) (81 mg, 99%) as a purple oil: m/z 280 (M+H)⁺ (ES⁺).

To a solution of (**5**) (57 mg, 0.250 mmol) in DCM (6.0 mL) was added saturated aq NaHCO₃ solution (4.0 mL) The mixture was stirred vigorously and was cooled to 0°C and trichloromethylchloroformate (0.091 mL, 0.750 mmol) was added in one portion. The resulting mixture was stirred at 0°C for 1.5 hr. The biphasic mixture was separated and the organic extract was dried and the solvents removed under reduced pressure to afford an oil, which was dried under high vacuum, at 35°C for 35 min. The resulting oil was taken up into THF (5.0 mL), and then added to 4-(1-(4-aminonaphthalen-1-yloxy)ethyl)pyridin-2-amine (**39**) (81 mg, 0.290 mmol). DIPEA (179 µL, 1.029 mmol) was added, and the reaction mixture was stirred at RT for 16 hr. Water (15 mL) and EtOAc (10 mL) were added to the reaction mixture and the layers were separated. The aq layer was extracted with EtOAc (15 mL). The combined organic extracts were washed with brine (20 mL), dried and the solvents removed under reduced pressure. The resulting residue was dissolved in MeOH (5.0 mL) and AcOH (2.0 mL) and subjected to SCX capture and release. The crude mixture was purified by column chromatography (SiO₂, 12 g, 0-10% MeOH in DCM, gradient elution) to give the title compound, **Intermediate G,** (63 mg, 38%) as a beige powder: m/z 535 (M+H)⁺ (ES⁺).

### Example 44: N-(4-(1-(4-(3-(3-tert-Butyl-1-p-tolyl-1H-pyrazol-5-yl)ureido)naphthalen-1-yloxy)ethyl)pyridin-2-yl)-2-methoxyacetamide:

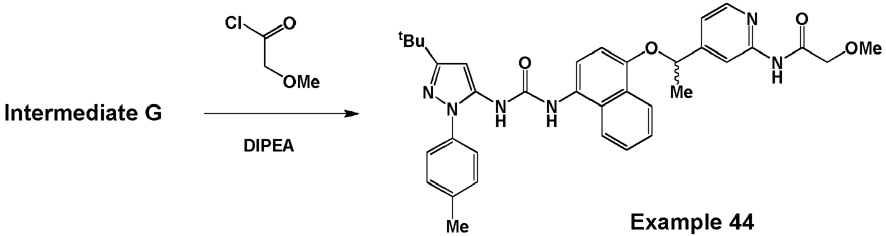

Methoxyacetyl chloride (17.5 µL, 0.192 mmol) was added dropwise to a solution of **Intermediate G** (41 mg, 0.077 mmol) and DIPEA (40.1 µL, 0.230 mmol) in DCM (3 mL) under nitrogen at 0°C. After 15 min the reaction mixture was warmed to RT and was stirred for 1.5 hr. A solution of NH₃ (1% in MeOH, 1.5 mL) was added and stirring continued for a further 2 hr. The reaction mixture was evaporated *in vacuo* and the residue was subjected to SCX capture and release. Fractions containing the desired material were combined, evaporated *in vacuo* and purified by flash column chromatography (SiO₂, 12 g, 3-6% MeOH in DCM, gradient elution; then SiO₂, 12 g, 0-40% EtOAc in ether, gradient elution) to give the title compound, **Example 44,** as a beige solid (24 mg, 51%): m/z 607 (M+H)⁺ (ES⁺); ¹H NMR (400 MHz, DMSO d₆) δ: 1.26 (9H, s), 1.67 (3H, d), 2.38 (3H, s), 4.03 (2H, s), 5.75 (1H, q), 6.32 (1H, s), 6.81 (1H, d), 7.22 (1H, dd), 7.35 (2H, m), 7.42 (2H, m), 7.48 (1H, s), 7.59 (2H, m), 7.88 (1H, m), 8.24 (1H, br s), 8.27 (1H, d), 8.36 (1H, m), 8.59 (1H, br s), 8.76 (1H, br s), 9.99 (1H, br s).

### Intermediate H: 1-(4-(1-(2-Aminopyridin-4-yl)-2-methylpropan-2-yloxy)naphthalen-1-yl) -3-(3-tert-butyl-1-p-tolyl-1H-pyrazol-5-yl)urea.

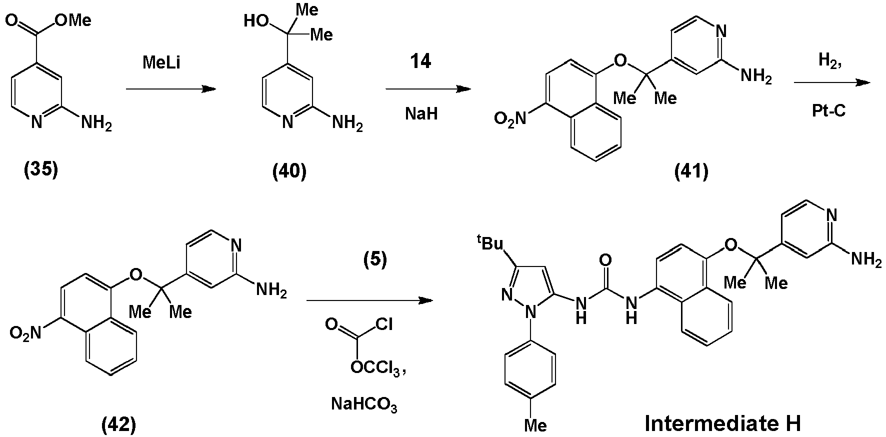

To a stirred solution of methyl-2-aminopyridine-4-carboxylate (**35**) (2.00 g, 13 mmol) in THF (200 mL), at -78°C under nitrogen, was added methyllithium (1.6 M in diethyl ether, 33 mL, 53 mmol) over 10 min. After 30 min the viscous reaction mixture was warmed to 0°C for 3.5 hr and was quenched at 0°C by the cautious addition of isopropanol (15 mL). The mixture was warmed to RT, and brine (400 mL) and EtOAc (300 mL) were added. The aqueous layer was separated and extracted with EtOAc (3 x 200 mL) and the combined organic extracts were dried (MgSO₄) and evaporated *in vacuo.* The crude residue was purified by flash column chromatography (SiO₂, 120 g, 0-10% MeOH in EtOAc, gradient elution) to afford the 2-(2-aminopyridin-4-yl)propan-2-ol (**40**) (1.27 g, 63%) as a yellow amorphous solid: ¹H NMR (400 MHz, CD₃OD) δ: 1.47 (6H, s), 6.68-6.71 (2H, overlapping m), 7.80-7.81 (1H, dd).

To a stirred solution of (**40**) (1.55 g, 10.0 mmol) in DMF (30 mL), under nitrogen at 0°C, was added sodium hydride (60% wt, 0.61 g, 15.0 mmol) and the resulting mixture was stirred at 0°C for 5 min., A solution of 1-fluoro-4-nitronaphthalene (**14**) (1.95 g, 10 mmol) in DMF (30 mL) was added dropwise and the resulting dark-red mixture was stirred at 0°C for a further 5 min and then at RT for 2 hr. The reaction mixture was quenched by the addition of saturated aq NH₄Cl solution (10 mL). Water (150 mL) and EtOAc (150 mL) were added, and the layers were separated. The aq layer was extracted with EtOAc (3 x 100 mL) and the combined organic extracts were washed with brine, dried (MgSO₄) and evaporated *in vacuo.* The crude residue was purified by flash column chromatography (SiO₂, 80 g, 0-60% EtOAc in isohexane, gradient elution) to afford 4-(2-(4-nitronaphthalen-1-yloxy)propan-2-yl)pyridin-2-amine (**41**) (282 mg, 8%) as a red oil: m/z 324 (M+H)⁺ (ES⁺).

A solution of (**41**) (282 mg, 0.87 mmol) in MeOH (45 mL) was passed through a Thales H-cube (1.0 mL.min⁻¹, 30°C, 55 mm 10% Pt/C Cat-Cart, full hydrogen mode). The reaction mixture was evaporated *in vacuo* to afford 4-(2-(4-aminonaphthalen-1-yloxy)propan-2-yl)pyridin-2-amine (**42**) (253 mg, 89%) as a brown foam: m/z 294 (M+H)⁺ (ES⁺).

To a solution of (**5**) (447 mg, 1.75 mmol) in DCM (40 mL) was added saturated aq NaHCO₃ solution (27 mL). The mixture was stirred vigorously and was cooled to 0°C and then trichloromethylchloroformate (0.63 mL, 5.25 mmol) was added in one portion. The resulting mixture was stirred at 0°C for 1.5 hr. The biphasic mixture was separated and the organic layer was dried (MgSO₄) and evaporated *in vacuo.* The oily residue which was taken up in THF (15 mL) and was added to a solution of (**42**) (253 mg, 0.86 mmol) in THF (2.0 mL). Neat DIPEA (451 µL, 2.59 mmol) was added and the reaction mixture was stirred at RT for 2 hr. Water (30 mL) and EtOAc (20 mL) were added and the layers were separated. The aq layer was extracted with EtOAc (3 x 15 mL) and the combined organic layers were washed with brine (40 mL), dried (MgSO₄), and evaporated *in vacuo.* The residue was purified by flash column chromatography (SiO₂, 40 g, 0-10% MeOH in DCM, gradient elution) to afford the title compound, **Intermediate H,** (249 mg, 51%) as a purple amorphous solid: m/z 549 (M+H)⁺ (ES⁺).

### Example 45: N-(4-(2-(4-(3-(3-tert-Butyl-1-p-tolyl-1H-pyrazol-5-yl)ureido)naphthalen-1-yloxy)-2-methylpropyl)pyridin-2-yl)-2-methoxyacetamide:

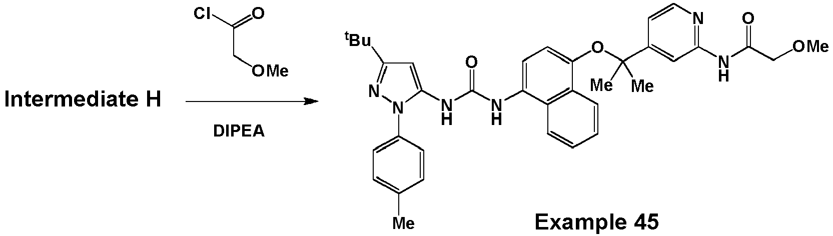

Methoxyacetyl chloride (28 µL, 0.30 mmol) was added dropwise under nitrogen to a solution of **Intermediate H** (66 mg, 0.12 mmol) in DCM (3.0 mL) and DIPEA (63 µL, 0.36 mmol) at 0°C. The reaction mixture was stirred at 0°C for 15 min and then at RT for 2.5 hr. A solution of NH₃ (1% in MeOH, 1.5 mL) was added and the mixture was stirred for 30 min and was then evaporated *in vacuo.* The residue was subjected to SCX capture and release then purified three times by column chromatography (SiO₂, 12 g, 0-7% MeOH in DCM, gradient elution; C₁₈, 12 g, 0-100% MeCN in water, gradient elution and SiO₂, 12 g, 0-65% EtOAc in Et₂O, gradient elution) to afford the title compound, **Example 45,** as a white solid (18 mg, 24%): m/z 621 (M+H)⁺ (ES⁺). ¹H NMR (400 MHz, DMSO-d₆) δ: 1.28 (9H, s), 1.39 (6H, s), 2.39 (3H, s), 3.22 (3H, s), 3.80 (1H, d), 4.16 (1H, d), 5.27 (1H, s), 6.41 (1H, s), 7.26 (1H, dd), 7.37 (2H, m), 7.46 (2H, m), 7.56 (1H, d), 7.60 (2H, overlapping m), 7.76 (1H, br s), 7.95 (1H, m), 8.01 (1H, d), 8.09 (1H, m), 8.22 (1H, d), 8.94 (1H, br s), 9.28 (1H, br s).

### Intermediate J: 1-(4-(1-(2-Aminopyridin-4-yl)propan-2-yloxy)naphthalen-1-yl)-3-(3-tert-butyl-1-p-tolyl-1H-pyrazol-5-yl)urea.

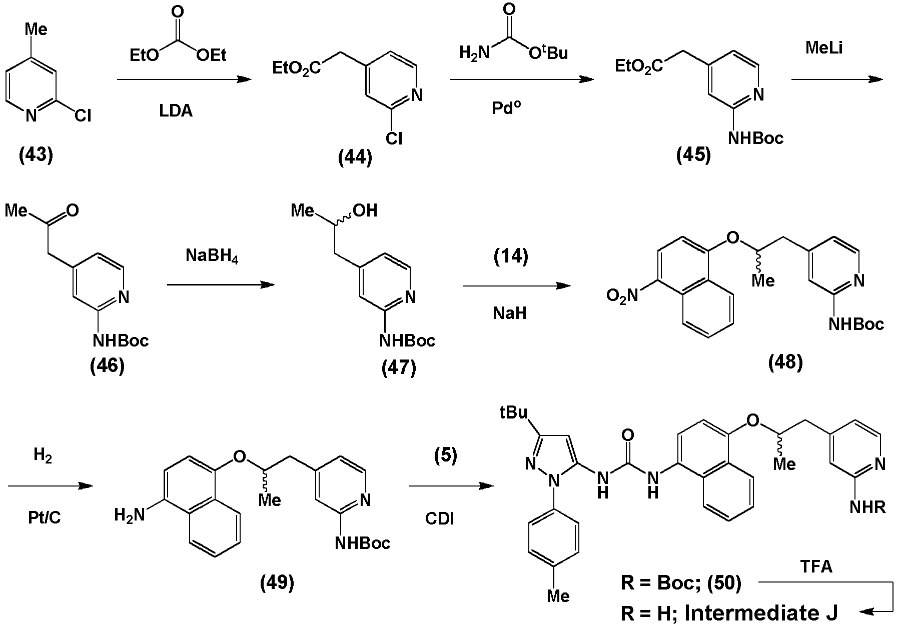

A solution of LDA (2.0 M in THF, 809 mL, 1.62 mol) was added dropwise over 3 hr to a stirred solution of 2-chloropicoline (**43**) (59.1 mL, 674 mmol) in THF (500 mL) at -78°C. The reaction mixture was stirred for a further 15 min and then diethylcarbonate (106 mL, 876 mmol) was added in a single portion. After 10 min the reaction mixture was allowed to warm to 0°C, was stirred at this temperature for 20 min and then a saturated aq solution of NH₄Cl (800 mL) was added. The mixture was extracted with ether and the organic phase was washed with water, dried (MgSO₄), and evaporated *in vacuo* to give a dark oil, (∼200g) which was purified in three batches by flash column chromatography (SiO₂, 330 g, 5-20% EtOAc in isohexane, gradient elution) to give ethyl 2-(2-chloropyridin-4-yl)acetate (**44**) (72 g, 51%): m/z 200 (M+H)⁺ (ES⁺)

A mixture of (**44**) (15.0 g, 75.0 mmol), *tert*-butylcarbamate (26.4 g, 225 mmol), Pd₂(dba)₃ (1.719 g, 1.88 mmol), caesium carbonate (36.7 g, 113 mmol) and Xantphos® (2.17 g, 3.76 mmol) in THF (100 mL) was purged with nitrogen and was then stirred at 65°C for 8 hr. The mixture was cooled to RT and was diluted with water and extracted with ether. The ether extracts were combined and washed with water and brine and then dried (MgSO₄), and evaporated *in vacuo.* The residue was purified by flash column chromatography (SiO₂, 330g, 5-20% EtOAc in isohexane, gradient elution) then subjected to SCX capture and release to provide ethyl 2-(2-(*tert*-butoxycarbonylamino)pyridin-4-yl)acetate (**45**) (12.0g, 51%): m/z 281 (M+H)⁺ (ES⁺).

A solution of methyllithium (1.6 M in diethyl ether, 17.4 mL, 27.9 mmol) was added dropwise over 12 min to a stirred solution of (**45**) (1.56 g, 5.58 mmol) in THF (140 mL) under nitrogen at - 78°C. After 3 hr isopropanol (5.0 mL) was added and the reaction mixture was partitioned between water and EtOAc. The aqueous layer was extracted twice with EtOAc and the combined organic extracts were washed with brine then dried (MgSO₄) and evaporated *in vacuo.* The residue was purified by flash column chromatography (SiO₂, 80g, 0-80% EtOAc in isohexane, gradient elution) to afford *tert-butyl* 4-(2-oxopropyl)pyridin-2-ylcarbamate (**46**) (300 mg, 21%) as a white solid: m/z 251.0 (M+H)⁺ (ES⁺).

To a stirred solution of (**46**) (300 mg, 1.20 mmol) in methanol (12 mL) at 0°C, under nitrogen, was added sodium borohydride (45.3 mg, 1.20 mmol). After 10 min the reaction mixture was warmed to RT and stirring continued for 75 min. The mixture was evaporated *in vacuo* and the residue was taken up into EtOAc and washed with aq NaHCO₃ solution. The aqueous layer was extracted twice with EtOAc and the combined organic extracts were washed with brine, then dried (MgSO₄) and evaporated *in vacuo.* The residue was purified by flash column chromatography (SiO₂, 12 g, 10-80% EtOAc in isohexane, gradient elution) to afford *tert-*butyl 4-(2-hydroxypropyl)pyridin-2-ylcarbamate (**47**) (262 mg, 85%) as a white powder; m/z 253.0 (M+H)⁺ (ES⁺).

Sodium hydride (119 mg, 2.97 mmol) was added to a stirred solution of (**47**) (250 mg, 0.991 mmol) in DMF (6.0 mL) at 0°C, under nitrogen and after 45 min a solution of 1-fluoro-4-nitronaphthalene (**14**) (189 mg, 0.991 mmol) in DMF (6.0 mL) was added dropwise, over 2 min. The resulting dark-brown reaction mixture was stirred at 0°C for 5 min, and was then warmed to RT. After 70 min, a saturated aq solution of NH₄Cl (3.0 mL) was added and the mixture was partitioned between water and EtOAc. The aq layer was extracted twice with EtOAc and the combined organic extracts were washed with brine, dried (MgSO₄) and evaporated *in vacuo.* The residue was purified by flash column chromatography (SiO₂, 40 g; 0-70% EtOAc in isohexane, gradient elution) to afford *tert-butyl* 4-(2-(4-nitronaphthalen-1-yloxy)propyl)pyridin-2-ylcarbamate (**48**) (293 mg, 65%) as an orange foam: m/z 424.0 (M+H)⁺ (ES⁺).

A solution of (**48**) (271 mg, 0.614 mmol) in MeOH (30 mL) was passed through a Thales H-cube (1.0 mL.min⁻¹, 30°C, 55 mm, 10% Pt/C Cat-Cart, full hydrogen mode) and the volatiles were removed *in vacuo* to afford *tert-butyl* 4-(2-(4-aminonaphthalen-1-yloxy)propyl)pyridin-2-ylcarbamate (**49**) (241 mg, 100%) as a purple foam: m/z 394.0 (M+H)⁺ (ES⁺).

A solution of (**5**) (212 mg, 0.923 mmol) in DCM (1 mL) was added to a stirred solution of CDI (150 mg, 0.923 mmol) in DCM (1 mL), under nitrogen at RT over 5 min. After 1.5 hr a solution of (**49**) (242 mg, 0.615 mmol) in DCM (2 mL) was added and stirring was continued at RT for 16 hr. A second portion of 3-*tert*-butyl-1-*p*-tolyl-1*H*-pyrazol-5-amine (141 mg, 0.615 mmol) in DCM (1 mL) was processed in a similar manner to the first, by reaction with CDI (100 mg, 0.615 mmol) in DCM (1 mL) and the resulting adduct was then added to the reaction mixture. After a further 2.5 hr the mixture was partitioned between water and DCM. The aqueous layer was extracted with DCM and the combined organic extracts were washed with brine and then dried (MgSO₄) and evaporated *in vacuo.* The residue was purified by flash column chromatography (SiO₂, 40 g, 0-100% EtOAc in isohexane, gradient elution) to afford *tert-*butyl 4-(2-(4-(3-(3-*tert*-butyl-1-*p*-tolyl-1*H*-pyrazol-5-yl)ureido)naphthalen -1-yloxy)propyl)pyridin-2-ylcarbamate (**50**) (163 mg, 40%) as a purple foam: m/z 649 (M+H)⁺ (ES⁺).

To a stirred solution of the *tert-butyl* carbamate (**50**) (158 mg, 0.244 mmol) in DCM (4.0 mL) at 0°C under nitrogen was added TFA (2.0 mL). After 5 min the reaction mixture was warmed to RT and stirred for a further 2 hr. The mixture was evaporated *in vacuo* and the residue was subjected to SCX capture and release to afford the title compound, **Intermediate J,** (138 mg, >100%): m/z 549 (M+H)⁺ (ES⁺).

### Example 46: N-(4-(2-(4-(3-(3-tert-Butyl-1-p-tolyl-1H-pyrazol-5-yl)ureido)naphthalen-1-yloxy)propyl)pyridin-2-yl)-2-methoxyacetamide:

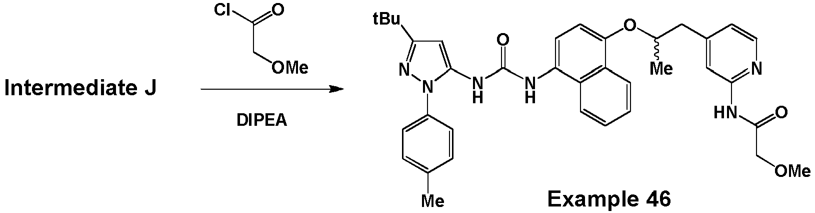

Methoxyacetyl chloride (17 µL, 0.18 mmol) was added dropwise to a stirred solution of **Intermediate J** (40 mg, 0.073 mmol) and DIPEA (38 µL, 0.22 mmol) in DCM (3 mL) at 0°C under nitrogen. After 20 min the reaction mixture was warmed to RT. After a further 4 hr a solution of NH₃ (1% in MeOH, 3 mL) was added and stirring continued for 1 hr. The reaction mixture was evaporated *in vacuo* and the residue was subjected to SCX capture and release and was then purified by flash column chromatography (SiO₂, 12 g, 0-5% MeOH in DCM, gradient elution) to give the title compound, **Example 46,** (5.6 mg, 12%): m/z 621 (M+H)⁺ (ES⁺). ¹H NMR (400 MHz, CDCl₃) δ: 1.31 (9H, s), 1.45 (3H, d), 2.22 (3H, s), 3.06 (1H, dd), 3.20 (1H, dd), 3.47 (3H, s), 3.95 (2H, s), 4.86 (1H, m), 6.42 (1H, s), 6.50 (1H, br s), 6.56 (1H, br s), 6.71 (1H, d), 6.89 (2H, br d), 6.97 (2H, br d), 7.00 (1H, dd) 7.31 (1H, d), 7.49 (2H, m), 7.81 (1H, br m), 8.17 (1H, d), 8.19 (1H, brs), 8.26 (1H, m), 8.83 (1H, brs).

### Intermediate K: 1-(4-(2-(2-Aminopyridin-4-yl)propoxy)naphthalen-1-yl)-3-(3-tert-butyl-1-p-tolyl-1H-pyrazol-5-yl)urea

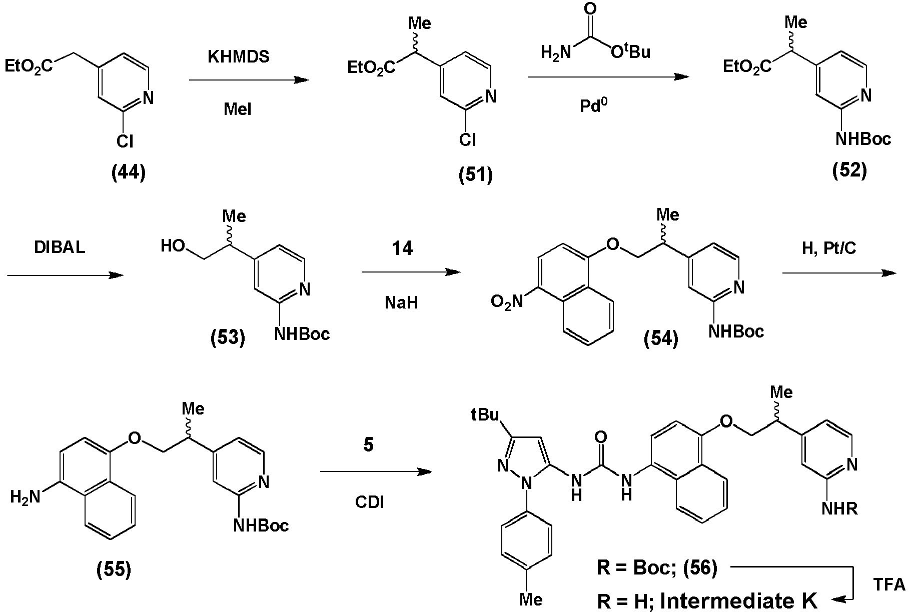

To a stirred solution of ethyl 2-(2-chloropyridin-4-yl)acetate (**44**) (2.5 g, 12.52 mmol) in THF (25 mL), under nitrogen at -78 °C was added a solution of KHMDS (0.5M in toluene, 26.3 mL, 13.2 mmol). The mixture was warmed to RT for 10 min, then re-cooled to -78 °C and methyl iodide (0.820 mL, 13.15 mmol) added in a single portion and the mixture allowed to warm to RT. Saturated aq. NH₄Cl solution was added and the mixture was diluted with ether. The organic layer was washed with water and brine and then dried and evaporated *in vacuo.* The residue was purified by flash column chromatography (SiO₂, 100g, 5-10% EtOAc in isohexane, gradient elution) to afford ethyl 2-(2-chloropyridin-4-yl)propanoate (**51**) (1.57 g, 56%): m/z 214 (M+H)⁺ (ES⁺).

A mixture of (**51**) (1.55 g, 7.25 mmol), *tert*-butylcarbamate (2.55 g, 21.76 mmol), Pd₂dba₃ (0.166 g, 0.181 mmol), caesium carbonate (3.55 g, 10.88 mmol) and Xantphos (0.210 g, 0.363 mmol) in THF (10 mL) was purged with nitrogen and heated at 65°C for 48 hr. The mixture was then cooled to RT, diluted with water and extracted with ether. The ether layer was washed with water and brine and then dried and evaporated *in vacuo.* The residue was purified by flash column chromatography (SiO₂, 100g, 5-10% EtOAc in isohexane, gradient elution) to afford ethyl 2-(2-(*tert*-butoxycarbonylamino)pyridin-4-yl)propanoate (**52**) (1.35 g, 61%): m/z 295 (M+H)⁺ (ES⁺)

A solution of DIBAL (1M in DCM, 13.8 mL, 13.8 mmol) was added dropwise over 10 min to a stirred solution of the ester (**52**) (1.35 g, 4.59 mmol) in THF (25 mL) under nitrogen at -78 °C. The reaction mixture was warmed to RT and then re-cooled to -78 °C and a second aliquot of DIBAL (1M in DCM, 4.5 mL, 4.5 mmol) was added. The reaction mixture was allowed to warm to RT, then cooled to 0°C and water (5 mL) and then MgSO₄ were added. The mixture was diluted with DCM and filtered to remove the solids. The filter cake was washed with EtOAc, MeOH and DCM and the combined filtrate and washings were evaporated *in vacuo.* The residue was purified by flash column chromatography (SiO₂, 40g, 25-50% EtOAc in isohexane, gradient elution) to afford *tert-butyl* 4-(1-hydroxypropan-2-yl)pyridin-2-ylcarbamate (**53**) (0.62 g, 50%): m/z 253 (M+H)⁺ (ES⁺).

To a solution of the alcohol (**53**) (0.62 g, 2.46 mmol) in DMF (4.0 mL) was added sodium hydride (60% dispersion in mineral oil, 0.246 g, 6.14 mmol) in a single portion and the mixture sonicated under a flow of nitrogen and then stirred at RT for 30 min. The resulting yellow suspension was cooled to 0°C and a solution of 1-fluoro-4-nitronaphthalene (**14**) (470 mg, 2.46 mmol) in DMF (2.0 mL) was added over 10 min. The reaction mixture was warmed to RT and glacial acetic (1.0 mL) was added. The mixture was poured onto saturated aq NaHCO₃ solution and extracted with EtOAc. The organic layer was washed with saturated aq Na₂CO₃, solution, three times with water and twice with brine then dried (MgSO₄) and evaporated *in vacuo* to give a yellow solid. The solid was suspended in MeOH (20 mL) and sonicated and the insoluble residue was collected by filtration and was washed with MeOH (10 mL) and then ether to afford *tert-butyl* 4-(1-(4-nitronaphthalen-1-yloxy)propan-2-yl)pyridin-2-ylcarbamate (**54**) as an off white solid (0.73 g, 67%): m/z 424 (M+H)⁺ (ES⁺).

A solution of the nitroarene (**54**) (0.61 g, 1.441 mmol) in a mixture of MeOH (20.0 mL), AcOH (5.0 mL) and EtOAc (10.0 mL) was passed through a Thales H-Cube (1.0 mL.min⁻¹, 45°C, 55 mm, 10% Pt/C Cat-Cart, full hydrogen mode). The solvent was evaporated *in vacuo* and the residue was partitioned between saturated aq NaHCO₃ solution and EtOAc. The organic layer was washed with water and brine and then dried and evaporated *in vacuo* to furnish *tert-*butyl 4-(1-(4-aminonaphthalen-1-yloxy)propan-2-yl)pyridin-2-ylcarbamate (**55**) (610 mg, 97%): m/z 394 (M+H)⁺ (ES⁺).

A solution of (**5**) 612 mg, 2.67 mmol) in DCM (2.0 mL) was added dropwise over 1.5 hr to a suspension of CDI (433 mg, 2.67 mmol) in DCM (2.0 mL) under nitrogen and the mixtre was stirred at RT for 1 hr. A solution of the amine (**55**) (700 mg, 1.78 mmol) in DCM (4.0 mL) was added in a single portion and the reaction mixture was stirred for 16 hr, during which time a precipitate formed. The mixture was taken up in DCM (10 mL) and was purified by flash column chromatography (SiO₂, 80g, 0-20% EtOAc in isohexane, gradient elution) to afford *tert-butyl* 4-(1-(4-(3-(3-*tert*-butyl-1-*p*-tolyl-1*H*-pyrazol-5-yl)ureido)naphthalen-1-yloxy)propan-2-yl)pyridin-2-ylcarbamate (**56**) (590 mg, 50%): m/z 649 (M+H)⁺ (ES⁺)

To a suspension of the *tert-butyl* carbamate (**56**) (0.57 g, 0.88 mmol) in DCM (10.0 mL) was added TFA (5.0 mL) and the resulting dark green solution stirred at RT for 1 hr. The mixture was evaporated *in vacuo* and the residue was dissolved in MeOH (10.0 mL) and subjected to SCX capture and release to afford the title compound, **Intermediate K,** as a red oil (488 mg, 98%): m/z 549 (M+H)⁺ (ES⁺).

### Example 47: N-(4-(1-(4-(3-(3-tert-Butyl-1-p-tolyl-1H-pyrazol-5-yl)ureido)naphthalen-1-yloxy)propan-2-yl)pyridin-2-yl)-2-methoxyacetamide:

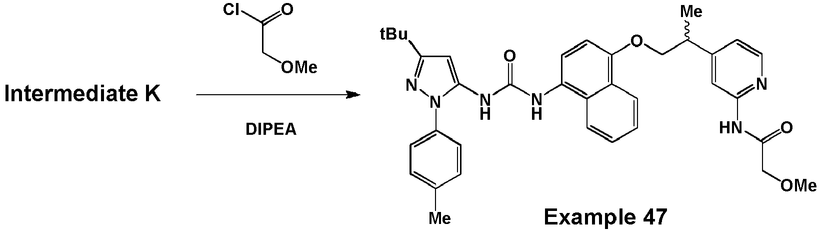

To a stirred solution of **Intermediate K** (58 mg, 0.106 mmol) in DCM (2.0 mL) was added methoxyacetyl chloride (9.7 µL, 0.106 mmol) followed by DIPEA (18.4 µL, 0.106 mmol) and the mixture stirred at RT for 1 hr. A solution of NH₃ (1% in MeOH, 3.0 mL) was added and the mixture was stirred for 30 min and was then evaporated *in vacuo.* The residue was subjected to SCX capture and release and the product was triturated with MeCN (5.0 mL) and was washed with MeCN (5.0 mL) and then ether (5.0 mL) to afford the title compound (**Example 47**) as a white solid (32 mg, 48%): m/z 621 (M+H)⁺ (ES⁺); ¹H NMR (400 MHz, DMSO-d₆) δ: 1.27 (9H, s), 1.42 (3H, d), 2.39 (3H, s), 3.38 (3H, s), 3.40 (1H, m), 4.07 (2H, s), 4.21-4.30 (2H, overlapping m), 6.35 (1H, s), 6.95 (1H, d), 7.22 (1H, dd), 7.35 (2H, d), 7.40-7.47 (3H, overlapping m), 7.54 (1H, m), 7.60 (1H, d), 7.86 (1H, d), 8.07 (1H, d), 8.20 (1H, br s), 8.25 (1H, d), 8.55 (1H, br s), 8.75 (1H, d), 9.94 (1H, br s).

### Intermediate L: 1-(4-(2-(2-aminopyridin-4-yl)-2-methylpropoxy)naphthalen-1-yl)-3-(3-tert-butyl-1-p-tolyl-1H-pyrazol-5-yl)urea.

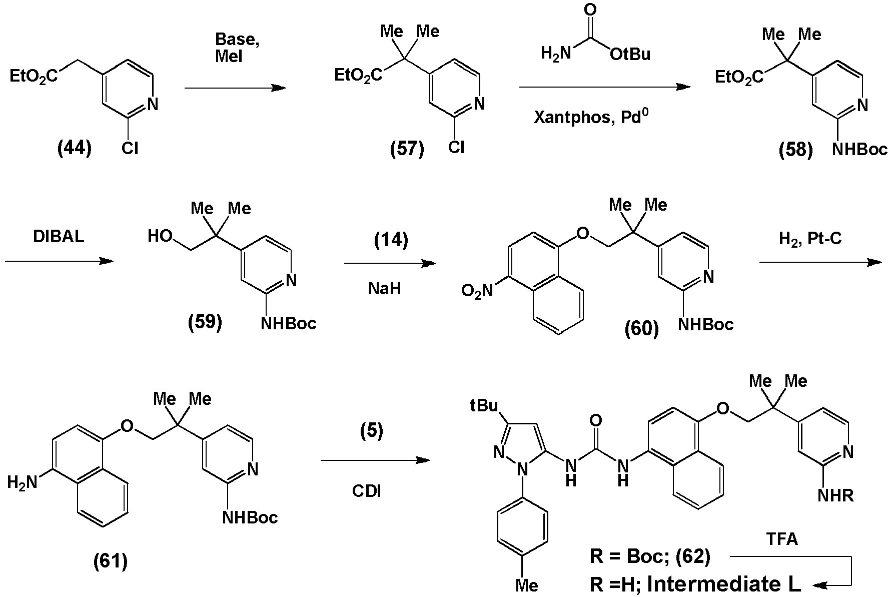

To a stirred solution of ethyl 2-(2-chloropyridin-4-yl)acetate (**44**) (3.47 g, 17.4 mmol) in THF (40 mL), under nitrogen at -78°C was added a solution of KHMDS (36.5 mL, 0.5M in toluene, 18.25 mmol) in a single portion The mixture was warmed to RT for 10 min and then re-cooled to -78 °C, and treated with methyl iodide (1.14 mL, 18.3 mmol). The mixture was warmed to RT during which time a precipitated was thrown down. The mixture was cooled to -78 °C and a further aliquot of the KHMDS solution (36.5 mL, 0.5M in toluene, 18.25 mmol) was added. The mixture was warmed to RT for 10 min and the suspension then re-cooled to -78°C, and a second aliquot of methyl iodide (1.1 mL, 18.3 mmol) was added. The mixture was warmed to RT and sodium hydride (0.730 g, 18.25 mmol) was added and the mixture stirred at this temperature for 1 hr and then a third aliquot of methyl iodide (1.1 mL, 18 3mmol) was added. After 1 hr, a saturated aq solution of NH₄Cl was added and the mixture was extracted with ether. The combined organic layers were washed with water and brine and then dried and evaporated *in vacuo.* The residue was purified by flash column chromatography (SiO₂, 100g, 5-10% EtOAc in isohexane, gradient elution) to afford ethyl 2-(2-chloropyridin-4-yl)-2-methylpropanoate (**57**) (2.81 g, 67%): m/z 228 (M+H)⁺ (ES⁺).

A mixture of (**57**) (2.80 g, 12.3 mmol), *tert*-butylcarbamate (4.32 g, 36.9 mmol), Pd₂(dba)₃ (281 mg, 0.307 mmol), Xantphos (355 mg, 0.615 mmol) and caesium carbonate (6.01 g, 18.5 mmol) in THF (10.0 mL) was purged with nitrogen and then stirred at 65°C for 72 hr. The mixture was cooled to RT and was diluted with water and extracted with ether. The organic layer was washed with water and brine and was then dried, and evaporated *in vacuo.* The residue was purified by flash column chromatography (SiO₂, 120g, 5-8% EtOAc in isohexane, gradient elution) to give ethyl 2-(2-(*tert*-butoxycarbonylamino)pyridin-4-yl)acetate (**58**) (1.47 g, 37%): m/z 309 (M+H)⁺ (ES⁺).

To a stirred solution of the ester (**58**) (1.43 g, 4.64 mmol) in THF (25 mL) at -78°C under nitrogen was added a solution of DIBAL (18.5 mL, 1M in DCM, 18.5 mmol) dropwise over 10 min. The reaction mixture was warmed to RT then cooled to 0 °C and water (5.0 mL) was added, followed by MgSO₄. The mixture was diluted with DCM and filtered and the filter cake was washed consecutively with EtOAc, MeOH and DCM. The filtrate and washings were combined, and evaporated *in vacuo* and the residue was purified by flash column chromatography (SiO₂, 40 g, 25-50% EtOAc in isohexane, gradient elution) to yield *tert-butyl* 4-(1-hydroxy-2-methylpropan-2-yl)pyridin-2-ylcarbamate (**59**) (1.13 g, 86%): m/z 267 (M+H)⁺ (ES⁺).

To a stirred solution of the alcohol (**59**) (1.11 g, 4.17 mmol) in DMF (10.0 mL) at 0°C under nitrogen was added sodium hydride (350 mg, 8.75 mmol) in a single portion. The mixture was warmed to RT for 30 min and was then sonicated, flushed with nitrogen and re-cooled to 0°C. A solution of 1-fluoro-4-nitronaphthalene (**14**) (797 mg, 4.17 mmol) in DMF (4.0 mL) was added over 5 min and the reaction mixture allowed to warm to RT and after 30 min glacial acetic acid (1.0 mL) was added. The reaction mixture was poured onto saturated aq NaHCO₃ solution and extracted twice with EtOAc. The combined organic extracts were washed three times with water and with brine and then dried (MgSO₄), and evaporated *in vacuo.* The residue was triturated from EtOAc and washed with ether (20 mL) to afford *tert-butyl* 4-(2-methyl-1-(4-nitronaphthalen-1-yloxy)propan-2-yl)pyridin-2-ylcarbamate (**60**) as a yellow solid (1.23 g, 64 %): m/z 438 (M+H)⁺ (ES⁺).

A solution of the nitroarene (**60**) (1.15 g, 2.63 mmol) in a mixture of MeOH (40 mL), AcOH (10 mL) and DCM (20 mL) was passed through a Thales H-cube (1.0 mL.min⁻¹, 45°C, 55 mm 10% Pt/C Cat-Cart, full hydrogen mode). The solvent was evaporated *in vacuo* and the residue subjected to SCX capture and release to furnish *tert-butyl* 4-(1-(4-aminonaphthalen-1-yloxy)-2-methylpropan-2-yl)pyridin-2-ylcarbamate (**61**) (1.15 g, 84%): m/z 408 (M+H)⁺ (ES⁺).

A solution of (**5**) (760 mg, 3.31 mmol) in DCM (2.0 mL) was added dropwise over 1.5 hr to a suspension of CDI (537 mg, 3.31 mmol) in DCM (2.0 mL) under nitrogen and the solution was stirred at RT for 1 hr. A solution of the naphthylamine (**61**) (1.00 g, 2.21 mmol) in DCM (4.0 mL) was added in a single portion and the solution was stirred for 16 hr, during which time a precipitate formed. The reaction mixture was taken up in DCM (10 mL) and purified by flash column chromatography (SiO₂, 80 g, 20-80% EtOAc in isohexane, gradient elution) to afford *tert-*butyl 4-(1-(4-(3-(3-*tert*-butyl-1-*p*-tolyl-1*H*-pyrazol-5-yl)ureido)naphthalen-1-yl oxy)-2-methylpropan-2-yl)pyridin-2-ylcarbamate (**62**) (780 mg, 52%): m/z 663 (M+H)⁺ (ES⁺)

To a suspension of (**62**) (780 mg, 1.18 mmol) in DCM (10 mL) was added TFA (8.0 mL) and the resulting dark green solution stirred at RT for 2 hr. The mixture was evaporated *in vacuo* and the residue was taken up in MeOH (10 mL) and subjected to SCX capture and release to afford the title compound, **Intermediate L,** (690 mg, 100%): m/z 563 (M+H)⁺ (ES⁺).

### Example 48: N-(4-(1-(4-(3-(3-tert-Butyl-1-p-tolyl-1-pyrazol-5-yl)ureido)naphthalen-1-yloxy)-2-methylpropan-2-yl)pyridin-2-yl)-2-methoxyacetamide:

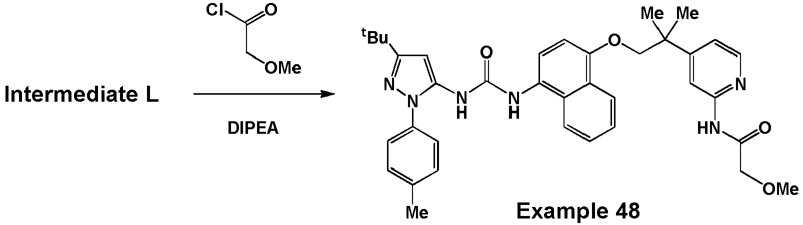

To a solution of **Intermediate L** (52 mg, 0.092 mmol) in DCM (2.0 mL) was added methoxyacetyl chloride (8.5 µL, 0.092 mmol) followed by DIPEA (16. µL, 0.092 mmol) and the mixture was stirred at RT for 1 hr. A solution of NH₃ (1% in MeOH, 3.0 mL) was added and the mixture was stirred for 30 min and was then evaporated *in vacuo.* The residue was purified by flash column chromatography (SiO₂,12g, 0-100% EtOAc in isohexane, gradient elution) to afford the title compound, **Example 48,** (32 mg, 53%): m/z 635 (M+H)⁺ (ES⁺); ¹H NMR (400 MHz, DMSO-d₆) δ 1.27 (9H, s), 1.50 (6H, s), 2.39 (3H, s), 3.37 (3H, s), 4.06 (2H, s), 4.19 (2H, s), 6.35 (1H, s), 6.95 (1H, d), 7.30 (1H, dd), 7.34 (2H, m), 7.43 (2H, m), 7.45 (1H, m), 7.53 (1H, m), 7.61 (1H, d), 7.87 (1H, d), 8.02 (1H, dd), 8.25 (1H, d), 8.34 (1H, br s) 8.54 (1H, brs), 8.75 (1H, br s), 9.90 (1H, br s).

### Intermediate M: N-(4-((4-aminonaphthalen-1-yloxy)methyl)pyridin-2-yl)-2-methoxy acetamide.

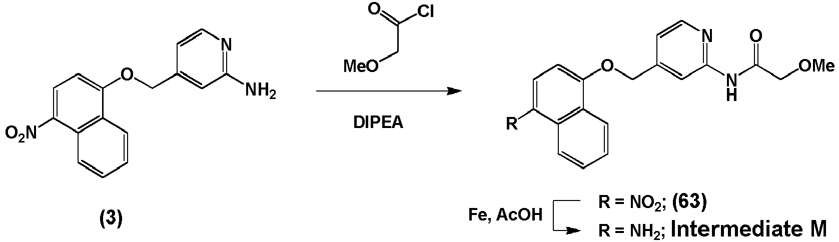

Methoxyacetyl chloride (24.2 mL, 264 mmol) was added dropwise over 10 min to a stirred suspension of the amine (3) (60.0 g, 203 mmol) in a mixture of DCM (300 mL), THF (450 mL) and DIPEA (53.1 mL, 305 mmol) under nitrogen at -5°C. After 10 min the reaction mixture was allowed to warm to RT during which time a dark solution formed. After 30 min a solution of NH₃ in MeOH (7M, 30 mL) was added and stirring continued for 1 hr during which time a precipitate formed. The suspension was evaporated *in vacuo* and the residue was triturated with water (500 mL). The precipitate was collected by filtration, and washed with water (300 mL) and diethyl ether (500 mL) and was then dried *in vacuo* at 60°C to furnish 2-methoxy-*N*-(4-((4-nitronaphthalen-1-yloxy)methyl)pyridin-2-yl)acetamide (**63**) as a yellow solid (68 g, 88%): m/z 368 (M+H)⁺ (ES⁺).

A suspension of the nitroarene (**63**) (30.0 g, 82.0 mmol) and iron powder (22.8 g, 408 mmol) in AcOH (300 mL) was heated at 50°C for 1.5 hr, and was then cooled to RT. Solid sodium carbonate was added portionwise to the reaction mixture until effervescence was no longer observed. The mixture was extracted with ethyl acetate (2 x 700 mL) and the combined organic extracts were washed with saturated aqueous sodium carbonate and with brine and then dried (MgSO₄). Evaporation *in vacuo* furnished the title compound, **Intermediate M,** as a brown solid (25.0 g, 77%): m/z 338 (M+H)⁺ (ES⁺).

### Intermediate N: 3-tert-Butyl-1-(4-((tert-butyldimethylsilyloxy)methyl)phenyl)-1H-pyrazol-5-amine.

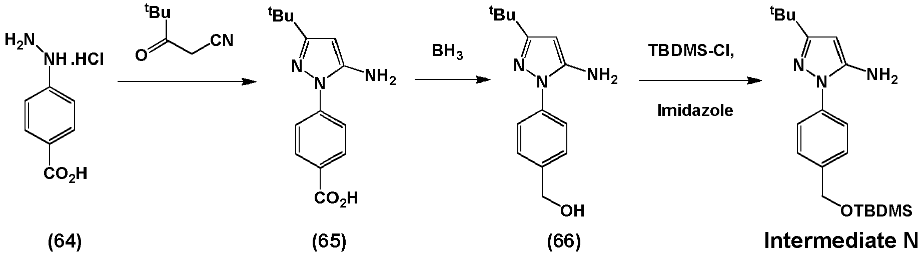

To a suspension of 4-hydrazinobenzoic acid (**64**) (1.00 g, 5.30 mmol) in EtOH (20 mL) containing conc hydrochloric acid (0.5 mL) was added pivaloylacetonitrile (0.730 g, 5.83 mmol) and the mixture heated to reflux for 5 hr. The reaction mixture was stirred at RT for 64 hr and the solvent evaporated *in vacuo.* The residue was suspended in THF and an aqueous solution of LiOH (1M, 30 mL, 30 mmol) was added and the mixture was stirred at RT for 2 hr. The THF was removed by evaporation *in vacuo* and the resulting aqueous solution was diluted with AcOH and subjected to SCX capture and release. Fractions containing the desired compound were combined and evaporated *in vacuo.* The residue was dissolved in DCM and was dried (MgSO₄) and evaporated *in vacuo.* Co-evaporation with acetonitrile *in vacuo* furnished 4-(5-amino-3-tert-butyl-1H-pyrazol-1-yl)benzoic acid (**65**) as an orange solid (1.50 g, >100 % recovery): m/z 260 (M+H)⁺ (ES⁺); 258 (M-H)⁻ (ES-).

To a stirred solution of the benzoic acid (**65**) (1.50 g, 5.7 mmol) in THF at 0°C was added a solution of borane (2M in THF, 17.4 mL, 34.8 mmol). The reaction mixture was warmed to RT and was stirred for 16 hr. An additional aliquot of the BH₃ solution (2M in THF, 8.0 mL, mmole) was added and stirring continued for a further 3 hr. The reaction mixture was cooled to 0°C and 1M hydrochloric acid was added to quench the reaction. The solution was neutralized and extracted into EtOAc. The extracts were washed with aq Na₂CO₃ solution and brine and then dried and evaporated *in vacuo* to afford (4-(5-amino-3-*tert*-butyl-1*H*-pyrazol-1-yl)phenyl)methanol (**66**) (0.64 g, 45 %).

To a stirred solution of the benzyl alcohol (**66**) (640 mg, 2.61 mmol) and imidazole (266 mg, 3.91 mmol) in DMF (5.0 mL) at RT was added TBDMS-Cl (590 mg, 3.91 mmol). After 3 hr the reaction mixture was diluted with water and extracted with Et₂O. The organic layer was washed with brine, dried (MgSO₄) and then evaporated *in vacuo* to afford the title compound, **Intermediate N,** (875 mg, 89%): m/z 360 (M+H)⁺ (ES⁺).

### Example 49: N-(4-((4-(3-(3-tert-Butyl-1-(4-(hydroxymethyl)phenyl)-1H-pyrazol-5-yl) ureido)naphthalen-1-yloxy)methyl)pyridin-2-yl)-2-methoxyacetamide:

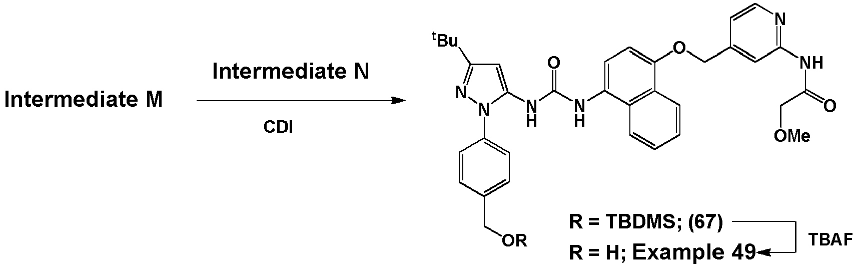

The pyrazole amine **Intermediate N** (100 mg, 0.278 mmol) was added portionwise to a stirred suspension of CDI (45.1 mg, 0.278 mmol) in DCM (0.5 mL) over 1hr and the mixture was stirred at RT for 16 hr. A solution of, **Intermediate M,** (47 mg, 0.139 mmol) in DCM (0.5 mL) was added dropwise over 2 hr and after a further 2hr the mixture was evaporated *in vacuo* and the residue was purified by flash column chromatography (SiO₂, 12g, 30-70% EtOAc in isohexane, gradient elution) to yield *N*-(4-((4-(3-(3-*tert*-butyl-1-(4-((*tert*-butyl dimethylsilyloxy)methyl)phenyl)-1*H*-pyrazol-5-yl)ureido)naphthalen-1-yloxy)methyl)pyridin-2-yl)-2-methoxyacetamide (**67**) (42 mg, 20 %): m/z 723 (M+H)⁺ (ES⁺).

To a solution of the silyl ether (**67**) (42 mg, 58 µmol) in THF under nitrogen at -5°C was added a solution of TBAF (1M in THF, 58 µL, 58 µmol). The mixture was warmed to RT and a second aliquot of TBAF (1M in THF, 58 µL, 58 µmol) was added. After 1 hr the mixture was diluted with EtOAc and washed with saturated aq ammonium chloride. The aq layer was extracted with EtOAc and the combined organic layers were washed with water and brine and then dried and evaporated *in vacuo.* The residue was purified by flash column chromatography (SiO₂, 12 g, 2-6% MeOH in DCM, gradient elution) to provide the title compound, **Example 49,** (23 mg, 63%): m/z 609 (M+H)⁺ (ES⁺); ¹H NMR (400 MHz, DMSO-d₆) δ: 1.28 (9 H, s), 3.37 (3H, s) 4.08 (2H, s), 4.59 (2H, d), 5.32 (1H, t), 5.39 (2H, s), 6.36 (1H, s), 7.02 (1H, d), 7.29 (1H, dd), 7.48 (2H, m), 7.52 (2H, m), 7.54-7.63 (3H, overlapping m), 7.93 (1H, m), 8.30-8.37 (3H, overlapping m), 8.63 (1H, s), 8.81 (1H, s), 10.02 (1H, br s).

### Intermediate P: 1-(4-((2-Aminopyrimidin-4-yl)methoxy)naphthalen-1-yl)-3-(3-tert-butyl-1-p-tolyl-1H-pyrazol-5-yl)urea.

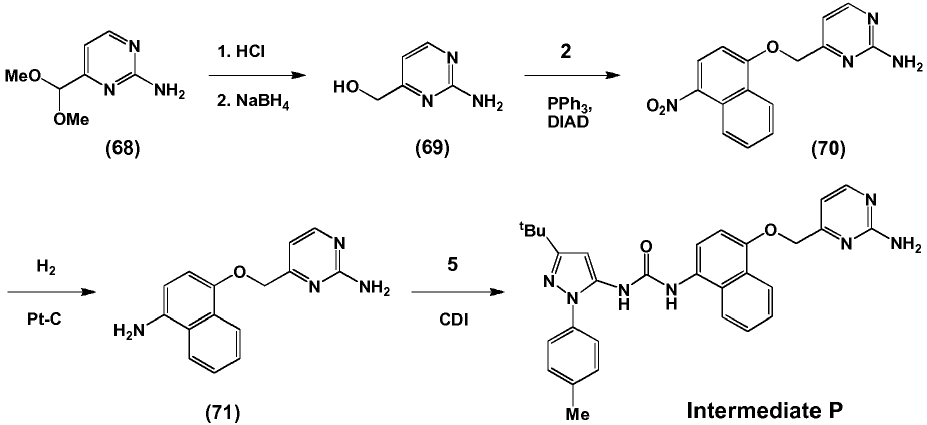

Hydrochloric acid (2M, 207 mL, 414 mmol) was added to 4-(dimethoxymethyl)pyrimidin-2-amine (**68**) (WO 2007/096764) (14.0 g, 83 mmol) and the mixture heated at 48°C for 16 hr. The mixture was cooled to RT and was neutralized with solid Na₂CO₃ which produced a precipitate at pH 7. The suspension was diluted with EtOAc (300 mL) and the solid removed by filtration. The organic layer was separated and the aqueous layer was extracted with 1% MeOH in THF (4 x 300 mL). The organics were combined, dried and then evaporated *in vacuo.* The residue (ca. 4.0 g) was suspended in a mixture of MeOH (100 mL), THF (100 mL) and water (100 mL) and treated with NaBH₄ (1.57 g, 41.4 mmol). After stirring for 1 hr a solution of NaOH (1M, 20 mL) was added and the mixture was allowed to stand at RT for 48 hr. The solvents were evaporated to give a yellow solid which was partitioned between water (50 mL) and EtOAc (100 mL). The solid which formed at the interface was removed by filtration and the aq layer was extracted with THF (3 x 300 mL) then dried and evaporated to give a yellow solid. The material was suspended in THF (100 mL) and MeOH (50 mL) and absorbed onto silica gel (20 g) and subjected to column chromatography (80 g, 15% MeOH in DCM isocratic elution) to give (2-aminopyrimidin-4-yl)methanol (**69**) as an off-white solid (720 mg, 7%): m/z 126 (M+H)⁺ (ES⁺).

To a stirred mixture of (**69**) (700 mg, 3.92 mmol), 4-nitronaphthol (**2**) (741 mg, 3.92 mmol) and PPh₃ (1.23 g, 4.70 mmol) in THF (20 mL) under nitrogen at -50°C was added DIAD (996 µL, 4.70 mmol) dropwise over 5 min. The mixture was allowed to warm to RT and stirred for 1 hr during which time a yellow precipitate formed. The suspension was stirred overnight and the volatiles were evaporated *in vacuo.* The residue was triturated from MeOH (50 mL) and the pale yellow solid collected by filtration and washed with diethyl ether (50 mL) to give 4-((4-nitronaphthalen-1-yloxy)methyl)pyrimidin-2-amine (**70**) (1.10 g, 93 %): m/z 297 (M+H)⁺ (ES⁺).

A solution of the nitroarene (**70**) (1.10 g, 3.71 mmol) in a a mixture of DCM (50 mL) and AcOH (40 mL) was passed through a Thales H-cube (1.0 mL min⁻¹, 55 mm, 10% Pt/C, 40°C, full hydrogen mode). LC-MS analysis of the resulting solution showed a mixture comprising of mainly starting material and ca. 20% product. The DCM was removed by evaporation *in vacuo* and the solution re-subjected to reduction using the same conditions at RT. The volatiles were evaporated *in vacuo* to give 4-((4-aminonaphthalen-1-yloxy)methyl)pyrimidin-2-amine (**71**) (ca. 70% by LC-MS) as a purple solid (0.90 g, 64 % yield): m/z 267 (M+H)⁺ (ES⁺).

A solution of (**5**) (980 mg, 4.26 mmol) in DCM (4.0 mL) was added dropwise over 1 hr, to a suspension of CDI (690 mg, 4.26 mmol) in DCM (3.0 mL) and the mixture was stirred at RT for 2 hr. This solution was then added dropwise to a solution of the naphthylamine (**71**) (900 mg, 2.37 mmol) in DCM (10 mL), such that after each 1.0 mL aliquot was added, the reaction was stirred for 1 hr. The reaction was quenched with MeOH (20 mL), and silica was added (20 g) and the volatiles were evaporated *in vacuo.* The residue was subjected to purification by column chromatography (100 g, 50 to 100% EtOAc in isohexane, gradient elution). The resulting product was triturated from DCM (20 mL) and the solid collected, and washed with diethyl ether (50 mL) to give the title compound, **Intermediate P,** as a purple solid (480 mg, 38 %): m/z 523 (M+H)⁺ (ES⁺).

### Example 50: N-(4-((4-(3-(3-tert-Butyl-1-p-tolyl-1H-pyrazol-5-yl)ureido)naphthalen-1-yloxy)methyl) pyrimidin-2-yl)-2-methoxyacetamide:

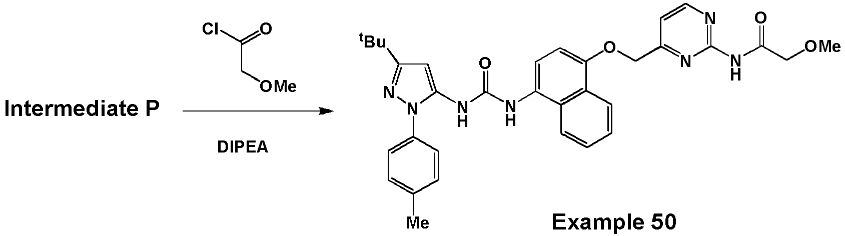

A suspension of **Intermediate P** (52 mg, 0.10 mmol) in DCM (1.0 mL) and DMF (100 µL) was treated with methoxyacetyl chloride (27 µL, 0.30 mmol) followed by DIPEA (52 µL, 0.30 mmol) and the mixture stirred overnight at RT. The volatiles were evaporated *in vacuo* and the residue suspended in a mixture of MeOH (2.0 mL) and AcOH (2.0 mL). The suspension was subjected to SCX capture and release under standard conditions. As only a low recovery of material was obtained the SCX cartridge was extracted with MeOH (50 mL) and the solvent evaporated, to give an off white solid. This was triturated from MeOH (1.0 mL) and diethyl ether (5.0 mL) to give the title compound, **Example 50,** as a white solid (12 mg, 19 %): m/z 594 (M+H)⁺ (ES⁺). ¹H NMR (400 MHz, DMSO-d₆) δ: 1.27 (9H, s), 2.40 (3H, s), 3.34 (3H, s), 4.24 (2H, s), 5.34 (2H, s), 6.35 (1H, s), 7.02 (1H, d), 7.35 (2H, d), 7.44 (3H, m), 7.60 (3H, m), 7.95 (1H, m), 8.36 (1H, m), 8.59 (1H, br s), 8.68 (1H, d), 8.80 (1H, br s), 10.44 (1H, br s).

### Intermediate Q: 1-(4-(2-Aminopyridin-4-yloxy)naphthalen-1-yl)-3-(3-tert-butyl-1-p-tolyl-1H-pyrazol-5-yl)urea.

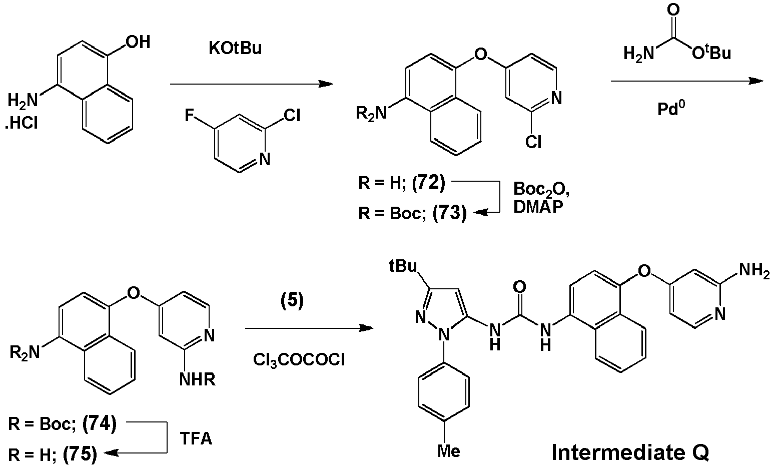

To a stirred solution of 2-chloro-4-fluoropyridine (1.26 g, 9.58 mmol) and 4-amino-1-naphthol hydrochloride (750 mg, 3.83 mmol) in NMP (40 mL), at -20°C, was added potassium *tert-*butoxide (1.290 g, 11.50 mmol). The reaction mixture was allowed to warm to RT and after 2.5 hr the reaction mixture was diluted with water (100 mL) and extracted with EtOAc (100 mL then 2 x 80 mL). The combined organic extracts were washed with brine (150 mL), dried and evaporated *in vacuo.* The crude product was subjected to SCX capture and release and the solvent was removed *in vacuo* to give 4-(2-chloropyridin-4-yloxy)naphthalen-1-amine (**72**) (1.02 g, 92%) as a brown solid: m/z 271 (M+H)⁺ (ES⁺).

To a stirred solution of (**72**) (1.02 g, 3.76 mmol) in THF (30 mL) at 0°C was added DMAP (0.034 g, 0.282 mmol) and di-*tert*-butyl dicarbonate (0.904 g, 4.14 mmol) and the reaction mixture stirred at 0°C for 30 min, and then at RT for 1.5 hr. The mixture was cooled to 0°C, and a further aliquot of di-*tert*-butyl dicarbonate (0.904 g, 4.14 mmol) was added and stirring continued at 0°C for 15 min and then at RT for 16 hr. The reaction mixture was diluted with water (40 mL) and extracted with EtOAc (2 x 40 mL). The combined organic extracts were washed with brine (75 mL), dried and evaporated *in vacuo.* The crude material was purified by flash column chromatography (SiO₂; 80 g, 0-40% EtOAc in isohexane, gradient elution) to give 4-(2-chloropyridin-4-yloxy)naphthalen-1-*N*,*N*-di-*tert*-butylcarbamate (**73**) (892 mg, 48%) as a purple solid: m/z 471 (M+H)⁺ (ES⁺).

A mixture of the chloropyridine (**73**) (892 mg, 1.89 mmol), *tert-butyl* carbamate (666 mg, 5.68 mmol), caesium carbonate (926 mg, 2.84 mmol), Pd₂(dba)₃ (43 mg, 0.047 mmol) and XantPhos (55 mg, 0.095 mmol) was suspended in THF (10 mL). The reaction mixture was purged with nitrogen, and was then heated at reflux for 15 hr. The mixture was cooled to RT, was diluted with water (35 mL) and extracted with EtOAc (35 mL, 25 mL). The combined organic extracts were washed with brine (50 mL), dried and evaporated *in vacuo.* The crude material was purified by flash column chromatography (SiO₂; 80 g, 0-30% EtOAc in isohexane, gradient elution) to give *tert*-butyl 4-(4-(*N*,*N*-di-*tert*-butylcarbamyl)naphthalen-1-yloxy)pyridin-2-ylcarbamate (**74**) (289 mg, 28%) as a white solid: m/z 552 (M+H)⁺ (ES⁺).

To a stirred solution of the bis *tert-butyl* carbamate (**74**) (289 mg, 0.524 mmol) in DCM (8.0 mL), at 0°C, was added TFA (4.0 mL). The resulting mixture was stirred and allowed to warm to RT. After 5 hr, the volatiles were removed *in vacuo* and the residue was taken up in MeOH (5 mL) and subjected to SCX capture and release. The solvent was removed *in vacuo* to afford 4-(4-aminonaphthalen-1-yloxy)pyridin-2-amine (**75**) (116 mg, 85%) as a brown-orange oil: m/z 252 (M+H)⁺ (ES⁺).

To a solution of (**5**) (206 mg, 0.900 mmol) in DCM (20 mL) was added a saturated aq. solution of NaHCO₃ (14 mL). The mixture was stirred vigorously, cooled to 0°C and trichloromethylchloroformate (0.326 mL, 2.70 mmol) was added in one portion. The reaction mixture was stirred vigorously at 0°C for a further 80 min. The layers were separated and the organic layer was dried, evaporated *in vacuo* and the resulting orange oil was dried further for 30 min under high vacuum. The resulting crude isocyanate was then taken up into THF (6.0 mL) and kept under nitrogen at 0°C. To a stirred solution of (**75**) (116 mg, 0.462 mmol) and DIPEA (241 µL, 1.385 mmol) in THF (3.0 mL), at 0°C, was added an aliquot of the isocyanate solution prepared above (2.0 mL, 0.30 mmol) and the resulting mixture vigorously stirred and alllowed to warm to RT. Two additional aliquots of the isocyanate solution were added to the reaction mixture, the first after 1.5 hr, (1.0 mL, 0.15 mmol) and the second after 3.5 hr (0.50 mL, 0.075 mmol). After a further 20 hr water (30 mL) was added and the mixture was extracted with EtOAc (2 x 30 mL). The combined organic extracts were washed with brine (50 mL) then dried and evaporated *in vacuo.* The residue was purified by flash column chromatography (SiO₂; 12 g, 25-100% [5% MeOH in EtOAc] in isohexane, gradient elution) to furnish the title compound, **Intermediate Q**, (127 mg, 49%) as a brown oil: m/z 507 (M+H)⁺ (ES⁺).

### Example 51: N-(4-(4-(3-(3-tert-Butyl-1-p-tolyl-1H-pyrazol-5-yl)ureido)naphthalen-1-yloxy)pyridin-2-yl)-2-(methylsulfonyl)acetamide:

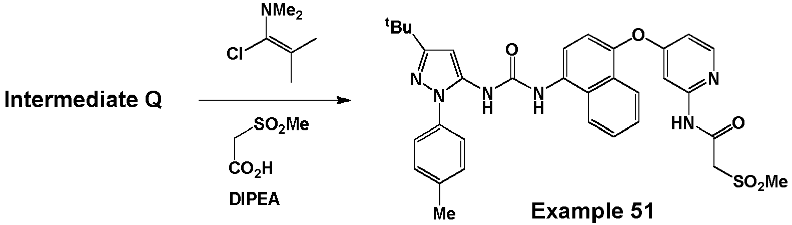

To a suspension of methanesulfonylacetic acid (40 mg, 0.29 mmol) in DCM (2.0 mL) under nitrogen was added 1-chloro-*N*,*N*,2-trimethylpropenylamine (48 µL, 0.37 mmol), and the mixture was stirred at RT for 2 hr. The resulting mixture was added to a solution of **Intermediate Q** (37 mg, 0.07 mmol) and DIPEA (51 µL, 0.29 mmol) in DCM (2.0 mL) and stirring continued at RT for 3 hr. The reaction mixture was treated with 1% NH₃ in MeOH (3.0 mL) for 45 min and was then evaporated *in vacuo.* The residue was subjected to SCX capture and release and was then purified by flash column chromatography (SiO₂, 12 g, 0-70% [5% MeOH in EtOAc] in isohexane, gradient elution) to afford the title compound, **Example 51,** as a white powder (13 mg, 28%): m/z 627 (M+H)⁺ (ES⁺). ¹H NMR (400 MHz, DMSO-d₆) δ: 1.29 (9H, s), 2.39 (3H, s), 3.10 (3H, s), 4.36 (2H, s), 6.40 (1H, s), 6.71 (1H, dd), 7.33-7.38 (3H, overlapping m), 7.47 (2H, m), 7.56 (1H, m), 7.65 (1H, m), 7.69 (1H, m), 7.85 (1H, d), 7.96 (1H, d), 8.11 (1H, d), 8.22 (1H, d), 8.91 (1H, brs), 9.23 (1H, brs), 10.96 (1H, brs).

### Example 52: 4-(4-(3-(3-tert-Butyl-1-p-tolyl-1H-pyrazol-5-yl)ureido)naphthalen-1-yloxy) pyridin-2-ylurea:

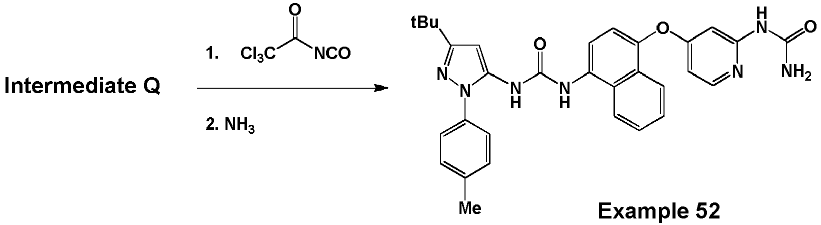

To a solution of **Intermediate Q** (50 mg, 0.099 mmol) in dry pyridine (1.5 mL) at 0°C under nitrogen was added trichloroacetyl isocyanate (15 µL, 0.12 mmol) and after 30 min at 0°C the reaction mixture was warmed to RT. After 24 hr a further aliquot of trichloroacetyl isocyanate (29 µL, 0.25 mmol) was added and after 42 hr the reaction was quenched by addition of 1% NH₃ in MeOH (2.0 mL). After an additional 30 min the resulting mixture was evaporated *in vacuo* and the residue was purified by flash column chromatography (SiO₂, 12 g, [5% MeOH in EtOAc] in isohexane, 0-75%, gradient elution) to afford the title compound, **Example 52** as a white solid (7 mg, 13%): R^{t} 2.21 min (Method 2); m/z 550 (M+H)⁺ (ES⁺); ¹H NMR (400 MHz, DMSO-*d₆*) δ: 1.28 (9H, s), 2.40 (3H, s), 6.41 (1H, s), 6.52 (1H, dd), 6.95 (1H, d), 7.31 (1H, d), 7.37 (2H, d), 7.45-7.47 (2H, m), 7.54-7.61 (1H, m), 7.62-7.67 (1H, m), 7.83 (1H, dd), 7.95 (1H, d), 8.06 (1H, d), 8.09 (1H, d), 8.81 (1H, s), 9.03 (1H, s), 9.15 (1H, s).

### Example 53: 1-(3-(tert-Butyl)-1-(p-tolyl)-1H-pyrazol-5-yl)-3-(4-((2-(3-methyl)ureido) pyridin-4-yl)oxy)naphthalen-1-yl)urea:

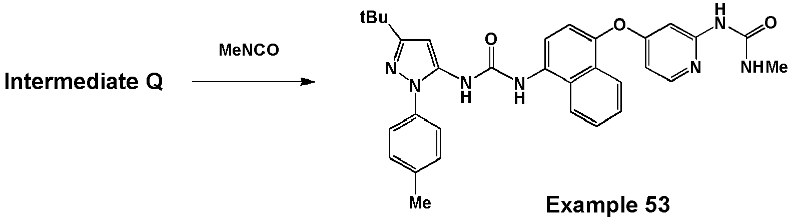

To a solution of **Intermediate Q** (50 mg, 0.099 mmol) in pyridine (1.0 mL) was added methyl isocyanate (50 µL, 0.806 mmol) and the mixture maintained at RT for 24 hr. Additional aliquots of methyl isocyanate (150 µL, 0.26 mmol) and pyridine (0.5 mL) were added and the reaction mixture maintained at RT for 96 hr and then partitioned between DCM (30 mL) and saturated aq. NaHCO₃ (10 mL). The aqueous layer was separated and extracted with DCM (10 mL) and the combined organic extracts were dried (MgSO₄) and evaporated *in vacuo.* The residue was purified by flash column chromatography (SiO₂, 12 g, MeOH in DCM, 0-10%, gradient elution) to afford the title compound, **Example 53** as a white solid (12 mg, 21%): R^{t} 5.24 min (Method 1 basic); m/z 564 (M+H)⁺ (ES⁺); ¹H NMR (400 MHz, DMSO-*d*₆) δ: 1.28 (9H, s), 2.39 (3H, s), 2.65 (3H, d), 6.40 (1H, s), 6.54 (1H, dd), 6.87 (1H, d), 7.31 (1H, d), 7.37 (2H, d), 7.46 (2H, m), 7.58 (1H, m), 7.64 (1H, m), 7.81 (1H, d), 7.88 (1H, br s), 7.95 (1H, d), 8.05 (1H, d), 8.08 (1H, d), 8.76 (1H, s), 9.09 (1H, s), 9.11 (1H, s).

### Example 54: 3-(4-((4-(3-(3-(tert-Butyl)-1-(p-tolyl)-1H-pyrazol-5-yl)ureido)naphthalen-1-yl)oxy)pyridin-2-yl)-1,1-dimethylurea:

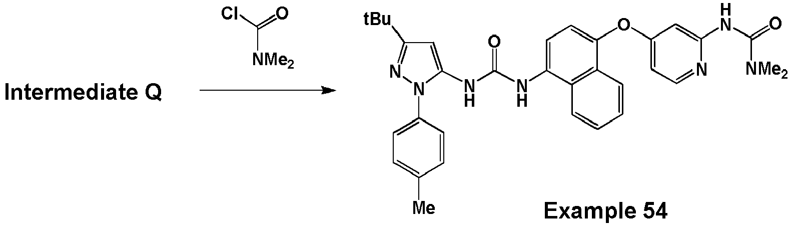

To a solution of **Intermediate Q** (50 mg, 0.099 mmol) and DIPEA (34 µL, 0.20 mmol) in dry pyridine (1.5 mL) was added dimethylcarbamoyl chloride (18 µL, 0.20 mmol) and the reaction mixture maintained at RT for 64 hr. The reaction was quenched by the addition of 1% NH₃ in MeOH (2.0 mL) and after 30 min the resulting mixture was evaporated *in vacuo.* The residue was purified by flash column chromatography (SiO₂, 12 g, MeOH in DCM, 0-5%, gradient elution; then SiO₂, 4 g, [5% MeOH in EtOAc] in isohexane, 50-90%, gradient elution) to afford the title compound, **Example 54** as an off-white solid (5 mg, 8%): R^{t} 5.15 min (Method 1 basic); m/z 578 (M+H)⁺ (ES⁺); ¹H NMR (400 MHz, DMSO-*d*₆) δ: 1.28 (9H, s), 2.40 (3H, s), 2.86 (6H, s), 6.41 (1H, s), 6.67 (1H, br s), 7.32 (2H, d), 7.37 (2H, d), 7.47 (2H, d), 7.58 (1H, t), 7.65 (1H, t), 7.84 (1H, d), 7.97 (1H, d), 8.09-8.13 (2H, m), 8.82 (1H, s), 9.00 (1H, br s), 9.15 (1H, s).

### Example 55: N-(4-(4-(3-(3-tert-Butyl-1-p-tolyl-1H-pyrazol-5-yl)ureido)naphthalen-1-yloxy)pyridin-2-yl)morpholine-4-carboxamide:

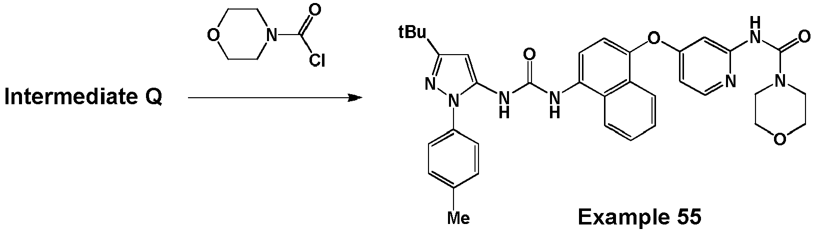

To a solution of **Intermediate Q** (50 mg, 0.099 mmol) and DIPEA (52 µL, 0.30 mmol) in dry pyridine (1.5 mL) under nitrogen at 0°C was added morpholine-4-carbonyl chloride (14 µL, 0.12 mmol) dropwise The reaction mixture was maintained at 0°C for 15 min, was warmed to 40°C for 4 hr and was then left at RT for 20 hr. The reaction mixture was reheated to 40°C for 3 hr and was then quenched by the addition of 1% NH₃ in MeOH (2.0 mL). After 45 min the resulting mixture was evaporated *in vacuo* and the residue was purified by flash column chromatography (SiO₂, 12 g, [5% MeOH in EtOAc] in isohexane, 0-80%, gradient elution) to afford the title compound, **Example 55** as a beige powder (16 mg, 25%): R^{t} 2.18 min (Method 2); m/z 620 (M+H)⁺ (ES⁺); ¹H NMR (400 MHz, DMSO-*d*₆) δ: 1.28 (9H, s), 2.40 (3H, s), 3.36 (4H, t), 3.53 (4H, t), 6.41 (1H, s), 6.61 (1H, dd), 7.31 (1H, d), 7.34-7.41 (3H, m), 7.46 (2H, d), 7.55-7.59 (1H, m), 7.63-7.68 (1H, m), 7.84 (1H, dd), 7.96 (1H, d), 8.08 (1H, d), 8.11 (1H, d), 8.80 (1H, s), 9.13 (1H, s), 9.25 (1H, s).

### Example 56: N-(4-((4-(3-(3-(tert-Butyl)-1-(p-tolyl)-1H-pyrazol-5-yl)ureido)naphthalen-1-yl)oxy)pyridin-2-yl)-4-methylpiperazine-1-carboxamide:

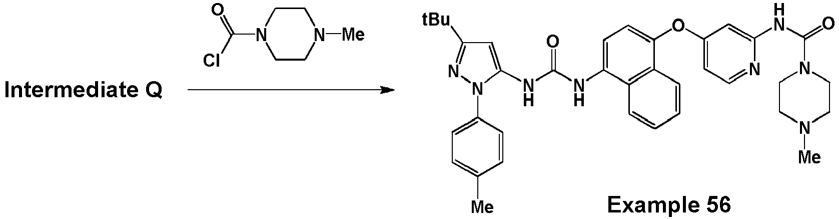

To a solution of **Intermediate Q** (70 mg, 0.138 mmol) and DIPEA (120 µL, 0.691 mmol) in dry pyridine (1.5 mL) was added 4-methylpiperazine-1-carbonyl chloride hydrochloride (138 mg, 0.691 mmol) and the mixture maintained at RT for 64 hr. The reaction was quenched by the addition of 1% NH₃ in MeOH (2.0 mL) and after 30 min the resulting mixture was evaporated *in vacuo* and the residue was taken up into EtOAc. The organic solution was washed with water and brine and was dried (MgSO₄) and evaporated *in vacuo.* The residue was purified by flash column chromatography (SiO₂, [7% NH₃ in MeOH] in DCM, 0-5%, gradient elution) to afford the title compound, **Example 56** as a pale brown solid (28 mg, 31%): R^{t} 5.15 min (Method 1 basic); m/z 633 (M+H)⁺ (ES⁺); ¹H NMR (400 MHz, DMSO-*d*₆) δ: 1.28 (9H, s), 2.15 (3H, s), 2.23 (4H, t), 2.40 (3H, s), 3.37 (4H, t), 6.41 (1H, s), 6.58 (1H, dd), 6.95 (1H, d), 7.31 (1H, d), 7.38 (2H, d), 7.46 (2H, d), 7.57 (1H, t), 7.65 (1H, t), 7.84 (1H, d), 7.95 (1H, d), 8.07-8.11 (2H, m), 8.79 (1H, s), 9.12 (1H, s), 9.19 (1H, s).

### Example 57: N-(3-(Imidazol-1-yl)propyl)-N'-4-(4-(3-(3-tert-butyl-1-p-tolyl-1H-pyrazol-5-yl)ureido)naphthalen-1-yloxy)pyridin-2-ylurea:

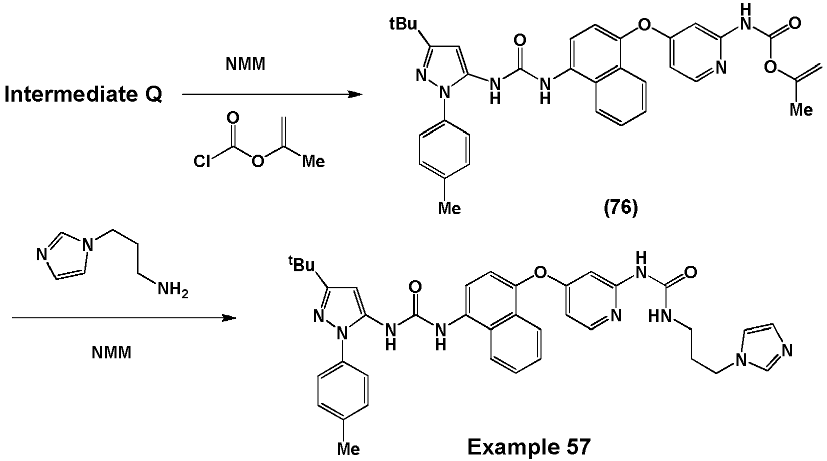

To a solution of **Intermediate Q** (1.1 g, 2.2 mmol) and *N*-methyl morpholine (290 µL, 2.6 mmol) in THF (20 mL) at -78°C was added dropwise a solution of prop-1-en-2-yl carbonochloridate (280 µL, 2.6 mmol) in THF (10 mL) and on completion of the addition the mixture was warmed to RT. After 16 hr an additional aliquot of *N*-methyl morpholine (290 µL, 2.6 mmol) was added, the reaction mixture was cooled to -78°C and a solution of prop-1-en-2-yl carbonochloridate (280 µL, 2.6 mmol) in THF (5 mL) was added. The reaction mixture was maintained at RT for a further 2 hr and was then quenched by the addition of NH₃ in MeOH (7 M, 4.0 mL). After 1 hr the resulting mixture was diluted with EtOAc (30 mL) and was washed with water (30 mL) and brine (30 mL) and then dried and evaporated *in vacuo.* The residue was triturated with ethyl acetate (20 mL) and was purified by flash column chromatography (SiO₂, EtOAc in DCM, 20-50%, gradient elution) to afford prop-1-en-2-yl 4-(4-(3-(3-*tert*-butyl-1-p-tolyl-1*H-*pyrazol-5-yl)ureido)naphthalen-1-yloxy)pyridin-2-ylcarbamate (**76**) as an off-white solid (0.60 g, 84% pure by HPLC, 40%) R^{t} 2.78 min (Method 2); m/z 591 (M+H)⁺ (ES⁺); 589 (M-H)⁻ (ES-).

To a solution of (**76**) (50 mg, 85 µmol) and *N*-methyl morpholine (1.0 µL, 9 µmol) in THF (5.0mL)) was added 3-(1*H*-imidazol-1-yl)propan-1-amine (10.6 mg, 85 µmol) and the reaction mixture heated to 55°C for 16 hr. The resulting mixture was evaporated *in vacuo* and the residue was purified by flash column chromatography (SiO₂, 4 g, MeOH in DCM, 2-5%, gradient elution then SiO₂, 12 g, 4% [1% NH₃ in MeOH] in DCM, isocratic elution) to afford the title compound, **Example 57,** as a purple solid (9 mg, 16%): R^{t} 5.37 min (Method 1 basic); m/z 658 (M+H)⁺ (ES⁺): ¹H NMR (400 MHz, DMSO-*d*₆) δ: 1.28 (9H, s), 1.85 (2H, m), 2.39 (3H, s), 3.06 (2H, m), 3.95 (2H, m), 6.40 (1H, s), 6.56 (1H, dd), 6.86 (1H, t), 6.89 (1H, d), 7.16 (1H, t), 7.31 (1H, d), 7.37 (2H, d), 7.46 (2H, d), 7.57 (1H, m), 7.60 (1H, t), 7.64 (1H, m), 7.81 (1H, d), 7.95 (1H, d), 8.06-8.10 (3H, overlapping m), 8.77 (1H, s), 9.07 (1H, s), 9.12 (1H, s).

### Example 58: N-(4-(4-(3-(3-tert-Butyl-1-p-tolyl-1H-pyrazol-5-yl)ureido)naphthalen-1-yloxy)pyridin-2-yl)-2-(2-methoxyacetamido)acetamide:

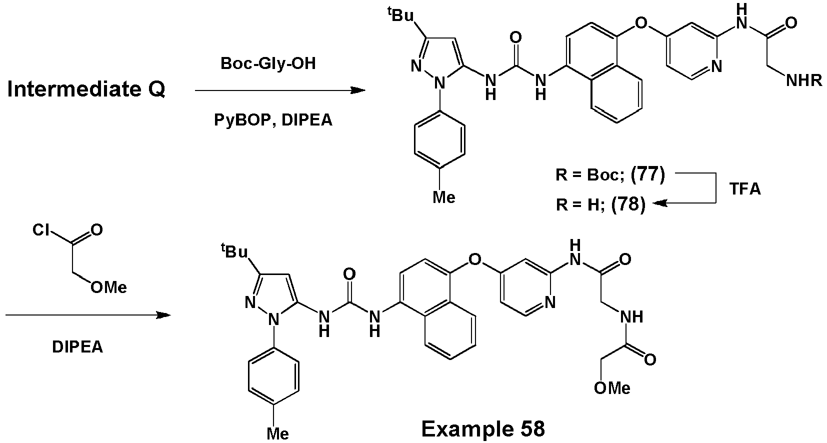

To a mixture of 2-(*tert*-butoxycarbonylamino)acetic acid [Boc-Gly-OH] (415 mg, 2.37 mmol), PyBOP (1.23 g, 2.37 mmol) and DIPEA (413 µL, 2.37 mmol) in dry DMF (12 mL) at 0°C under nitrogen was added **Intermediate Q** (300 mg, 0.592 mmol) and the mixture warmed to 50°C for 16 hr. The resulting mixture was cooled to RT and was partitioned between EtOAc (60 mL) and saturated aq NaHCO₃ solution (80 mL). The aq layer was extracted with EtOAc (60 mL) and the combined organic extracts were washed with brine (80 mL), dried and evaporated *in vacuo.* The residue was purified by flash column chromatography (SiO₂, 40 g, EtOAc in isohexane, 0-65%, gradient elution) to afford *tert*-butyl 2-(4-(4-(3-(3-*tert*-butyl-1-*p*-tolyl-1*H*-pyrazol-5-yl)ureido)naphthalen-1-yloxy)pyridin-2-ylamino)-2-oxoethylcarbamate (**77**) as a purple solid (197 mg, 92% purity, 46%); R^{t} 2.65 min (Method 2); m/z 664 (M+H)⁺ (ES⁺).

To a stirred solution of the carbamate (**77**) (187 mg, 92% pure, 0.259 mmol) in dry DCM (6.0 mL) 0°C under nitrogen was added TFA (2.0 mL) and the reaction mixture maintained at 0°C for 20 min and then warmed to RT for 3 hr. The resulting mixture was evaporated *in vacuo* and the residue was purified by SCX capture and release to afford 2-amino-*N*-(4-(4-(3-(3-*tert*-butyl-1-p-tolyl-1H-pyrazol-5-yl)ureido)naphthalen-1-yloxy)pyridin-2-yl)acetamide (**87**) as a brown solid (130 mg, 87%); R^{t} 1.82 min (Method 2); m/z 564 (M+H)⁺ (ES⁺).

To a solution of (**78**) (35 mg, 62 µol) and DIPEA (43.3 µl, 248 µmol) in dry THF (2.5 mL) under nitrogen at 0°C was added 2-methoxyacetyl chloride (17.0 µl, 186 µmol) and the reaction mixture maintained at 0°C for 15 min and then warmed to RT. After 1.75 hr the reaction was quenched by the addition of a 1% solution of NH₃ in MeOH (2.0 mL) and the resulting mixture kept at RT for 1 hr and was then evaporated *in vacuo.* The residue was subjected to SCX capture and release and the crude product so obtained was purified by flash column chromatography (SiO₂, 12 g, [5% MeOH in EtOAc] in isohexane, 25-100%, gradient elution) to afford the title compound, **Example 58,** as a beige solid (14 mg, 34%); R^{t} 2.28 min (Method 2); m/z 636 (M+H)⁺ (ES⁺); ¹H NMR (400 MHz, DMSO-d₆) δ: 1.28 (9H, s), 2.40 (3H, s), 3.30 (3H, s), 3.82 (2H, s), 3.90 (2H, d), 6.41 (1H, s), 6.72 (1H, dd), 7.33 (1H, d), 7.38 (2H, d), 7.47 (2H, m), 7.57 (2H, m), 7.64 (1H, m), 7.83 (1H, dd), 7.92 (1H, t), 7.96 (1H, d), 8.09 (1H, d), 8.20 (1H, d), 8.82 (1H, brs), 9.14 (1H, brs), 10.59 (1H, brs).

### Example 59: 4-(2-(4-(3-(3-tert-Butyl-1-p-tolyl-1H-pyrazol-5-yl)ureido)naphthalen-1-yloxy)ethyl)-3-(pyridin-2-yl)urea:

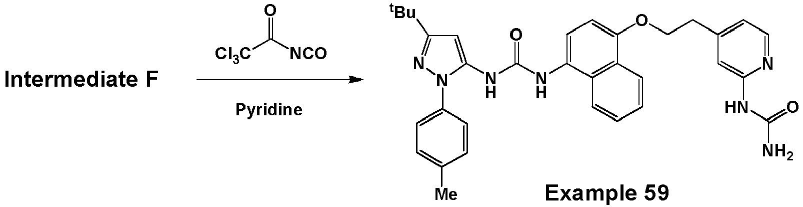

To a solution of **Intermediate F** (50 mg, 0.094 mmol) in pyridine (1.0 mL) was added trichloroacetylisocyanate (12 µL, 0.103 mmol) and the mixture was stirred at RT until judged to be complete by LC-MS. The solvent was evaporated *in vacuo* and the resulting residue was subjected to SCX capture and release and then triturated from DCM (10 mL) to give the title compound, **Example 59,** as an off white solid (25 mg, 44%): m/z 578 (M+H)⁺ (ES⁺). ¹H NMR (400 MHz, DMSO-d₆) δ: 1.26 (9 H, s), 2.38 (3 H, s), 3.12 (2 H, t), 4.35 (2 H, t), 6.34 (1H, s), 6.94-6.99 (2 H, m), 7.19 (1H, dd), 7.33-7.35 (2 H, m), 7.41-7.50 (5 H, m), 7.52-7.56 (1H, m), 7.60 (1H, d), 7.87 (1H, d), 8.09-8.13 (2 H, m), 8.54 (1H, s), 8.75 (1H, s), 9.08 (1H, s).

### Example 60: (R)-N-(4-(4-(3-(3-tert-Butyl-1-p-tolyl-1H-pyrazol-5-yl)ureido)naphthalen-1-yloxy)pyridin-2-yl)-3-(dimethylamino)pyrrolidine-1-carboxamide:

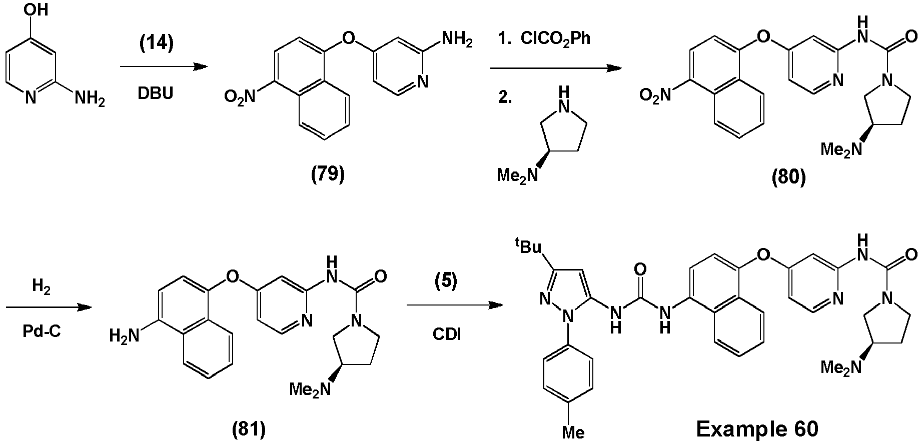

To a stirred suspension of 2-aminopyridin-4-ol (53.9 g, 489 mmol) in MeCN (500 mL) was added DBU (102 mL, 678 mmol) dropwise over 30 min. The resulting solution was maintained at RT for 30 min and was then treated dropwise with a solution of 1-fluoro-4-nitronaphthalene (**14**) (72.0 g, 377 mmol) in acetonitrile (400 mL) over 50 min. The reaction mixture was stirred overnight at RT and was then heated to 50°C for 2 hr. The heating was removed and the stirred mixture was diluted cautiously with water (600 mL) after which it was allowed to cool to RT over 2 hr and was then cooled further to 0°C. The yellow precipitate so produced was collected by filtration and was washed sequentially with a mixture of water and acetonitrile (1:1, 2 x 100 mL) and then with water (500 mL) to give the 4-(4-nitronaphthalen-1-yloxy)pyridin-2-amine (**77**), as a yellow solid (76.0 g, 70%): m/z 283 (M+H)⁺ (ES⁺).

To a solution of (**77**) (200 mg, 0.71 mmol) and Et₃N (0.20 mL, 1.42 mmol) in dry THF was added phenyl carbonochloridate (98 µL, 0.78 mmol) and the reaction mixture maintained at RT for 1 hr. An aliquot of (*R*)-*N*,*N*-dimethylpyrrolidin-3-amine (270 µL, 2.13 mmol) was added to this mixture and after 15 hr at RT a second aliquot of (*R*)-*N*,*N*-dimethylpyrrolidin-3-amine (100 µL, 0.79 mmol) was added and the mixture was maintained at RT for a further 1 hr. The resulting mixture was partitioned between aq. NH₄Cl (10 mL) and DCM (10 mL) and the aq layer was separated and extracted with DCM (3 x 10 mL) The combined organic extracts were dried and evaporated *in vacuo.* The residue was purified by flash column chromatography (SiO₂, 40 g, [5% MeOH in DCM] in DCM, 0-100%, gradient elution and then, 10% [2% NH₃ (7M in MeOH) in MeOH] in DCM, isocratic elution and finally, 30% MeOH in DCM, isocratic elution) to afford (*R*)-3-(dimethylamino)-*N*-(4-(4-nitronaphthalen-1-yloxy)pyridin-2-yl)pyrrolidine-1-carboxamide, (**78**), as a yellow/brown solid (250 mg, 81%); R^{t} 1.54 min (Method 2); m/z 422 (M+H)⁺ (ES⁺).

A solution of (**78**) (250 mg, 0.593 mmol) in MeOH (40 mL) containing AcOH (4 drops) was subjected to hydrogenation by passage through a Thales H-cube (1.0 mL min⁻¹, 25°C, 70 mm 10% Pt/C Cat-Cart, full hydrogen mode) and was then evaporated *in vacuo.* The crude product was partitioned between DCM (20 mL) and aq NaHCO₃ solution (10 mL) and the organic layer was separated and washed with brine (10 mL), dried and evaporated *in vacuo* to afford (*R*)-*N-*(4-(4-aminonaphthalen-1-yloxy)pyridin-2-yl)-3-(dimethylamino)pyrrolidine-1-carboxamide, (**79**) as a green amorphous solid (200 mg, 78%, 90% pure); R^{t} 1.13 min (Method 2); m/z 392 (M+H)⁺ (ES⁺), which was used directly in the next step.

To a solution of CDI (100 mg, 0.370 mmol) in dry DCM (1.0 mL) was added (**5**) (85 mg, 0.370 mmol), portionwise, over 20min and the resulting solution maintained at RT for 2.5 hr. A portion of this solution (0.70 mL) was added to a solution of (**79**) (100 mg, 0.255 mmol) in DCM (1.0 mL) and the mixture was maintained at RT for 18 hr. The reaction was quenched by the addition of MeOH (3.0 mL) and the mixture was evaporated *in vacuo.* The residue was purified by flash column chromatography (SiO₂, 4 g, [5% NH₃ (7M in MeOH) in DCM] in DCM, 0-100%, gradient elution) to afford the title compound, **Example 60**, as a brown glass (52 mg, 30%); R^{t} 4.92 min (Method 1 basic); m/z 647 (M+H)⁺ (ES⁺); ¹H NMR (400 MHz, DMSO-d₆) δ: 1.28 (9H, s), 1.61 (1H, m), 1.98 (1H, m), 2.12 (6H, s), 2.39 (3H, s), 2.58 (1H, m), 3.03 (1H, m), 3.25 (1H, m), 3.49 (1H, m), 3.58 (1H, m), 6.41 (1H, s), 6.60 (1H, dd), 7.30 (1H, d), 7.37 (2H, d), 7.44-7.47 (3H, overlapping m), 7.56 (1H, m), 7.64 (1H, m), 7.83 (1H, d), 7.95 (1H, d), 8.08 (1H, d),, 8.10 (1H, d), 8.70 (1H, s), 8.79 (1H, s), 9.12 (1H, s).

### Biology Section

### Definitions

"Inhibition" refers to a reduction in the level of normally observed biological activity. The reduction may be partial or complete, i.e. the biological activity may be decreased but still occur or may be decreased to such an extent that it is effectively blocked completely.

"Competitive inhibition" refers to inhibition where the substrate and inhibitor cannot bind to the enzyme at the same time. This usually results from the inhibitor having an affinity for the active site of an enzyme where the substrate also binds; the substrate and inhibitor compete for access to the enzyme's active site. This type of inhibition can be overcome by sufficiently high concentrations of substrate, i.e., by out-competing the inhibitor. Competitive inhibitors are often similar in structure to the real substrate.

"Uncompetitive inhibition" refers to inhibition where the inhibitor binds only to the substrate-enzyme complex. In uncompetitive inhibition both maximum velocity (Vmax) and binding efficiency (Km) decrease.

"Mixed inhibition" refers to inhibition where the inhibitor can bind to the enzyme at the same time as the enzyme's substrate. However, the binding of the inhibitor affects the binding of the substrate, and vice versa. This type of inhibition can be reduced, but not overcome by increasing concentrations of substrate. Although it is possible for mixed-type inhibitors to bind in the active site, this type of inhibition generally results from an allosteric effect where the inhibitor binds to a different site on an enzyme. Inhibitor binding to this allosteric site changes the conformation (i.e., tertiary structure or three-dimensional shape) of the enzyme so that the affinity of the substrate for the active site is reduced.

"Non-competitive inhibition" refers to a form of mixed inhibition where the binding of the inhibitor to the enzyme reduces its activity but does not affect the binding of substrate. As a result, the extent of inhibition depends only on the concentration of the inhibitor.

"Influenza" refers to a disease caused by viral infection with influenza virus of either the A, B or C strain.

"Infection" refers to the growth of a parasitic organism within the body. Mere contact with or entry into the body of the parasitic organism is but the first step in infection. For the purposes of this patent application "parasitic organism" includes "virus".

### Experimental Methods and Results

### Human Phospho-Kinase Array

BEAS2B cells (human bronchial epithelial cells, ATCC) in 75-T flask were infected with 2 MOI (multiplicity of infection of 2) of H1 N1 (Strain A/PR/8/34, HPA, Salisbury, UK), and incubated for 2 hr at 37°C. The cells were rinsed with PBS and collected by scraping. Cellular extracts were prepared with Lysis buffer provided in the Proteome Profiler TM kit, human phosphor-kinase array (R&D Systems) following instruction. The array membranes were incubated with diluted cell extracts overnight, and then incubated with Detection antibody cocktail A for 2hr and streptavidin-HRP for another 30 min. Phosphorylated kinases were visualized by exposure to chemiluminescent reagents, and the image was acquired by image analyser. The density of each spot was calculated by the image analyser, and the value was expressed as the ratio between non-treatment and Influenza treatment. A total of 46 phosphorylated kinases and transcription factors were evaluated.

### HCK Enzyme Assay

The enzyme inhibitory activity of compound was determined by fluorescence resonance energy transfer (FRET) using synthetic peptides labelled with both donor and acceptor fluorophores (Z'-LYTE, Invitrogen). HCK enzyme (Invitrogen) was incubated with test compound for 2 hr at RT. The FRET peptides (Z'-LYTE™ kit-Tyrosine 2 peptide; 2 µM) and 15µM of ATP solution were then added to the enzymes/compound mixtures and incubated for 1 hr. After development reagent was added, the mixtures were incubated for one hour and the assay protocol was completed by detection of the fluorescence levels in a microplate reader (Varioskan® Flash, ThermoFisher Scientific).

### p38α and c-Src Enzyme Assay

The enzyme inhibitory activity of compound was also determined using Z'-LYTE system (Invitrogen) as shown above. For p38 MAPKα (MAPK14: Invitrogen), enzyme activity was evaluated indirectly by determining activation/phosphorylation of the down-stream molecule, MAPKAP-K2. The p38 MAPKα protein was mixed with its inactive target MAPKAP-K2 (Invitrogen) and compound for 2 hr at RT. The FRET peptide (Z'-LYTE™ kit-Ser/Thr 4 peptide; 2 µM), which is a phosphorylation target for MAPKAP-K2, and ATP (10 µM) were then added to the enzymes/compound mixture and incubated for 1 hr. For c-SRC assay, the FRET peptides (Z'-LYTE™ kit-Tyrosine 2 peptide; 2 µM) and 200 µM of ATP solutions were then added to the c-Src enzyme (invitrogen)/compound mixtures and incubated for 1 hr.

After development reagent was added, the mixtures were incubated for 1 hr and the assay protocol was completed by detection of the fluorescence levels in a microplate reader (Varioskan® Flash, ThermoFisher Scientific).

### Influenza Induced CPE Assay

MDCK (Madin-Darby canine kidney) cells were infected at an MOI of 0.1 with influenza (H1N1A/PR/8/34, HPA, Salisbury, UK) in the FCS-free media containing 1.5 µg/mL TPCK treated trypsin and incubated for 1 hr at 37°C for adsorption. The cells were then washed with PBS, fresh media added and the cells were incubated for 2 days (44-56 hr). Where appropriate cells were pre-incubated with compound for 2 hr, and then added again after washout of the virus The cells were then washed with 10% FCS DMEM, and incubated in 10% FCS DMEM containing 0.25 mg/mL of MTT for 2hr. The media were then removed, 200 µL of DMSO added to each well and the plates were shaken lightly for 1 hr prior to reading the absorbance at 550 nm.

The percentage inhibition for each well was calculated compared with vehicle. The IC₅₀ value was calculated from the concentration-response curve generated by the serial dilutions of compound.

### In Vivo Testing: Influenza virus propagation in mouse lung

Mice (n=6 per group) were first exposed to cigarette smoke for 14 days (3 times per day). On day 15 mice were dosed intranasally with influenza (H3N1 Memphis 71). Mice were dosed intratracheally, once daily with 2 µg of the Example or its vehicle on days 14, 15, 16, 17 and 18. On day 18 the animals were sacrificed and the lungs examined to estimate the viral titre using a plaque assay.

To perform the plaque assay serial dilutions were added to near confluent monolayers of cells. One hour after initial infection the monolayer was overlaid with molten agarose, which was allowed to set, and the cells incubated with influenza virus. The cells were then fixed in formaldehyde and subsequently stained with a dye. The plaques were counted and a titre determined in plaque forming units (pfu).

### MTT assay (MDCK cells)

MDCK cells were incubated with the compounds for 54 hrs in the FCS-free media containing 1.5 µg/mL TPCK treated trypsin. The cells were then washed with 10% FCS DMEM, and incubated in 10% FCS DMEM containing 0.25 mg/mL MTT for 2hr. The media were then removed, 200 µL of DMSO added to each well and the plates were shaken lightly for 1 hr prior to reading the absorbance at 550 nm.

### RNA Interference

BEAS2B cells (ex, ATCC) were seeded into 24 well plates. Two HCK siRNAs (Invitrogen Ltd., Paisley, UK), 100 nM each, were transfected into the cells with Lipofectamine LTX (Invitrogen). The siRNAs (12 µL each, 100 mM stock solutions) were added to LHC basal medium (1200 µL) and incubated for 5 min. Lipofectamine (30 mL) was added to LHC basal medium (1200 µL) with Plus Reagent (24 µL; Invitrogen) and incubated for 5 min. Both solutions were then mixed together gently and incubated at RT for 30 min. 100 mL of the siRNA complexes was added per well and incubated for 48 hr at 37°C, 5% CO₂. siRNA-Negative control (200 nM, Invitrogen) were also transfected for comparison.

These cells were then infected with H1 N1 virus (A/WSN/33) at an MOI of 1 for 1 hr. The media was removed by aspiration and the wells were washed twice with warmed LHC basal media. The plate was further incubated at 37°C for 48 hr. The supernatant was collected and the virus titre estimated by a CPE assay in MDCK cells as follows. An aliquot, (20 µL) of supernatant was collected and 10-fold serial dilutions were prepared in FCS-free DMEM containing 1.5 µg/mL of TPCK treated trypsin. All titrations were performed by infecting confluent MDCK cell monolayers (96 well plates) with the serially diluted supernatant preparations (10⁻¹-10⁻⁵). The resultant cytopathic effects (CPE) were assessed by visual inspection 3 days after infection. The amount of virus required to infect 50% of MDCK cells was calculated for each treatment and is reported as log[TCID₅₀] (U/20µL).

### Results

It was discovered that an interesting pattern of protein phosphorylation occurs in BEAS2B cells following inoculation with influenza virus. The pattern shows that virus inoculation is associated with a limited number of proteins in which the intensity of phosphorylation increases by more than 1.5-fold. One of these proteins is p38MAPKα and its downstream target molecule, HSP27. In addition, the Src kinase family (e.g. LCK, HCK and c-Src, Fyn) is dominantly phosphorylated. The following table (**Tab**le **1**) shows those proteins which exhibit an increased level of phosphorylation following influenza virus infection and the extent of the increase of the level of phosphorylation observed.

**Table 1**

| **Proteins Phospylated on Inoculation (Fold Induction)** | |
|---|---|
| **1.25 ∼1.5 fold induction** | **>1.5 fold induction** |
| GSK3alpha (1.3) | p38MAPKalpha (1.7) |
| MEK1/2 (1.4) | mTOR (1.9) |
| c-Src (1.4) | HSP27 (1.7) |
| Fyn (1.3) | LCK (1.9) |
| JNK (pan) (1.5) | HCK (1.7) |
| | CHK2 (1.8) |

On the basis of the different levels of phosphorylation following influenza virus infection, the relationship between the ability of compounds to inhibit p38α, HCK or c-SRC activity and the ability of the compounds to inhibit influenza-induced cytopathic effect (CPE) responses in Madin-Darby canine kidney (MDCK) cells was initially investigated. The compounds used were compounds of formula (I), which the inventors had previously identified as having kinase modulatory activity. The results of the investigations are summarised below (**Table 2**), which also includes results from a maximum toleration test (MTT) assay conducted using MDCK cells.

**Table 2: Effects on enzyme activities, influenza-induced CPE and in a maximum toleration test assay**

| **Test Compound** | **IC₅₀ (nM)** | | | | **MTT assay¹** | |
|---|---|---|---|---|---|---|
| **Example No.** | **p38α** | **HCK** | **c-SRC** | **Influenza-Induced CPE** | **4 hr** | **24hr** |
| **1** | 3 | 47 | 162 | 6 | -ve | -ve |
| **41** | 3.3 | 586 | 16307 | NE² | -ve | -ve |
| **2** | 2.8 | 17 | 45 | 11 | -ve | -ve |
| **34** | 2.1 | 742 | >17301 | NE² | +ve | -ve |
| **40** | 0.3 | 276 | >17331 | 97 | -ve | -ve |
| **36** | 14.4 | >1570 | 9331 | NE² | -ve | -ve |
| **20** | 0.7 | 176 | 161 | 486** | -ve | +ve |
| **15** | 14.4 | 65 | 28 | NE² | -ve | -ve |
| **16** | 10.3 | 20 | 28 | 166 | +ve | +ve |
| **49** | 5 | 24 | 155 | 32 | -ve | +ve |
| **17** | 2.7 | 60 | 149 | 64 | -ve | -ve |
| **32** | 1 | 183 | 111 | NE³ | -ve | -ve |

| | | | | | | |
|---|---|---|---|---|---|---|
| **1**) Conducted at a final concentration of 10 µg/mL, <30% = -ve,> 30% = +ve; **2**) No effect at at 0.2 µg/mL **3**) No effect at 0.04 µg/mL **Linear regression otherwise non-linear regression for calculation of IC₅₀; | | | | | | |

The data in **Table 2,** and summarised in the figures disclosed herein, demonstrate that there is no detectable correlation between the ability of compounds to prevent influenza-induced CPE responses and their potency as inhibitors of either p38α or the c-SRC enzymes.

However, surprisingly and in contrast to the aforementioned observation, a very strong correlation was observed between the ability of compounds to prevent influenza-induced CPE responses and their potency as inhibitors of the HCK enzyme (p=0.004).

This strong correlation is very good evidence of a link between inhibition of the p59-HCK enzyme and inhibition of influenza virus infection.

To confirm the relevance of our *in vitro* studies, the ability of compound **Example 1** to inhibit influenza virus viral load *in vivo* in the mouse (*versus* the influenza H3N1, Memphis 71 strain) was investigated further. The results obtained from this profiling study are presented in below (**Table 3**) and demonstrate that treatment led to a decrease in viral load of approximately 80% *versus* vehicle controls.

**Table 3: Effects of treatment with compound Example 1 on viral load in the mouse lung**

| **Treatment** | **Lung viral load¹** |
|---|---|
| **Vehicle** | 46.6 ± 16.6 |
| **Example 1** | 8.1 ± 1.9 |

| | |
|---|---|
| **1**) pfu / g lung tissue (x 10⁶) | |

In view of the findings discussed above, the role of HCK in flu infection was investigated in human bronchial epithelial cells using specific RNA interference as an alternative strategy to decrease the activity of the enzyme.

HCK protein expression was decreased by more than 40% by HCK-specific siRNA transfection in BEAS2B cells. The cells were infected with influenza H1N1 (A/WSN/33) at an MOI of 1 for 48 hr and viral load was determined by titration assay using MDCK cells. The control cells that had been transfected with non-coding siRNA showed a similar viral titre to non-transfected controls, whereas the viral titre was significantly (p<0.05) reduced in HCK-KD cells (**Table 4**).

**Table 4**

| **Treatment** | **n values** | **Log, TCID₅₀ /20µL (mean ± SEM)** |
|---|---|---|
| Non treatment | 3 | 4.4 ± 0.20 |
| Non-coding siRNA (Negative control) | 3 | 4.7 ± 0.15 |
| HCK-siRNA | 4 | 3.8 ± 0.17* |
| * P<0.05 *versus* non-coding siRNA control (Mann-Whitney U test) | | |

## Claims

1. A compound capable of inhibiting hemopoietic cell kinase (p59-HCK) activity for use in the treatment or prophylaxis of infection by influenza virus (A, B and C strains) wherein the compound is not *N*-(4-(4-(3-(3-*tert*-butyl-1-*p*-tolyl-1*H*-pyrazol-5-yl) ureido) naphthalen-1-yloxy)pyridin-2-yl)-2-methoxyacetamide or a pharmaceutically acceptable salt or solvate thereof.

2. Use of a compound capable of inhibiting hemopoietic cell kinase (p59-HCK) activity for the manufacture of a medicament for the treatment or prophylaxis of infection by influenza virus (A, B and C strains) wherein the compound is not *N*-(4-(4-(3-(3-*tert*-butyl-1-*p*-tolyl-1*H*-pyrazol-5-yl) ureido) naphthalen-1-yloxy)pyridin-2-yl)-2-methoxyacetamide or a pharmaceutically acceptable salt or solvate thereof.

3. A compound for use or use according to claim 1 or claim 2 wherein the compound is a chemical inhibitor of p59-HCK activity.

4. A compound for use or use according to claim 3 wherein the chemical inhibitor is a compound of formula (I) 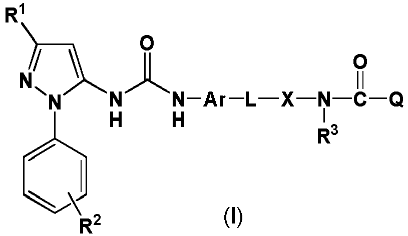
wherein R¹ is C₁₋₆ alkyl optionally substituted by a hydroxyl group;
R² is H or C₁₋₆ alkyl optionally substituted by a hydroxyl group;
R³ is H, C₁₋₆ alkyl or C₀₋₃ alkylC₃₋₆ cycloalkyl;
Ar is a naphthyl or a phenyl ring either of which may be optionally substituted by one or more groups independently selected from C₁₋₆ alkyl, C₁₋₆ alkoxy, amino, C₁₋₄ mono or C₂₋₈ di-alkyl amino;
L is a saturated or unsaturated branched or unbranched C₁₋₈ alkylene chain, wherein one or more carbons are optionally replaced by -O- and the chain is optionally substituted by one or more halogen atoms;
X is 5 or 6 membered heteroaryl group containing at least one nitrogen atom and optionally including 1 or 2 further heteroatoms selected from O, S and N;
Q is selected from:
a) a saturated or unsaturated, branched or unbranched C₁₋₁₀ alkyl chain, wherein at least one carbon is replaced by a heteroatom selected from O, N, S(O)ₚ, wherein said chain is optionally, substituted by one or more groups independently selected from oxo, halogen, an aryl group, a heteroaryl group, a heterocyclyl group or a C₃₋₈ cycloalkyl group,
each aryl, heteroaryl, heterocyclyl or C₃₋₈ cycloalkyl group bearing 0 to 3 substituents selected from halogen, hydroxyl, C₁₋₆ alkyl, C₁₋₆ alkoxy, C₁₋₆ haloalkyl, amino, C₁₋₄ mono or C₂₋₈ di-alkyl amino, C₁₋₄ mono or C₂₋₈ di-acyl amino, S(O)_{q}C₁₋₆ alkyl, C₀₋₆ alkylC(O)C₁₋₆ alkyl or C₀₋₆ alkylC(O)C₁₋₆ heteroalkyl,
with the proviso that the atom linked directly to the carbonyl in -NR³C(O)- is not an oxygen or a sulfur atom; and
b) a C₀₋₈ alkyl-heterocycle said heterocyclyl group comprising at least one heteroatom selected from O, N, and S, and which is optionally substituted by one, two or three groups independently selected from halogen, hydroxyl, C₁₋₆ alkyl, C₁₋₆ alkoxy, C₁₋₆ haloalkyl, amino, C₁₋₄ mono and C₂₋₈ di-alkyl amino, C₁₋₄ mono or C₂₋₈ di-acyl amino, S(O)_{q}C₁₋₆ alkyl, C₀₋₆ alkylC(O)C₁₋₆ alkyl, C₀₋₆ alkylC(O)NC₀₋₆ alkyl C₀₋₆ alkyl or C₀₋₆ alkylC(O)C₀₋₆ heteroalkyl;
p is 0, 1 or 2; and
q is 0, 1 or 2;
or a pharmaceutically acceptable salt thereof.

5. A compound for use or use according to claim 1 or claim 2 wherein the compound is *N-*(4-((4-(3-(3-*tert*-Butyl-1-*p*-tolyl-1*H*-pyrazol-5-yl)ureido)naphthalen-1-yloxy)methyl)pyridin-2-yl)-2-methoxyacetamide or a pharmaceutically acceptable salt thereof.

6. A compound for use or use according to claim 1 or claim 2 wherein the compound is a biochemical inhibitor of p59-HCK activity.

7. A compound for use or use according to claim 6 wherein the compound is an RNAi molecule.

8. A compound for use or use according to any one of claims 1 to 7 wherein the compound is a competitive inhibitor of p59 HCK activity.

9. A compound for use or use according to any one of claims 1 to 8, wherein the treatment or prophylaxis of infection by influenza virus is in at-risk patients selected from:
i. pregnant women or patients undergoing chemotherapy; or
ii. patients suffering complications (pulmonary and systemic) arising from infection by influenza virus (A, B and C strains); or
iii. patients with chronic diseases, such as diabetes, congestive heart failure, renal failure, chronic obstructive pulmonary disease.

10. A compound or use according to any one of claims 1 to 9, wherein the compound is administered in combination with one or more anti-viral drugs selected from zanamivir, oseltamivir, laninamivir, peramivir, ribavarin and an (exogenous) interferon.

11. A composition for separate, simultaneous or sequential use comprising a compound capable of inhibiting p59-HCK activity and one or more anti-viral drugs selected from zanamivir, oseltamivir, laninamivir, peramivir and ribavarin, wherein the compound is not *N*-(4-(4-(3-(3-*tert-*butyl-1-*p*-tolyl-1*H*-pyrazol-5-yl) ureido) naphthalen-1-yloxy)pyridin-2-yl)-2-methoxyacetamide or a pharmaceutically acceptable salt or solvate thereof.

## Patentansprüche

1. Zum Inhibieren der Aktivität von hämopoietischer Zellkinase (p59-HCK) fähige Verbindung zur Verwendung bei der Behandlung oder Prophylaxe einer Infektion durch Influenzavirus (A-, B- und C-Stämme), wobei es sich bei der Verbindung nicht um *N*-(4-(4-(3-(3*-tert.-*Butyl-1-*p*-tolyl-1*H*-pyrazol-5-yl)ureido)naphthalin-1-yloxy)pyridin-2-yl)-2-methoxyacetamid oder ein pharmazeutisch unbedenkliches Salz oder Solvat davon handelt.

2. Verwendung einer zum Inhibieren der Aktivität der hämopoietischen Zellkinase (p59-HCK) fähigen Verbindung zur Herstellung eines Medikaments zur Behandlung oder Prophylaxe einer Infektion durch Influenzavirus (A-, Bund C-Stämme), wobei es sich bei der Verbindung nicht um *N*-(4-(4-(3-(3-*tert*.-Butyl-1-p-tolyl-1*H*-pyrazol-5-yl)ureido)naphthalin-1-yloxy)pyridin-2-yl)-2-methoxyacetamid oder ein pharmazeutisch unbedenkliches Salz oder Solvat davon handelt.

3. Verbindung zur Verwendung nach Anspruch 1 oder Anspruch 2, wobei es sich bei der Verbindung um einen chemischen Inhibitor der p59-HCK-Aktivität handelt.

4. Verbindung zur Verwendung oder Verwendung nach Anspruch 3, wobei es sich bei dem chemischen Inhibitor um eine Verbindung der Formel (I) 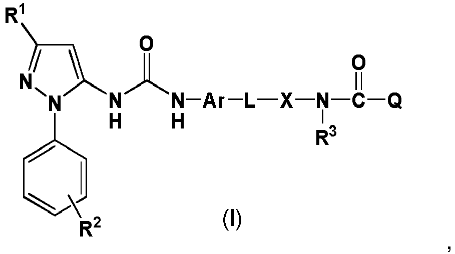
wobei R¹ für gegebenenfalls durch eine Hydroxylgruppe substituiertes C₁₋₆-Alkyl steht,
R² für H oder gegebenenfalls durch eine Hydroxylgruppe substituiertes C₁₋₆-Alkyl steht,
R³ für H, C₁₋₆-Alkyl oder C₀₋₃-Alkyl-C₃₋₆-Cycloalkyl steht, Ar für einen Naphthyl- oder Phenylring steht, die jeweils gegebenenfalls durch eine oder mehrere unabhängig voneinander aus C₁₋₆-Alkyl, C₁₋₆-Alkoxy, Amino, C₁₋₄-Mono- und C₂₋₈-Dialkylamino ausgewählte Gruppen substituiert sein können,
L für eine gesättigte oder ungesättigte verzweigte oder geradkettige C₁₋₈-Alkylenkette steht, wobei ein oder mehrere Kohlenstoffe gegebenenfalls durch -0- ersetzt sind und die Kette gegebenenfalls durch ein oder mehrere Halogenatome substituiert ist,
X für eine 5- oder 6-gliedrige Heteroarylgruppe mit mindestens einem Stickstoffatom und gegebenenfalls 1 oder 2 weiteren, aus 0, S und N ausgewählten Heteroatomen steht,
Q ausgewählt ist aus:
a) einer gesättigten oder ungesättigten, verzweigten oder geradkettigen C₁₋₁₀-Alkylkette, wobei mindestens ein Kohlenstoff durch ein aus 0, N, S(O)ₚ ausgewähltes Heteroatom ersetzt ist, wobei die Kette gegebenenfalls durch eine oder mehrere unabhängig voneinander aus Oxo, Halogen, einer Arylgruppe, einer Heteroarylgruppe, einer Heterocyclylgruppe und einer C₃₋₈-Cycloalkylgruppe ausgewählte Gruppen substituiert ist,
Aryl-, Heteroaryl-, Heterocyclyl- und C₃₋₈-Cycloalkylgruppen jeweils 0 bis 3 aus Halogen, Hydroxy, C₁₋₆-Alkyl, C₁₋₆-Alkoxy, C₁₋₆-Halogenalkyl, Amino, C₁₋₄-Mono- oder C₁₋₈-Dialkylamino, C₁₋₄-Mono- oder C₂₋₈-Diacylamino, S(O)_{q}-C₁₋₆-Alkyl, C₀₋₆-Alkyl-C(O)-C₁₋₆-alkyl und C₀₋₆-Alkyl-C(O)-C₁₋₆-heteroalkyl ausgewählte Substituenten tragen, mit der Maßgabe, dass es sich bei dem direkt an das Carbonyl in -NR³C(O)-gebundene Atom nicht um ein Sauerstoff- oder ein Schwefelatom handelt, und
b) einem C₀₋₈-Alkyl-heterocyclus, wobei die Heterocyclylgruppe mindestens ein aus 0, N und S ausgewähltes Heteroatom umfasst, und welcher gegebenenfalls durch eine, zwei oder drei unabhängig voneinander aus Halogen, Hydroxy, C₁₋₆-Alkyl, C₁₋₆-Alkoxy, C₁₋₆-Halogenalkyl, Amino, C₁₋₄-Mono- und C₂₋₈-Dialkylamino, C₁₋₄-Mono- und C₂₋₈-Diacylamino, S(O)_{q}-C₁₋₆-Alkyl, C₀₋₆-Alkyl-C (0) -C₁₋₆-alkyl, C₀₋₆-Alkyl-C(O)N-C₀₋₆-alkyl-C₀₋₆-alkyl oder C₀₋₆-Alkyl-C(O)-C₀₋₆-heteroalkyl ausgewählte Gruppen substituiert ist,
p für 0, 1 oder 2 steht und
q für 0, 1 oder 2 steht,
oder ein pharmazeutisch unbedenkliches Salz davon handelt.

5. Verbindung zur Verwendung oder Verwendung nach Anspruch 1 oder Anspruch 2, wobei es sich bei der Verbindung um *N*-(4-((4-(3-(3-*tert*.-Butyl-1-*p*-tolyl-1*H-*pyrazol-5-yl)ureido)naphthalin-1-yloxy)methyl)pyridin-2-yl)-2-methoxyacetamid oder ein pharmazeutisch unbedenkliches Salz davon handelt.

6. Verbindung zur Verwendung oder Verwendung nach Anspruch 1 oder Anspruch 2, wobei es sich bei der Verbindung um einen biochemischen Inhibitor der p59-HCK-Aktivität handelt.

7. Verbindung zur Verwendung oder Verwendung nach Anspruch 6, wobei es sich bei der Verbindung um ein RNAi-Molekül handelt.

8. Verbindung zur Verwendung oder Verwendung nach einem der Ansprüche 1 bis 7, wobei es sich bei der Verbindung um einen kompetitiven Inhibitor der p59-HCK-Aktivität handelt.

9. Verbindung zur Verwendung oder Verwendung nach einem der Ansprüche 1 bis 8, wobei die Behandlung bzw. Prophylaxe der Infektion durch Influenzavirus bei Risikopatienten ausgewählt aus:
i. schwangeren Frauen oder Patienten, die sich einer Chemotherapie unterziehen, oder
ii. Patienten, die an von einer Infektion durch Influenzavirus (A-, B- und C-Stämme) herrührenden Komplikationen (pulmonal und systemisch) leiden, oder
iii. Patienten mit chronischen Krankheiten wie Diabetes, dekompensierter Herzinsuffizienz, Niereninsuffizienz, chronischer obstruktiver Lungenkrankheit leiden,
erfolgt.

10. Verbindung oder Verwendung nach einem der Ansprüche 1 bis 9, wobei die Verbindung in Kombination mit einem oder mehreren aus Zanamivir, Oseltamivir, Laninamivir, Peramivir, Ribavarin und einem (exogenen) Interferon ausgewählten antiviralen Arzneimitteln verabreicht wird.

11. Zusammensetzung zur getrennten, gleichzeitigen oder aufeinanderfolgenden Anwendung, umfassend eine zum Inhibieren der p59-HCK-Akvitität fähige Verbindung und ein oder mehrere aus Zanamivir, Oseltamivir, Laninamivir, Peramivir und Ribavarin ausgewählte antivirale Arzneimittel, wobei es sich bei der Verbindung nicht um *N*-(4-(4-(3-(3-*tert*.-Butyl-1-*p-*tolyl-1*H*-pyrazol-5-yl)ureido)naphthalin-1-yloxy)pyridin-2-yl)-2-methoxyacetamid oder ein pharmazeutisch unbedenkliches Salz oder Solvat davon handelt.

## Revendications

1. Composé capable d'inhiber l'activité cellule hématopoïétique kinase (p59-HCK) pour utilisation dans le traitement ou la prophylaxie d'une infection par le virus de la grippe (souches A, B et C), le composé n'étant pas le *N*-(4-(4-(3-(3-*tert*-butyl-1-*p*-tolyl-1*H*-pyrazol-5-yl)uréido)naphtalén-1-yloxy)pyridin-2-yl)-2-méthoxyacétamide ou un sel pharmaceutiquement acceptable ou un solvate de celui-ci.

2. Utilisation d'un composé capable d'inhiber l'activité cellule hématopoïétique kinase (p59-HCK) pour la fabrication d'un médicament pour le traitement ou la prophylaxie d'une infection par le virus de la grippe (souches A, B et C), le composé n'étant pas le *N*-(4-(4-(3-(3-*tert*-butyl-1-*p*-tolyl-1*H*-pyrazol-5-yl)uréido)-naphtalén-1-yloxy)pyridin-2-yl)-2-méthoxyacétamide ou un sel pharmaceutiquement acceptable ou un solvate de celui-ci.

3. Composé pour utilisation ou utilisation selon la revendication 1 ou la revendication 2, le composé étant un inhibiteur chimique de l'activité p59-HCK.

4. Composé pour utilisation ou utilisation selon la revendication 3 où l'inhibiteur chimique est un composé de formule (I) 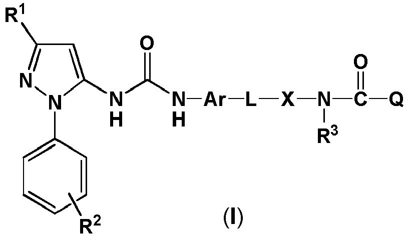
dans lequel R¹ est alkyle en C₁₋₆ facultativement substitué par un groupe hydroxyle ;
R² est H ou alkyle en C₁₋₆ facultativement substitué par un groupe hydroxyle ;
R³ est H, alkyle en C₁₋₆ ou (alkyle en C₀₋₃)-(cycloalkyle en C₃₋₆) ;
Ar est un cycle naphtyle ou phényle dont chacun peut être facultativement substitué par un ou plusieurs groupes indépendamment choisis parmi alkyle en C₁₋₆, alcoxy en C₁₋₆, amino, (monoalkyle en C₁₋₄) - ou (dialkyle en C₂₋₈) - amino ;
L est une chaîne alkylène en C₁₋₈ saturée ou insaturée, ramifiée ou non ramifiée, dans laquelle un ou plusieurs carbones sont facultativement remplacés par -0- et la chaîne est facultativement substituée par un ou plusieurs atomes d'halogène ;
X est un groupe hétéroaryle de 5 ou 6 chaînons contenant au moins un atome d'azote et comprenant facultativement 1 ou 2 hétéroatomes supplémentaires choisis parmi 0, S et N ;
Q est choisi parmi :
a) une chaîne alkyle en C₁₋₁₀ saturée ou insaturée, ramifiée ou non ramifiée, dans laquelle au moins un carbone est remplacé par un hétéroatome choisi parmi 0, N, S(O)ₚ, ladite chaîne étant facultativement substituée par un ou plusieurs groupes indépendamment choisis parmi oxo, halogène, un groupe aryle, un groupe hétéroaryle, un groupe hétérocyclyle ou un groupe cycloalkyle en C₃₋₈, chaque groupe aryle, hétéroaryle, hétérocyclyle ou cycloalkyle en C₃₋₈ comportant 0 à 3 substituants choisis parmi halogène, hydroxyle, alkyle en C₁₋₆, alcoxy en C₁₋₆, halogénoalkyle en C₁₋₆, amino, (monoalkyle en C₁₋₄) - ou (dialkyle en C₂₋₈)-amino, (monoacyle en C₁₋₄) - ou (diacyle en C₂₋₈) -amino, S(O)_{q}(alkyle en C₁₋₆), (alkyle en C₀₋₆) - C(O)-(alkyle en C₁₋₆) ou (alkyle en C₀₋₆)-C(O)-(hétéroalkyle en C₁₋₆),
à condition que l'atome directement lié au carbonyle dans -NR³C(O)- ne soit pas un atome d'oxygène ou de soufre ; et
b) un (alkyle en C₀₋₈)-hétérocycle, ledit groupe hétérocyclyle comprenant au moins un hétéroatome choisi parmi 0, N et S, et qui est facultativement substitué par un, deux ou trois groupes indépendamment choisis parmi halogène, hydroxyle, alkyle en C₁₋₆, alcoxy en C₁₋₆, halogénoalkyle en C₁₋₆, amino, (monoalkyle en C₁₋₄)- ou (dialkyle en C₂₋₈)-amino, (monoacyle en C₁-₄)- ou (diacyle en C₂₋₈)-amino, S(O)_{q}(alkyle en C₁₋₆), (alkyle en C₀₋₆)-C(O)-(alkyle en C₁₋₆), (alkyle en C₀₋₆)-C(O)N-(alkyle en C₀₋₆) ou (alkyle en C₀₋₆)-C(O)-(hétéroalkyle en C₀₋₆);
p est 0, 1 ou 2 ; et
q est 0, 1 ou 2 ;
ou sel pharmaceutiquement acceptable de celui-ci.

5. Composé pour utilisation ou utilisation selon la revendication 1 ou la revendication 2, le composé étant le *N*-(4-((4-(3-(3-*tert*-butyl-1-p-tolyl-1*H*-pyrazol-5-yl)ureido)naphtalén-1-yloxy)méthyl)pyridin-2-yl)-2-méthoxyacétamide ou un sel pharmaceutiquement acceptable de celui-ci.

6. Composé pour utilisation ou utilisation selon la revendication 1 ou la revendication 2, le composé étant un inhibiteur biochimique de l'activité p59-HCK.

7. Composé pour utilisation ou utilisation selon la revendication 6, le composé étant une molécule d'ARNi.

8. Composé pour utilisation ou utilisation selon l'une quelconque des revendications 1 à 7, le composé étant un inhibiteur compétitif de l'activité p59 HCK.

9. Composé pour utilisation ou utilisation selon l'une quelconque des revendications 1 à 8, le traitement ou la prophylaxie d'une infection par le virus de la grippe étant chez des patients à risque choisis parmi :
i. les femmes enceintes ou les patients sous chimiothérapie ; ou
ii. les patients souffrant de complications (pulmonaires et systémiques) consécutives à une infection par le virus de la grippe (souches A, B et C) ; ou
iii. les patients atteints de maladies chroniques, telles que le diabète, l'insuffisance cardiaque congestive, l'insuffisance rénale, la bronchopneumopathie chronique obstructive.

10. Composé ou utilisation selon l'une quelconque des revendications 1 à 9, le composé étant administré en combinaison avec un ou plusieurs médicaments antiviraux choisis parmi le zanamivir, l'oseltamivir, le laninamivir, le péramivir, la ribavarine et un interféron (exogène).

11. Composition pour utilisation séparée, simultanée ou séquentielle comprenant un composé capable d'inhiber l'activité p59-HCK et un ou plusieurs médicaments antiviraux choisis parmi le zanamivir, l'oseltamivir, le laninamivir, le péramivir et la ribavarine, dans laquelle le composé n'est pas le *N*-(4-(4-(3-(3-*tert*-butyl-1-*p-*tolyl-1*H*-pyrazol-5-yl)uréido)naphtalén-1-yloxy)pyridin-2-yl)-2-méthoxyacétamide ou un sel pharmaceutiquement acceptable ou solvate de celui-ci.
